(19) Europäisches Patentamt
European Patent Office
Office européen des brevets

(11) EP 4 524 135 A1

(12) EUROPEAN PATENT APPLICATION
published in accordance with Art. 153(4) EPC

(43) Date of publication:
**19.03.2025 Bulletin 2025/12**

(21) Application number: **23802999.5**

(22) Date of filing: **11.05.2023**

(51) International Patent Classification (IPC):
***C07D 401/14*** (2006.01) ***C07D 487/04*** (2006.01)
***C07D 491/04*** (2006.01) ***C07D 471/04*** (2006.01)
***C07D 498/04*** (2006.01) ***C07D 513/04*** (2006.01)
***C07D 519/00*** (2006.01) ***C07D 491/048*** (2006.01)
***A61K 31/63*** (2006.01) ***A61K 31/438*** (2006.01)
***A61K 31/38*** (2006.01) ***A61K 31/41*** (2006.01)
***A61P 35/00*** (2006.01)

(52) Cooperative Patent Classification (CPC):
**A61K 31/38; A61K 31/41; A61K 31/438; A61K 31/455; A61K 31/495; A61K 31/497; A61K 31/506; A61K 31/63; A61P 35/00; C07D 401/04; C07D 401/12; C07D 401/14; C07D 405/04; C07D 405/14; C07D 413/14;** (Cont.)

(86) International application number:
**PCT/CN2023/093528**

(87) International publication number:
**WO 2023/217230 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**13.05.2022 CN 202210520744**
**25.07.2022 CN 202210878192**
**27.09.2022 CN 202211183092**
**02.12.2022 CN 202211537992**
**16.01.2023 CN 202310071265**
**22.03.2023 CN 202310288871**
**27.04.2023 CN 202310475180**

(71) Applicant: **Shanghai Apeiron Therapeutics Company Limited**
**Shanghai 201210 (CN)**

(72) Inventors:
- **XIAO, Yisong**
**Shanghai 201210 (CN)**
- **GU, Xiaohui**
**Shanghai 201210 (CN)**
- **LAI, Kunmin**
**Shanghai 201210 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) KINESIN KIF18A INHIBITOR AND USE THEREOF

(57) The present invention provides a KIF18 inhibitor and its synthetic method. Compounds of the present invention are capable of modulating the KIF18A protein, thereby influencing cell cycle and proliferation processes for the treatment of cancers and cancer-related diseases. The present invention also encompasses pharmaceutical compositions containing the compounds and methods for treating conditions associated with KIF 18A activity.

EP 4 524 135 A1

(52) Cooperative Patent Classification (CPC): (Cont.)
**C07D 471/04; C07D 487/04; C07D 491/04; C07D 491/048; C07D 498/04; C07D 513/04; C07D 519/00**

## Description

This application claims priority from the following applications:

1) A Chinese patent application submitted to China National Intellectual Property Administration on May 13, 2022, with the application number 202210520744.0 and the invention name "Kinesin KIF18A Inhibitor and Use Thereof";
2) A Chinese patent application submitted to China National Intellectual Property Administration on July 25, 2022, with the application number 202210878192.0 and the invention name "Kinesin KIF18A Inhibitor and Use Thereof";
3) A Chinese patent application submitted to China National Intellectual Property Administration on September 27, 2022, with the application number 202211183092.2 and the invention name "Kinesin KIF18A Inhibitor and Use Thereof";
4) A Chinese patent application submitted to China National Intellectual Property Administration on December 02, 2022, with the application number 202211537992.2 and the invention name "Kinesin KIF18A Inhibitor and Use Thereof";
5) A Chinese patent application submitted to China National Intellectual Property Administration on January 16, 2023, with the application number 202310071265.X and the invention name "Kinesin KIF18A Inhibitor and Use Thereof";
6) A Chinese patent application submitted to China National Intellectual Property Administration on March 22, 2023, with the application number 202310288871.7 and the invention name "Kinesin KIF18A Inhibitor and Use Thereof";
7) A Chinese patent application submitted to China National Intellectual Property Administration on April 27, 2023, with the application number 202310475180.8 and the invention name "Kinesin KIF18A Inhibitor and Use Thereof";

The entire contents of which are incorporated herein by reference.

## TECHNICAL FIELD

The present invention belongs to the field of medicine and relates to a class of kinesin KIF18A inhibitors and their use for inhibiting cancer cell proliferation and treating cancers.

## BACKGROUND ART

KIF18A is a member of the kinesin-8 family. It moves towards the plus ends of microtubules within cells using microtubules as tracks, relying on the energy released from ATP hydrolysis. Upon reaching the plus ends of microtubules, KIF 18A regulates the dynamic instability of microtubules and exerts an activity similar to that of microtubule depolymerase. During mitosis, KIF18A regulates spindle microtubule dynamics and chromosome amplitude, playing a critical role in the timely completion of chromosome alignment at mitosis, maintenance of genomic stability and successful completion of mitosis.

The KIF18A gene belongs to the kinesin-8 subfamily and is a plus-end directed motor. KIF18A is believed to influence the dynamics of the plus ends of centromeric microtubules, thereby regulating correct chromosome orientation and spindle tension. Depletion of human KIF18A results in longer spindles, increased chromosome oscillation at metaphase and activated mitotic spindle assembly checkpoint in HeLa cervical cancer cells (MI Mayr et al., Current Biology 17, 488-98, 2007). KIF18A appears to be a viable target for cancer therapy. KIF18A is overexpressed in multiple types of cancers, including but not limited to colon, breast, lung, pancreatic, prostatic, bladder, head, neck, cervical and ovarian cancers. Furthermore, in cancer cell lines, gene deletion or knockout or KIF18A inhibition affects the mitotic spindle apparatus. In particular, inhibition of KIF18A has been found to induce mitotic cell arrest, a vulnerability known to promote mitotic cell death via apoptosis, mitotic catastrophe or death following polyphasic-driven lethality or interphase mitotic slippage. As a result, there is a strong interest in finding inhibitors of KIF 18A protein. Therefore, inhibition of KIF18A ATPase activity is a promising approach to developing new anticancer agents.

## CONTENT OF THE INVENTION

The present invention belongs to the field of medicine and relates to a class of kinesin KIF18A inhibitors, specifically to the said compounds or their stereoisomers, tautomers, mesomers, racemates, enantiomers, diastereoisomers or their mixture forms or pharmaceutically acceptable salts, co-crystals, metabolites, solvates, prodrugs or isotopic labels, their preparation methods and pharmaceutical compositions containing such compounds and their use as therapeutic agents, especially the use to inhibit cancer cell proliferation and treat cancers.

The present invention provides a novel class of compounds that modulate KIF18A protein, alone or in microtubule-bound complexes, for the treatment of KIF18A-mediated disorders and/or diseases, including cancer, inflammation or ciliopathy.

The compounds of the present invention exhibit MT-based regulatory activity on KIF 18A, specifically inhibitory activity on KIF 18A. To this end, the present invention also provides the use of these compounds and their pharmaceutically acceptable salts in the preparation and manufacture of pharmaceutical compositions or medicines for therapeutic, prophylactic, acute or chronic treatment of KIF 18A-mediated diseases and disorders (including but not limited to cancers).

The present invention provides an example: a compound having the structure of formula (I), or its pharmaceutically acceptable salts, stereoisomers, isotope isomers, prodrugs, hydrates, or solvates:

A compound having the structure of formula (I), or its pharmaceutically acceptable salts, stereoisomers, isotope isomers, prodrugs, hydrates, or solvates:

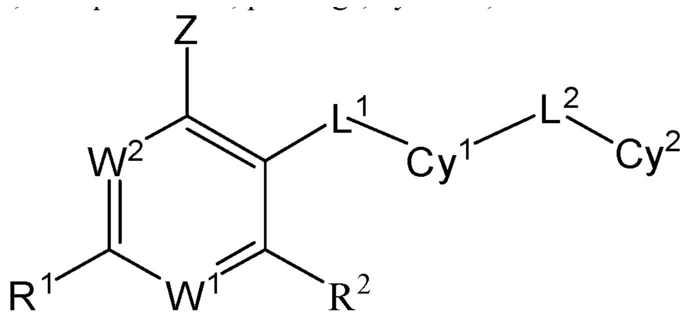

Formula (I)

wherein, $W^1$ represents $CR^{W1}$ or N;

wherein, $W^2$ represents $CR^{W2}$ or N;

wherein, Z represents $-CR^TR^{T'}$ or $-NR^SR^{S'}$;

wherein, $R^T$ and $R^{T'}$, together with the C atom they are attached to, form a ring having a structure selected from the following:

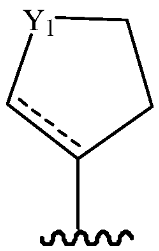 、 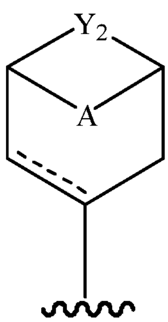 and/or 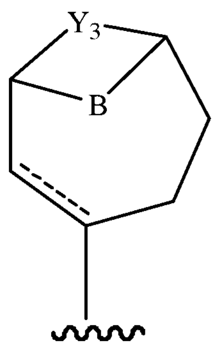 ;

wherein, $R^S$ and $R^{S'}$, together with the N atom they are attached to, form a ring having a structure selected from the following:

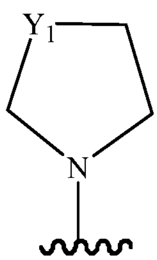 、 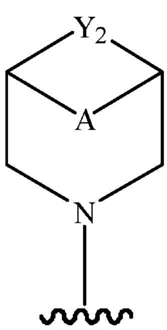 and/or 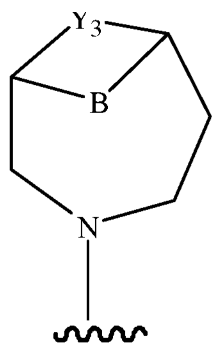

wherein, $Y^1$, $Y^2$ and $Y^3$ each independently represent $-(CR^aR^b)_o-(NR^a)_p-(CR^{a'}R^{b'})_q-$;

wherein, A and B each independently represent $-(CR^aR^b)m-$;

Wherein $L^1$ represents -C(O)NH-, -HNC(O)-, 5-6 membered heteroaryl,

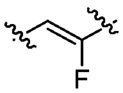, 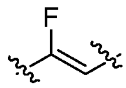, 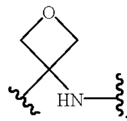, 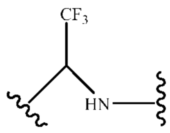, 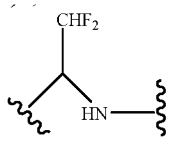, 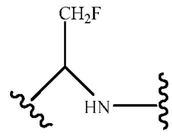;

wherein, $L^2$ represents absence, -$C_1$-$C_6$ alkylene-, -$NR^a$-, -$NR^a$($C_1$-$C_6$ alkylene)-, -C(O)$NR^a$($C_1$-$C_6$ alkylene)-, -O-, -O-($C_1$-$C_6$ alkylene), -S-, -S(O)-, -$S(O)_2$-, -$S(O)_2NR^a$- or -S(O)(NRa)-;
wherein, $R^1$ represents $L^3$-$R^3$;
wherein, $R^2$ represents hydrogen, halogen, cyano, nitro, hydroxy $C_1$-$C_6$ alkyl, $C_1$-$C_6$ alkyl, $C_3$-$C_8$ cycloalkyl, $C_1$-$C_6$ haloalkyl, -$OR^a$, -$SO_3R^a$, -$SR^a$, -$SF_5$, -S(O)$R^a$, -O- $C_1$-$C_6$ haloalkyl or -$NR^aR^b$;
wherein, $L^3$ represents absence, $C_1$-$C_6$ alkylene, -$NR^a$-, -$NR^aSO_2$-, -$SO_2NR^a$-, -S(=O)($NR^a$)-, -P(O)$(OR^a)_2$, -$NR^a$-P(O)$(OR^a)_2$ or -$NR^a$P(O)$(R^a)_2$;
wherein, $R^3$ represents absence, hydrogen, or $C_1$-$C_6$ alkyl or $C_3$-$C_6$ cycloalkyl substituted with 0-3 substituents selected from halogen, -$OR^a$, -$NR^aR^b$, cyano and -O-$C_1$-$C_6$ haloalkyl;
wherein, $R^{W1}$ and $R^{W2}$ each independently represent hydrogen, halogen, cyano, nitro, hydroxy $C_1$-$C_6$ alkyl, $C_1$-$C_6$ alkyl, $C_3$-$C_8$ cycloalkyl, $C_1$-$C_6$ haloalkyl, -$OR^a$, -$SO_3R^a$, -S(O)$R^a$, -O-$C_1$-$C_6$ haloalkyl, -$SR^a$, -$SF_5$ or -$NR^aR^b$;
Wherein $Cy^1$ represents groups with the following structure;

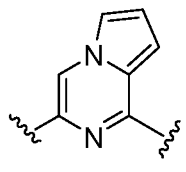 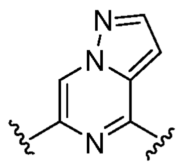 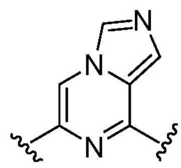 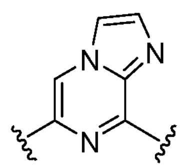

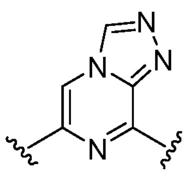 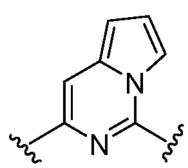 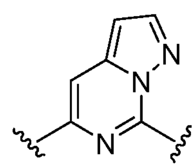 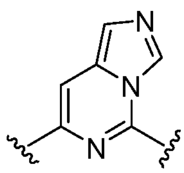

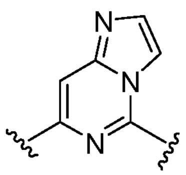 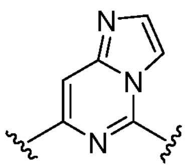 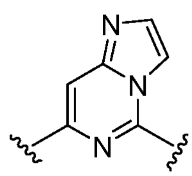 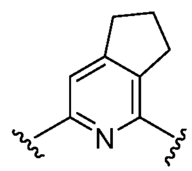

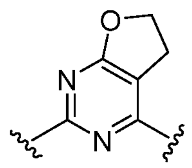 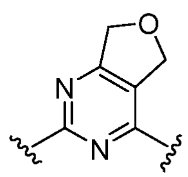 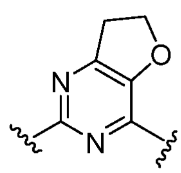 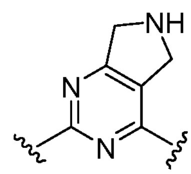

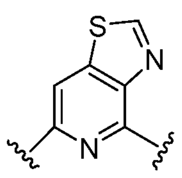 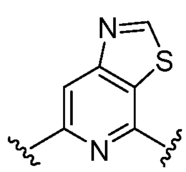 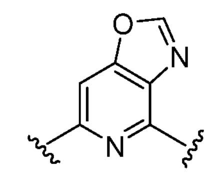 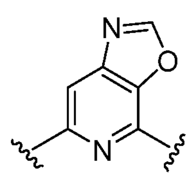

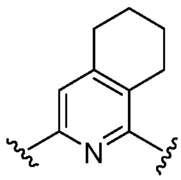
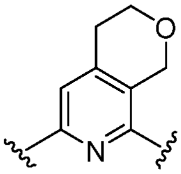
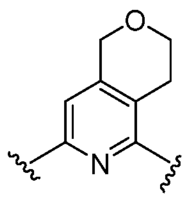
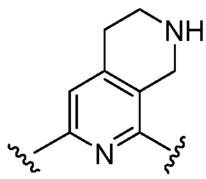

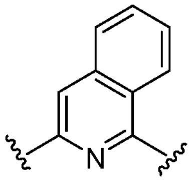
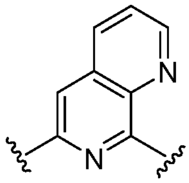
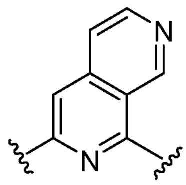
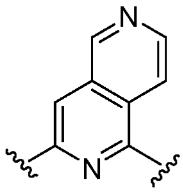

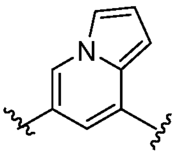
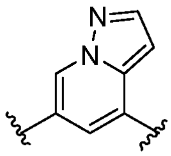
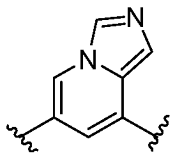
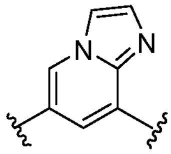

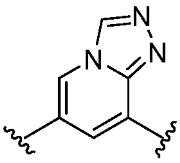
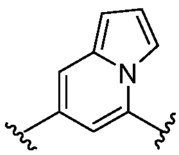
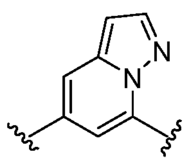
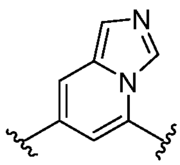

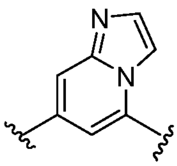
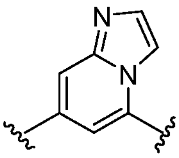
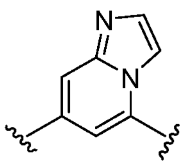
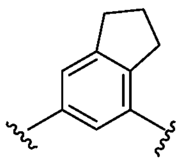

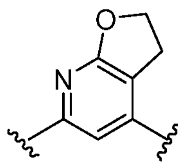
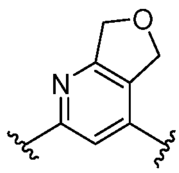
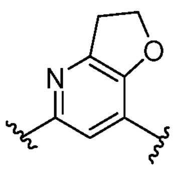
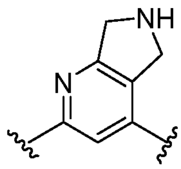

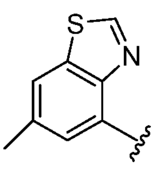
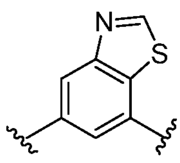
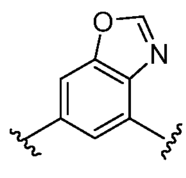
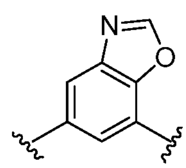

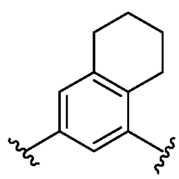
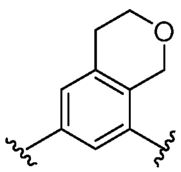
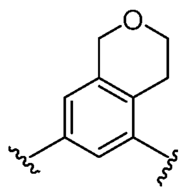
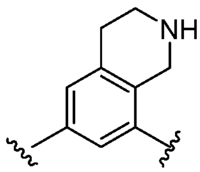

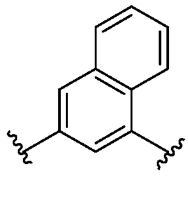 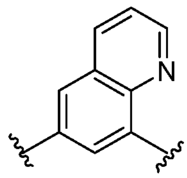 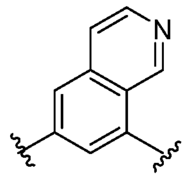 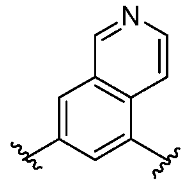

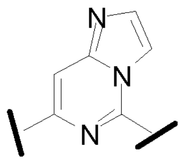 、 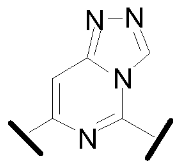 、 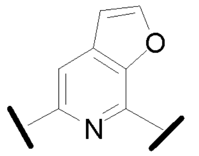 、 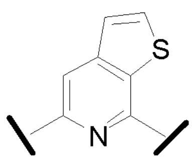

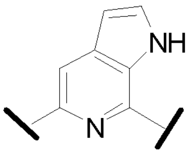 、 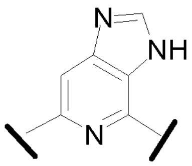 、 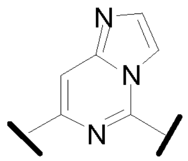 、 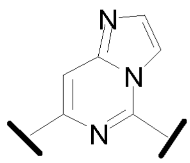

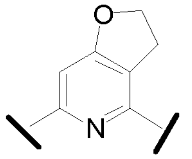 、 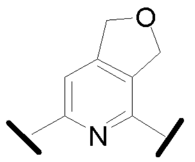 、 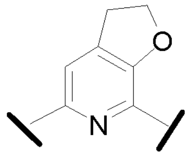 、 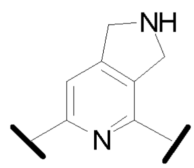

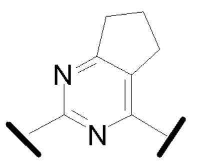 、 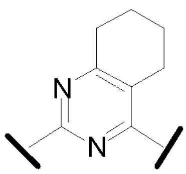 、 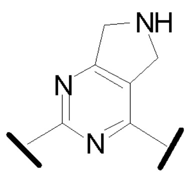 、 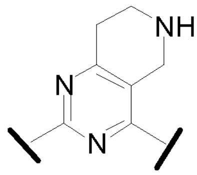

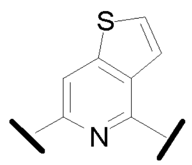 、 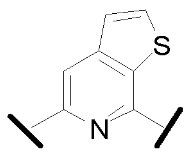 、 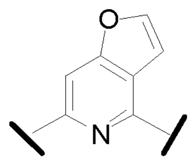 、 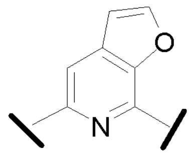

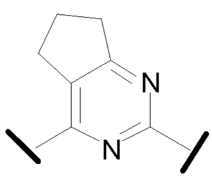 、 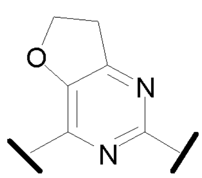 、 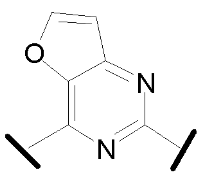 、 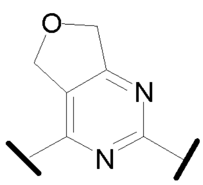

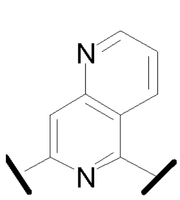 、 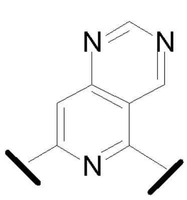 、 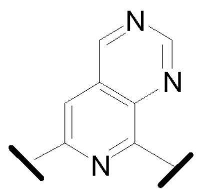 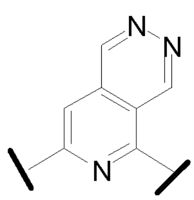

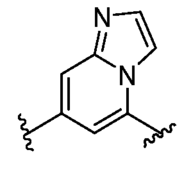 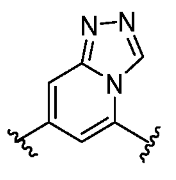 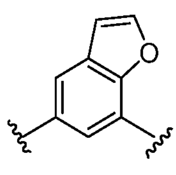 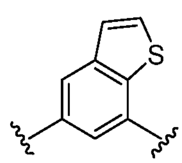

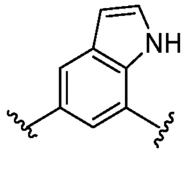 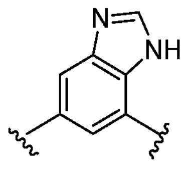 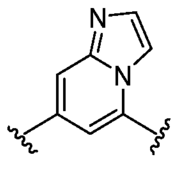 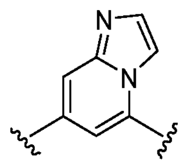

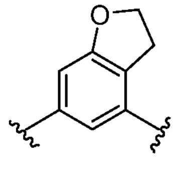 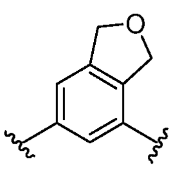 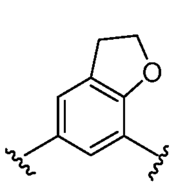 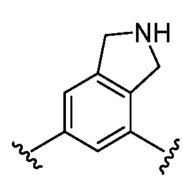

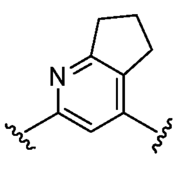 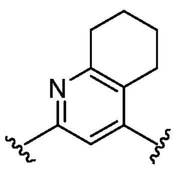 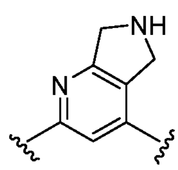 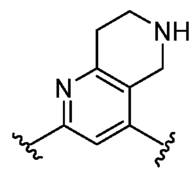

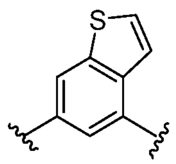 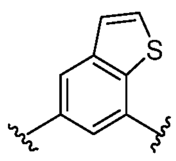 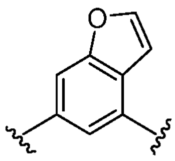 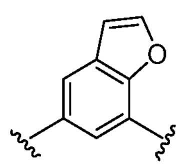

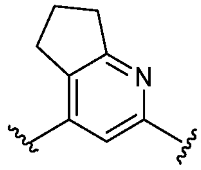 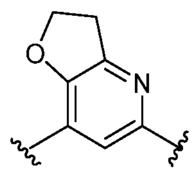 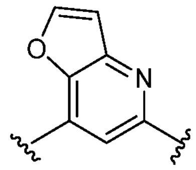 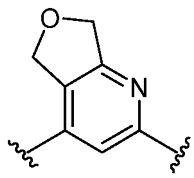

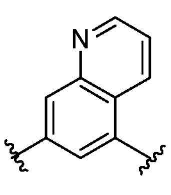 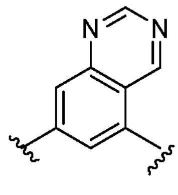 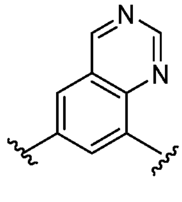 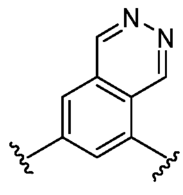

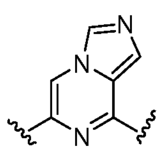 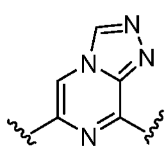 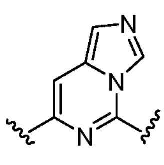 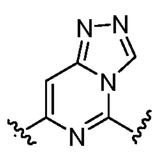

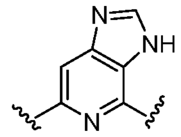 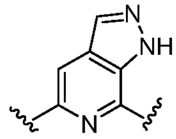 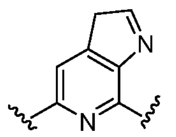 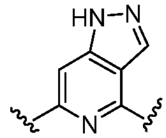

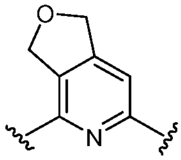 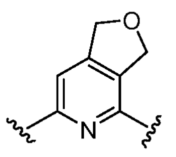 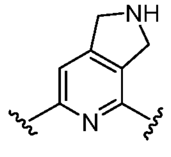 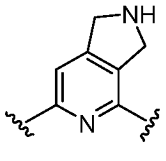

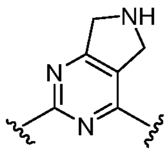 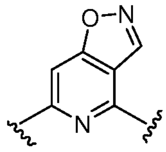 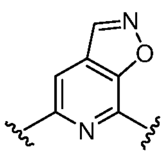 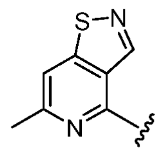

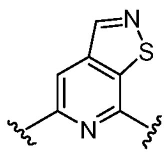 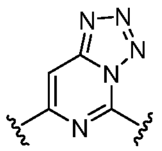 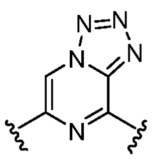 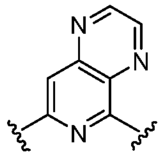

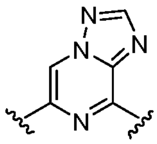 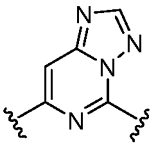 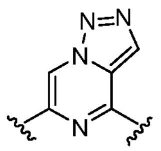 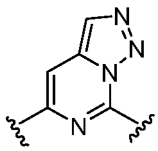

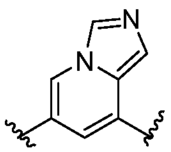 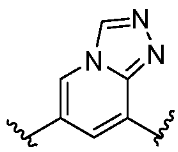 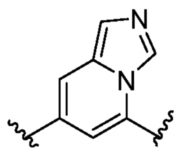 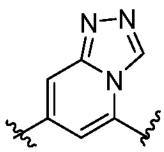

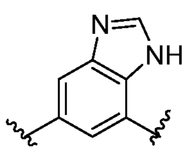 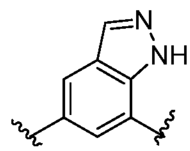 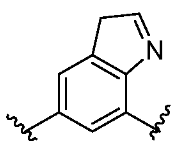 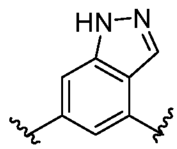

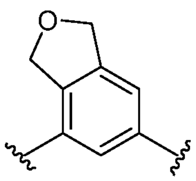 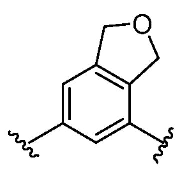 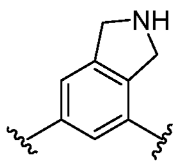 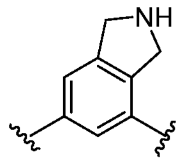

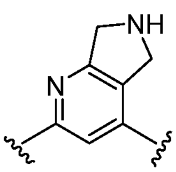 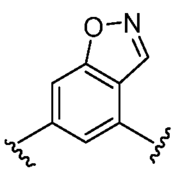 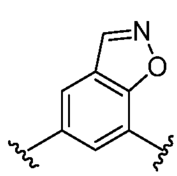 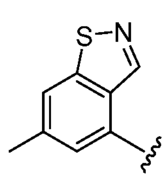

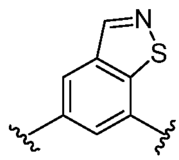 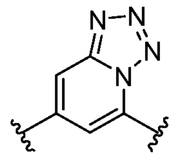 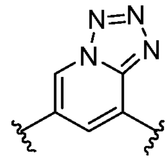 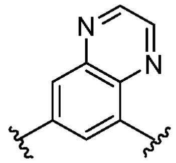

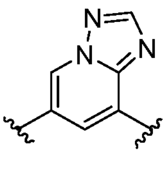 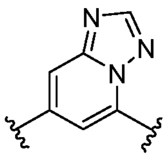 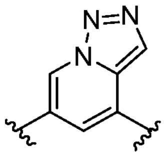 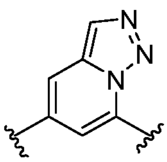

The $Cy^1$ may be optionally substituted with 0-3 substituents selected from the following: halogen, $C_1$-$C_6$ alkyl, $C_1$-$C_6$ haloalkyl, hydroxy $C_1$-$C_6$ alkyl, $OR^a$, -O-$C_1$-$C_6$ haloalkyl, 5-6-membered heteroaryl, phenyl, -$SR^a$, -$SF_5$, cyano, nitro, -$NR^aR^b$, -$NR^aC(O)R^b$, -$C(O)NR^aR^b$, -$OC(O)R^a$, -$C(O)R^a$, -$P(O)R^aR^b$, -$C(O)OR^a$, -$S(O)R^a$, -$S(O)_2R^a$, and -$S(O)_2N$-$R^aR^b$;

wherein, $Cy^2$ represents a 3-12-membered saturated or unsaturated monocyclic or bicyclic ring which may optionally contain 0-3 heteroatoms selected from O, N and S; the $Cy^2$ may be optionally substituted with 0-3 substituents selected from the following: halogen, $C_1$-$C_6$ alkyl, $C_1$-$C_6$ haloalkyl, hydroxy $C_1$-$C_6$ alkyl, -$OR^a$, -O- $C_1$-$C_6$ haloalkyl, -$SR^a$, -$SF_5$, cyano, nitro, -$NR^aR^b$, -$NR^aC(O)R^b$, -$C(O)NR^aR^b$, -$OC(O)R^a$, -$C(O)OR^a$, -$S(O)R^a$, -$S(O)_2R^a$ and -$S(O)_2NR^aR^b$;

wherein, $R^a$, $R^b$, $R^{a'}$ and $R^{b'}$ each independently represent hydrogen, halogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N; or $R^{a'}$ and $R^{b'}$, together with the atom they are attached to, form a 3-6-membered saturated or unsaturated ring which may optionally contain 0-2 heteroatoms selected from O, S and N;

wherein, the dashed line represents a single or double bond;

wherein, m, o, p and q represent integers from 0 to 3.

In a preferred example of the present invention, $W^1$ represents CH or N.

In a preferred example of the present invention, $W^2$ represents CH or N.

In a preferred example of the present invention, $L^3$ represents -$NR^aSO_2$-, -$SO_2NR^a$- or -$S(=O)(NR^a)$-.

In a preferred example of the present invention, $L^3$ represents -$NR^aSO_2$-.

In a preferred example of the present invention, $R^3$ represents hydrogen, or $C_1$-$C_6$ alkyl or $C_3$-$C_6$ cycloalkyl substituted with 0-3 substituents selected from halogen, -$OR^a$, -$NR^aR^b$, cyano, and -O-$C_1$-$C_6$ haloalkyl.

In a preferred example of the present invention, $R^3$ represents $C_1$-$C_6$ alkyl substituted with 0-3 substituents selected from halogen, -$OR^a$, -$NR^aR^b$, cyano and -O- $C_1$-$C_6$ haloalkyl.

In a preferred example of the present invention, Z represents $NR^SR^{S'}$.

In a preferred example of the present invention, Z represents:

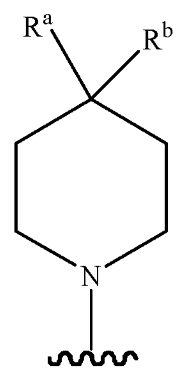 、 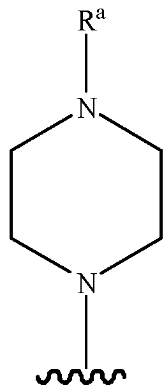 、 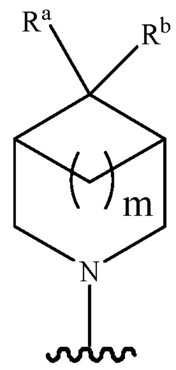 and/or 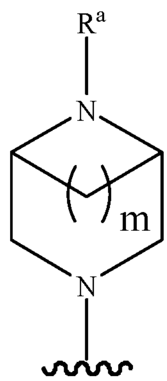 ;

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring

which may optionally contain 0-2 heteroatoms selected from O, S and N.

In a preferred example of the present invention, Z represents:

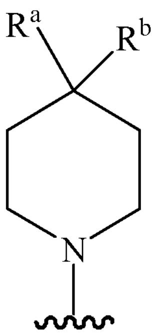,

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N.

In a preferred example of the present invention, Z represents:

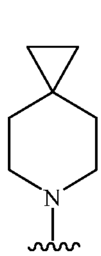 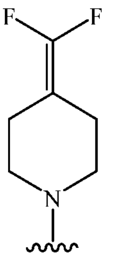

In the preferred example of the present invention, $L^1$ represents -C(O)NH-, -HNC(O)-, 5-6 membered heteroaryl,

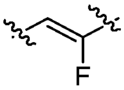, 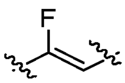, 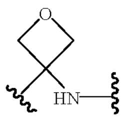,

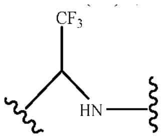, 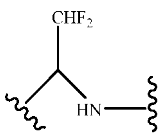, 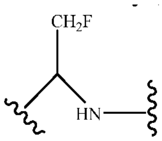.

In a preferred example of the present invention, $L^1$ represents -C(O)NH-.

In a preferred example of the present invention, $Cy^1$ represents the following groups:

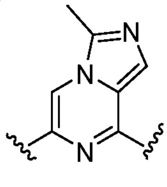 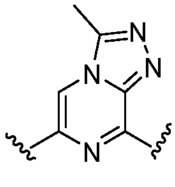 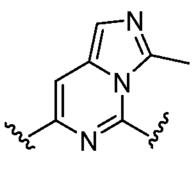 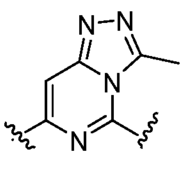

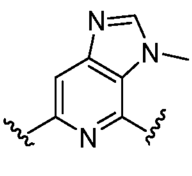 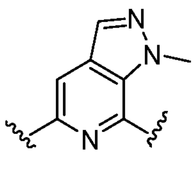 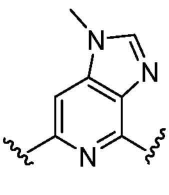 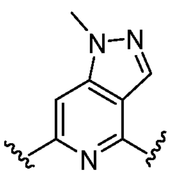

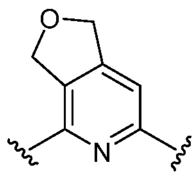 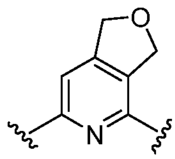 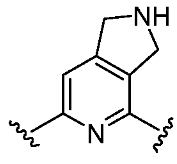 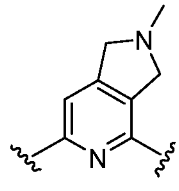

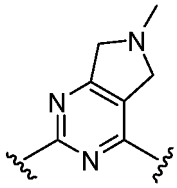 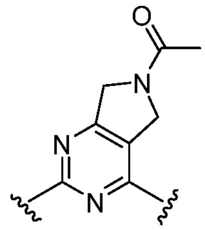 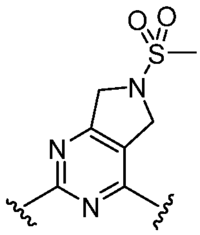 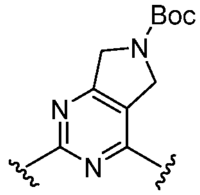

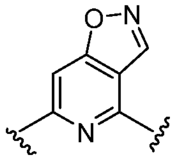 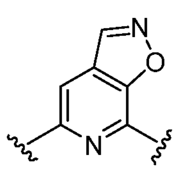 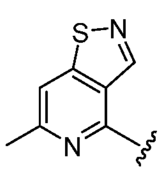 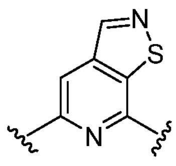

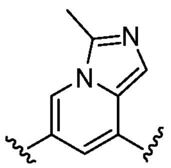 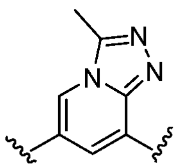 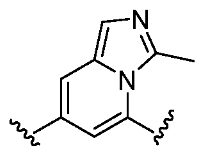 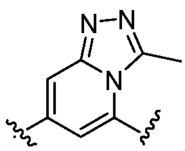

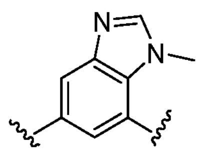 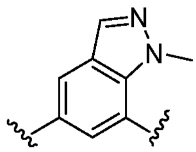 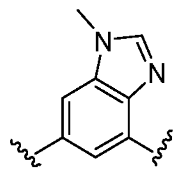 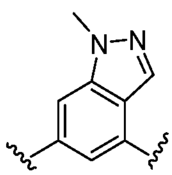

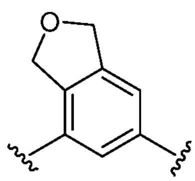 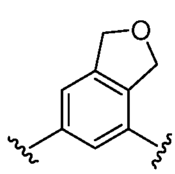 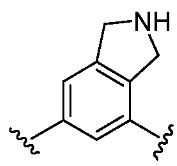 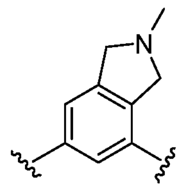

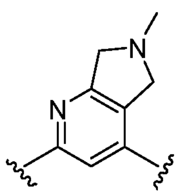 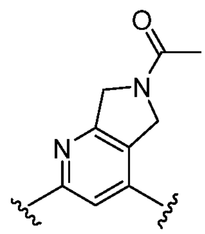 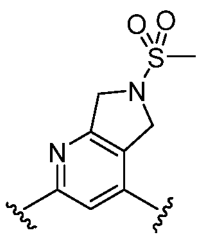 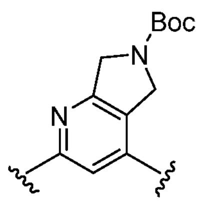

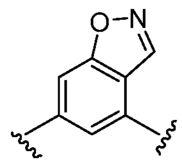 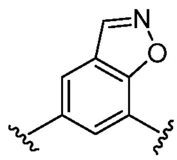 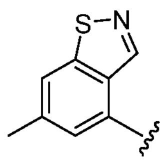 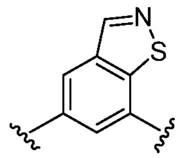 ;

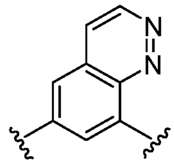 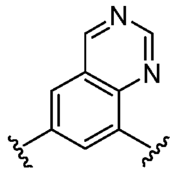 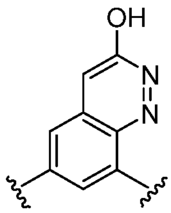 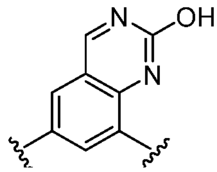

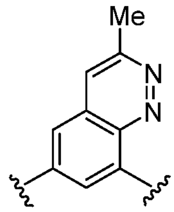 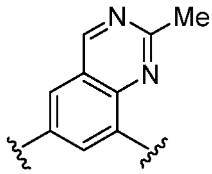 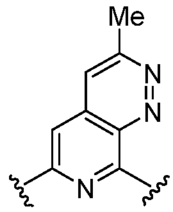 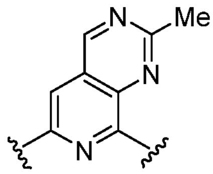

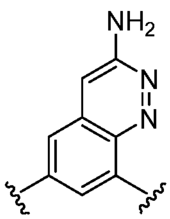 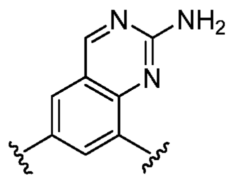 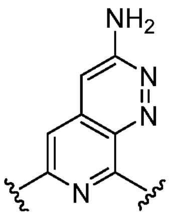 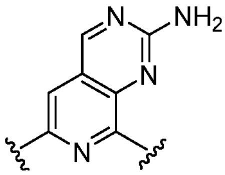

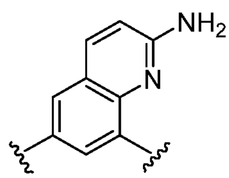 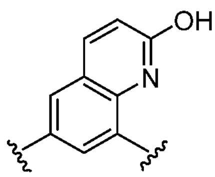 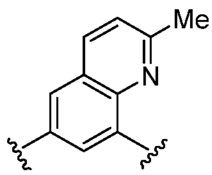 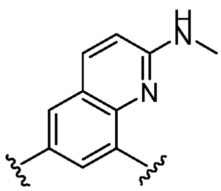

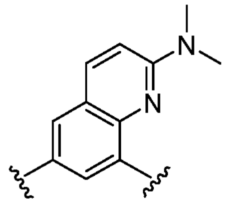 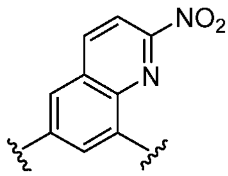 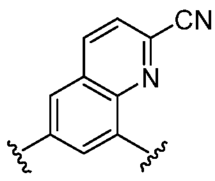 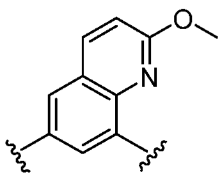

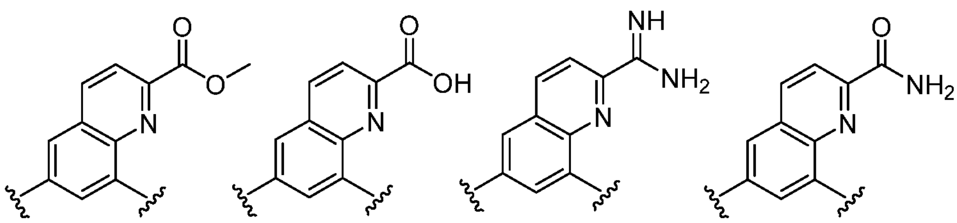

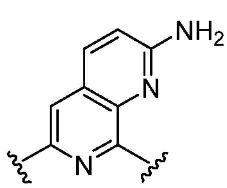
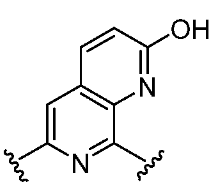
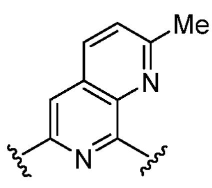
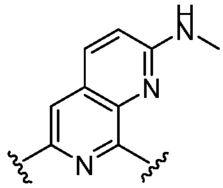

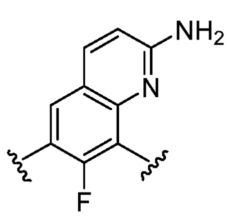
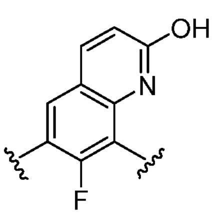
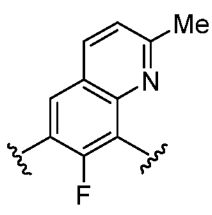
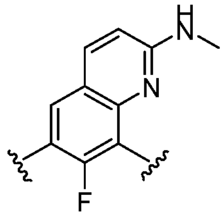

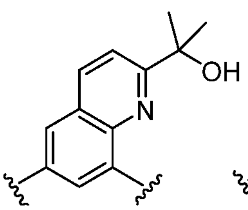
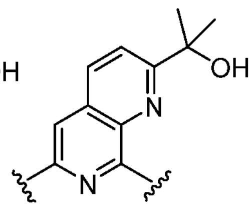
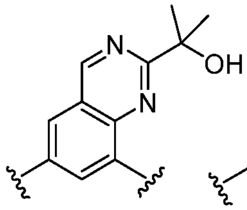
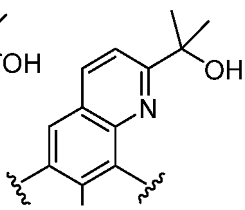

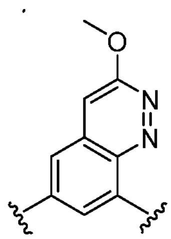
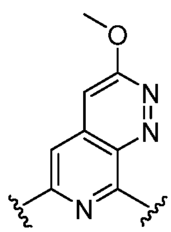
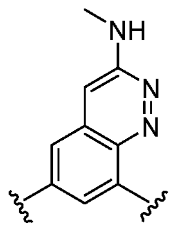
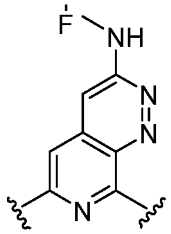

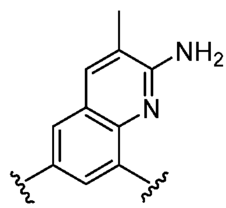
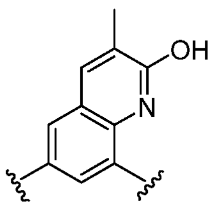
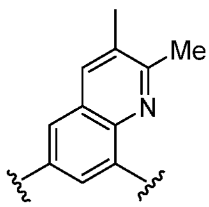
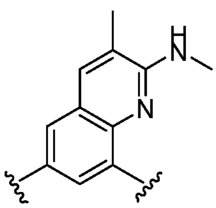

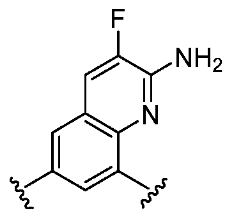
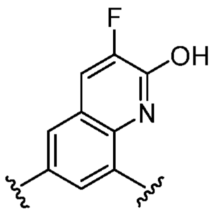
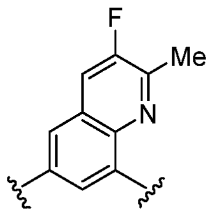
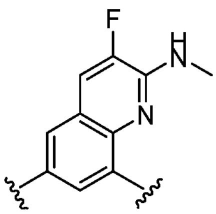

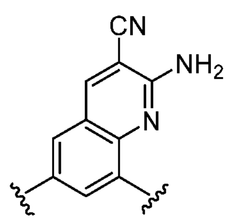
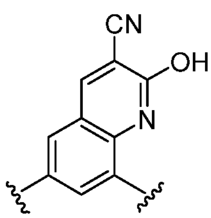
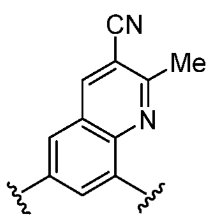
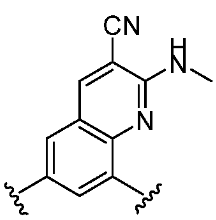

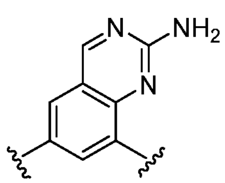 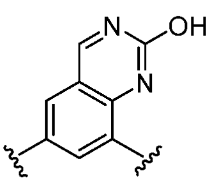 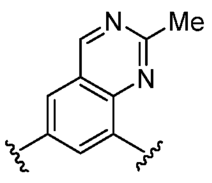 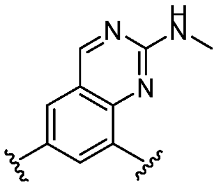

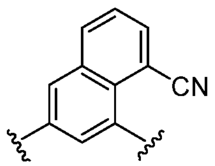 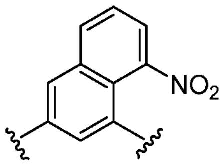 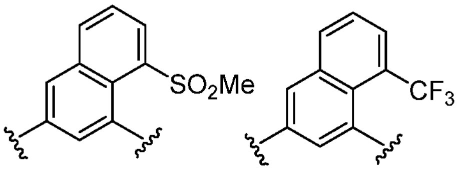

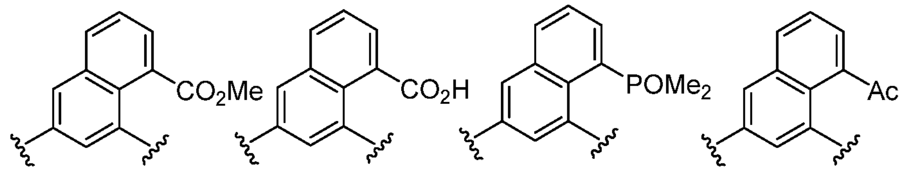

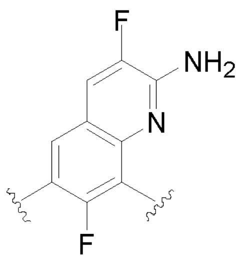 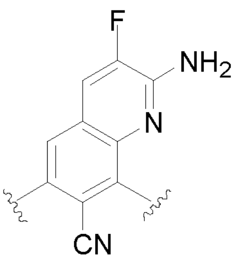 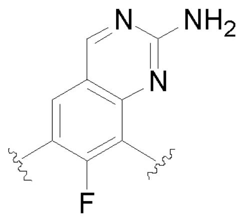 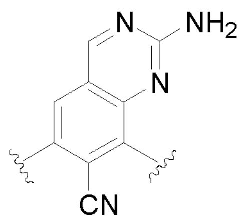

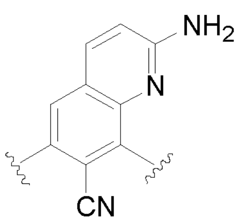 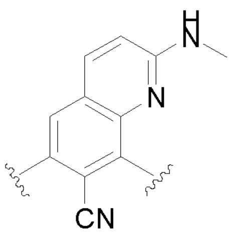 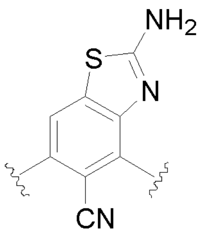 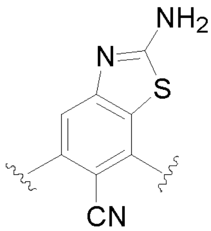

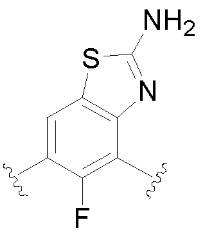 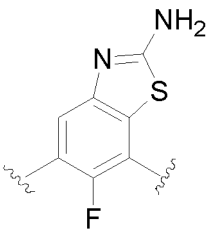

In the preferred example of the present invention, $L^1$ represents -C(O)NH-, -HNC(O)-, 5-6 membered heteroaryl,

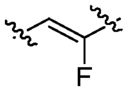 , 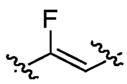 , 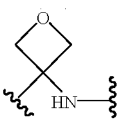 ,

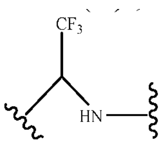, 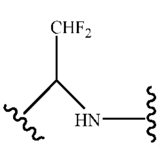, 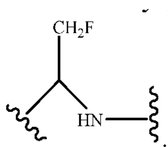.

In a preferred example of the present invention, $L^1$ represents any one of the following groups:

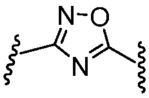 i¢ 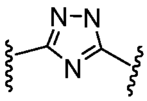 i¢ 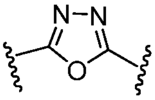 i¢ 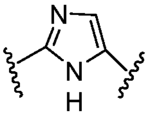 i¢ 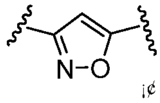 i¢

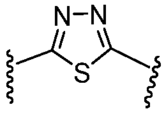 i¢ 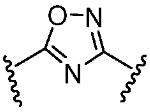 i¢ 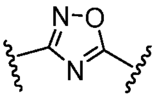 i¢ 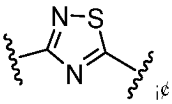 i¢ 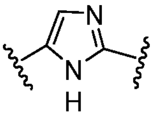

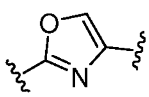 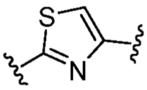 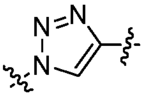 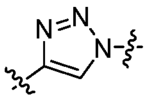 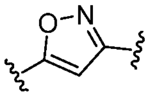

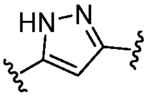 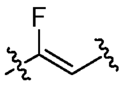 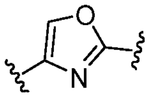 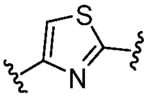 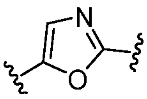

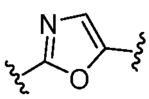 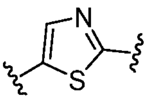 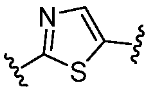 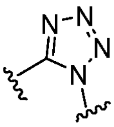 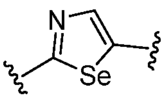

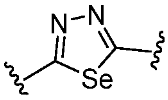 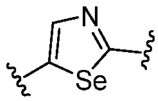 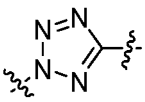 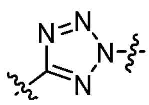 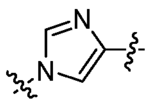

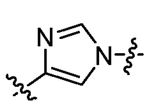 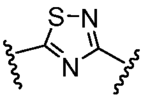

In a preferred example of the present invention, $L^2$ represents absence, -$C_1$-$C_6$ alkylene- or -NH-.

In a preferred example of the present invention, $L^2$ represents absence.

In a preferred example of the present invention, $Cy^2$ represents a saturated, partially saturated or unsaturated 3-, 4-, 5-, 6-, 7-membered monocyclic ring, or a 3-, 4-, 5-, 6-, 7-membered fused ring, which contains 0-3 N heteroatoms and 0-2 O or S heteroatoms and is substituted with 0-3 substituents selected from the following: halogen, cyano, nitro, hydroxy $C_1$-$C_6$ alkyl, $C_1$-$C_6$ alkyl, $C_3$-$C_8$ cycloalkyl, $C_1$-$C_6$ haloalkyl, -$OR^a$, -$SO_3R^a$, -S(O)$R^a$, -O- $C_1$-$C_6$ haloalkyl, -$SR^a$, -$SF_5$ or $NR^aR^b$-substituted morpholinyl, piperidinyl, azetidine, pyrrolidinyl, cyclopropyl, cyclobutyl, cyclopentyl, cyclohexyl, piperazinyl or tetrahydrofuryl; wherein, $R^a$ and $R^b$ each independently represent hydrogen, halogen, $C_1$-$C_6$ alkyl, $C_3$-$C_8$ cycloalkyl, $C_1$-$C_6$ haloalkyl or hydroxy $C_1$-$C_6$ alkyl; or $R^a$ and $R^b$, together with the atom they are attached to, form a 3-6 membered ring which contains 0-2 heteroatoms selected from O, N and S.

In a preferred example of the present invention, $Cy^2$ represents:

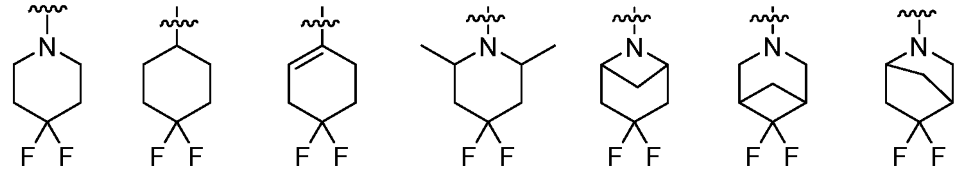

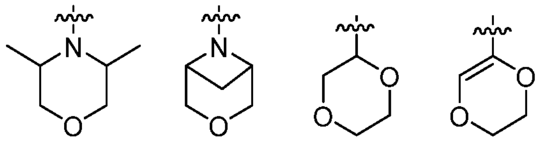

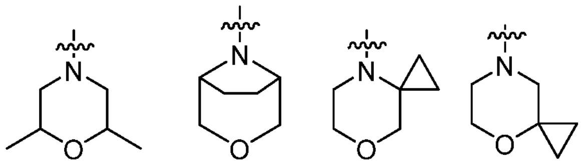

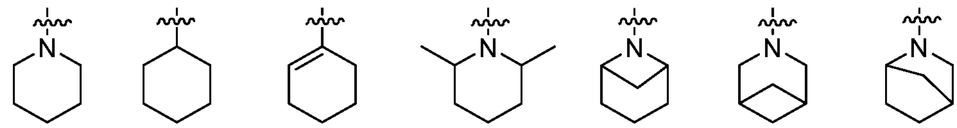

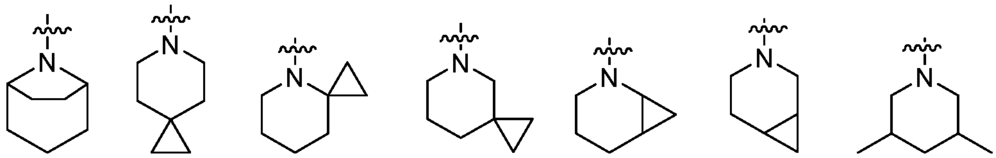

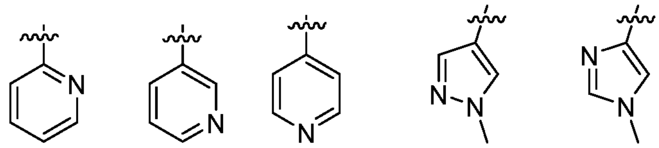

In particular, the present invention provides compounds having the following structures:

| | | | |
|---|---|---|---|
| 1 | 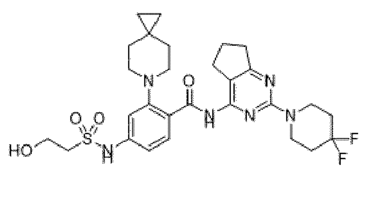 | 2 | 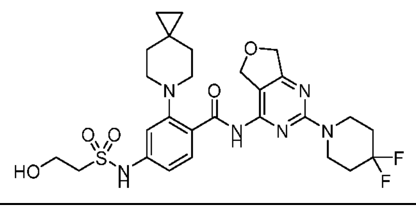 |
| 3 | 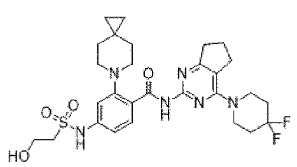 | 4 | 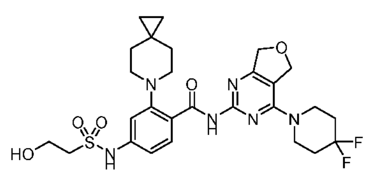 |
| 5 | 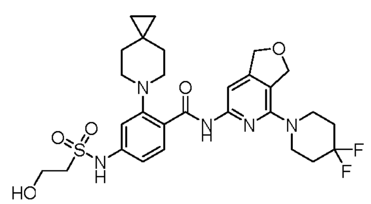 | 6 | 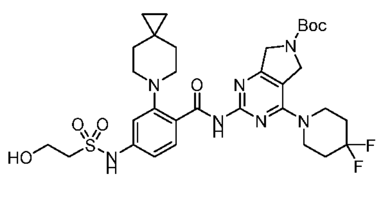 |

(continued)

| | | | |
|---|---|---|---|
| 7 | 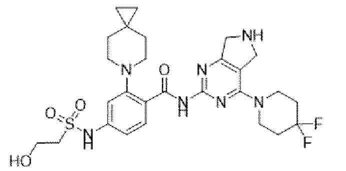 | 8 | 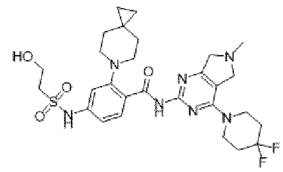 |
| 9 | 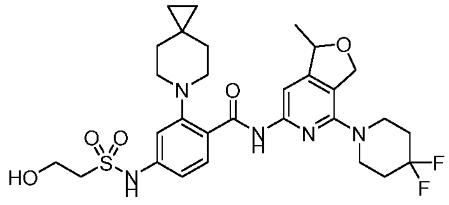 | 10 | 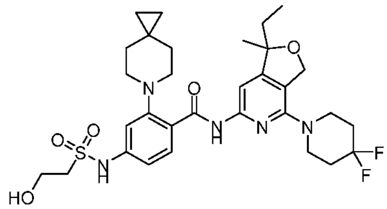 |
| 11 | 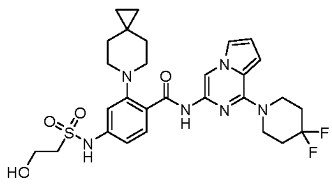 | 12 | 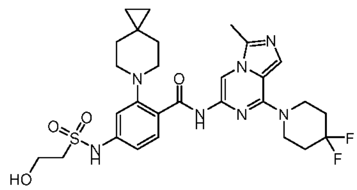 |
| 13 | 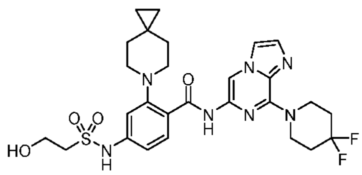 | 14 | 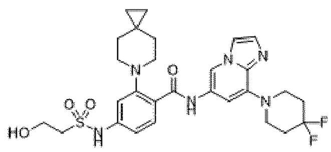 |
| 15 | 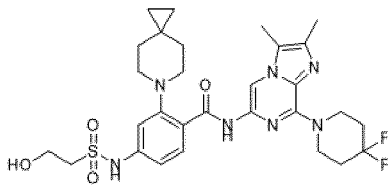 | 16 | 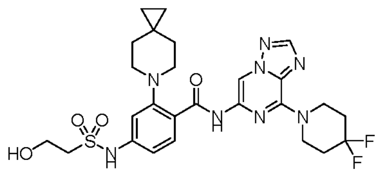 |
| 17 | 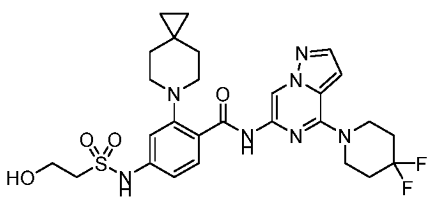 | 18 | 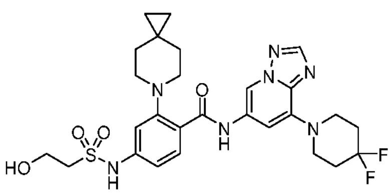 |
| 19 | 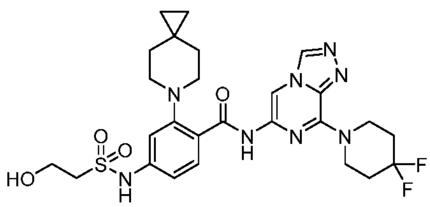 | 20 | 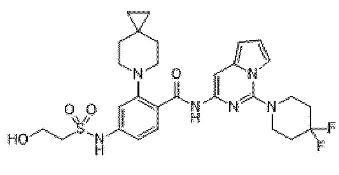 |
| 21 | 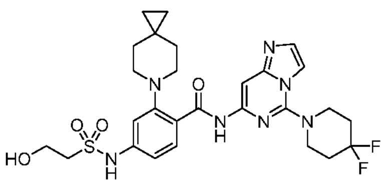 | 22 | 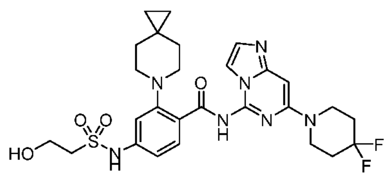 |

(continued)

| | | | |
|---|---|---|---|
| 23 | 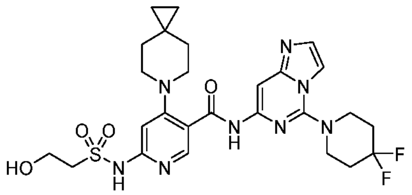 | 24 | 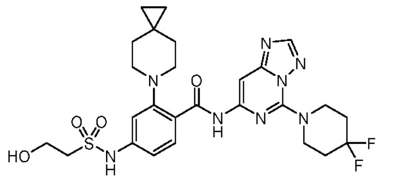 |
| 25 | 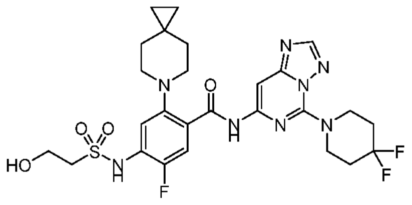 | 26 | 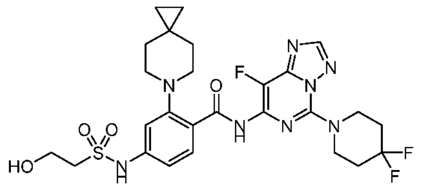 |
| 27 | 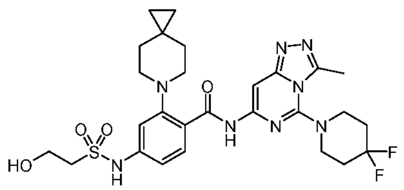 | 28 | 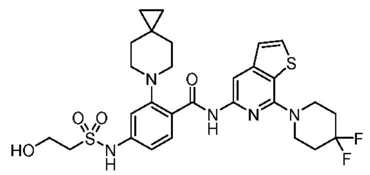 |
| 29 | 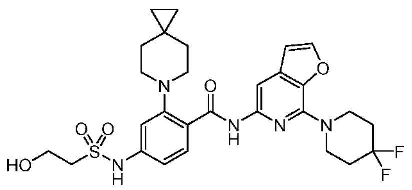 | 30 | 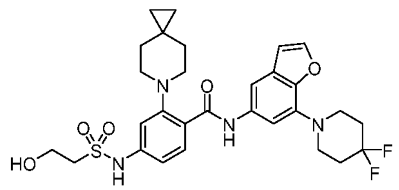 |
| 31 | 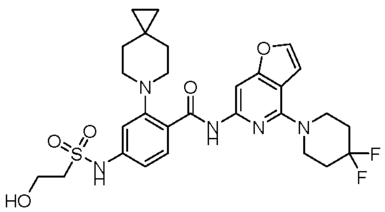 | 32 | 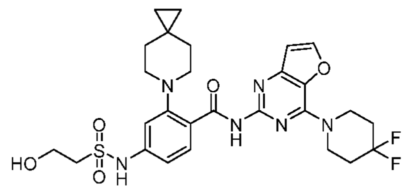 |
| 33 | 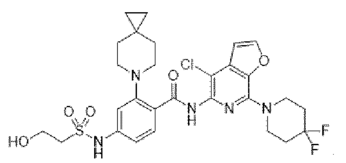 | 34 | 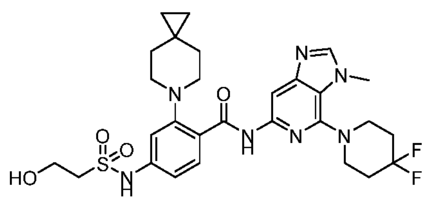 |
| 35 | 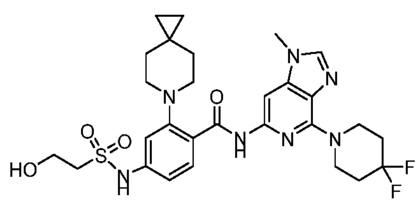 | 36 | 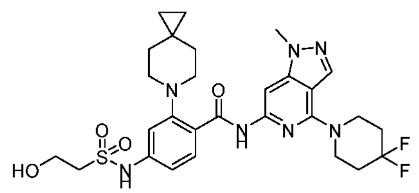 |
| 37 | 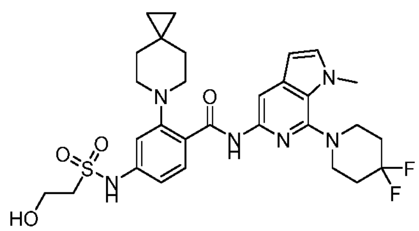 | 38 | 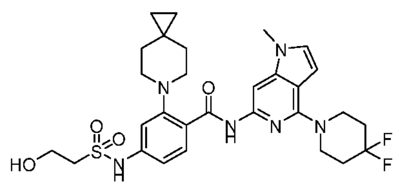 |

(continued)

| | | | |
|---|---|---|---|
| 39 | 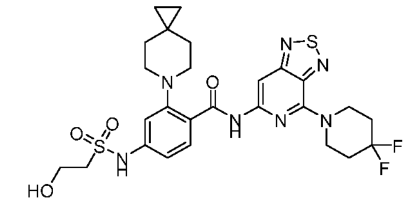 | 40 | 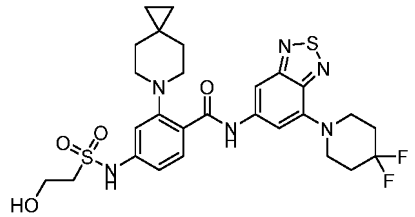 |
| 41 | 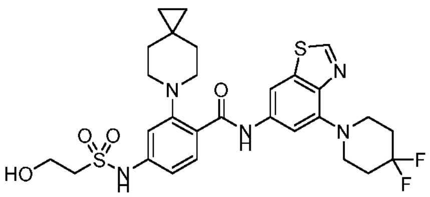 | 42 | 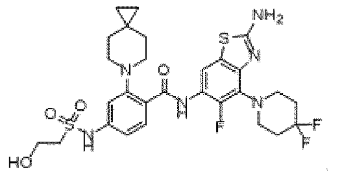 |
| 43 | 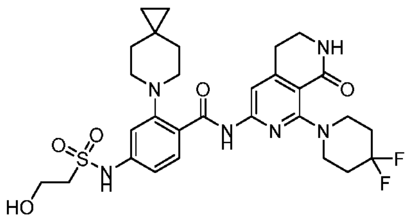 | 44 | 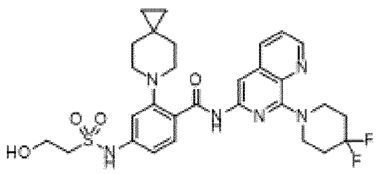 |
| 45 | 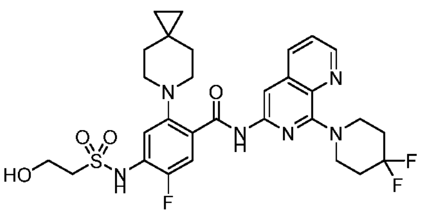 | 46 | 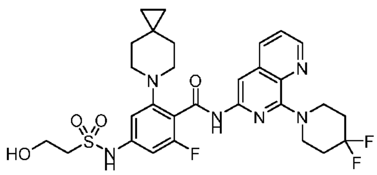 |
| 47 | 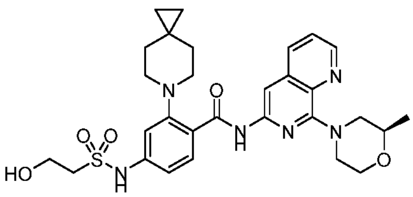 | 48 | 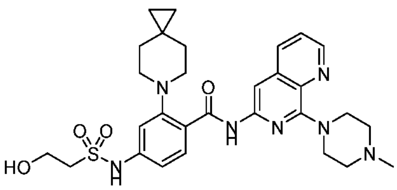 |
| 49 | 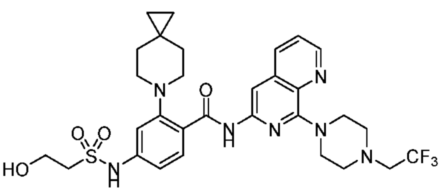 | 50 | 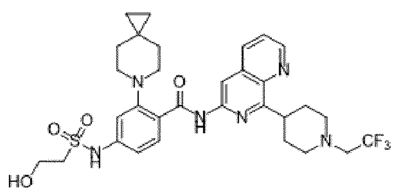 |
| 51 | 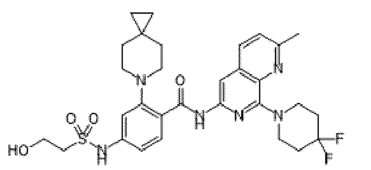 | 52 | 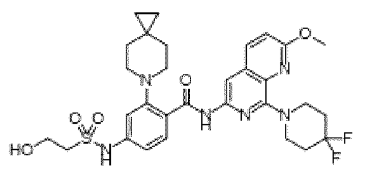 |
| 53 | 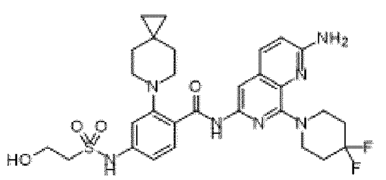 | 54 | 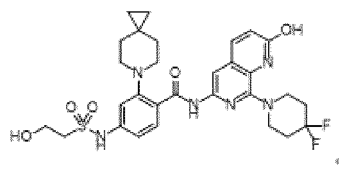 |

(continued)

| | | | |
|---|---|---|---|
| 55 | 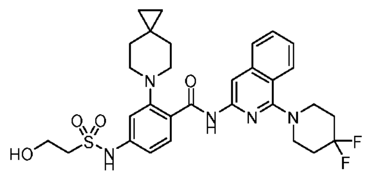 | 56 | 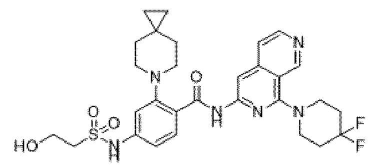 |
| 57 | 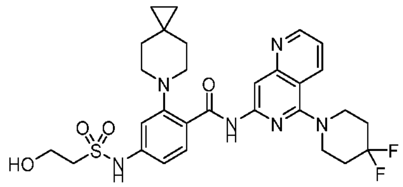 | 58 | 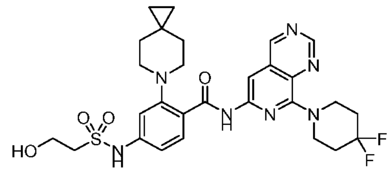 |
| 59 | 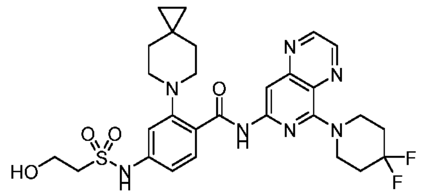 | 60 | 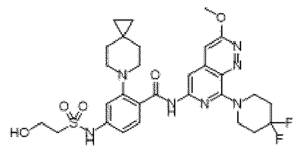 |
| 61 | 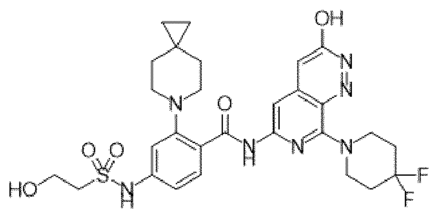 | 62 | 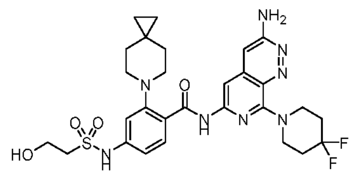 |
| 63 | 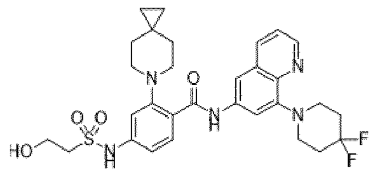 | 64 | 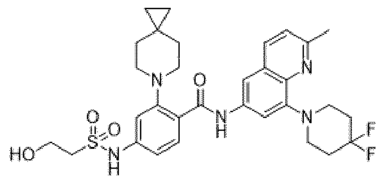 |
| 65 | 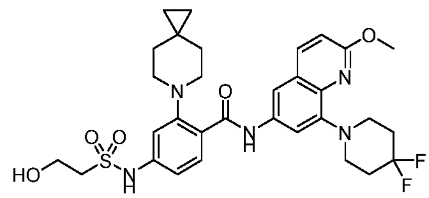 | 66 | 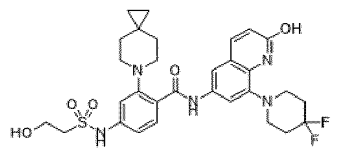 |
| 67 | 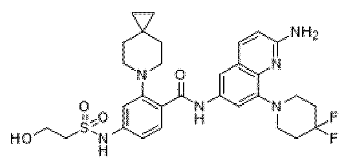 | 68 | 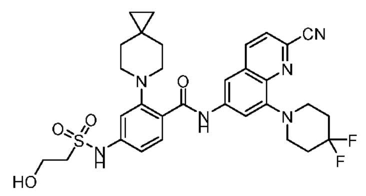 |
| 69 | 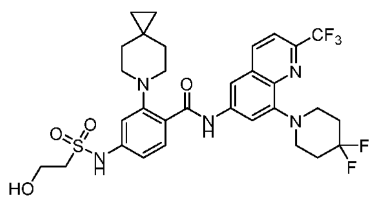 | 70 | 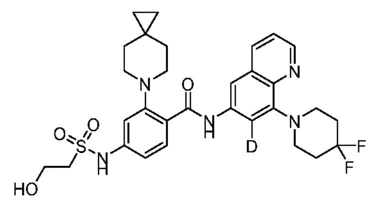 |

(continued)

| | | | |
|---|---|---|---|
| 71 | 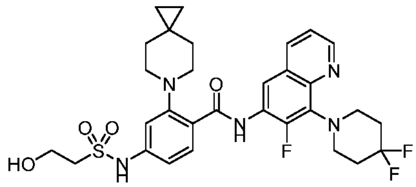 | 72 | 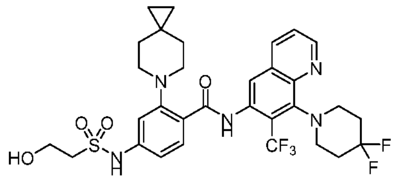 |
| 73 | 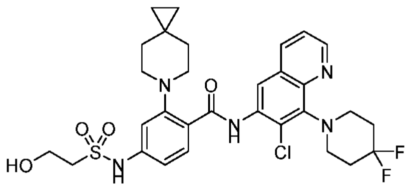 | 74 | 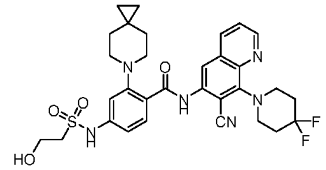 |
| 75 | 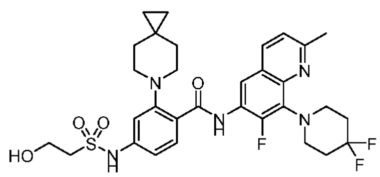 | 76 | 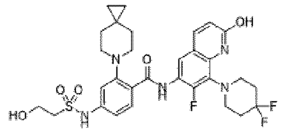 |
| 77 | 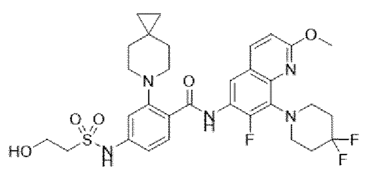 | 78 | 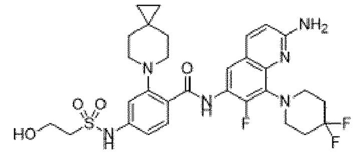 |
| 79 | 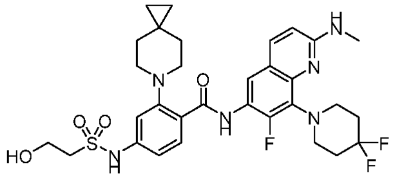 | 80 | 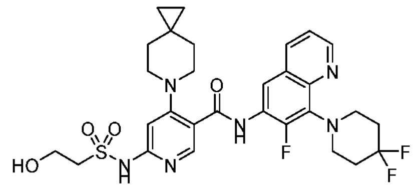 |
| 81 | 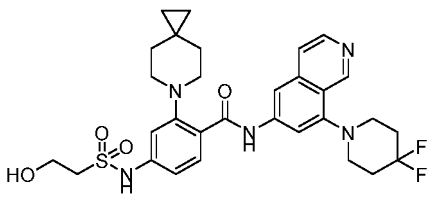 | 82 | 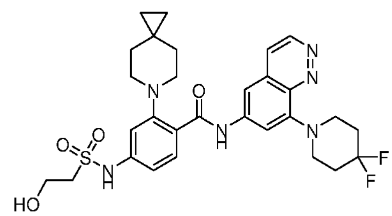 |
| 83 | 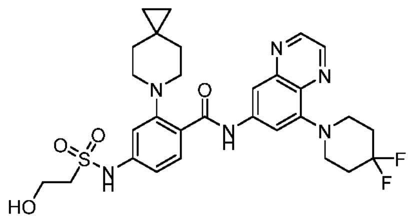 | 84 | 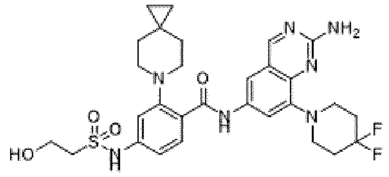 |
| 85 | 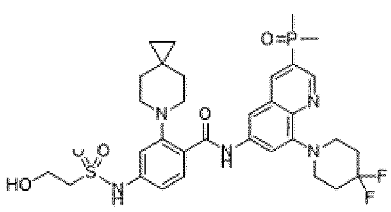 | 86 | 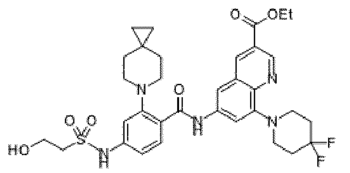 |

(continued)

| | | | |
|---|---|---|---|
| 87 | 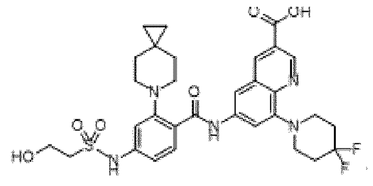 | 88 | 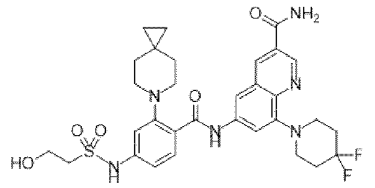 |
| 89 | 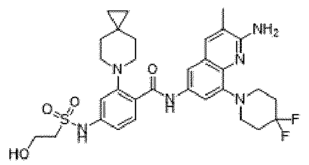 | 90 | 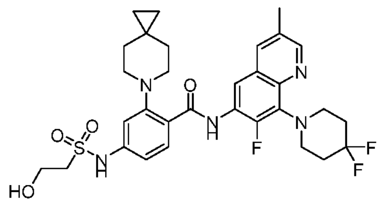 |
| 91 | 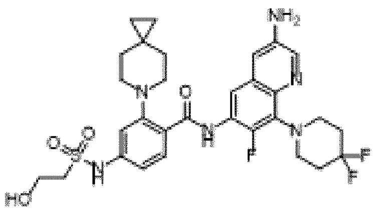 | 92 | 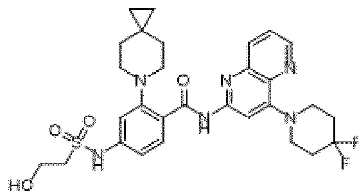 |
| 93 | 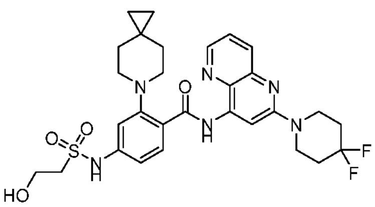 | 94 | 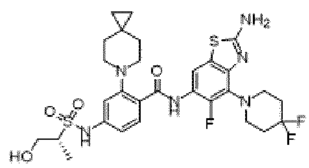 |
| 95 | 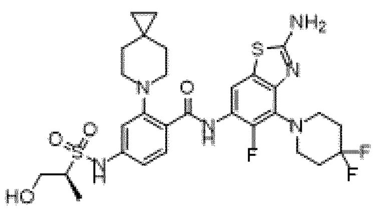 | 96 | 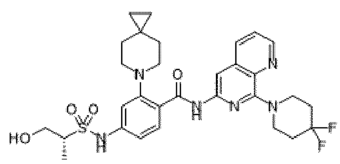 |
| 97 | 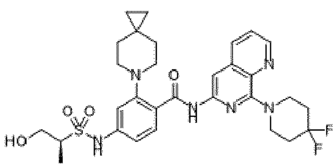 | 98 | 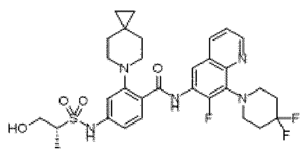 |
| 99 | 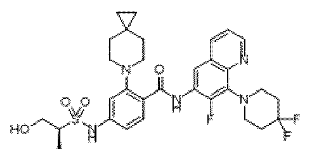 | 100 | 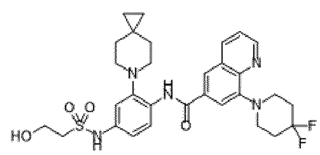 |
| 101 | 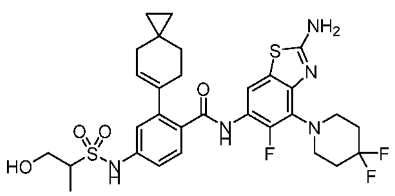 | 102 | 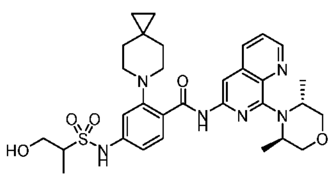 |

In addition, the present invention provides a pharmaceutical composition that contains any one of the compounds

in the examples of the present invention or its pharmaceutically acceptable salts, stereoisomers, isotope isomers, prodrugs, hydrates or solvates and pharmaceutically acceptable carriers.

In addition, the present invention provides a method for treating tumors by inhibiting KIF 18A, comprising administering any one of the compounds of the present invention or its pharmaceutically acceptable salts, stereoisomers, isotope isomers, prodrugs, hydrates or solvates to an individual in need.

**Definitions**

Unless indicated to the contrary, the compounds of the present invention may be extended to include, in addition to their specific structures, their pharmaceutically acceptable salts, stereoisomers, isotope isomers (e.g. deuterated compounds), solvates, hydrates, prodrugs and metabolites, meaning that the pharmaceutically acceptable salts, stereoisomers, isotope isomers, solvates, hydrates, prodrugs and metabolites of these compounds also fall within the scope of protection.

Unless otherwise mentioned, the following terms used in this patent specification and claims have the meanings discussed below. Furthermore, many of the groups defined herein may be optionally substituted. A list of typical substituents is presented in Definitions by way of example and is not intended to limit the substituents defined elsewhere in this patent specification and claims.

The term "alkyl" refers to a linear or branched saturated aliphatic hydrocarbyl or linker, comprising 1-20 carbon atoms, preferably 1-12 carbon atoms, more preferably 1-8 carbon atoms, 1-6 carbon atoms or 1-4 carbon atoms. "Lower alkyl" refers in particular to an alkyl comprising 1-4 carbon atoms. Examples of alkyl include $-(CH_2)_3-$, methyl, trifluoromethyl, ethyl, propyl, isopropyl, n-butyl, isobutyl, tert-butyl and pentyl. Alkyl may be either substituted or unsubstituted. Typical substituents include cycloalkyl, aryl, heteroaryl, heterocycloalkyl, hydroxyl, alkoxy, aryloxy, mercapto, alkylthio, arylthio, cyano, halo, carbonyl, thiocarbonyl, O-carbamoyl, N-carbamoyl, O-thiocarbamoyl, N-thiocarbamoyl, C-acylamino, N-amido, C-carboxyl, O-carboxyl, nitro, silyl, amino and $-NR^xR^y$; wherein, $R^x$ and $R^y$ are independently selected from hydrogen, alkyl, cycloalkyl, aryl, carbonyl, acetyl, sulfonyl, trifluoromethanesulfonyl and a fused 5- or 6-membered heterocyclyl ring.

The term "alkenyl" refers to a linear or branched hydrocarbyl containing one or more double bonds and typically comprising 2-20 carbon atoms. For example, "$C_2$-$C_6$ alkenyl" comprises 2-6 carbon atoms. Examples of alkenyl include, but are not limited to, vinyl, propenyl, butenyl and 1-methyl-2-buten-1-yl.

The term "alkynyl" refers to a linear or branched hydrocarbyl containing one or more triple bonds and typically comprising 2-20 carbon atoms. For example, "C2-C6 alkynyl" comprises 2-6 carbon atoms. Examples of alkynyl include, but are not limited to, ethynyl, 1-propynyl and 1-butynyl.

The term "alkoxy" or "alkyloxy" refers to -O-alkyl. "$C_1$-$C_6$ alkoxy" (or alkyloxy) is intended to include $C_1$, $C_2$, $C_3$, $C_4$, $C_5$ and $C_6$ alkoxy. Examples of alkoxy include, but are not limited to, methoxy, ethoxy, propoxy (e.g., n-propoxy and isopropoxy) and tert-butoxy. Similarly, "alkylthio" or "thioalkoxy" refers to sulfur-bridged alkyl comprising a specified number of carbon atoms as defined above; for example, methyl-S- and ethyl-S-.

The term "cycloalkyl" refers to a 3- to 8-membered all-carbon monocyclic or bicyclic ring, a 5-/6- or 6-/6-membered fused all-carbon bicyclic ring, or a fused polycyclic ring (in a "fused" ring system, each ring shares at least one adjacent carbon atom with other rings) group, in which one or more of the rings may contain one or more double bonds but none of such rings has an intact conjugated π-electron system, or two rings forms a spiro by sharing one carbon. Examples of cycloalkyl include, but are not limited to, cyclopropane, cyclobutane, cyclopentane, cyclopentene, cyclohexane, cyclohexadiene, adamantane, cycloheptane and cycloheptatriene. Bicyclic alkyl includes bridged, spiro or fused-ring cycloalkyl. Illustrative examples of cycloalkyl are derived from but not limited to the following:

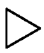 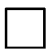 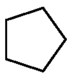 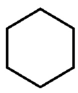 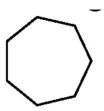 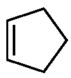 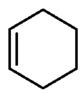 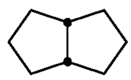

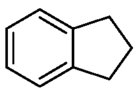 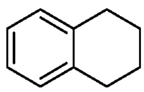 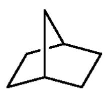 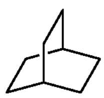 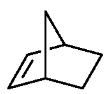 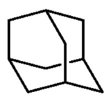

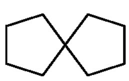 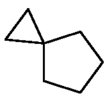 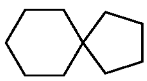 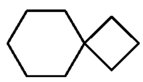

The term "aryl" refers to an all-carbon monocyclic or fused-ring polycyclic group comprising 6-12 carbon atoms, with an intact conjugated $\pi$-electron system. Examples of aryl include, but are not limited to, phenyl, naphthyl and anthracenyl. Aryl may be either substituted or unsubstituted. Typical substituents include halo, trihalomethyl, alkyl, hydroxyl, alkoxy, aryloxy, mercapto, alkylthio, arylthio, cyano, nitro, carbonyl, thiocarbonyl, C-carboxyl, O-carbamoyl, N-carbamoyl, O-thiocarbamoyl, N-thiocarbamoyl, C-acylamino, N-amido, sulfinyl, sulfonyl, amino and $-NR^aR^b$, wherein $R^a$ and $R^b$ are as defined above. The aryl-fused saturated or unsaturated cycloalkyl/saturated or unsaturated heterocycloalkyl may be regarded as a special substituent of aryl, and typical examples include but are not limited to:

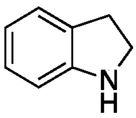 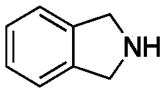 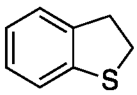 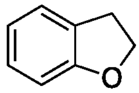 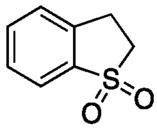

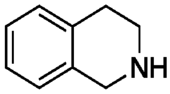 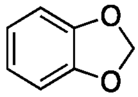

The term "heteroaryl" refers to a monocyclic or fused ring comprising 5-12 ring atoms, with one, two, three or four ring heteroatoms selected from N, O and S and the remaining ring atoms being C, having an intact conjugated $\pi$-electron system. Typical examples of heteroaryl include, but are not limited to, acridinyl, azetidinyl, azocinyl, benzimidazolyl, benzofuryl, benzothiofuranyl, benzothienyl, benzoxazolyl, benzoxazolinyl, benzothiazolyl, benzotriazolyl, benzotetrazolyl, benzoisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofura[2,3-b]tetrahydrofuryl, furanyl, furazanyl, imidazolidinyl, imidazolinyl, imidazolyl, 1H-indazolyl, imidazopyridinyl, indolenyl, dihydroindolyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridoxazolyl, pyridinoimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolyl, 4H-quinolizinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienoxazolyl, thienoimidazolyl, thienyl, triazinyl, 1,2,3-triazolyl, 1,2,4-triazolyl, 1,2,5-triazolyl, 1,3,4-triazolyl and xanthyl, quinolyl, isoquinolinyl, phthalazinyl, quinazolinyl, indolyl, isoindolyl, dihydroindolyl, 1H-indazolyl, benzimidazolyl, 1,2,3,4-tetrahydroquinolyl, 1,2,3,4-tetrahydroisoquinolyl, 5,6,7,8-tetrahydro-quinolyl, 2,3-dihydro-benzofuranyl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and monocyclic "heteroaryl", including but not limited to "-phenylbipyridyl-", "-phenylbipyrimidinyl", "-pyridylbiphenyl", "-pyridylbipyrimidinyl-" and "-pyrimidinylbiphenyl-"; wherein the present invention also includes fused ring and spiro ring compounds containing, for example, heterocyclic rings mentioned above.

A pharmaceutically acceptable heteroaryl is sufficiently stable to be linked to a compound of the present invention for formulation into a pharmaceutical composition and subsequent administration to patients in need.

Unless otherwise defined, the definitions of substituents in the present invention are independent and not interrelated. For example, the definition of $R^a$ (or $R^b$) is independent across different substituents. Specifically, when one definition is selected for $R^a$ (or $R^b$) in a substituent, it does not imply that the same definition applies to $R^a$ (or $R^b$) in other substituents. More specifically, when $R^a$ (or $R^b$) is defined as hydrogen in $NR^aR^b$, for example (non-exhaustive), it does not imply that $R^a$ (or $R^b$) in $-C(O)-NR^aR^b$ must be hydrogen.

"Halo" or "halogen" includes fluorine, chlorine, bromine and iodine. "Haloalkyl" is intended to include branched and linear saturated aliphatic hydrocarbyl having a specified number of carbon atoms substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl also include "fluoroalkyl" which is intended to include branched and linear saturated aliphatic hydrocarbyl having a specified number of carbon atoms and substituted with 1 or more fluorine atoms.

"Haloalkoxy" or "haloalkyloxy" refers to oxygen-bridged haloalkyl having a specified number of carbon atoms as defined above. For example, "C1-C6 haloalkoxy" is intended to include $C_1$, $C_2$, $C_3$, $C_4$, $C_5$ and $C_6$ haloalkoxy. Examples of haloalkoxy include, but are not limited to, trifluoromethoxy, 2,2,2-trifluoroethoxy and pentafluoroethoxy. Similarly,

"haloalkylthio" or "thiohaloalkoxy" refers to sulfur-bridged haloalkyl comprising a specified number of carbon atoms as defined above; for example, trifluoromethyl-S- and pentafluoroethyl-S-.

In the present disclosure, the expression Cx1-Cx2 is used when referring to some substituents, which means that the number of carbon atoms in the substituent may be x1 to x2. For example, $C_0$-$C_8$ means that the group contains 0, 1, 2, 3, 4, 5, 6, 7 or 8 carbon atoms, $C_1$-$C_8$ means that the group contains 1, 2, 3, 4, 5, 6, 7 or 8 carbon atoms, $C_2$-$C_8$ means that the group contains 2, 3, 4, 5, 6, 7 or 8 carbon atoms, $C_3$-$C_8$ means that the group contains 3, 4, 5, 6, 7 or 8 carbon atoms, $C_4$-$C_8$ means that the group contains 4, 5, 6, 7 or 8 carbon atoms, $C_0$-$C_6$ means that the group contains 0, 1, 2, 3, 4, 5 or 6 carbon atoms, $C_1$-$C_6$ means that the group contains 1, 2, 3, 4, 5 or 6 carbon atoms, $C_2$-$C_6$ means that the group contains 2, 3, 4, 5 or 6 carbon atoms, and $C_3$-$C_6$ means that the group contains 3, 4, 5 or 6 carbon atoms.

In the present disclosure, the expression " x1-x2-membered ring" is used when referring to cyclic groups (e.g., aryl, heteroaryl, cycloalkyl and heterocycloalkyl), which means that the number of ring atoms in the group may be x1 to x2. For example, 3-12-membered cyclic group may be a 3, 4, 5, 6, 7, 8, 9, 10, 11 or 12-membered ring comprising 3, 4, 5, 6, 7, 8, 9, 10, 11 or 12 ring atoms; 3-6-membered cyclic group may be a 3, 4, 5 or 6-membered ring comprising 3, 4, 5 or 6 ring atoms; 3-8-membered cyclic group may be a 3, 4, 5, 6, 7 or 8-membered ring comprising 3, 4, 5, 6, 7 or 8 ring atoms; 3-9-membered cyclic group may be a 3, 4, 5, 6, 7, 8 or 9-membered ring comprising 3, 4, 5, 6, 7, 8 or 9 ring atoms; 4-7-membered cyclic group may be a 4, 5, 6 or 7-membered ring comprising 4, 5, 6 or 7 ring atoms; 5-8-membered cyclic group may be a 5, 6, 7 or 8-membered ring comprising 5, 6, 7 or 8 ring atoms; 5-12-membered cyclic group may be a 5, 6, 7, 8, 9, 10, 11 or 12-membered ring comprising 5, 6, 7, 8, 9, 10, 11 or 12 ring atoms; 6-12-membered cyclic group may be a 6, 7, 8, 9, 10, 11 or 12-membered ring comprising 6, 7, 8, 9, 10, 11 or 12 ring atoms. The ring atoms may be carbon atoms or heteroatoms, such as heteroatoms selected from N, O and S. The heterocyclic rings may contain 1, 2, 3, 4, 5, 6, 7, 8, 9, 10 or more ring heteroatoms, such as heteroatoms selected from N, O and S.

In the present invention, one or more halogens may be each independently selected from fluorine, chlorine, bromine and iodine.

The term "substituted" as used herein means that at least one hydrogen atom is substituted by a non-hydrogen group, provided that normal valence is maintained and the substitution results in a stable compound. The term "cyclic double bond" as used herein refers to a double bond formed between two adjacent ring atoms (e.g. C=C, C=N or N=N).

Where nitrogen atoms (e.g. amines) are present on the compounds of the invention, these nitrogen atoms may be converted to N-oxides by treatment with an oxidizing agent (e.g. mCPBA and/or hydrogen peroxide) to obtain other compounds of the invention. Therefore, the nitrogen atoms shown and claimed are deemed to encompass the nitrogen atoms shown and their N-oxides to obtain derivatives of the present invention.

When any variable occurs more than once in any composition or formula of a compound, its definition in each occurrence is independent of its definition in every other occurrence Therefore, for example, if a group is shown substituted with 0-3 Rs, the group may be optionally substituted with up to three R groups, with R defined independently in each occurrence. Furthermore, combinations of substituents and/or variables are permitted only if such combinations result in stable compounds.

The term "patient" as used herein refers to an organism to be treated by a method of the present invention. Such organisms preferably include, but are not limited to, mammals (e.g. rodents, apes/monkeys, horses, cattle, pigs, dogs and cats) and most preferably humans.

The term "effective amount" as used herein refers to an amount of a drug or agent (i.e., a compound of the invention) that will elicit, for example, a biological or medical response in a tissue, a system, an animal or a human sought by a researcher or clinician. Furthermore, the term "therapeutically effective amount" refers to an amount that results in improved treatment, cure, prevention or alleviation of a disease, condition or side effect, or reduction in the progression rate of a disease or condition, as compared to a corresponding subject who does not receive such amount. An effective amount may be administered in one or more doses and is not intended to be limited to a particular formulation or route of administration. The term also includes an amount effective to enhance normal physiological functions within its range.

The term "treatment" as used herein includes its broad sense and encompasses both therapeutic and/or prophylactic treatment of an object. Specifically, "treatment" includes any treatment that results in the alleviation, inhibition, elimination and relief and/or prevention of a condition, disease and disorder, such as alleviation, reduction, regulation, relief, elimination, prophylaxis, prevention or remission of symptoms thereof. The therapeutic treatment includes alleviating, inhibiting or relieving symptoms or conditions of a disease; inhibiting the occurrence of complications; alleviating the underlying metabolic syndrome; inhibiting the occurrence of a disease or condition such as controlling the progression of the disease or condition; alleviating a disease or condition; reducing a disease or condition; alleviating complications caused by a disease or condition, or treating signs caused by a disease or condition. The prophylactic treatment includes a prior treatment to prevent, block or delay, slow the onset or progression of a disease or condition or reduce the severity of the disease or condition.

Similarly, the term "therapeutic agent" also includes agents or reagents used in the therapeutic and/or prophylactic treatment of an object.

The terms "pharmaceutical" or "pharmaceutically acceptable" as used herein refer to compounds, substances,

compositions and/or dosage forms that, within the scope of sound medical judgment, are suitable for contact with human and animal tissues without excessive toxicity, irritation, allergic reactions and/or other issues or complications, and commensurate with a reasonable benefit/risk ratio.

Specific Pharmaceutical and Medical Terms

The term "cancer" as used herein refers to an abnormal growth of cells that is uncontrollable and, under certain conditions, capable of metastasis (spread). This type of cancer includes, but is not limited to, solid tumors [such as bladder, intestine, brain, chest, uterus, heart, kidney, lung, lymphoid tissue (lymphoma), ovary, pancreas or other endocrine organs (e.g. thyroid gland), prostate and skin (melanoma)] or blood tumors (e.g. non-leukemic leukemia).

The term "combined administration" or similar terms thereof, as used herein, refers to the administration of several selected therapeutic agents to a patient in the same or different modes of administration and at the same or different times.

The term "enhancement" or "capable of enhancing" as used herein refers to an expected outcome of an increase or extension in either potency or duration. Thus, in the context of enhancing the therapeutic effect of a drug, the term "capable of enhancing" refers to the ability of the drug to increase or extend its potency or duration within the system. The term "enhancement value" as used herein refers to the ability to maximize the efficacy of another therapeutic agent in an ideal system.

The term "immune disease" refers to a disease or condition resulting from an adverse or harmful response to endogenous or exogenous antigens, typically leading to cellular dysfunction, or functional damage and impairment, or damage to organs or tissues that may produce immune symptoms.

The term "kit" is synonymous with "product packaging."

The terms "object", "subject" or "patient" include both mammals and non-mammals. Mammals include, but are not limited to, mammals including humans and non-human primates (such as orangutans, apes and monkeys), agricultural animals (such as cattle, horses, goats, sheep and pigs), domestic animals (such as rabbits and dogs), and laboratory animals including rodents (such as rats, mice and guinea pigs). Non-mammals include, but are not limited to, birds and fish. **In** a preferred example, the selected mammal is human.

As used herein, a compound or pharmaceutical composition, after administered, can relieve a disease, symptom or condition, particularly to reduce severity, delay onset, slow down progression or shorten duration. This applies whether the administration is fixed or temporary, continuous or intermittent, and can be attributed to or associated with the administration.

Routes of Administration

Suitable routes of administration include, but are not limited to, oral, intravenous, rectal, aerosol, parenteral, ocular, pulmonary, transdermal, vaginal, ear canal, nasal and topical. In addition, by way of example only, routes of parenteral administration include intramuscular, subcutaneous, intravenous, intramedullary, ventricular, intraperitoneal, intralymphatic and intranasal.

The mode of administration of the compounds of the present invention may be topical. In certain examples, long-acting formulations are administered by (subcutaneous or intramuscular) implantation or intramuscular injection. In another example, administration is achieved by a targeted drug delivery system. For example, liposomes coated with organ-specific antibodies. In this example, the liposomes are selectively directed to and absorbed by specific organs.

Pharmaceutical Composition and Dosage

The term "pharmaceutical carrier" as used herein refers to a pharmaceutical substance, composition or vehicle, such as a liquid or solid filler, a diluent, an excipient, a manufacturing aid (e.g., lubricant, talc, magnesium stearate, calcium stearate or zinc stearate or stearic acid) or a solvent-encapsulated substance, used to carry or deliver the target compound from one organ or part of the body to another. Each carrier must be "acceptable" in the sense of being compatible with other ingredients of the formulation and harmless to patients.

The term "pharmaceutical composition" refers to a composition comprising a compound of the present invention and optionally a pharmaceutical carrier. The "pharmaceutical carrier" refers to a medium generally accepted in the art for delivering a biologically active agent to animals (particularly mammals), including (i.e.) adjuvants, excipients or vehicles such as diluents, preservatives, fillers, flow regulators, disintegrants, wetting agents, emulsifiers, suspending agents, sweeteners, corrigents, perfuming agents, antibacterial agents, antifungal agents, lubricants and dispersants, depending on the mode of administration and nature of the dosage form.

The pharmaceutical composition of the present invention may comprise a therapeutically effective amount of one or more compounds described in the present invention formulated with optionally one or more pharmaceutical carriers

(additives) and/or diluents, and optionally one or more other therapeutic agents. The compounds of the present invention may be administered by any suitable means for any of the above uses, for example orally, such as in the form of tablets, pills, powders, granules, elixirs, tinctures, suspensions (including nanosuspensions, microsuspensions and spray-dried dispersions), syrups and emulsions; sublingually; buccally; parenterally, such as by subcutaneous, intravenous, intramuscular or intrasternal injection or infusion techniques (e.g. in the form of sterile injectable aqueous or non-aqueous solutions or suspensions); nasally, including administering to the nasal membrane, such as by inhalation spray; topically, such as in the form of creams or ointments; rectally, such as in the form of suppositories; or intratumorally. They may be administered alone; however, they are typically administered via a drug carrier selected based on the chosen route of administration and standard pharmaceutical practice.

Pharmaceutical carriers are formulated based on various factors known to those skilled in the art. These factors include, but are not limited to: the type and nature of the active agent formulated; the subject to whom the composition containing the active agent is to be administered; the intended route of administration of the composition; and the targeted therapeutic indication. Pharmaceutical carriers include aqueous and non-aqueous liquid media and various solid and semisolid dosage forms.

The above-mentioned carriers may comprise many different ingredients and additives in addition to the active agent. These ingredients are included in the formulation for various reasons known to those skilled in the art, such as stabilizing active agents and binding agents. Descriptions of suitable pharmaceutical carriers and factors involved in carrier selection can be obtained from several readily available sources, such as Allen L. V. Jr. et al. Remington: The Science and Practice of Pharmacy( 2 Volumes), 22nd Edition (2012), Pharmaceutical Press.

Dosage regimens of the compounds of the present invention will, of course, vary depending on known factors such as the pharmacodynamic properties of the particular agent and its mode and route of administration; the species, age, sex, health status, medical condition and weight of the recipient; the nature and severity of symptoms; the type of concurrent treatment; the frequency of treatment; the route of administration, renal and hepatic function of the patient and expected effects. According to general guidance, the daily oral dose of each active ingredient when used for a given effect should range from about 0.001 mg/day to 10-5000 mg/day, preferably from about 0.01 mg/day to 1000 mg/day, and most preferably from about 0.1 mg/day to 250 mg/day. During constant-rate infusion, the most preferred intravenous dose should range from about 0.01 mg/kg/min to 10 mg/kg/min. The compounds of the present invention may be administered in a single daily dose, or divided into two, three or four doses daily.

The compounds are typically administered in the form of mixtures with suitable pharmaceutical diluents, excipients or carriers (collectively referred to herein as pharmaceutical carriers) appropriately selected according to the intended form of administration (e.g., oral tablets, capsules, elixirs and syrups) and conventional pharmaceutical practice.

A dosage form (pharmaceutical composition) suitable for administration may contain about 1 mg-2000 mg of active ingredient per dosage unit. In any of these pharmaceutical compositions, the active ingredient will generally be present in an amount of about 0.1-95% by weight based on the total weight of the composition.

The scope of the present invention includes pharmaceutical compositions comprising a therapeutically effective amount of at least one compound of the invention as an active ingredient, alone or in combination with a pharmaceutical carrier. Optionally, the compounds of the invention may be used alone, in combination with other compounds of the invention or in combination with one or more other therapeutic agents (e.g. anticancer agents or other pharmaceutically active substances).

The compounds of the present invention (which may be used in a suitable hydrated form) and/or the pharmaceutical compositions of the present invention are formulated into pharmaceutical dosage forms by conventional methods known to those skilled in the art, regardless of the selected route of administration.

The actual dose level of the active ingredient in a pharmaceutical composition of the present invention may be adjusted to reach an amount effective for achieving the desired therapeutic response, composition and mode of administration and ensuring nontoxicity for a particular patient.

The dosage level will be selected depending on a variety of factors, including the activity of the particular compound of the invention or its ester, salt or amide; route of administration; duration of administration; the excretion rate of the particular compound; the rate and extent of absorption; duration of treatment; other medications, compounds and/or substances used in combination with the particular compound; factors well known in the medical art such as age, sex, weight, condition, general health and previous medical history of the patient to be treated.

A physician or veterinarian of ordinary skill in the art can readily determine and prescribe an effective amount of a desired pharmaceutical composition. For example, to achieve a desired therapeutic effect, a physician or veterinarian may start with a dose level lower than the desired level for any compound of the present invention incorporated in a pharmaceutical composition, and then gradually increase the dose level until the desired effect is achieved. Typically, a suitable daily dose for a compound of the invention will be the lowest dose of the compound effective to produce a therapeutic effect. This effective dose usually depends on the factors mentioned above. Typically, oral, intravenous, intracerebroventricular and subcutaneous doses for compounds of the invention range from about 0.01 to 50 mg/kg body

weight per day for use in patients. If desired, an effective daily dose of the active compound may be administered in two, three, four, five, six or more sub-doses at a suitable interval throughout the day, optionally in unit dosage form. In certain examples of the present invention, administration is once daily.

Although the compounds of the present invention may be administered alone, they are preferably administered in the form of pharmaceutical formulations (compositions).

Kit/Product Packaging

For use in the treatment of the above indications, kit/product packaging is also described herein. A kit may consist of a delivery device, a drug pack, or a container box which may be divided into several compartments to accommodate one or more containers such as vials, tubes and the like, each containing a separate ingredient involved in a method described herein. Suitable containers include bottles, vials, syringes and test tubes. These containers are made of acceptable materials such as glass or plastic.

For example, a container may contain one or more compounds described herein, either as a pharmaceutical ingredient or in a mixture with other ingredients described herein. The container may have a sterile outlet (e.g. the container may be an intravenous infusion bag or bottle, and its stopper can be pierced by a hypodermic needle). A kit may contain a compound and a description, label or instructions for use for a method described herein.

A typical kit may comprise one or more containers, each containing one or more materials (e.g. reagents, concentrated stock solutions and/or devices) to accommodate commercial promotion and user needs for the use of the compound. These materials include, but are not limited to, buffers, diluents, filters, needles, syringes, delivery devices, bags, containers, bottles and/or test tubes, accompanied by a list of contents and/or instructions for use, and a description of built-in packaging (if any). The entire set of instructions should be included in the kit.

A label may appear on or closely associated with a container. The presence of a label on a container means that letters, numbers or other features are pasted, molded and engraved onto the container; the label may also appear inside a container box or transport box containing multiple containers, such as in a product insert. A label may be used to indicate a specific therapeutic use of the contents. The label may also carry instructions for the use of the contents, such as described in the methods above.

All features described in the specification (including any stated claim, abstract and figure) and/or all steps involved in any method or process may exist in any combination unless some features or steps are mutually exclusive in the same combination.

The above-mentioned features in the present invention or the features mentioned in examples may be combined arbitrarily. All features disclosed in the specification may be used in conjunction with any composition form. Each feature disclosed in the specification may be replaced by any alternative feature that provides the same, equivalent or similar purpose. Therefore, unless otherwise specified, the features disclosed are only general examples of equivalent or similar features.

The present invention will be further described below in conjunction with specific examples. It should be noted that these examples are not intended to define the scope of the present invention but merely to describe the present invention. The experimental methods with no specific conditions indicated in the following examples usually follow conventional conditions or the conditions recommended by manufacturers. Unless otherwise stated, all percentages, ratios, proportions or parts are measured by weight.

The unit of weight-to-volume percentage in the present invention is well known to those skilled in the art, for example, it refers to the weight of solute in 100 mL of solution. Unless otherwise defined, all technical and scientific terms used herein have the same meanings as those familiar to those skilled in the art. In addition, any methods and materials similar or equivalent to those described herein may be used in the methods of the present invention. The preferred methods and materials described herein are for exemplary purposes only.

## SPECIFIC IMPLEMENTATIONS

The present invention is further described through the following specific examples, which are not intended to limit the scope of the present invention to these examples. Experimental methods with no specific conditions indicated in the following examples should be selected according to conventional methods and conditions, or commercial specifications.

NMR is measured using Bruker AVANCE-400 NMR spectrometer. Solvents used for measurement are indicated in the spectrum analysis.

MS measurement is conducted using Agilent 1200-G1956A/1200-6110A/1200-6140A/1260-6125B/Prime-6125B/1260-6120 liquid chromatograph-mass spectrometer (LC/MS), SHIMADZU 20A-2010/20A-2020 LC/MS and Waters ACQ-QDA LC/MS.

HPLC analysis is performed using a SHIMADZU 20A high performance liquid chromatograph.

SFC analysis and determination are conducted using Waters UPCC with PDA Detector and QDa Detector ultra-high performance convergence chromatograph, Waters UPC[2] with PDA detector ultra-high performance convergence chromatograph, Agilent 1260 with DAD detector high-performance liquid chromatograph, Shimadzu LC-20AB with PDA detector high-performance liquid chromatograph and Shimadzu LC-20AD with PDA detector high-performance liquid chromatograph.

Preparative HPLC separation is conducted using Shimadzu LC-20AP pump, Shimadzu LH-40 Liquid Handler, Shimadzu SPD-20A Detector, Gilson GX-281 Liquid Handler, Gilson 322 pump and Gilson 156 UV Detector preparative chromatographs.

SFC separation is conducted using The Berger MG II, MG III, Sepiatec's Prep SFC 100 system, Waters Prep 80Q SFC SYSTEM, Prep 150 AP SFC SYSTEM, Prep 200 SFC SYSTEM and Prep 350 SFC SYSTEM.

Flash column chromatography separation is conducted using Biotage IsoleraOne flash-preparative chromatograph.

The GF254 acrylic adhesive silica gel plate manufactured by Anhui Liangchen Silicon Material Co., Ltd. is used for thin-layer chromatography (TLC). The specification of the TLC silica gel plate is 0.25 mm and that for products separated and purified by TLC is 0.5 mm.

Pressurized hydrogenation reaction is conducted using hydrogenation bottles and hydrogen gas cylinders.

Microwave reaction is conducted using Biotage Initiator + microwave synthesizer. The glove box used is customized by DELLIX.

The present invention is further described through the following examples, which are not intended to impose any limitations on the present invention. The compounds of the present invention can be prepared by a variety of synthetic methods known to those skilled in the art, including the specific examples listed below, examples formed by their combination with other chemical synthesis methods and equivalent alternatives well known to those skilled in the art. Preferred examples include, but are not limited to, the examples of the present invention. It will be apparent to those skilled in the art that various changes and improvements can be made to specific examples of the present invention without departing from the spirit and scope of the present invention.

**Intermediate A1: 2-(4,4-difluoropiperidin-1-yl)-6-methylpyrimidin-4-amine**

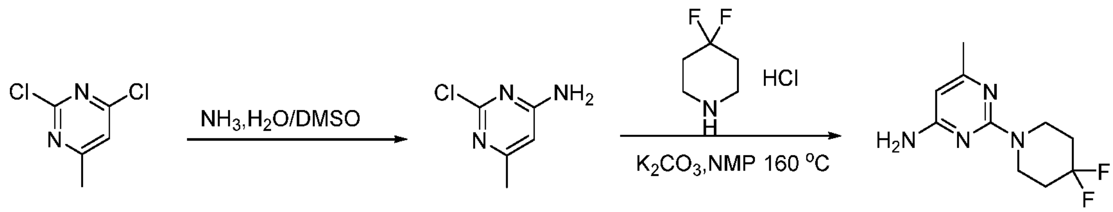


Step 1: To a solution of 2,4-dichloro-6-methylpyrimidine (3.00 g, 18.40 mmol) in DMSO (30 mL) was added liquor ammonia (2.5 mL, 18.04 mmol) at 25°C, and the reaction solution was stirred at 25°C for 16 h. The reaction solution was extracted with ethyl acetate (50 mL×3). The combined organic layer was dried over anhydrous sodium sulfate, filtered, concentrated and purified by slurrying (ethyl acetate: petroleum ether=1:1, 10 mL) to give 2-chloro-6-methylpyrimidin-4-amine (2.00 g, yield: 75%) as a white solid. LCMS (ESI): $[M+H]^+$ = 144.1.

Step 2: To a solution of 2-chloro-6-methylpyrimidin-4-amine (7.50 g, 41.79 mmol) and 4,4-difluoropiperidine hydrochloride (12.00 g, 76.13 mmol) in *N*-methylpyrrolidone (75 mL) was added N,N-Diisopropylethylamine (25 mL) at 25°C. The reaction solution was stirred at 160°C for 6 h.The reaction mixture was concentrateddissolved in dichloromethane (50 mL), and purified by flash column chromatography (silica gel, 0-10% gradient of methanol/dichloromethane) to give a yellow oil crude. The crude product was dissolved in ethyl acetate (50 mL) and washed with water (50 mL×2). The organic phase was dried over anhydrous sodium sulfate, filtered, and concentrated to give a pale yellow solid. The yellow solid was stirred in petroleum ether (30 mL) for 30 min. The white precipitate was filtered to give 2-(4,4-difluoropiperidin-1-yl)-6-methylpyrimidin-4-amine (3.00 g, yield: 38%). LCMS (ESI): $[M+H]^+$ = 229.1.

**Intermediate A2: 4-Bromo-N-(2-(4,4-difluoropiperidin-1-yl)-6-methylpyrimidin-4-yl)-2-(6-azaspiro [2.5]oct-6-yl) benzamide**

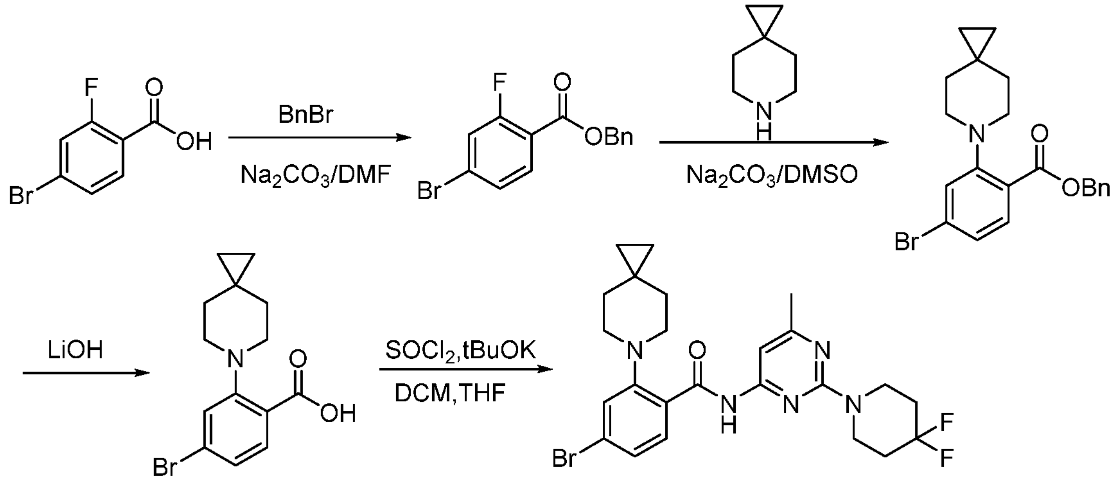


Step 1: To a stirred solution of 4-bromo-2-fluorobenzoic acid (20.00 g, 90.41 mmol), and sodium carbonate (14.52 g, 135.61 mmol) in N,N-dimethylformamide (200 mL) was addedbenzyl bromide (17.18 g, 99.45 mmol) at 30°C. The reaction mixture was stirred at 30°C for 15 h. The reaction mixture was quenched with water (400 mL) and extracted with methyl tert-butyl ether (3×300 mL).The combined organic layer was washed with saline (400 mL), dried over anhydrous sodium sulfate, filtered, and concentrated under reduced pressure to give benzyl 4-bromo-2-fluorobenzoate (29.00 g) as a white solid crude. LCMS (ESI): $[M+H]^+$ = 308.

Step 2: To a solution of benzyl 4-bromo-2-fluorobenzoate (20.00 g, 59.79 mmol) in DMSO (120 mL) was add sodium carbonate (21.82 g, 203.8 mmol), and 6-azaspiro[2.5]octane hydrochloride (13.38 g, 89.69 mmol). The reaction mixture was stirred at 100°C for 15 h. The reaction mixture was quenched with water (200 mL) and extracted with methyl tert-butyl ether (3 × 200 mL). The combined organic layer was washed with saline (3×200 mL), dried over anhydrous sodium sulfate, filtered, and concentrated under reduced pressure to give benzyl 4-bromo-2-(6-azaspiro[2.5]octan-6-yl) benzoate (23.00 g, yield: 89%) as a red oil. LCMS (ESI): $[M+H]^+$ = 402.1.

Step 3: To a solution of benzyl 4-bromo-2-(6-azaspiro[2.5]octan-6-yl)benzoate (7.00 g, 16.24 mmol) in methanol/water (60 mL/15 mL) was added lithium hydroxide monohydrate (1.15 g, 48.72 mmol), and the reaction solution was stirred at 35°C for 15 h. The reaction mixture was concentrated, dissolved the residue in water (50 mL), washed the aqueous phase with ethyl acetate (3 × 70 mL), adjusted the pH to 5 with aqueous hydrogen chloride solution (1 M, 20 mL), and extracted with ethyl acetate (3 × 50 mL). The organic layer was washed with saturated aqueous sodium chloride solution (100 mL), dried over anhydrous sodium sulfate, and concentrated to give 2-(6-azaspiro[2.5]octan-6-yl)-4-bromobenzoic acid (4.00 g, yield: 73%) as a red solid. LCMS (ESI): $[M+H]^+$ = 311.7.

Step 4: To a solution of 4-bromo-2-(6-azaspiro[2.5]oct-6-yl) benzoic acid (1000 mg, 3.22 mmol) in dichloromethane (10 mL) and N,N-dimethylformamide (0.2 mL) was added thionyl chloride (1.10 g, 9.67 mmol), and the reaction solution was stirred at ambient temperature for 2 h. The reaction solution was concentrated, and toluene (10 mL) was added, then concentrated. The operation was repeated for 3 times. To a solution of potassium tert-butoxide (1096 mg, 9.67 mmol) in THF (10 mL) were added 2-(4,4-difluoropiperidin-1-yl)-6-methylpyrimidin-4-amine (735 mg, 3.22 mmol). and the crude product from the above step in the solution of THF (20 mL).The reaction solution was stirred at ambient temperature for 4 h. The reaction solution was filtered, concentrated, and purified by flash column chromatography (silica gel, 0-25% gradient of ethyl acetate/petroleum ether) to give 4-bromo-N-(2-(4,4-difluoropiperidin-1-yl)-6-methylpyrimidin-4-yl)-2-(6-azaspiro[2.5] oct-6-yl)benzamide (600 mg, yield: 35%) as a pale yellow solid.

**Intermediate A3: 4-Nitro-2-(6-azaspiro[2.5]octan-6-yl)benzoyl chloride**

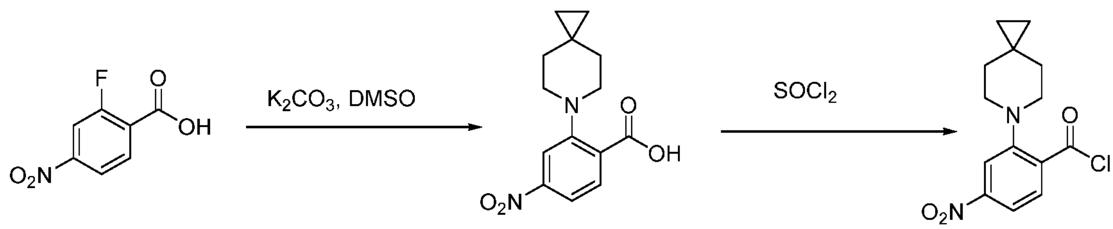

Step 1: To a stirred solution of 2-fluoro-4-nitrobenzoic acid (10.00 g, 53.48 mmol), potassium carbonate (22.60 g, 160.45 mmol) in DMSO (50 mL) was added 6-azaspiro[2.5]octane hydrochloride (8.69 g, 58.83 mmol). Then the reaction was stirred at 140°C for 12 h. 2 M aqueous hydrochloric acid solution was added dropwise to the reaction solution to adjust the pH to about 5. Then, the solution was filtered and dried the cake to give 4-nitro-2-(6-azaspiro[2.5]octan-6-yl)benzoic acid (10.60 g) as a yellow solid.

Step 2: 4-nitro-2-(6-azaspiro[2.5]octan-6-yl)benzoic acid (500 mg, 1.81 mmol) was added to the solvent of thionyl chloride (5 mL). Then the reaction was stirred at 90°C for 1 h. The reaction solution was concentrated with dichloromethane to give 4-nitro-2-(6-azaspiro[2.5]octan-6-yl)benzoyl chloride (620 mg) as a yellow solid compound. LCMS (ESI): $[M+H]^+$ = 291.1.

**Intermediate A4: Ethyl 2-sulfamoylacetate**

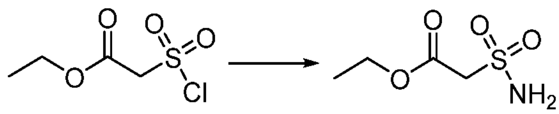

Step 1: Ethyl 2-(chlorosulfonyl)acetate (5.00 g, 26.53 mmol) was added to the solvent of dichloromethane (25 mL), and the reaction solution was cooled to 0°C.hexamethyldisilazane (4.32 g, 26.53 mmol) was slowly added dropwise to the reaction solution. The reaction solution was stirred at 25°C for 1 h. The reaction solution was concentrated to dryness to give the residue. The residue was dissolved in ethanol (20 mL) at 0°C, and The reaction solution was stirred at 25°C for 1 h. The reaction solution was concentrated to dryness to give ethyl 2-sulfamoylacetate (4.20 g, yield: 94%) as a white solid.

**Intermediate A5: 4,6-dichloro-1-methyl-1,3-dihydrofuro[3,4-c]pyridine**

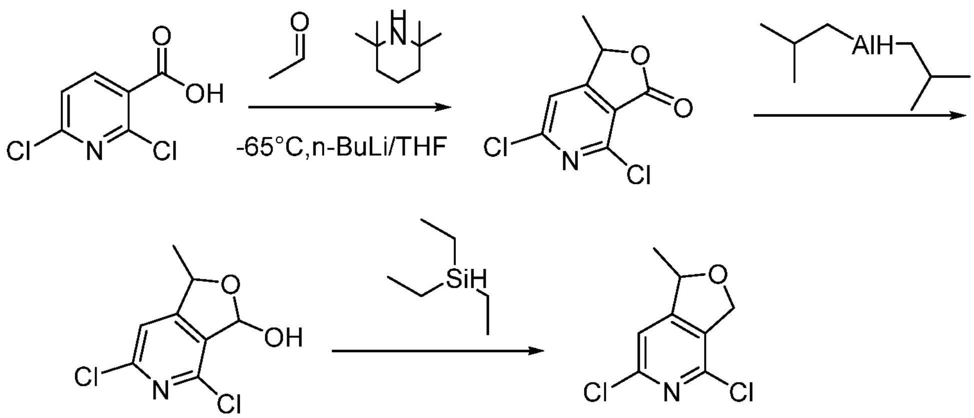


Step 1: To the solvent of THF (40 mL) was added 2,2,6,6-tetramethylpiperidine (8.91 g, 62.50 mmol) under nitrogen atmosphere and the reaction solution was stirred at -70°C for 1 h. Then, n-butyllithium solution (72 mL, 125 mmol, 2.5M n-hexane solution) was slowly added dropwise and the mixture was stirred at -70°C for 3 h. 2,6-dichloronicotinic acid (6.0 g, 31.2 mmol) in THF (40 mL) was added slowlyinto the reaction system by injecting. The solution was stirred at -70°C for 1 h, then was slowly heated to ambient temperature and stirred for 4 h. The reaction temperature was controled to -70°C again and acetaldehyde solution (13.76 g, 312.50 mmol) was slowly added dropwise, then heated to ambient temperature and stirred for 24 h. The reaction was adjusted the pH to 2 with diluted hydrochloric acid (200 mL, 6M aqueous solution). extracted with ethyl acetate (200 mL × 3), the organic phases was combined, washed once with saturated saline (100 mL), dried over anhydrous sodium sulfate, filtered, and concentrated under reduced pressure to give the crude. The crude product was slurried with methanol (50 mL), stired for 1 h, and filtered to give 4,6-dichloro-1-methylhydrofuro[3,4-c]pyridin-3-one (4.00 g, 59%) as a white solid. LCMS (ESI): $[M+H]^+$ = 218.0

Step 2: 4,6-dichloro-1-methylhydrofurano[3,4-c]pyridin-3-one (4.00 g, 18.30 mmol) was dissolved in THF (40 mL) under nitrogen atmosphere and the solution was stirred at -78°C for 1 h. Then, diisobutylaluminium hydride (46 mL, 45.87 mmol, 1.0M n-hexane solution) was slowly added dropwise. The reaction mixture was stirred at -78°C for 1.5 h and quenched by dropwise addition of saturated ammonium chloride (30 mL) at -78°C. The mixture was slowly heated to

ambient temperature and stirred for 30 min. The reaction mixture was filtered and extracted with ethyl acetate (30 mL×3). The combined organic layer was dried over sodium sulfate and concentrated to dryness in vacuo. The residue was purified by column chromatography to give 4,6-dichloro-1-methyl-1,3-dihydrofuro[3,4-c]pyridin-3-ol (2.40 g, 59%). LCMS (ESI): $[M+H]^+$ = 221.0

Step 3: To a stirred solution of 4,6-dichloro-1-methyl-1,3-dihydrofuro[3,4-c]pyridin-3-ol (2.40 g, 10.80 mmol) in dichloromethane (30 mL)were added trifluoroacetic acid (6.15 g, 54.00 mmol) and triethylsilane (3.76 g, 32.40 mmol), and the reaction mixture was stirred at 25°C for 5 h. The solvent was removed under reduced pressure, the residue was dissolved in water (50 mL), then the pH was adjusted to 8 with saturated aqueous sodium bicarbonate solution and extracted with ethyl acetate (50 mL × 3). The combined organic layer was washed with saturated aqueous sodium chloride solution (50 mL), dried over anhydrous sodium sulfate, concentrated under reduced pressure, and The crude product was purified by flash column chromatography (silica gel, 0-20% gradient of ethyl acetate/petroleum ether) to give 4,6-dichloro-1-methyl-1,3-dihydrofuro[3,4-c]pyridine (1.70 g, 77%) as a colorless oil. LCMS (ESI): $[M+H]^+$ = 204.0

**Intermediate A6: 4,6-dichloro-1-ethyl-1-methyl-1,3-dihydrofuro[3,4-c]pyridine**

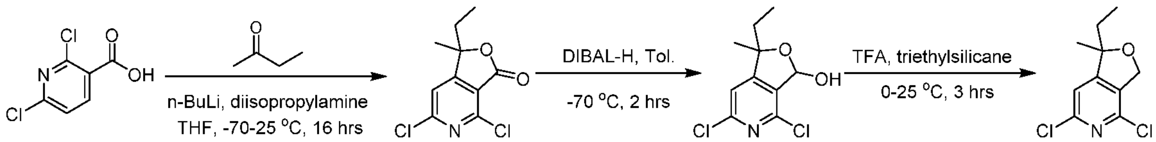


Step 1: To a stirred solution of diisopropylamine (15.8 g, 156 mmol) in THF (50 mL) was added n-butyllithium (52.1 mL, 130 mmol) dropwise at -70°C and stirred at -70°C for 40 min. The solution of 2,6-dichloronicotinic acid (10.0 g, 52.1 mmol) in THF (100.00 mL) was added dropwise to the reaction system at -70°C and stirred at -70°C for 3 h. Then, butanone (33.8 g, 468 mmol) was added dropwise to the reaction at -70°C, heated to 25°C, and stirred for 16 h. The reaction was quenched with saturated ammonium chloride solution (200 mL), diluted with water (100 mL), extracted with ethyl acetate (200 mL × 2), dried over anhydrous sodium sulfate , filtered, and concentrated under reduced pressure to afford the residue. The residue was purified by flash column chromatography (silica gel, 0-11% gradient of THF/petroleum ether) to give 4,6-dichloro-1-ethyl-1-methylfuro[3,4-c]pyridin-3(1H)-one (1.90 g, 7.29 mmol, yield: 14%) as a yellow solid. LCMS (ESI): $[M+H]^+$ = 246.0. H NMR (400 MHz, DMSO-$d_6$) δ 8.11 (s, 1H), 2.10 - 1.93 (m, 2H), 1.63 (s, 3H), 0.71 (t, *J* = 7.6 Hz, 3H)

Step 2: To a stirred solution of 4,6-dichloro-1-ethyl-1-methylfuro[3,4-c]pyridin-3(1H)-one (1.90 g, 7.72 mmol) in toluene (40 mL), was added diisobutylaluminium hydride (17.76 mL, 17.76 mmol) dropwise at -70°C, and the mixture was stirred at -70°C for 2 h. The reaction solution was quenched with water (200 mL), extracted with ethyl acetate (200 mL×2) after filtration, The combined organic layer was dried over anhydrous sodium sulfate , filtered, and concentrated under reduced pressure. The residue was purified by flash column chromatography (silica gel, 0-15% gradient of THF/petroleum ether) to give 4,6-dichloro-1-ethyl-1-methyl-1,3-dihydrofuro[3,4-c]pyridine-3-phenol (1.80 g, 7.02 mmol, yield: 91%) as a yellow oil. LCMS (ESI): $[M+H]^+$ = 247.9. $^1$H NMR (400 MHz, DMSO-$d_6$) δ ppm 7.69 (d, *J* = 2.8 Hz, 1H), 7.23 - 7.11 (m, 1H), 6.29 (dd, *J* = 6.8, 14.0 Hz, 1H), 1.81 - 1.76 (m, 2H), 1.53 - 1.38 (m, 3H), 0.86 - 0.60 (m, 3H)

Step 3: To a stirred solution of 4,6-dichloro-1-ethyl-1-methyl-1,3-dihydrofuro[3,4-c]pyridine-3-phenol (1.80 g, 7.25 mmol) in dichloromethane (36 mL), was added trifluoroacetic acid (3.52 g, 36.3 mmol) at 0°C and stirred at 0°C for 30 min, triethylsilane (2.56 g, 21.7 mmol) was added at 0°C, heated to 25°C, and stirred for 3 h. The reaction solution was poured into water (50 mL). The pH was adjusted to 7 with saturated sodium bicarbonate solution, extracted with ethyl acetate (100 mL×2), The combined organic layer was dried over anhydrous sodium sulfate , filtered, and concentrated under reduced pressure. The residue was purified by flash column chromatography (silica gel, 0-5% gradient of THF/petroleum ether) to give 4,6-dichloro-1-ethyl-1-methyl-1,3-dihydrofuro[3,4-c]pyridine (1.40 g, 5.94 mmol, yield: 82%) as a white solid. LCMS (ESI): $[M+H]^+$ = 232.0. $^1$H NMR (400 MHz, DMSO-$d_6$) δ ppm 7.64 (s, 1H), 5.09 - 4.90 (m, 2H), 1.80 (td, *J* = 7.2, 14.0 Hz, 2H), 1.42 (s, 3H), 0.73 (t, *J*= 7.6 Hz, 3H)

**Intermediate A7: 1,3-Dibromopyrrolo[1,2-a]pyrazine**

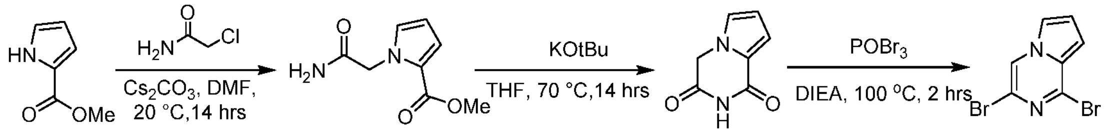

Step 1: To a stirred solution of methyl 2-pyrrolecarboxylate (37.0 g, 296 mmol) in N,N-dimethylformamide (400 mL) were added 2-chloroacetamide (33.2 g, 355 mmol) and cesium carbonate (128 g, 384 mmol) , and the mixture was stirred the mixture at 20°C for 14 h. The reaction solution was poured into ice water (500 mL) and extracted with ethyl acetate (300 mL×3). The combined organic layer was washed with saturated saline (300 mL×3), dried over anhydrous sodium sulfate, filtered, and concentrated to give 1-(2-amino-2-oxyethylene)-1H-pyrrole-2-carboxylic acid methyl ester (17.4 g crude) as a white solid. LCMS (ESI): $[M+H]^+$ = 182.9.

Step 2: To a solution of 1-(2-amino-2-oxyethylene)-1H-pyrrole-2-carboxylic acid methyl ester (17.4 g, 95.5 mmol) in THF (200 mL) was added potassium tert-butoxide (32.7 g, 288 mmol) , and the mixture was stirred at 70°C for 14 h. The pH of the reaction solution was adjusted to 6 with 2 eN hydrochloric acid and extracted with ethyl acetate (200 mL×3). The combined the organic layer was dried over anhydrous sodium sulfate, filtered, and concentrated under reduced pressure to give the residue. The residue was slurried with ethanol and filtered to give pyrrolo[1,2-a]pyrazine-1,3(2H,4H)-dione (6.50 g, 43.3 mmol, yield: 45%) as a brown solid. LCMS (ESI): $[M+H]^+$ = 151.1. $^1H$ NMR (400 MHz, DMSO-d6) δ ppm 11.31 (br s, 1H), 7.17 (s, 1H), 6.89 (m, 1H), 6.35 (m, 1H), 4.99 (s, 2H)

Step 3: To a stirred solution of pyrrolo[1,2-a]pyrazine-1,3(2H,4H)-dione (6.50 g, 43.3 mmol) in dioxane (60 mL). was added phosphorus oxybromide (24.8 g, 86.6 mmol). The reaction mixture was heated to 100°C and stirred at 100°C for 2 h. The reaction solution was concentrated, ethyl acetate was added , and the pH was adjusted to 7 with aqueous sodium bicarbonate solution. The mixture was extracted with ethyl acetate (200 mL×3), The combined organic layer was washed with saturated saline (200 mL), dried over anhydrous sodium sulfate, filtered, and concentrated under reduced pressure to give the residue. The residue was purified by flash column chromatography (silica gel, 0-5% gradient of ethyl acetate/petroleum ether) to give 1,3-dibromopyrrolo[1,2-a]pyrazine (3.19 g, 11.6 mmol, yield: 27%) as a yellow solid. LCMS (ESI): $[M+H]^+$ = 276.9.

**Intermediate A8: 6,8-Dichloro-3-methylimidazo[1,5-a]pyrazine**

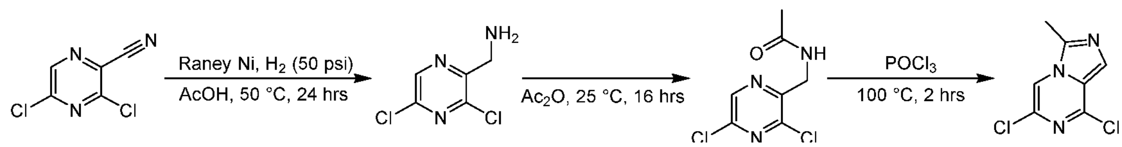


Step 1: To a stirred solution of 3,5-dichloropyrazine-2-carbonitrile (25.0 g, 144 mmol) in acetic acid (250 mL)was added Raney nickel (2.50 g). The mixture was replaced with hydrogen three times, heated to 50°C under hydrogen atmosphere (50 psi), and stired at 50°C for 24 h. The reaction solution was filtered and concentrated under reduced pressure to give (3,5-dichloropyrazin-2-yl)methanamine (12.3 g, 69.1 mmol, yield: 48%) as a green solid. LCMS (ESI): $[M+H]^+$ = 177.9.

Step 2: (3,5-dichloropyrazin-2-yl)methanamine (12.0 g, 67.4 mmol) was dissolved in acetic anhydride (60 mL) and the mixture was stirred at 25°C for 16 h. Ethyl acetate (250 mL) was added to the reaction solution and washed with saturated aqueous sodium bicarbonate solution. The combined organic layer was washed with saturated saline (250 mL), dried over anhydrous sodium sulfate, filtered, and concentrated under reduced pressure to give N-((3,5-dichloropyrazin-2-yl)methyl)acetamide (10.8 g, 49.1 mmol, yield: 73%) as a yellow solid. LCMS (ESI): $[M+H]^+$ = 219.9.

Step 3: N-((3,5-dichloropyrazin-2-yl)methyl)acetamide (10.8 g, 49.1 mmol) was added to phosphorus oxychloride (110 mL). The mixture was stirred at 100°C for 2 h. The reaction solution was added dropwise to water (500 mL), extracted with ethyl acetate (300 mL×3), and washed with saturated aqueous sodium bicarbonate solution. The combinedorganic layerwas washed with saturated saline (300 mL), dried over anhydrous sodium sulfate, filtered and concentrated under reduced pressure to give a residue. The residue was purified by flash column chromatography (silica gel, 0-100% gradient of ethyl acetate/petroleum ether) to give 6,8-dichloro-3-methylimidazo[1,5-a]pyrazine (2.7 g, 13.3 mmol, yield: 27%) as a yellow solid. LCMS (ESI): $[M+H]^+$ = 201.9.

**Intermediate A9: 6,8-Dibromo-[1,2,4]triazolo[1,5-a]pyrazine**

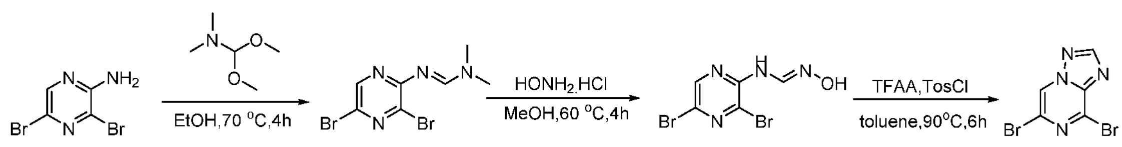

Step 1: To a stirred solution of 3,5-dibromopyrazine-2-amine (20.00 g, 79.08 mmol) in ethanol (200 mL) was added N,N-dimethylformamide dimethyl acetal (48.08 g, 395.41 mmol). The mixture was heated to 70°C under nitrogen atmosphere, reacted at 70°C for 4 h.The reaction solution was cooled to ambient temperature to precipitate a large amount of solids, filtered, and The cake was dried to give (E)-N'-(3,5-dibromopyrazine-2-yl)-N,N-dimethylformamide (23.00 g, yield: 94%). LCMS (SI): $[M+H]^+$ = 308.6

Step 2: To a stirred solution of (E)-N'-(3,5-dibromopyrazine-2-yl)-N,N-dimethylformamide (20.00 g, 64.94 mmol) in methanol (200 mL)wasadded hydroxylamine hydrochloride (6.77 g, 97.41 mmol). The mixture was heated to 60°C and reacted at 60°C for 16 h under nitrogen atmosphere. The reaction solution was cooled to ambient temperature, filtered and the cake was dried to give (E)-N-(3,5-dibromopyrazin-2-yl)-N'-hydroxyformamide (10.00 g, yield: 52%) as a bright yellow solid. LCMS (ESI): $[M+H]^+$ = 295.6.

Step 3: To a stirred solution of (E)-N-(3,5-dibromopyrazin-2-yl)-N'-hydroxyformamide (10.00 g, 33.79 mmol) in toluene (100 mL) wereadded trifluoroacetic anhydride (35.49 g, 168.97 mmol) and p-toluenesulfonyl chloride (7.23 g, 37.17 mmol). The mixture wasstirred at 90°C under nitrogen atmospherefor 16 h. The reaction solution was slowly pourede into saturated sodium bicarbonate (50 mL) and extracted with ethyl acetate (50 mL*3). The combined organic layer was washed once with saturated saline (50 mL), dried over anhydrous sodium sulfate, concentrated under reduced pressure, and purified by flash column chromatography (silica gel, 5-20% gradient of ethyl acetate/petroleum ether) to give 6,8-dibromo-[1,2,4]triazolo[1,5-a]pyrazine (5.8 g, yield: 62%) as a yellow solid. LCMS (ESI): $[M+H]^+$ =444.2

**Intermediate A10: 6-Bromo-8-chloro-[1,2,4]triazolo[4,3-a]pyrazine**

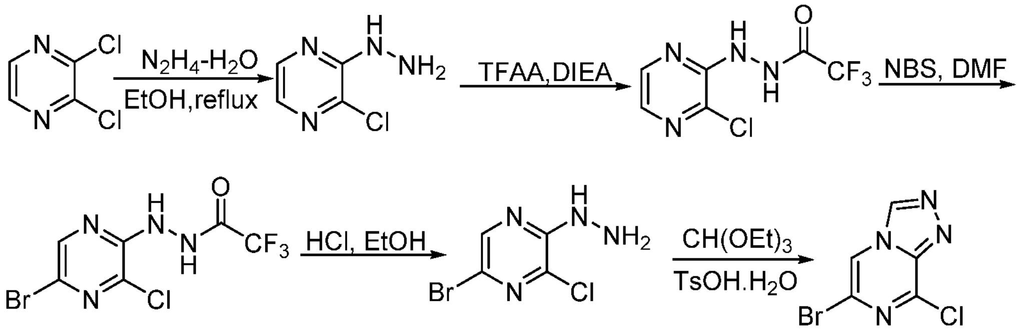


Step 1: To a stirred solution of 2,3-dichloropyrazine (25.00 g, 167.81 mmol) in ethanol (50 mL)was added hydrazine hydrate (80%, 21.53 g, 344.00 mmol). The reaction solutionwas stirrd at 85°C for 2 h. The reaction solution was concentrated , filtered, washed the solid with water (50 mL×3), and dried to give 2-chloro-3-hydrazinopyrazine (19.00 g, 78.32%) as an orange solid. LCMS (ESI): $[M+H]^+$ =145.1, 147.1.

Step 2: To a stirred solution of 2-chloro-3-hydrazinopyrazine (19.00 g, 131.43 mmol) in dichloromethane (50 mL) was added N,N-diisopropylethylamine (34.67 g, 262.87 mmol). The reaction solution was cooled to 0°C. Trifluoroacetic anhydride (30.67 g, 144.58 mmol) was added dropwise to the reaction solution, and the reaction solution was stirred at 25°C for 1 h. The reaction solution was poured into water (100 mL), extracted with ethyl acetate (50 mL × 3), The combined organic layer was washed with the saturated aqueous sodium chloride solution (50 mL × 3), dried over anhydrous sodium sulfate, and concentrated to obtain the crude product. The crude product was purified by flash column chromatography (silica gel, 0-25% gradient of ethyl acetate/petroleum ether) to give N'-(3-chloropyrazin-2-yl)-2,2,2-trifluoroacetohydrazide (14.00 g, 44.28%) as a pale yellow solid. LCMS (ESI): $[M+H]^+$ =241.0

Step 3: N'-(3-chloropyrazin-2-yl)-2,2,2-trifluoroacetohydrazide (14.00 g, 58.20 mmol) was added to N,N-dimethylformamide (45 mL), and The reaction solution was cooled to 0°C. N-bromosuccinimide (11.39 g, 64.02 mmol) was slowly added to the reaction solution in 3 portions, and the reaction solution was stirred at 25°C for 4 h. The reaction solution was quenched slowly with water (50 mL), extracted with ethyl acetate (50 mL × 3), The combined organic layer was washed with the saturated aqueous sodium chloride solution (50 mL × 3), dried over anhydrous sodium sulfate, and concentrated to obtain the crude product. The crude product was purified by flash column chromatography (silica gel, 0-25% gradient of ethyl acetate/petroleum ether) to give N'-(5-bromo-3-chloropyrazin-2-yl)-2,2,2-trifluoroacetohydrazide (5.90 g, 31.73%) as a white solid. LCMS (ESI): $[M+H]^+$ = 321.0.

Step 4: To a stirred solution of N'-(5-bromo-3-chloropyrazin-2-yl)-2,2,2-trifluoroacetohydrazide (5.90 g, 18.47 mmol) in ethanol (50 mL)was added concentrated hydrochloric acid (12 M, 10 mL) and the reaction solution was stirred at 80°C for 4 h. The reaction solution was concentrated to give the crude solution, which pH was neutralized to 7 with saturated aqueous sodium bicarbonate solution, extracted with ethyl acetate (50 mL×3), the combined organic layer was

dried over anhydrous sodium sulfate, and concentrated to give 5-bromo-3-chloro-2-hydrazinopyrazine (3.50 g, 85%) as a brown solid. LCMS (ESI): $[M+H]^+$ = 225.0.

Step 5: To a stirred solution of 5-bromo-3-chloro-2-hydrazinopyrazine (3.50 g, 15.73 mmol) in triethyl orthoformate (10 mL)was added p-toluenesulfonic acid monohydrate (138 mg, 0.79 mmol) and the reaction solution was stirredat 130°C for 4 h.The reactionsolution was poured into water (50 mL), extracted with ethyl acetate (50 mL×3), the combined organic layer was dried over anhydrous sodium sulfate and concentrated to give 6-bromo-8-chloro-[1,2,4] triazolo[4,3-a]pyrazine (2.10 g, 57.17%) as a brown solid. LCMS (ESI): $[M+H]^+$ =234.9

**Intermediate A11: 5,7-Dichloroimidazol[1,2-c]pyrimidine**

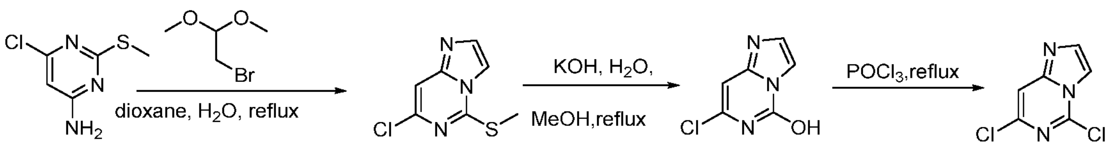


Step 1: To a stirred solution of 6-chloro-2-(methylthio)pyrimidin-4-amine (5.00 g, 28.47 mmol), in dioxane/-water=4/1 (50 mL) was added 2-bromo-1,1-dimethoxyethane (5.77 g, 34.16 mmol). The mixture was stirred at 100°C for 12 h. The reaction solution was poured into water (100 mL), and extracted with ethyl acetate (50 mL×3). The combined organic layer was dried over anhydrous sodium sulfate, filtered, and concentrated to give 7-chloro-5-(methylthio)imidazo [1,2-c]pyrimidine (7.90 g) as a brown solid compound. LCMS (ESI): $[M+H]^+$ = 200.0.

Step 2: To a stirred solution of 7-chloro-5-(methylthio)imidazo[1,2-c]pyrimidine (7.90 g, 39.57 mmol) in methanol (20 mL) was added aqueous potassium hydroxide solution (60 mL, 2 M). The mixture was stirred at 100°C for 12 h. The reaction solution was extracted with ethyl acetate (50 mL×3), The combined organic layer was dried over anhydrous sodium sulfate, filtered, and concentrated to give 7-chloroimidazo[1,2-c]pyrimidin-5-ol (3.50 g) as a yellow solid compound. LCMS (ESI): $[M+H]^+$ = 170.0.

Step 3: 7-chloroimidazo[1,2-c]pyrimidin-5-ol (3.40 g, 20.05 mmol)was added to phosphorus oxychloride (20 mL). The mixture was stirred at 90°C for 2 h. The reaction solution was concentrated, and the pH was adjusted to about 8 by adding saturated aqueous sodium bicarbonate solution (100 mL). Then, extracted with ethyl acetate (30 mL × 3). the combined organic layer was dried over anhydrous sodium sulfate, filtered, and concentrated to give 5,7-dichloroimidazol [1,2-c]pyrimidine (2.80 g) as a brown solid compound. LCMS (ESI): $[M+H]^+$ = 188.0.

**Intermediate A12: 5,7-Dichloro-[1,2,4]triazolo[4,3-c]pyrimidine**

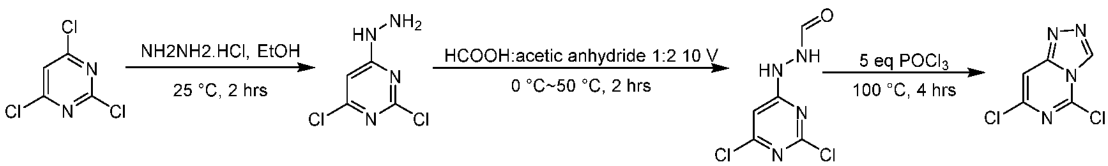


Step 1: To a stirred solution of 2,4,6-trichloropyrimidine (35.0 g, 191 mmol) and triethylamine (57.9 g, 572 mmol) in ethanol (700 mL) was added hydrazine monohydrochloride (12.0 mL, 28.5 mmol) , and the mixture was stirred at 25°C for 2 h. The reaction solution was concentrated under reduced pressure and was slurried with ethanol (200 mL) to give 2,4-dichloro-6-hydrazinopyrimidine (60.0 g, 175 mmol, yield: 86%) as a yellow solid. LCMS (ESI): $[M+H]^+$ = 179.1

Step 2: To the solvent of acetic anhydride (200 mL) was added formic acid (100 mL) dropwise at 0°C. The mixture was stirred at 0°C for 10 min, and then heated to 50°C, and stirred at 50°C for 15 min. Then, the solution was cooled to 0°C and 2,4-dichloro-6-hydrazinopyrimidine (55.0 g, 307 mmol) was added to the mixture solution, which was stirred 0-25°C for another 2 h. The reaction solution was diluted with water (500 mL) and extracted with ethyl acetate (800 mL × 3). The combined organic layer was dried over anhydrous magnesium sulfate, filtered, and concentrated to give the residue. The residue was slurried with methyl tert-butyl ether (200 mL) to give N-[(2,6-dichloropyrimidin-4-yl)amino]formamide (22.0 g, 106 mmol, yield: 35%) as a pink solid. LCMS (ESI): $[M+H]^+$ = 207.1

Step 3: N'-(2,6-dichloropyrimidin-4-yl)formylhydrazine (22.0 g, 106 mmol) was added to the solvent of phosphorus oxychloride (180 mL, 1937 mmol) under nitrogen atmosphere. The mixture was heated to 100°C, and stirred for 4 h. Most of the phosphorus oxychloride was removed, The remainder solution was poured slowly into water (500 mL) . The

solution was extracted with dichloromethane (500×3 mL) and the combined organic layer was washed with saturated sodium chloride (500 mL) and dried over anhydrous sodium sulfate, filtered, and concentrated to give the residue. The residue was purified by flash column chromatography (silica gel, 0-50% gradient of ethyl acetate/petroleum ether) to give 5,7-dichloro-[1,2,4]triazolo[4,3-c]pyrimidine (1.04 g, 5.3 mmol, yield: 5%) as a pale brown solid. LCMS (ESI): $[M+H]^+$ = 189.1.

**Intermediate A13: 5,7-dichloro-3-methyl-[1,2,4]triazolo[4,3-c]pyrimidine**

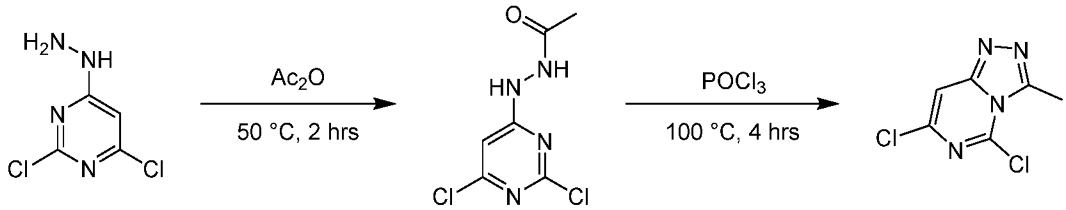


Step 1: A2,4-dichloro-6-hydrazinopyrimidine (30.0 g, 168 mmol) was added to the solvent of acetic anhydride (30 mL). The mixture was heated to 50°C, and stirred at 50°C for 1 h. water (500 mL) was added to the reaction mixture, and extracted with ethyl acetate (200 mL × 3). The combined organic layer was dried over anhydrous magnesium sulfate, filtered, and concentrated under reduced pressure to give the residue. The residue was sluuried with methyl tert-butyl ether (200 mL) and filtered to give N'-(2,6-dichloropyrimidin-4-yl)acetylhydrazine (6.00 g, 27.1 mmol, yield: 16%) as a yellow solid. LCMS (ESI): $[M+H]^+$ = 221.0

Step 2: N'-(2,6-dichloropyrimidin-4-yl)acetylhydrazine (6.00 g, 27.1 mmol) was slowly added to the solvent of phosphorus oxychloride (60 mL), and the reaction mixture was stirred at 100°C for 4 h. The reaction mixture was quenched with water (200 mL) at 25°C and extracted with ethyl acetate (200 mL×2). The combined organic layer was dried over anhydrous magnesium sulfate, filtered, and concentrated to give the residue. The residue was purified by flash column chromatography (silica gel, 0-25% gradient of ethyl acetate/petroleum ether) to give 5,7-dichloro-3-methyl-[1,2,4]triazolo [4,3-c]pyrimidine (2.00 g, 9.85 mmol, yield: 36%) as a yellow solid. LCMS (ESI): $[M+H]^+$ = 203.0. $^1H$ NMR (400 MHz, DMSO-$d_6$) δ ppm 8.11 (s, 1H), 2.55 (s, 3H)

**Intermediate A14: 5,7-Dibromothieno[2,3-c]pyridine**

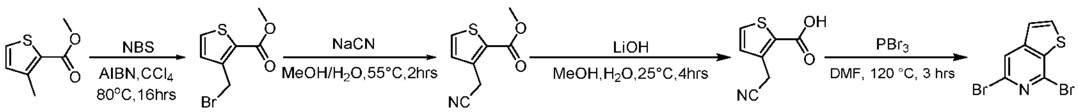


Step 1: To a solution of methyl 3-methylthiophene-2-carboxylate (20.0 g, 128 mmol) in carbon tetrachloride (400 mL) were added 2,2'-azobis(2-methylpropanonitrile) (6.31 g, 38.4 mmol) and N-bromosuccinimide (27.3 g, 153 mmol) . The mixture was heated to 80°C, stirrd for at 80°C 16 h. water (200 mL) was added to the reaction mixture, and extracted with ethyl acetate (200 mL×2). The combined organic layer was dried over anhydrous magnesium sulfate, filtered and concentrated under reduced pressure to give methyl 3-(bromomethyl)thiophene-2-carboxylate (43.0 g, 128 mmol, crude) as a yellow oil

Step 2: To the stirred solution of methyl 3-(bromomethyl)thiophene-2-carboxylate (43.0 g, 182 mmol) in methanol (350 mL) was added the solution of sodium cyanide (11.6 g, 237 mmol) in water (170 mL) under an ice bath. The reaction mixture was heated to 55°C, and stirred at 55°C for 2 h. Water (100 mL) was added to the reaction solution and extracted with dichloromethane (300 mL×3). The combined organic layer was dried over anhydrous magnesium sulfate, filtered and concentrated under reduced pressure to give the residue. The residue was purified by flash column chromatography (silica gel, 0-4% gradient of ethyl acetate/petroleum ether) to give methyl 3-(cyanomethyl)thiophene-2-carboxylate (8.5 g, 182 mmol, yield: 26%) as a white solid. LCMS (ESI): $[M+H]^+$ = 182.0

Step 3: To a stirred solution of methyl 3-(cyanomethyl)thiophene-2-carboxylate (8.50 g, 46.9 mmol) in methanol (45.0 mL) and water (45.0 mL) was added lithium hydroxide monohydrate (5.96 g, 140 mmol). The mixture was stirred at 25°C for 4 h. The reaction mixture was concentrated under reduced pressure to give 3-(cyanomethyl)thiophene-2-carboxylic acid (6.00 g, 46.9 mmol, crude) as a white solid. LCMS (ESI): $[M+H]^+$ = 168.0

Step 4: To a solution of bromophos (20.0 mL) in N,N-dimethylformamide (4.00 mL) was added 3-(cyanomethyl)thiophene-2-carboxylic acid (2.70 g, 16.17 mmol) at 0°C. The reaction mixture was heated to 120°C, and stirred at 120°C for 3 h. The reaction mixture was cooled to ambient temperature with an ice-water bath. The reaction mixture was poured into ice water and the precipitate was collected by filtration to give 5,7-dibromothieno[2,3-c]pyridine (2.1 g, crude) as a brown solid. LCMS (ESI): $[M+H]^+$ = 293.7

**Intermediate A15: 5,7-dibromofuro[2,3-c]pyridine**

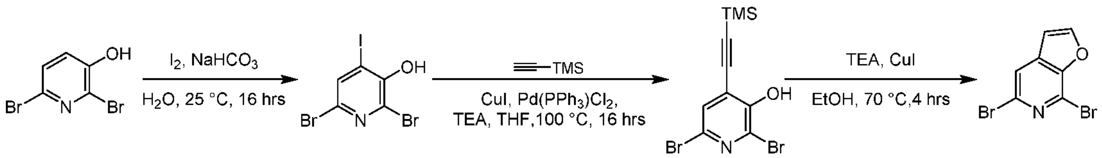


Step 1: To a solution of 6-dibromopyridin-3-ol (10.0 g, 39.5 mmol) and sodium bicarbonate (11.7 g, 138 mmol) in water (200 mL) was added iodine (17.2 g, 67.2 mmol). The mixture was stirred at 25°C for 16 h. The reaction solution was diluted with saturated sodium thiosulfate solution (200 mL) and the pH was adjusted to 2 with diluted hydrochloric acid. The solution was extracted with dichloromethane (900 mL*2). The combined organic layer was washed with saturated saline (1000 mL), dried over sodium sulfate, filtered, and concentrated under reduced pressure to give the residue. The residue was purified by flash column chromatography (silica gel, 0-0% gradient of ethyl acetate/petroleum ether) to give 2,6-dibromo-4-iodopyridin-3-phenol (12.0 g, 39.5 mmol, yield: 76%) as a white solid. LCMS (ESI): $[M+H]^+$ = 379.7.

Step 2: To a solution of 2,6-dibromo-4-iodopyridin-3-phenol (6.00 g, 15.8 mmol), ethynyltrimethylsilane (2.33 g, 23.7 mmol), cuprous iodide (0.31 g, 1.58 mmol) and triethylamine (4.91 g, 47.5 mmol) in chloroform (2.00 mL) and THF (120 mL) was added bis(triphenylphosphine)palladium(II) dichloride (1.12 g, 1.58 mmol) . The mixture was replaced with nitrogen 3 times and the mixture was heated to 100°C and stirred at 100°C for 16 h. water (100 mL) was addedto the reaction solution and extracted with ethyl acetate (300 mL × 2). The combined organic layer was dried over anhydrous sodium sulfate, filtered and concentrated under reduced pressure to give the residue. The residue was purified by flash column chromatography (silica gel, 0-1% gradient of ethyl acetate/petroleum ether) to give 2,6-dibromo-4-((trimethylsilyl)ethynyl)pyridine-3-phenol (1.0 g, 15.8 mmol, yield: 18.1%) as a white solid. LCMS (ESI): $[M+H]^+$ = 349.8.

Step 3: To the solvent of triethylamine (10.0 mL)/ethanol (10.0 mL) were added 2,6-dibromo-4-((trimethylsilyl)ethynyl)pyridine-3-phenol (1.20 g, 3.44 mmol) and cuprous iodide (0.33 g, 1.72 mmol). The mixture was replaced with nitrogen 3 times. The mixture was heated to 70°C under nitrogen atmosphere, and stirred for 4 h. water (20 mL) was added to the reaction solution and extracted with dichloromethane (60 mL × 2). The combined organic layer was dried over anhydrous sodium sulfate, filtered and concentrated under reduced pressure to give the residue. The residue was purified by flash column chromatography (silica gel, 0-1% gradient of ethyl acetate/petroleum ether) to give 5,7-dibromofuro[2,3-c]pyridine (0.40 g, 3.44 mmol, yield: 42%) as a yellow solid. LCMS (ESI): $[M+H]^+$ = 277.8

**Intermediate A16: 4,6-Dichlorofuro[3,2-c]pyridine**

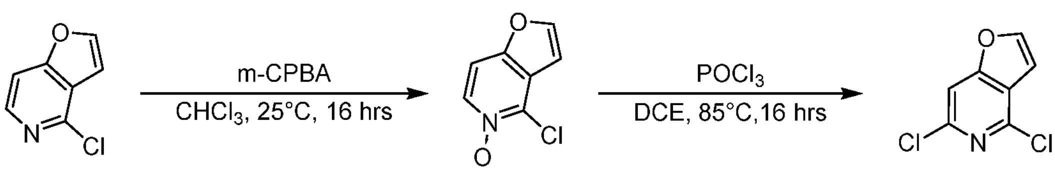


Step 1: To a stirred solution of 4-chlorofuro[3,2-c]pyridine (5.00 g, 32.6 mmol) in chloroform (100 mL) was added meta-chloroperoxybenzoic acid (9.92 g, 48.8 mmol). The mixture was stirred at 25°C for 16 h. Water (100 mL) was added to the reaction solution and extracted with ethyl acetate (100 mL × 3). The combined organic layer was dried over anhydrous sodium sulfate, filtered and concentrated under reduced pressure to give the residue. The residue was purified by flash column chromatography (silica gel, 0-2% gradient of methanol/dichloromethane) to give 4-chlorofuro[3,2-c]pyridine 5-oxide (3.00 g, 32.5 mmol, yield: 54%) as a brown solid. LCMS (ESI): $[M+H]^+$ =170.0

Step 2: To a stirred solvent of 1,2-dichloroethane (30.0 mL) and phosphorus oxychloride (3.00 mL) was slowly added 4-chlorofuro[3,2-c]pyridine 5-oxide (3.00 g, 17.7 mmol) . The mixture was heated to 85°C and stirrd for 16 h. water (20 mL) was added to the reaction solution and extracted with dichloromethane (20 mL × 3). The combined organic layer was dried over anhydrous sodium sulfate, filtered and concentrated under reduced pressure to give the residue. The

residue was purified by flash column chromatography (silica gel, 0-35% gradient of THF/petroleum ether) to give 4,6-dichlorofuro[3,2-c]pyridine (1.00 g, 17.7 mmol, yield: 30%) as a yellow solid. LCMS (ESI): $[M+H]^+$ =188.0

**Intermediate A17: 4,6-Dichloro-[1,2,5]thiadiazolo[3,4-c]pyridine**

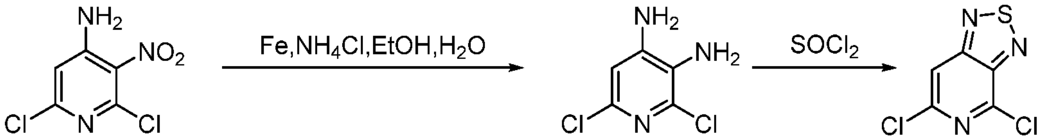


Step 1: To a stirred solution of iron powder (27.1 g, 480.76 mmol), ammonium chloride (26.0 g, 480.76 mmol) in ethanol (100 mL) and water (20 mL) was added 4-amino-2,6-dichloro-3-nitropyridine (10 g, 48.08 mmol).The mixture was stirredat 60°C for 2 h. The reaction solution was filtered and concentrated, then extracted with ethyl acetate (100 mL × 3). The combined organic layer was dried over anhydrous sodium sulfate, filtered, and concentrated to give 2,6-dichloropyridine-3,4-diamine (8 g, 44.94 mmol, yield: 93%) as a pale yellow solid compound. LCMS (ESI): $[M+H]^+$ = 178.0.

Step 2: 2,6-dichloropyridine-3,4-diamine (8 g, 44.94 mmol) was added to the solvent of thionyl chloride (80 mL). The mixture was stirred at 90°C for 12 h. The reaction solution was concentrated, and saturated aqueous sodium bicarbonate solution (200 mL) was added to the solution, which pH was adjusted to be close to 8. Then, extracted with ethyl acetate (100 mL × 3), and the combined organic layer was dried over anhydrous sodium sulfate, filtered and concentrated to give the residue which was purified by column chromatography (silica gel, 0-100% gradient of ethyl acetate/petroleum ether) to give 4,6-dichloro-[1,2,5]thiadiazolo[3,4-c]pyridine (2.1 g, 10.19 mmol, yield: 23%) as a yellow solid compound. LCMS (ESI): $[M+H]^+$ = 223.1.

**Intermediate A18: 6-Bromo-4-fluorobenzo[c]1,2,5-thiadiazole**

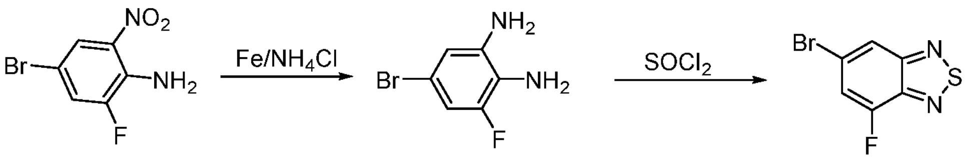


Step 1: To a stirred solution of 4-bromo-2-fluoro-6-nitroaniline (5.00 g, 21.30 mmol) in THF (50 mL)ethanol (10 mL), and water (5 mL), were added iron powder (6.00 g, 106.30 mmol) and ammonium chloride (1.72 g, 31.90 mmol), and the mixture was stirred at 90°C for 22 h. The reaction solution was cooled to ambient temperature, filtered and the solid was washed with ethanol until the washings passing through the filter are colorless. The filtrate was concentrated, and the residue was dissolved in ethyl acetate (50.00 mL), and extracted 3 times. the combined organic layer was washed once with saturated saline (10 mL), dried over anhydrous sodium sulfate, filtered, and concentrated under reduced pressure to give 5-bromo-3-fluorophenylenediamine (3.10 g, 71%) as a brown solid. LCMS (ESI): $[M+H]^+$ = 205.0

Step 2: To a stirred solution of 5-bromo-3-fluorophenylenediamine (3.00 g, 14.50 mmol) in dichloromethane (30.00 mL)were added triethylamine (5.89 g, 58.00 mmol) and thionyl chloride (2.81 g, 31.9 mmol). The mixture was refluxed at 60°C for 12 h. The mixture was concentrated, and he residue was dissolved in water (20.00 mL), and extracted with ethyl acetate (30.00 mL) 3 times. The combined organic layer was washed once with saturated saline (30 mL), dried over anhydrous sodium sulfate, filtered, concentrated under reduced pressure, and the crude was purified by flash column chromatography (silica gel, 0-50% ethyl acetate/petroleum ether) to give 6-bromo-4-fluorobenzo[c]1,2,5-thiadiazole (3.10 g, 91%). $^1H$ NMR (400 MHz, DMSO) δ 8.34 (d, *J* = 1.6 Hz, 1H), 7.89 - 7.86 (m, 1H)

**Intermediate A19: 6-Bromo-4-iodo-1,3-benzothiazole-2-amine**

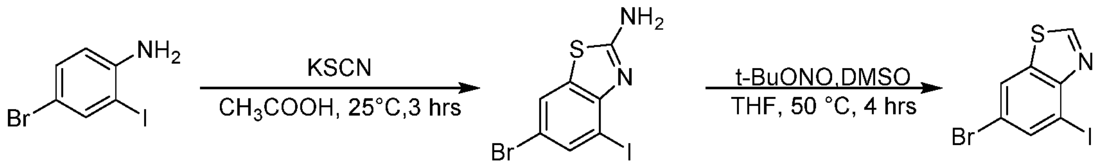


Step 1: To a solution of 4-bromo-2-iodoaniline (10.0 g, 33.6 mmol) in acetic acid (100 mL) was added potassium thiocyanate (16.3 g, 168 mmol) at 25°C. The mixture was stirred at 20°C for 10 min, liquid bromine (5.36 g, 33.57 mmol) was added dropwise and the reaction solution was stirred at 25°C for 3 h.The reaction solution was filtered, and the cake was washed with acetic acid (30 mL), then the cake was dissolved in hot water (100 mL), and the pH was adjusted to 11 with liquor ammonia. The resulting precipitate was filtered, washed with water, and dried to give 6-bromo-4-iodo-1,3-benzothiazole-2-amine (3.30 g, 9.41 mmol, yield: 28%) as a yellow solid product. LCMS (ESI): $[M+H]^+$ = 356.6

Step 2: To a stirred solution of 6-bromo-4-iodo-1,3-benzothiazole-2-amine (1.20 g, 3.38 mmol) in THF (20 mL), was added tert-butyl nitrite (0.42 g, 4.06 mmol) and DMSO (2.00 mL) dropwise at 25°C , and the reaction solution was stirred at 50°C for 4 h. water (50 mL) was added to the reaction solution and extracted with ethyl acetate (80 mL × 3). The combined organic layer was dried over anhydrous magnesium sulfate, filtered and concentrated under reduced pressure to give the residue. The residue was purified by flash column chromatography (silica gel, 0-6% gradient of THF/petroleum ether) to give 6-bromo-4-iodo-1,3-benzothiazole-2-amine (0.69 g, 2.03 mmol, yield: 60%) as a brown solid product. LCMS (ESI): $[M+H]^+$ = 339.6

**Intermediate A20: 6,8-Dichloro-2-[(4-methoxyphenyl)methyl]-2,3,4-trihydropyrido[3,4-c]pyridin-1-one**

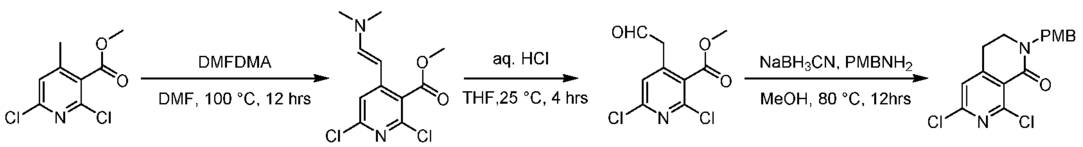


Step 1: To a solution of methyl 2,6-dichloro-4-methylpyridine-3-carboxylate (3.00 g, 13.6 mmol) in N,N-diisopropylethylamine (30 mL) was added N,N-dimethylformamide dimethyl acetal (4.97 g, 40.9 mmol) at 25°C, and the mixture was stirred at 100°C for 12 h. The reaction solution was slowly added to water (60 mL) dropwise, the yellow precipitate was separated, then precipitate was collected, and dried with an oil pump to give methyl 4-[(1E)-2-(dimethylamino)vinyl]-2,6-dichloropyridine-3-carboxylate (3.60 g, 39.3 mmol, yield: 96%) as a yellow solid crude.

Step 2: To a stirred solution of methyl 4-[(1E)-2-(dimethylamino)vinyl]-2,6-dichloropyridine-3-carboxylate (2.10 g, 7.63 mmol) in THF (30 mL), was added concentrated hydrochloric acid (18 mL) dropwise at 25°C, and the reaction solution was stirred at 25°C for 4 h. saline (100 mL) was added to the reaction solution and extracted with ethyl acetate (100 mL × 3). The combined organic layer was dried over anhydrous magnesium sulfate, filtered and concentrated under reduced pressure to give methyl 2,6-dichloro-4-(2-oxoethylidene)pyridine-3-carboxylate (1.80 g, 7.25 mmol, yield: 95%) as a yellow oil crude. $^1$H NMR (400 MHz, CHLOROFORM-d) δ ppm 9.65 (s, 1H), 7.18 - 7.10 (m, 1H), 3.88 (s, 3H), 3.76 (s, 2H)

Step 3: To a stirred solution of methyl 2,6-dichloro-4-(2-oxoethylidene)pyridine-3-carboxylate (1.8 g, 7.26 mmol) in isopropanol (18 mL) and methanol (18 mL) was added 4-methoxybenzylamine (1.01 g, 7.26 mmol) at 25°C, and the mixture was stirred at 25°C for 2 h. Then, acetic acid (1.31 g, 21.8 mmol) and sodium cyanoborohydride (1.35 g, 21.8 mmol) was added to the reaction solution, the reaction solution was heated to 80°C, and stirred for 12 h. saturated sodium bicarbonate solution (100 mL) was slowly poured into the reaction solution in portions to adjust the solution pH to 7-8, then ethyl acetate (150×2 mL) was added. The combined organic layer was washed with saturated sodium chloride (100 mL), dried over anhydrous magnesium sulfate, filtered and concentrated under reduced pressure to give the residue. The residue was purified by flash column chromatography (silica gel, 0-50% gradient of ethyl acetate/petroleum ether) to give 6,8-dichloro-2-[(4-methoxyphenyl)methyl]-2,3,4-trihydropyrido[3,4-c]pyridin-1-one (1.60 g, 4.72 mmol, yield: 65%) as a white solid. LCMS (ESI): $[M+H]^+$ = 336.6

**Intermediate A21: 6-Chloro-8-(4,4-difluoropiperidin-1-yl)pyrido[3,4-d]pyrimidine**

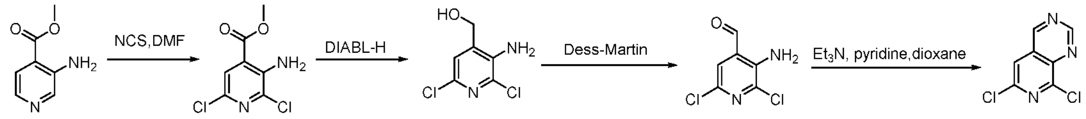


Step 1: To a stirred solution of methyl 3-aminopyridine-4-carboxylate (10 g, 65.73 mmol) in N,N-dimethylformamide (100 mL) was added N-chlorosuccinimide (19.3 g, 144.59 mmol) in portions . The reaction solution was stirred at 20°C for 12 h, pouredinto water (500 mL), extracted with ethyl acetate (100 mL×3), and the combined organic layer was dried over anhydrous sodium sulfate, filtered and concentrated to give the residue, which was purified by column chromatography (silica gel, 0-100% gradient of ethyl acetate/petroleum ether) to give methyl 3-amino-2,6-dichloroisonicotinate (11 g, 49.76 mmol, yield: 75.7%) as a white solid compound. LCMS (ESI): $[M+H]^+$ = 221.0.

Step 2: To a solution of 3-amino-2,6-dichloropyridine-4-carboxylic acid methyl ester (10 g, 45.24 mmol) in THF (200 mL) was added diisobutylaluminium hydride (113 mL, 113 mmol) dropwise at -78°C under nitrogen atmosphere. Then the reaction was heated to 20°C, and stirred at 20°C for 12 h. The reaction solution was quenched by adding sodium sulfate decahydrate, then, filtered and concentrated to give (3-amino-2,6-dichloropyridin-4-yl)methanol (6 g, 31.08 mmol, yield: 68.7%) as a white solid compound. LCMS (ESI): $[M+H]^+$ = 193.0.

Step 3: To a stirred solution of (3-amino-2,6-dichloro-4-pyridinyl)methane-1-ol (6 g, 31.08 mmol) in dichloromethane (60 mL) and THF (60 mL) was added Dess-Martin periodinane (20 g, 46.63 mmol) in portions . The reaction solution was stirred at 20°C for 12 h, and aqueous sodium bicarbonate solution (500 mL) was added to the reaction system to quench the reaction solution, which was then extracted with ethyl acetate (100 mL×3). The combined organic layer was dried over anhydrous sodium sulfate, filtered and concentrated to give the residue, which was purified by column chromatography (silica gel, 0-100% gradient of ethyl acetate/petroleum ether) to give the compound 3-amino-2,6-dichloroisonicotinaldehyde (2 g, 10.47 mmol, yield: 33.7%). LCMS (ESI): $[M+H]^+$ = 228.3.

Step 4: To a stirred solution of 3-amino-2,6-dichloropyridine-4-carbaldehyde (1.5 g, 7.85 mmol) and formamidine acetate (1.24 g, 11.78 mmol), in pyridine (3.3 g, 39.26 mmol) and tert-butanol (70 mL) were added 4,4-difluoropiperidine hydrochloride (6.25 g, 39.26 mmol) and triethylamine (8.11 g, 78.53 mmol). The mixture solution was stirred at 90°C for 12 h. the reaction solution was poured into water (50 mL), extracted with ethyl acetate (50 mL×3), and the combined organic layer was dried over anhydrous sodium sulfate, filtered and concentrated to give the residue, which was further purified by column chromatography (silica gel, 0-100% gradient of ethyl acetate/petroleum ether) to give 6-chloro-8-(4,4-difluoropiperidin-1-yl)pyrido[3,4-d]pyrimidine (700 mg, 2.46 mmol, yield: 31.3%) as a yellow solid compound. LCMS (ESI): $[M+H]^+$ = 285.10

**Intermediate A22: 6-Bromo-8-iodo-2-methoxyquinoline**

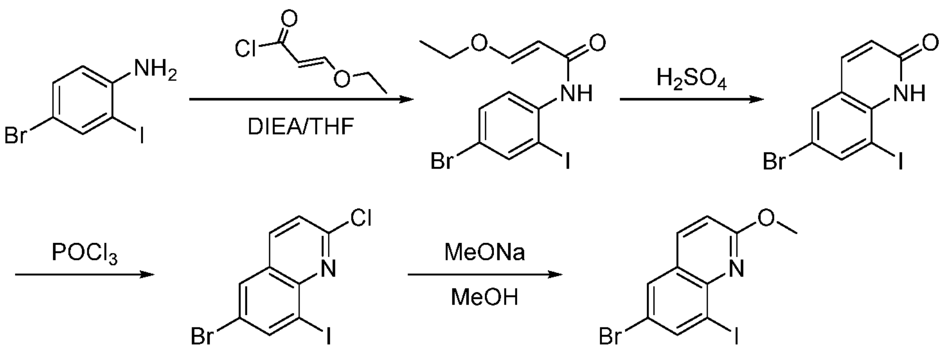


Step 1: To a stirred solution of 4-bromo-2-iodoaniline (25.00 g, 84.17 mmol) in THF (250 mL),were added (E)-3-ethoxyacryloyl chloride (22.70 g, 168.34 mmol) and N,N-diisopropylethylamine (43.70 g, 336.68 mmol), and the mixture was stirred at 25°C for 12 h. The reaction solution was slowly poured into water (200 mL), then extracted with ethyl acetate (200 mL × 3), The combined organic layer was washed once with saturated saline (100 mL), dried over anhydrous sodium sulfate, filtered and concentrated under reduced pressure to give a crude. The crude was slurried with methanol (50 mL) stirred for 1 h, and filtered to give (E)-N-4-bromo-2-iodophenyl-3-ethoxyacrylamide (17.00 g, 51%) as a white solid crude. LCMS (ESI): $[M+H]^+$ = 396.0.

Step 2: (E)-N-4-bromo-2-iodophenyl-3-ethoxyacrylamide (17.00 g, 42.90 mmol) was added to the solvent of concentrated sulfuric acid (34 mL) in portions. The reaction mixture was stirred at 25°C for 3 h. The reaction solution was slowly added to ice water (100 mL) and stired for 1 h until the precipitation of white solids. The mixture was filtered, and the

white solid was collected , and dried to give 6-bromo-8-iodoquinolin-2(1H)one (11.00 g, 73%) as a white solid. LCMS (ESI): $[M+H]^+$ = 350.0

Step 3: 6-bromo-8-iodoquinolin-2(1H)one (11.00 g, 31.40 mmol) was dissolved in phosphorus oxychloride (50 mL), and the reaction mixture was stirred at 100°C for 5 h. phosphorus oxychloride was removed under reduced pressure, the residue was dissolved in water (50 mL), then the pH of the aqueous layer was adjusted to 8 with saturated aqueous sodium bicarbonate solution (40 mL) and extracted with ethyl acetate (50 mL × 3).The combined organic layer was washed with saturated aqueous sodium chloride solution (50 mL), dried over anhydrous sodium sulfate, concentrated under reduced pressure, and the crude was purifed by flash column chromatography (silica gel, 0-50% gradient of ethyl acetate/petroleum ether) to give 6-bromo-2-chloro-8-iodoquinoline (8.00 g. 69%). LCMS (ESI): $[M+H]^+$ = 367.9

Step 4: To a stirred solution of 6-bromo-2-chloro-8-iodoquinoline (5.00 g, 13.50 mmol) in methanol (50 mL) was added sodium methoxide (5.10 g, 9.50 mmol) and the reactionmixture was stirred at 70°C for 8 h. The solvent was removed under reduced pressure, the residue was dissolved in water (100 mL), and extracted with ethyl acetate (50 mL×3). The combined organic layer was washed with saturated aqueous sodium chloride solution (50 mL), and dried over anhydrous sodium sulfate to give 6-bromo-8-iodo-2-methoxyquinoline (4.00 g, 80%) as a white solid. LCMS (ESI): $[M+H]^+$ = 364.0

**Intermediate A24: 6-Bromo-8-iodoquinoline-2-nitrile**

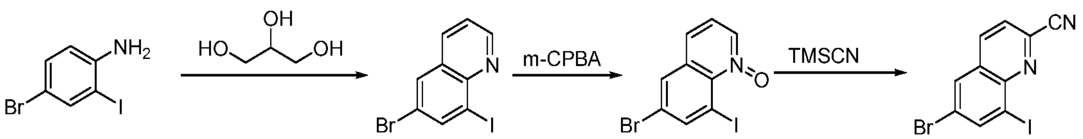


Step 1: , To a solution of 4-bromo-2-iodoaniline (5.00 g, 16.62 mmol) and glycerol (7.73 g, 83.08 mmol) in concentrated sulfuric acid (30 mL) and water (20 mL) was added sodium 3-nitrobenzenesulfonate (4.91 g, 21.60 mmol) . The reaction solution was stirred at 120°C for 16 h. The reaction solution was poured into water (100 mL), and the pH was adjusted to 10 with 2 M aqueous sodium hydroxide solution, the resulting solution was filtered, the solid was washed with water (50 mL×3), and dried to give 6-bromo-8-iodoquinoline (3.00 g, crude) as a pale yellow solid. LCMS (ESI): $[M+H]^+$ =333.9

Step 2: 6-bromo-8-iodoquinoline (3.00 g, 8.98 mmol) and meta-chloroperoxybenzoic acid (4.56 g, 22.16 mmol) were addd to dichloromethane (50 mL), and the reaction solution was stirred at 30°C for 16 h. the reaction solution was poured into saturated sodium bicarbonate solution (50 mL), extracted with dichloromethane (30 mL × 3). The combined organic layer was dried over anhydrous sodium sulfate, and concentrated to obtain the crude, which was purified by flash column chromatography (silica gel, 0-8% gradient of methanol/dichloromethane) to give 6-bromo-8-iodoquinoline-1-oxide (1.20 g, 3.44 mmol, yield: 38.2%) as a pale yellow solid. LCMS (ESI): $[M+H]^+$ =349.9

Step 3: 6-bromo-8-iodoquinoline-1-oxide (1.10 g, 3.14 mmol), trimethylsilyl cyanide (955 mg, 9.43 mmol), and iodobenzene acetate (3.10 g, 9.43 mmol) were added to 1,2-dichloroethane (10 mL), and the reaction solution was stirred at 80°C for 16 h. The reaction solution was poured into water (30 mL), extracted with dichloromethane (30 mL × 3), The combined organic layer was dried over anhydrous sodium sulfate, and concentrated to obtain the crude, which was purified by flash column chromatography (silica gel, 5-10% gradient of ethyl acetate/petroleum ether) to give 6-bromo-8-iodoquinoline-2-nitrile (800 mg, 2.23 mmol, yield: 70.9%) as a white solid. LCMS (ESI): $[M+H]^+$ =358.9

**Intermediate A25: 6-bromo-8-iodo-2-(trifluoromethyl)quinoline**

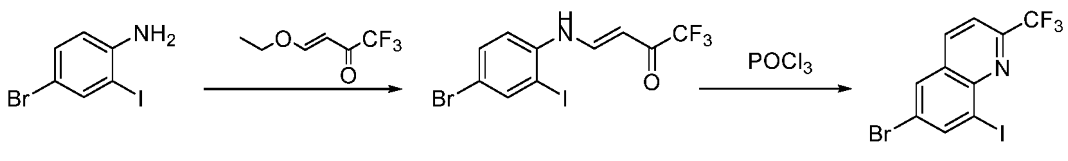


Step 1: To a solution of 4-bromo-2-iodoaniline (5.00 g, 16.78 mmol) in acetonitrile (50 mL) was added 4-ethoxy-1,1,1-trifluoro-3-buten-2-one (4.27 g, 25.17 mmol) at 25°C. The resulting reaction mixture was stirred at 50°C for 4 h. The residue was purified by flash column chromatography (silica gel, 0-20% gradient of ethyl acetate/petroleum ether) to give (3E)-4-[(4-bromo-2-iodophenyl)amino]-1,1,1-trifluorobut-3-en-2-one (6.00 g, 14.18 mmol, yield: 84.5%) as a yellow solid. LCMS (ESI): $[M+H]^+$ =421.9, $^1H$ NMR (400 MHz, DMSO) δ 11.81 (d, *J* = 12.9 Hz, 1H), 8.29 (dd, *J* = 12.9, 7.6 Hz, 1H),

8.13 (d, $J$ = 2.2 Hz, 1H), 7.68 (dd, $J$ = 8.8, 2.2 Hz, 1H), 7.59 (d, $J$ = 8.8 Hz, 1H), 5.79 (d, $J$ = 7.4 Hz, 1H).

Step 2: (3E)-4-[(4-bromo-2-iodophenyl)amino]-1,1,1-trifluorobut-3-en-2-one (3.00 g, 7.14 mmol) and 4-bromo-2-iodoaniline (2.15 g, 7.14 mmol) were added to phosphorus oxychloride (7.5 mL) at 25°C. The reaction mixture was stirred at 100°C for 16 h . The reaction mixture was concentrated, diluted with ethyl acetate (100 mL), and washed with saturated sodium bicarbonate solution (100 mL×3). The organic layer was dried with anhydrous sodium sulfate, filteredd, and concentrate to give the residue. The residue was purified by flash column chromatography (silica gel, 0-20% gradient of ethyl acetate/petroleum ether) to give 6-bromo-8-iodo-2-(trifluoromethyl)quinoline (2.30 g, 5.72 mmol, yield: 80.1%) as a pale yellow solid. LCMS (ESI): $[M+H]^+$ =402.0, $^1H$ NMR (400 MHz, DMSO) δ 8.70 - 8.62 (m, 2H), 8.51 (q, $J$ = 1.7 Hz, 1H), 8.09 (dd, $J$ = 8.5, 1.5 Hz, 1H).

**Intermediate A26: 6-bromo-7-fluoro-8-iodoquinoline**

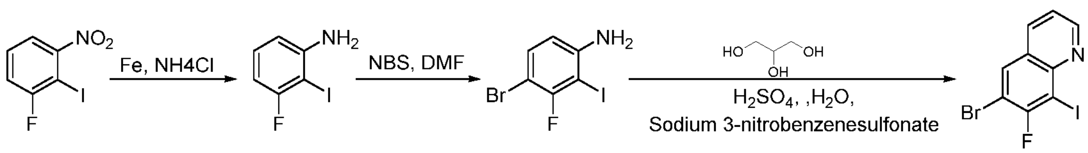


Step 1: To a stirred solution of iron powder (10.00 g, 177.90 mmol), ammonium chloride (9.60 g, 177.90 mmol) in ethanol (100 mL) and water (20 mL) was added 3-fluoro-2-iodo-1-nitrobenzene (9.50 g, 35.58 mmol) at 60°C for 1 h. the hot reaction solution was filtered, concentrated, extracted with ethyl acetate (50 mL × 3), and the combined organic layer was dried over anhydrous sodium sulfatefiltered and concentrated and the residue was purified by column chromatography (silica gel, 0-50% gradient of ethyl acetate/petroleum ether) to give 3-fluoro-2-iodoaniline (6.70 g, 79.5%) as a white solid compound. LCMS (ESI): $[M+H]^+$ = 238.0

Step 2: To a stirred solution of 3-fluoro-2-iodoaniline (6.70 g, 28.27 mmol) in N,N-dimethylformamide (60 mL) was added N-bromosuccinimide (5.0 g, 28.27 mmol). The reaction solution was stirred at 20°C for 1 h. The reaction solution was poured into water (300 mL), extracted with ethyl acetate (100 mL×3), and the combined organic layer was dried over anhydrous sodium sulfate, filtered and concentrated to give the residue, which was further purified by column chromatography (silica gel, 0-30% gradient of ethyl acetate/petroleum ether) to give 4-bromo-3-fluoro-2-iodoaniline (7.00 g, 78.7%) as a gray solid compound. LCMS (ESI): $[M+H]^+$ = 317.9.

Step 3: To a solution containing concentrated sulfuric acid (20 mL) and water (15 mL) were added glycerol (5.60 g, 59.83 mmol) and sodium 3-nitrobenzenesulfonate (5.40 g, 23.93 mmol). The mixture was heated to 110°C, then 4-bromo-3-fluoro-2-iodoaniline (6.30 g, 19.94 mmol) was slowly added , and the resulting mixture was stirred at 130°C for 12 h. The reaction solution was poured into ice water, extracted with ethyl acetate (50 mL×3), and the combined organic layer was dried over anhydrous sodium sulfate, filtered and concentrated to give the residue, which was further purified by column chromatography (silica gel, 0-50% gradient of ethyl acetate/petroleum ether) to give 6-bromo-7-fluoro-8-iodoquinoline (2.20 g, 31%) as a gray solid compound. LCMS (ESI): $[M+H]^+$ = 353.9.

**Intermediate A27:**

**8-(4,4-difluoropiperidinyl)-6-bromo-7-(trifluoromethyl)quinoline**

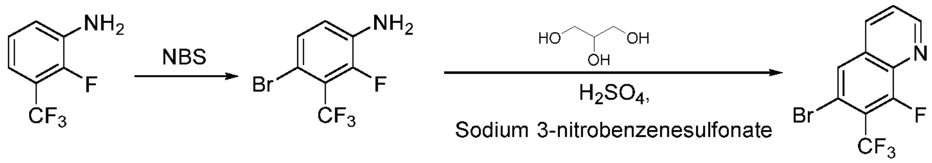


Step 1: To a solution of 2-fluoro-3-(trifluoromethyl)aniline (9.00 g, 50 mmol) in N,N-dimethylformamide (100 mL) was added N-bromosuccinimide (9.80 g, 55 mmol) at 25°C, and the reaction mixture was stirred at 25°C for 16 h. The reaction solution was diluted with ethyl acetate (300 mL), and washed with saturated sodium chloride solution (200 mL×3) and water (200 mL×2). The organic layer was dried with anhydrous sodium sulfate, filtered, and concentrated to give the residue. The residue was purified by flash column chromatography (silica gel, 0-15% gradient of ethyl acetate/petroleum ether) to give 4-bromo-2-fluoro-3-(trifluoromethyl)aniline (12.00 g, yield: 93%) as a violet solid. LCMS (ESI): $[M+H]^+$ =

259.9.

Step 2: glycerol (11.00 g, 116 mmol) and sodium 3-nitrobenzenesulfonate (9.70 g, 42 mmol) were added into water (70 mL) and concentrated sulfuric acid (30 mL). Then the mixture was heated to 100°C. 4-bromo-2-fluoro-3-(trifluoromethyl)aniline (10.00 g, 39.00 mmol) was slowly added to the reaction mixture. The reactionmixture was stirred at 100°C for 36 h. The reaction mixture was poured into crushed ice (400 mL), the pH was adjusted to 8 with saturated sodium bicarbonate solution, and extracted with ethyl acetate (200 mL×2). The combined organic layer was dried with anhydrous sodium sulfate, filtered, and concentrated to give the residue. The residue was purified by flash column chromatography (silica gel, 0-20% gradient of ethyl acetate/petroleum ether) to give 6-bromo-8-fluoro-7-(trifluoromethyl)quinoline (6.70 g, 59%) as a violet solid. LCMS (ESI): $[M+H]^+$ =296.0.

Step 3: To a solution of 6-bromo-8-fluoro-7-(trifluoromethyl)quinoline (3.7 g, 12 mmol) and 4,4-difluoropiperidine hydrochloride (3.0 g, 19 mmol) in DMSO (50 mL) was added cesium carbonate (5.3 g, 38 mmol) at 25°C, and the reactionmixture was stirred at 100°C for 24 h. The reaction mixture was filtered and diluted with ethyl acetate (200 mL). the organic phase was washed with saturated sodium chloride solution (50 mL×3), dried over anhydrous sodium sulfate, filtered, and concentrated to give a residue. the residue was stirred in a solution of petroleum ether: ethyl acetate (10:1, 11 mL) for 10 min, filtered, and concentrated to give a residue, which was further purified by flash column chromatography (silica gel, 0-50% gradient of ethyl acetate/petroleum ether) to give 8-(4,4-difluoropiperidinyl)-6-bromo-7-(trifluoromethyl) quinoline (450 mg, 9%) as a white solid. LCMS (ESI): $[M+H]^+$ =395.1.

**Intermediate A28: 6-bromo-7-chloro-8-fluoroquinoline**

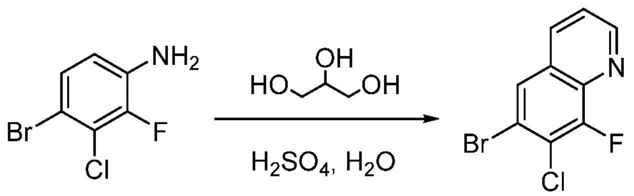


Step 1: glycerol (12.40 g, 133.65 mmol) and sodium 3-nitrobenzenesulfonate (12.20 g, 53.46 mmol) were added to water (60 mL) and concentrated sulfuric acid (80 mL). The mixture was heated to 100°C. 4-bromo-3-chloro-2-fluoroaniline (10.00 g, 44.55 mmol) was added to the mixture, and The mixture was stirred at 120°C for 12 h. The reaction solution was poured into water (300 mL), extracted with ethyl acetate (300 mL×3). The combined organic layer was dried over anhydrous sodium sulfate, filtered, and concentrated to give 6-bromo-7-chloro-8-fluoroquinoline (9.50 g, 36.47 mmol, yield: 81.9%) as a brown solid compound. LCMS (ESI): $[M+H]^+$ =260.1

**Intermediate A29: 6-Chloro-8-fluoroquinoline-7-nitrile**

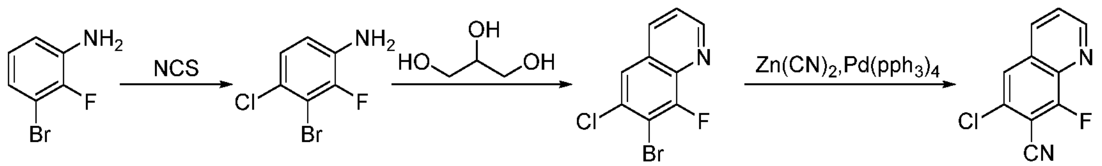


Step 1: To a solution of 3-bromo-2-fluoroaniline (10.00 g, 52.63 mmol) in N,N-dimethylacetamide (100 mL) was added chlorosuccinimide (7.00 g, 52.60 mmol) at 25°C, and the resulting reaction mixture was stirred at 55°C for 16 h. The reaction mixture was diluted with ethyl acetate (150 mL) and washed with saturated saline (150 mL×3).The organic phase was concentrated, dried, and filtered to give the residue. The residue was purifiedby flash column chromatography (silica gel, 0-30% gradient of ethyl acetate/petroleum ether) to give 3-bromo-4-chloro-2-fluoroaniline (6.00 g, 26.67 mmol, yield: 55.0%) as a violet solid. LCMS (ESI): $[M+H]^+$=226.0; $^1$H NMR (400 MHz, DMSO) δ 7.12 (dd, J = 8.8, 1.8 Hz, 1H), 6.82 - 6.74 (m, 1H), 5.60 (s, 2H)

Step 2: glycerol (10.00 g, 111.4 mmol) was dissolved in concentrated sulfuric acid (50 mL) and water (30 mL). The reaction was stirred at 100°C for 15 min, then the mixture was cooled to 40°C. 3-bromo-4-chloro-2-fluoroaniline (5.00 g, 22.3 mmol) and sodium 3-nitrobenzenesulfonate (5.57 g, 24.50 mmol) were added to the reaction mixture. The reactionmixture was stirred at 100°C for 16 h. The reaction mixture was poured into ice water (500 mL), the pH was

adjusted to 8 with potassium carbonate, and extracted with ethyl acetate (200 mL×2). The combined organic layer was dried over anhydrous sodium sulfate, filtered, and concentrated to give the crude.The crude product was purified by flash column chromatography (silica gel, 0-20% gradient of ethyl acetate/petroleum ether) to give 7-bromo-6-chloro-8-fluoroquinoline (3.80 g, 14.56 mmol, yield: 65.5%) as a gray solid. LCMS (ESI): $[M+H]^+$ =262.1; $^1H$ NMR (400 MHz, DMSO) δ 9.04 - 9.00 (m, 1H), 8.46 (dq, J = 8.4, 1.5 Hz, 1H), 8.26 (t, J = 2.0 Hz, 1H), 7.74 (dd, J = 8.4, 4.2 Hz, 1H).

Step 3: To a mixture of 7-bromo-6-chloro-8-fluoroquinoline (2.50 g, 9.60 mmol), zinc cyanide (5.17 g, 43.19 mmol) in N,N-dimethylformamide (40 mL) was added tetrakis(triphenylphosphine)palladium (1.13 g, 0.96 mmol) at 25°C. The resulting reaction mixture was bubbled by nitrogen for 1 min and stirred at 120°C for 16 h. The reaction solution was filtered, diluted with ethyl acetate (100 mL), and the organic phase was washed with saturated sodium chloride (50 mL × 3). The combined organic layer was dried , filtered, and concentrated to give the residue. The residue was purified by flash column chromatography (silica gel, 0-30% gradient of ethyl acetate/petroleum ether) to give 6-chloro-8-fluoroquinoline-7-nitrile (650 mg, 3.15 mmol, yield: 32.8%) as a white solid. LCMS (ESI): $[M+H]^+$ =207.1

**Intermediate A30: 6-Bromo-7-fluoro-8-iodo-2-methylquinoxaline**

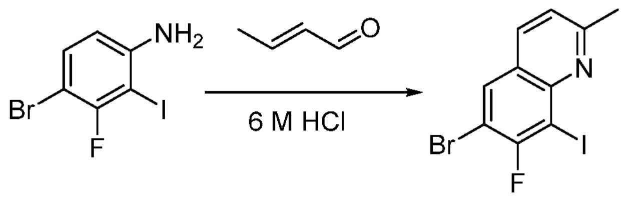


Step 1: To a stirred solution of (2E)crotonaldehyde (3.90 g, 56.00 mmol) in hydrochloric acid (116 mL, 6M) was added 4-bromo-3-fluoro-2-iodoaniline (4.40 g, 14.00 mmol). The solution was stirredat 100°C for 16 h. The reaction mixture was poured into crushed ice (500 mL) and the pH was adjusted to 8. The mixture was extrated with ethyl acetate (150 mL×3). The combined organic layer was dried over anhydrous sodium sulfate, filtered, and concentrated to give the residue. The residue was purified by flash column chromatography (silica gel, 0-20% ethyl acetate/petroleum ether) to give 6-bromo-7-fluoro-8-iodo-2-methylquinoxaline (1.00 g, 20%) as a white solid. LCMS (ESI): $[M+H]^+$ =365.9.

**Intermediate A31: 6-Bromo-8-fluoroisoquinoline**

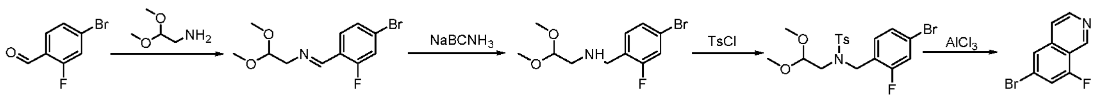


Step 1: To a stirred solution of 4-bromo-2-fluorobenzaldehyde (5.00 g, 24.63 mmol) in toluene (100 mL) wasadded 2,2-dimethoxyethan-1-amine (3.88 g, 36.94 mmol). The reaction solution was heated to 140°C, and stirred for 16 h. The reaction solution was cooled to ambient temperature and concentrated to give (E)-1-(4-bromo-2-fluorophenyl)-N-(2,2-dimethoxyethyl)methanamine (7.00 g, crude) as a brown oil crude for the next step without purification.

Step 2: (E)-1-(4-bromo-2-fluorophenyl)-N-(2,2-dimethoxyethyl)methanamine (3.00 g, 10.34 mmol) was dissolved in methanol (30 mL), Then the reaction solution was cooled to 0°C, sodium cyanoborohydride (270 mg, 7.24 mmol), was added. Then the reaction solution was heated to ambient temperature, and stirred for 2 h. ice water was added to the reaction solution, Then solution was concentrated and extracted with ethyl acetate (30 mL×3). The combined organic layer was washed with saturated sodium chloride solution, dried over anhydrous sodium sulfate, and concentrated to give the residue. The residue was purified by flash column chromatography (silica gel, 10-20% gradient of ethyl acetate/petroleum ether) to give N-(4-bromo-2-fluorobenzyl)-2,2-dimethoxyethan-1-amine (2.50 g, 8.50 mmol, yield: 83.3%) as a white solid. LCMS (ESI): $[M+H]^+$ = 292.0

Step 3:To a stirred solution of N-(4-bromo-2-fluorobenzyl)-2,2-dimethoxyethan-1-amine (2.50 g, 8.50 mmol) in dichloromethane (20 mL) were added 4-dimethylaminopyridine (156 mg, 1.28 mmol) and triethylamine (2.59 g, 25.5 mmol) successively. The reaction solution was cooled to 0°C under nitrogen atmosphere, and p-Toluenesulfonyl chloride (2.44 g, 12.75 mmol) was added. The reaction solution was heated to ambient temperature, and stirred for 16 h, then the solution was quenched with ice water (20 mL), and extracted with dichloromethane (30 mL×3). the combined organic layer was dried with anhydrous sodium sulfate, concentrated to give the residue. The residue was purified by flash column

chromatography (silica gel, 0-10% gradient of ethyl acetate/petroleum ether) to give N-(4-bromo-2-fluorobenzyl)-N-(2,2-dimethoxyethyl)-4-methylbenzenesulfonamide (3.04 g, 6.81 mmol, yield: 80.2%). LCMS (ESI): $[M+H]^+$ = 446.0

Step 4: To a stirred solution of aluminum trichloride (1.34 g, 10.08 mmol) in dichloromethane (20 mL) was added the solution of N-(4-bromo-2-fluorobenzyl)-N-(2,2-dimethoxyethyl)-4-methylbenzenesulfonamide (3.00 g, 6.72 mmol) in dichloromethane (10 mL) at 0°C under nitrogen atmosphere . The reaction solution was heated to ambient temperature, and stirred for 16 h, then the reaction solution was quenched with ice water (30 mL), the solution pH was adjusted to 10 with liquor ammonia, and extracted with dichloromethane (50 mL×3). The combined organic phases was dried over anhydrous sodium sulfate and concentrated to give the residue. The residue was purified by flash column chromatography (silica gel, 10-20% gradient of ethyl acetate/petroleum ether) to give 6-bromo-8-fluoroisoquinoline (1.36 g, 6.00 mmol, yield: 89.4%). LCMS (ESI): $[M+H]^+$ = 228.0

**Intermediate A32: 6-Bromo-8-fluorophosphorine**

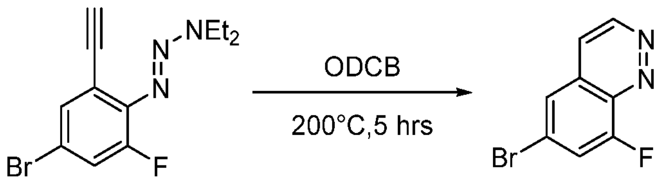


Step 1: (1E)-1-(4-bromo-2-ethynyl-6-fluorophenyl)-3,3-diethyltriaza-1-ene (2.00 g, 6.71 mmol) was added to 1,2-dichlorobenzene (20.0 mL). Then the reaction solution was stirred at 200°C for 5 h. The reaction mixture was purified by flash column chromatography (silica gel, 0-20% gradient of ethyl acetate/petroleum ether) to give 6-bromo-8-fluorophosphorine (500 mg, 2.14 mmol, yield: 33%) as a black solid. LCMS (ESI): [M+H]+ = 229.0

**Intermediate A33: 7-Bromo-5-iodoquinoxaline**

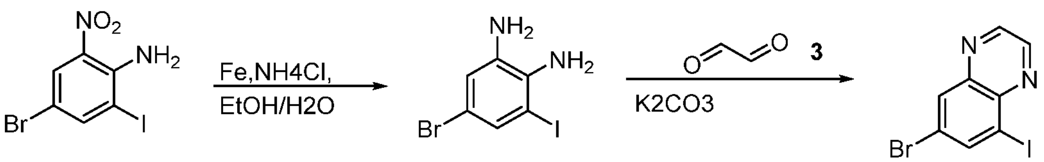


Step 1: To a mixture of 4-bromo-6-iodo-2-nitroaniline (5.00 g, 14.00 mmol) and iron (2.5 g, 44 mmol) in water (10 mL) and ethanol (50 mL) was added ammonium chloride (3.90 g, 73.00 mmol) at 25°C, and the resulting reaction mixture was stirred at 50°C for 1 h. The reaction mixture was filtered, and washed with ethanol (20 mL×2). The organic phase dried, filtered, and concentrated to give the brown solid residue (4.50 g, 98%) for the next step without purification. LCMS (ESI): $[M+H]^+$ =312.9.

Step 2: To a solution of 5-bromo-3-iodobenzene-1,2-diamine (4.50 g, 14.00 mmol) and glyoxal (1.70 g, 29.00 mmol) in ethanol (50 mL) was added potassium carbonate (6.00 g, 43.00 mmol) at 25°C. The reactionmixture was stirred at 80°C for 16 h.The reaction mixture was filtered and extracted with ethyl acetate (30 mL). The the filtrate was washed with saturated sodium chloride solution (20 mL×3). The organic layer was dried over anhydrous sodium sulfate, filtered, and concentrated to give the residue. The residue was purified by flash column chromatography (silica gel, 0-20% gradient of ethyl acetate/petroleum ether) to give 7-bromo-5-iodoquinoxaline (970 mg, 20%) as a pale yellow solid. LCMS (ESI): $[M+H]^+$ = 336.9. $^1H$ NMR (400 MHz, DMSO) δ 9.04 (d, *J* = 1.8 Hz, 1H), 8.98 (d, *J* = 1.8 Hz, 1H), 8.62 (d, *J* = 2.1 Hz, 1H), 8.40 (d, *J* = 2.1 Hz, 1H).

**Intermediate A34: 6-Bromo-7,8-difluoro-3-iodoquinoline**

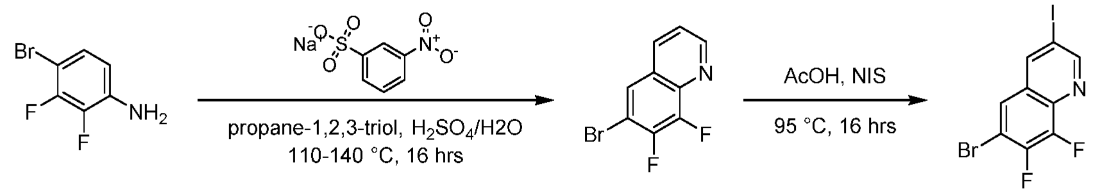


Step 1: To a stirred solution of sodium 3-nitrobenzenesulfonate (23.2 g, 102 mmol) and glycerol (32.2 g, 346 mmol) in pure water (308 mL) was added concentrated sulfuric acid (400 mL) dropwise , the reaction solution was heated to 110°C, 4-bromo-2,3-difluoroaniline (20.0 g, 96.2 mmol) was added portionwise, and stirted at 140°C for 16 h. the reaction solution was slowly added to 500 mL of ice water under stirring, and the pH was adjusted to 7 with sodium hydroxide solution (2000 mL, 600 g). After filtration, the cake was dispersed in water (1000 mL), extracted with ethyl acetate (1000 mL × 2), the organic phase was washed with saturated saline (400 mL), dried, filtered, and concentrated. The residue was slurried with methyl tert-butyl ether (250 mL) and filtered to give 6-bromo-7,8-difluoroquinoline (22.0 g, 88.5 mmol, yield: 92.0%) as a brown solid. LCMS (ESI): $[M+H]^+$ = 245.9; $^1$H NMR (400 MHz, DMSO-$d_6$) δ ppm 9.01 (d, *J* = 4.0 Hz, 1H), 8.44 (br d, *J* = 8.4 Hz, 1H), 8.34 (dd, *J* = 2.0, 7.2 Hz, 1H), 7.67 (dd, *J* = 4.4, 8.4 Hz, 1H)

Step 2: To a solution of 6-bromo-7,8-difluoroquinoline (9.50 g, 59%) in acetic acid (160 mL), was added N-iodosuccinimide (17.7 g, 78.7 mmol), and stired at 95°C for 16 h. The reaction solution was poured into saturated sodium bicarbonate solution (1500 mL), extracted with ethyl acetate (500 mL × 2). the organic phases was washed with saturated saline (400 mL), and dried, filtered, concentrated to give the residue. The residue was purified by flash column chromatography (silica gel, 0-7% gradient of THF/petroleum ether) to give 6-bromo-7,8-difluoro-3-iodoquinoline (6.40 g, 17.1 mmol, yield: 26.0%) as a brown solid. LCMS (ESI): $[M+H]^+$ = 371.7; $^1$H NMR (400 MHz, CHLOROFORM-d) δ ppm 9.02 (d, *J* = 1.6 Hz, 1H), 8.40 (s, 1H), 7.70 (dd, *J* = 2.4, 6.4 Hz, 1H)

**Example 1:**

**N-(2-(4,4-difluoropiperidin-1-yl)-6,7-dihydro-5H-cyclopenta[d]pyrimidin-4-yl)-4-((2-hydroxyethyl)sulfamoyl)-2-(6-azaspiro[2.5]octan-6-yl)benzamide**

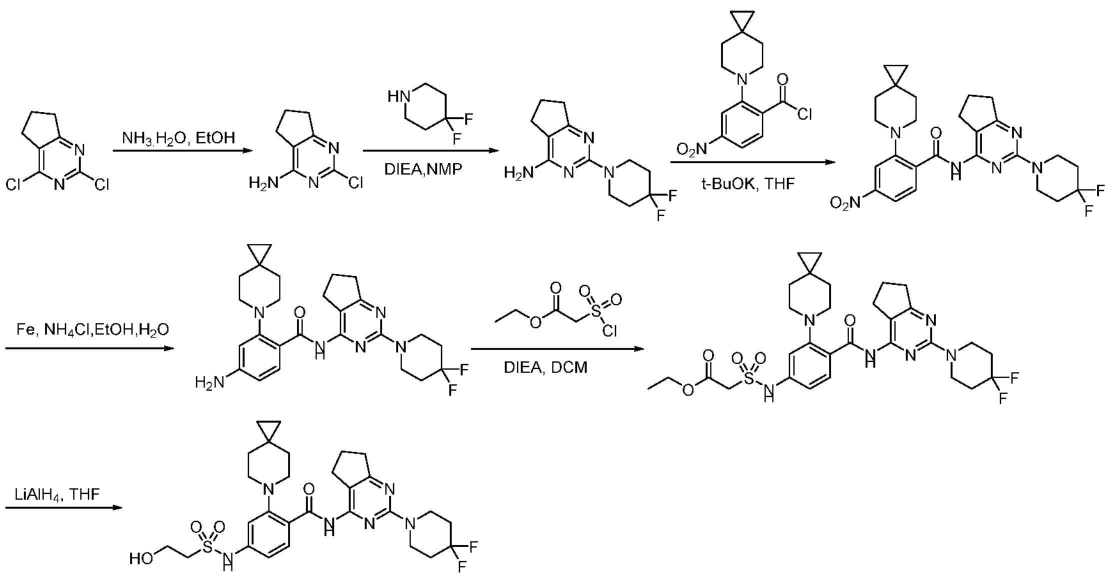


Step 1: To a stirred solution of 2,4-dichloro-6,7-dihydro-5H-cyclopenta[d]pyrimidine (5.00 g, 26.45 mmol) in ethanol (50 mL) was added liquor ammonia (9.90 g, 79.35 mmol). The reaction solution was stirred at 40°C for 12 h. The reaction solution was directly concentrated to give 2-chloro-6,7-dihydro-5H-cyclopenta[d]pyrimidin-4-amine (4.60 g) as a white solid compound. LCMS (ESI): $[M+H]^+$ = 170.1.

Step 2: To a stirred solution of 2-chloro-6,7-dihydro-5H-cyclopenta[d]pyrimidin-4-amine (4.60 g, 27.12 mmol), in dioxane (50 mL) were added 4,4-difluoropiperidine (3.90 g, 32.55 mmol) and N,N-diisopropylethylamine (10.70 g, 81.36 mmol). The reaction solution was stirred at 100°C for 12 h. The reaction solution was poured into water (100 mL), extracted

with ethyl acetate (100 mL×3), and the combined organic layer was dried over anhydrous sodium sulfate, filtered and concentrated to give the residue, which was further purified by column chromatography (silica gel, 0-50% gradient of ethyl acetate/petroleum ether) to give 2-(4,4-difluoropiperidin-1-yl)-6,7-dihydro-5H-cyclopenta[d]pyrimidin-4-amine (3.60 g) as a white solid compound. LCMS (ESI): $[M+H]^+$ = 255.1

Step 3: To a stirred solution of 2-(4,4-difluoropiperidin-1-yl)-6,7-dihydro-5H-cyclopenta[d]pyrimidin-4-amine (1.00 g, 3.93 mmol), potassium tert-butoxide (1.30 g, 11.80 mmol)in THF (10 mL) was added the solution of 4-nitro-2-(6-azaspiro[2.5]octan-6-yl)benzoyl chloride (2.10 g, 7.08 mmol) in THF (10 mL) . The reaction was stirred at ambient temperature for 30 min. The reaction solution was poured into water (50 mL), extracted with ethyl acetate (50 mL × 3), and the combined organic layer was dried over anhydrous sodium sulfate, filtered and concentrated to give the residue, which was further purified by column chromatography (silica gel, 0-100% gradient of ethyl acetate/petroleum ether) to give N-(2-(4,4-difluoropiperidin-1-yl)-6,7-dihydro-5H-cyclopenta[d]pyrimidin-4-yl)-4-nitr o-2-(6-azaspiro[2.5]octan-6-yl)benzamide (620 mg, containing isomers) as a yellow solid compound. LCMS (ESI): $[M+H]^+$ = 513.3.

Step 4: To a stirred solution of iron (341 mg, 6.05 mmol), ammonium chloride (327 mg, 6.05 mmol) in ethanol (5 mL), water (2 mL) and THF (5 mL) was added N-(2-(4,4-difluoropiperidin-1-yl)-6,7-dihydro-5H-cyclopenta[d]pyrimidin-4-yl)-4-nitr o-2-(6-azaspiro[2.5]octan-6-yl)benzamide (620 mg, 1.21 mmol). The reaction was stirred at 60°C for 30 min. the hot reaction solution was filtered, then poured into water (20 mL), extracted with ethyl acetate (20 mL × 3), and the combined organic layer was dried over anhydrous sodium sulfate, filtered and concentrated to give the residue, which was further purified by column chromatography (silica gel, 0-100% gradient of ethyl acetate/petroleum ether) to give 4-amino-N-(2-(4,4-difluoropiperidin-1-yl)-6,7-dihydro-5H-cyclopenta[d]pyrimidin-4-yl)-2-(6-azaspiro[2.5]octan-6-yl)benzamide (290 mg) as a yellow solid compound. LCMS (ESI): $[M+H]^+$ = 483.4.

Step 5: To a stirred solution containing 4-amino-N-(2-(4,4-difluoropiperidin-1-yl)-6,7-dihydro-5H-cyclopenta[d] pyrimidin-4-yl)-2-(6-azaspiro[2.5]octan-6-yl)benzamide (290 mg, 0.60 mmol), N,N-diisopropylethylamine (238 mg, 1.80 mmol) in dichloromethane (2 mL) was added ethyl chlorosulfonylacetate (136 mg, 0.721 mmol) dropwise at 0°C. Stir at 25°C for 1 h, The reaction solution was poured into water (30 mL), extracted withethyl acetate (30 mL × 3), and the combined organic layer was dried over anhydrous sodium sulfate, filtered and concentrated to give the residue, which was further purified by column chromatography (silica gel, 0-100% gradient of ethyl acetate/petroleum ether) to give 2-(N-(4-((2-(4,4-difluoropiperidin-1-yl)-6,7-dihydro-5H-cyclopentyl[d]pyrimidin-4-yl) carbamoyl)-3-(6-azaspiro[2.5]octan-6-yl)phenyl)sulfamoyl) ethyl acetate (100 mg) as a yellow solid compound. LCMS (ESI): $[M+H]^+$ = 633.3.

Step 6: To a reaction solution containing 2-(N-(4-((2-(4,4-difluoropiperidin-1-yl)-6,7-dihydro-5H-cyclopentyl[d] pyrimidin-4-yl) carbamoyl)-3-(6-azaspiro[2.5]octan-6-yl)phenyl)sulfamoyl) ethyl acetate (100 mg, 0.16 mmol) in THF (3 mL) was added lithium tetrahydridoaluminate (17 mg, 0.474 mmol). The reaction was stirred at 25°C for 30 min. saturated aqueous ammonium chloride solution (20 mL) was added dropwise to the reaction solution, which was extracted with ethyl acetate (20 mL × 3), and the combined organic layer was dried over anhydrous sodium sulfate, filtered and concentrated to give the residue, which was further purified by HPLC (C18, 30%-100% gradient of water/acetonitrile) to give N-(2-(4,4-difluoropiperidin-1-yl)-6,7-dihydro-5H-cyclopenta[d]pyrimidin-4-yl)-4-((2-hydroxyethyl)sulfamoyl)-2-(6-azaspiro[2.5]octan-6-yl)benzamide (16.3 mg) as a white solid compound. LCMS (ESI): $[M+H]^+$ = 591.3; $^1$H NMR (400 MHz, DMSO) δ 12.70 (s, 1H), 10.22 (s, 1H), 7.94 (d, *J* = 8.8 Hz, 1H), 7.24 (d, *J* = 2.4 Hz, 1H), 7.10 (dd, *J* = 8.4, 2.0 Hz, 1H), 4.96 (s, 1H), 3.75 (t, *J* = 6.8 Hz, 2H), 3.29-3.43 (m, 6H), 2.91-3.04 (m, 4H), 2.88 (t, *J* = 7.2 Hz, 2H), 2.78 (t, *J* = 7.6 Hz, 2H), 2.08 (s, 2H), 1.86 -2.03(m, 6H), 1.66-1.74 (m, 2H), 0.38 (s, 4H).

**Example 2:**

**N-(2-(4,4-difluoropiperidin-1-yl)-5,7-dihydrofuro[3,4-d]pyrimidin-4-yl)-4-((2-hyd roxyethyl)sulfonamide)-2-(6-azaspiro[2.5]octan-6-yl)benzamide**

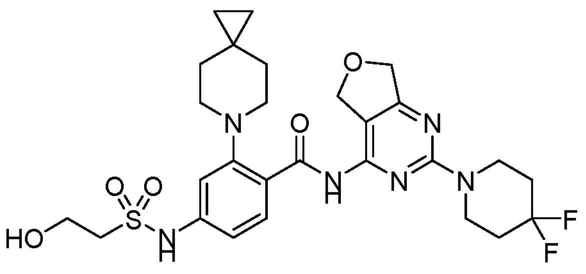

The preparations in Example 2 follow the synthetic route of Example 1: LCMS (ESI): $[M+H]^+$ = 593.4; $^1$H NMR

(500 MHz, DMSO) δ 13.29 (s, 1H), 10.32 (s, 1H), 9.06 (s, 1H), 8.01 (d, *J* = 7.2 Hz, 1H), 7.28-7.32 (m, 1H), 7.14 (dd, *J* = 6.8, 1.6 Hz, 1H), 5.19 (s, 3H), 4.79 (s, 2H), 3.87-4.01 (m, 4H), 3.75 (t, *J* = 5.2 Hz, 2H), 3.36 (t, *J* = 5.2 Hz, 2H), 2.92-3.04 (m, 4H), 1.93-2.09 (m, 4H), 1.61-1.84 (m, 3H), 1.54 (d, *J* = 5.6 Hz, 2H), 0.39 (s, 4H).

**Example 3:**

**N-(4-(4,4-difluoropiperidin-1-yl)-6,7-dihydro-5H-cyclopenta[d]pyrimidin-2-yl)-4-((2-hydroxyethyl)sulfonamide)-2-(6-azaspiro[2.5]oct-6-yl)benzamide**

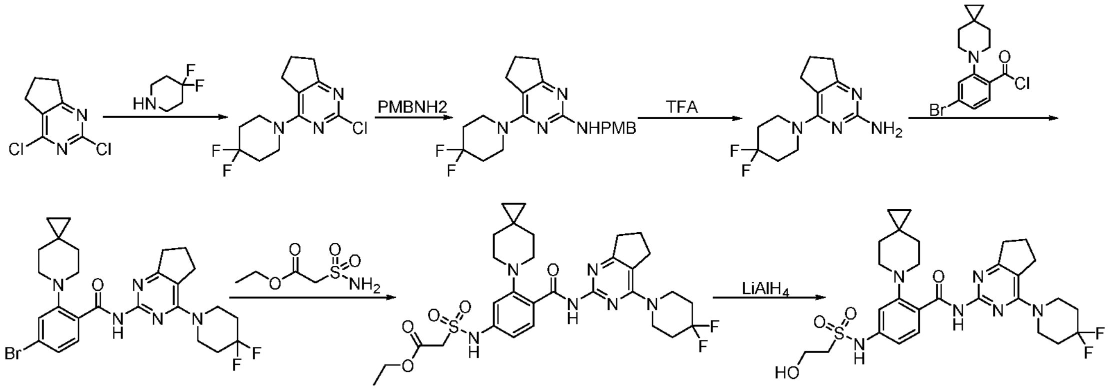


Step 1:2,4-dichloro-5,6-trimethylpyrimidine (2.00 g, 10.47 mmol), 4,4-difluoropiperidine hydrochloride (1.75 g, 10.99 mmol), and N,N-diisopropylethylamine (4.14 g, 31.42 mmol) were added to acetonitrile (10 mL), and the reaction solution was heated to 60°C and stirred for 4 h. The reaction solution was cooled to ambient temperature, diluted with water (50 mL) extracted with ethyl acetate (50 mL × 3), washed with saturated aqueous sodium chloride solution (50 mL × 3). The combined organic layer was dried over anhydrous sodium sulfate, and concentrated to give 2-chloro-4-(4,4-difluoropiperidin-1-yl)-6,7-dihydro-5H-cyclopenta[d]pyrimidine (2.60 g, yield: 90%) as a brown solid crude. LCMS (ESI): $[M+H]^+$= 274.1,276.1.

Step 2: To a stirred solution of 4-methoxybenzylamine (1.70 g, 12.28 mmol), and cesium carbonate (11.90 g, 35.80 mmol) in N,N-dimethylformamide (30 mL) was added 2-chloro-4-(4,4-difluoropiperidin-1-yl)-6,7-dihydro-5H-cyclopenta[d]pyrimidine (2.80 g, 10.23 mmol) , and the reaction solution was heated to 140°C and stirredfor 6 h. The reaction solution was cooled to ambient temperature, diluted with water (50 mL), extracted with dichloromethane (50 mL × 3), washed with saturated aqueous sodium chloride solution (50 mL × 3), The combined organic layer was dried over anhydrous sodium sulfate, and concentrated to give 4-(4,4-difluoropiperidin-1-yl)-N-(4-methoxybenzyl)-6,7-dihydro-5H-cyclopenta[d]py rimidine-2-amine (3.70 g, yield: 96%) as a brown solid crude. LCMS (ESI): $[M+H]^+$ = 375.3.

Step 3: 4-(4,4-difluoropiperidin-1-yl)-N-(4-methoxybenzyl)-6,7-dihydro-5H-cyclopenta[d]py rimidine-2-amine (3.70 g, 9.88 mmol) was added to THF (20 mL) and trifluoroacetic acid (20 mL), and the reaction solution was heated to 50°C and stirred for 30 min. The reaction solution was cooled to ambient temperature, diluted with water (50 mL), the pH was adjusted to 7 with saturated aqueous sodium bicarbonate solution, extracted with dichloromethane (50 mL×3), The combined organic layer was dried over anhydrous sodium sulfate and concentrated. The residue was purified by flash column chromatography (silica gel, 0-6% gradient of methanol/dichloromethane) to give 4-(4,4-difluoropiperidin-1-yl)-6,7-dihydro-5H-cyclopenta[d]pyrimidine-2-amine (1.50 g, 59%) as a pale yellow solid. LCMS (ESI): $[M+H]^+$= 255.3.

Step 4: To a stirred solution of 4-bromo-2-(6-azaspiro[2.5]oct-6-yl) benzoic acid (878 mg, 2.83 mmol) in dichloromethane (5 mL) were added a drop of N,N-dimethylformamide and thionyl chloride (1.13 g, 9.44 mmol)., The reaction solution was stirred at 25°C for 2 h. The reaction solution was concentrated to dryness to give the residue. To another solution of 4-(4,4-difluoropiperidin-1-yl)-6,7-dihydro-5H-cyclopenta[d]pyrimidine-2-amine (600 mg, 2.36 mmol) and potassium tert-butoxide (802 mg, 7.08 mmol) in THF (5 mL) was added the residue from the previous step in THF (5 mL)., and the reaction solution was stirred at 25°C for 2 h. The reaction solution was filtered under reduced pressure, concentrated, and purified by flash column chromatography (silica gel, 0-10% gradient of methanol/dichloromethane) to give 4-bromo-N-(4-(4,4-difluoropiperidin-1-yl)-6,7-dihydro-5H-cyclopenta[d]pyrimidin-2-yl)-2-(6-azaspiro[2.5]octan-6-yl) benzamide (450 mg, yield: 35%) as a brown solid. LCMS (ESI): $[M+H]^+$= 546.2, 548.2.

Step 5: potassium phosphate (159 mg, 0.73 mmol), (1S,2S)-N,N'-dimethyl-1,2-cyclohexanediamine (53 mg, 0.37 mmol) and ethyl 2-sulfamoylacetate (62 mg, 0.37 mmol) in N,N-dimethylformamide (4 mL) were added 4-bromo-N-(4-(4,4-difluoropiperidin-1-yl)-6,7-dihydro-5H-cyclopenta[d]pyrimidin-2-yl)-2-(6-azaspiro[2.5]octan-6-yl) benzamide

(200 mg, 0.37 mmol) and cuprous iodide (71 mg, 0.37 mmol) under nitrogen atmosphere, and the reaction solution was heated to 100°C and stired for 6 h. The reaction solution was cooled to ambient temperature, diluted with water (30 mL) , extracted with dichloromethane (30 mL×3), washed with saturated aqueous sodium chloride solution (30 mL×3). The combined organic layer was concentrated to give the residue. The residue was purified by flash column chromatography (silica gel, 0-10% gradient of methanol/dichloromethane) to give ethyl 2-(N-(4-((4-(4,4-difluoropiperidin-1-yl)-6,7-dihydro-5H-cyclopenta[d]pyrimidin-2-yl) carbamoyl)-3-(6-azaspiro[2.5]oct-6-yl)phenyl)sulfamoyl)acetate (100 mg, yield: 43%) as a pale yellow solid. LCMS (ESI): $[M+H]^+$= 633.4.

Step 6: ethyl 2-(N-(4-((4-(4,4-difluoropiperidin-1-yl)-6,7-dihydro-5H-cyclopenta[d]pyrimidin-2-yl) carbamoyl)-3-(6-azaspiro[2.5]oct-6-yl)phenyl)sulfamoyl)acetate (100 mg, 0.16 mmol) was added to THF (2 mL), and The reaction solution was cooled to 0°C. lithium aluminum hydride (12 mg, 0.32 mmol) was added to the reaction solution in 3 portions over 30 min, and the reaction solution was stirred at 0°C for 1 h. saturated aqueous ammonium chloride solution (30 mL) was added to the reaction solution, which was extracted with ethyl acetate (30 mL×3). The combined organic layer was dried over anhydrous sodium sulfate, and concentrated to give the residue. The residue was purified by preparative HPLC (40-60% gradient of acetonitrile/water) to give N-(4-(4,4-difluoropiperidin-1-yl)-6,7-dihydro-5H-cyclopenta[d]pyrimidin-2-yl)-4-((2-hydroxyethyl)sulfonamide)-2-(6-azaspiro[2.5]oct-6-yl)benzamide (5 mg, 5.98%). LCMS (ESI): $[M+H]^+$ = 591.4. $^1$HNMR (400 MHz, DMSO) δ ppm 13.20-13.00 (m,1H), 10.23 (s, 1H), 7.97(s, 1H), 7.23(s, 1H), 7.12-7.09(m, 1H), 4.96(s, 1H), 3.83-3.74(m,6H), 3.01-2.98(m, 6H), 2.97-2.80(m, 2H), 2.62-2.53(m, 2H), 2.10-2.00(m, 6H), 1.66(s,4H), 0.38(s, 4H).

### Example 4: N-(4-(4,4-difluoropiperidin-1-yl)-5,7-dihydrofuro[3,4-d]pyrimidin-2-yl)-4-(2-hyd roxyethylsulfonamido)-2-(6-azaspiro[2.5]octan-6-yl)benzamide

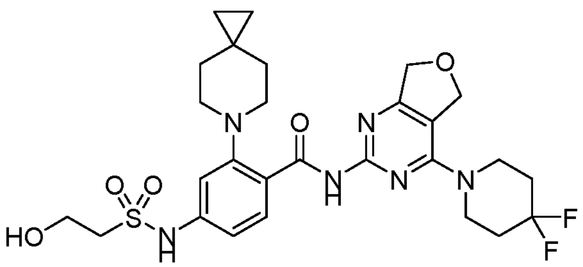

The preparations in **Example 4** follow the synthetic route of **Example 3:** LCMS (ESI): $[M+H]^+$ = 593.3. $^1$H NMR (400 MHz, DMSO-$d_6$) δ ppm 12.72 (s, 1H), 10.15 (s, 1H), 7.93 (br d, *J* =8.4 Hz, 1H), 7.20 (s, 1H), 7.07 (dd, *J* = 1.8, 8.6 Hz, 1H), 5.19 (s, 2H), 4.79 (s, 2H), 3.75 (t, *J* =6.5 Hz, 2H), 3.62 - 3.72 (m, 4H), 3.34 - 3.40 (m, 3H), 2.92 - 3.00 (m, 4H), 1.96 - 2.09 (m, 4H), 1.48 - 1.80 (m, 4H), 0.37 (s, 4H)

### Example 5: N-(4-(4,4-difluoropiperidin-1-yl)-1,3-dihydrofuro[3,4-c]pyridin-6-yl)-4-(2-hydrox yethylsulfonamido)-2-(6-azaspiro[2.5]octan-6-yl)benzamide

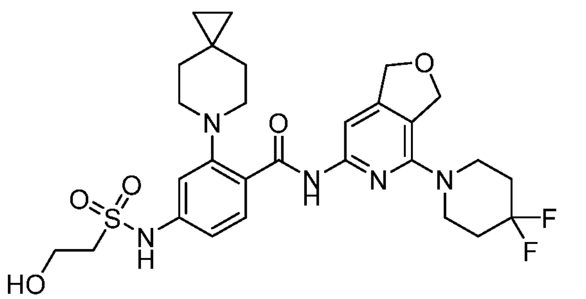

The preparations in **Example 5** follow the synthetic route of **Example 3:** LCMS (ESI): $[M+H]^+$ = 592.2. $^1$H NMR (400 MHz, DMSO-$d_6$) δ ppm 12.87 (s, 1H), 10.19 (s, 1H), 8.04 (d, *J* =8.6 Hz, 1H), 7.69 (s, 1H), 7.24 (d, *J* =1.6 Hz, 1H), 7.10 (dd, J = 1.9, 8.6 Hz, 1H), 5.07 (s, 2H), 4.94 (s, 2H), 3.75 (t, *J* =6.4 Hz, 2H), 3.57 (br t, *J* =4.9 Hz, 4H), 3.35 - 3.38 (m, 3H), 2.90 - 3.05(m, 4H), 1.96-2.08 (m, 4H), 1.37-1.93 (m, 4H), 0.38 (s, 4H)

**Example 6: Tert-butyl 4-(4,4-difluoropiperidin-1-yl)-2-(4-(2-hydroxyethylsulfonamido)-2-(6-azaspiro[2. 5]octan-6-yl)benzoylamino)-5H-pyrrolo[3,4-d]pyrimidine-6(7H)-carboxylate**

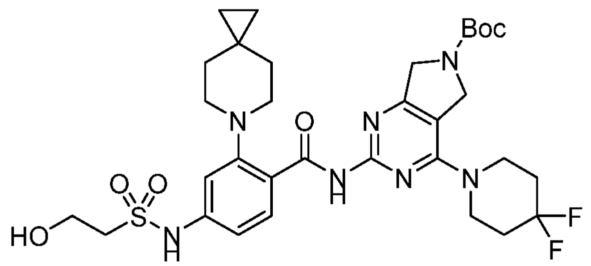

The preparations in **Example 6** follow the synthetic route of **Example 3:** LCMS (ESI): [M+H]+ = 692.5. $^1$H NMR (400 MHz, DMSO-d6) δ ppm 12.68 (br s, 1H), 10.17 (br s, 1H), 7.95 (d, *J* = 1.2 Hz, 1H), 7.22 (s, 1H), 7.09 (br d, *J* = 8.8 Hz, 1H), 5.08 - 4.84 (m, 1H), 4.80 - 4.62 (m, 2H), 4.49 - 4.27 (m, 2H), 3.76 (m, 7H), 2.96 (m, 4H), 2.15 - 1.98 (m, 4H), 1.73 - 1.58 (m, 4H), 1.50 - 1.44 (m, 9H), 0.38 (s, 4H)

**Example 7: N-(4-(4,4-difluoropiperidin-1-yl)-6,7-dihydro-5H-pyrrolo[3,4-d]pyrimidin-2-yl)-4 -(2-hydroxyethylsulfonamido)-2-(6-azaspiro[2.5]octan-6-yl)benzamide**

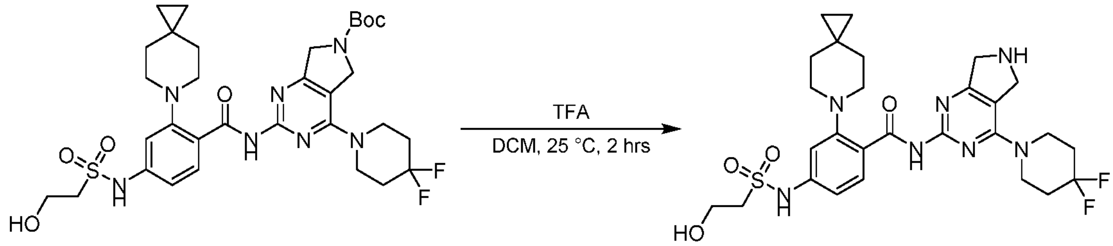


Step 1: To a solution of tert-butyl 4-(4,4-difluoropiperidin-1-yl)-2-(4-(2-hydroxyethylsulfonamido)-2-(6-azaspiro[2.5]oc tan-6-yl)benzoylamino)-5H-pyrrolo[3,4-d]pyrimidine-6(7H)-carboxylate (40.0 mg, 0.06 mmol) in dichloromethane (6.00 mL) was added trifluoroacetic acid (1.00 mL). The mixture was stirred at 25°C for 2 h. The mixture was purified by preparative HPLC (C18, 0-38% gradient of water (formic acid)/acetonitrile) to give N-(4-(4,4-difluoropiperidin-1-yl)-6,7-dihydro-5H-pyrrolo[3,4-d]pyrimidin-2-yl)-4-(2-hydroxyethylsulfonamido)-2-(6-azaspiro[2.5]octan-6-yl)benzamide (15 mg, 0.06 mmol, yield: 44%) as a white solid compound. LCMS (ESI): $[M+H]^+$ =592.5. $^1$H NMR (400 MHz, DMSO-d6) δ ppm 12.44 (s, 1H), 8.17 (s, 1H), 7.94 (br d, J = 8.4 Hz, 1H), 7.21 (s, 1H), 7.08 (dd, J = 2.0, 8.8 Hz, 1H), 4.30 - 4.21 (m, 2H), 3.96 - 3.88 (m, 2H), 3.79 - 3.70 (m, 6H), 3.36 - 3.34 (m, 2H), 3.01 - 2.93 (m, 4H), 2.10 - 1.98 (m, 4H), 1.83 - 1.46 (m, 4H), 0.38 (s, 4H)

**Example 8: N-(4-(4,4-difluoropiperidin-1-yl)-6-methyl-6,7-dihydro-5H-pyrrolo[3,4-d]pyrimid in-2-yl)-4-(2-hydroxyethylsulfonamido)-2-(6-azaspiro[2.5]octan-6-yl)benzamide**

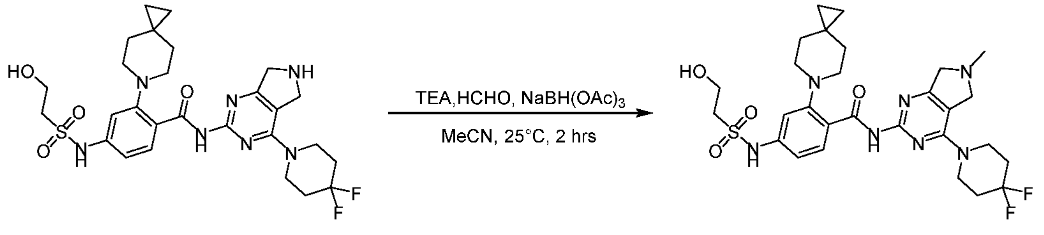


Step 1: To a solution of N-(4-(4,4-difluoropiperidin-1-yl)-6,7-dihydro-5H-pyrrolo[3,4-d]pyrimidin-2-yl)-4-(2-hydroxyethylsulfonamido)-2-(6-azaspiro[2.5]octan-6-yl)benzamide (40.0 mg, 0.07 mmol) and triethylamine (13.9 mg, 0.14 mmol) in acetonitrile (1.00 mL) were added aqueous formaldehyde solution (9.23 mg, 0.10 mmol) and sodium borohydride acetate (14.6 mg, 0.07 mmol). The mixture was stirred at 25°C for 2 h. the reaction solution was extracted with ethyl acetate, filtered under reduced pressure. the solid was slurried with pure water (5 mL) at 25°C for 1 h, and purified to give the crude, which was diluted with pure water (5 mL) and lyophilized to give N-(4-(4,4-difluoropiperidin-1-yl)-6-methyl-6,7-dihydro-5H-pyrrolo[3,4-d]pyrimidin-2 -yl)-4-(2-hydroxyethylsulfonamido)-2-(6-azaspiro[2.5]octan-6-yl)benzamide (12 mg, 0.07 mmol, yield: 29%) as a white solid. LCMS (ESI): $[M+H]^+$ =606.5. $^1H$ NMR (400 MHz, DMSO-d6) δ ppm 12.62 (br s, 1H), 10.16 (br s, 1H), 7.93 (br d, *J* = 8.0 Hz, 1H), 7.20 (br s, 1H), 7.12 - 7.02 (dd, *J* =1.6 Hz, 8.4Hz, 1H), 5.19 - 4.72 (m, 1H), 4.03 - 3.97 (m, 2H), 3.81 - 3.62 (m, 8H), 3.36 - 3.34 (m, 2H), 3.04 - 2.90 (m, 4H), 2.49 (s, 3H), 2.09 - 1.95 (m, 4H), 1.81 - 1.47 (m, 4H), 0.37 (s, 4H)

**Example 9: N-(4-(4,4-difluoropiperidin-1-yl)-1-methyl-1,3-dihydrofuro[3,4-c]pyridin-6-yl)-4-((2-hydroxyethyl) sulfonamido)-2-(6-azaspiro[2. 5]octan-6-yl)benzamide**

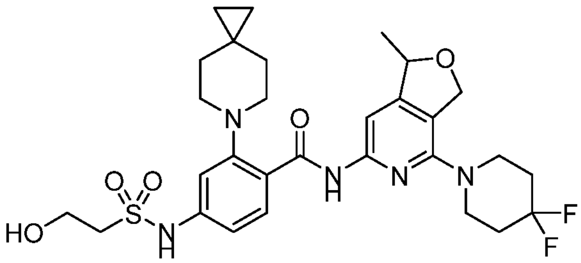

The preparations in **Example 9** follow the synthetic route of **Example 3:** LCMS (ESI): $[M+1]^+$ = 606.1. $^1H$ NMR (400 MHz, DMSO) δ 12.89 (s, 1H), 9.98 (s, 1H), 8.05 (d, J = 8.6 Hz, 1H), 7.63 (s, 1H), 7.26 (d, J = 2.0 Hz, 1H), 7.12 (dd, J = 8.8, 2.0 Hz, 1H), 6.25 - 4.36 (m, 4H), 3.76 (t, J = 6.4 Hz, 2H), 3.57 (dt, J = 7.2, 3.8 Hz, 4H), 3.33 (d, J = 3.2 Hz, 2H), 2.97 (d, J = 5.6 Hz, 4H), 2.11 - 2.00 (m, 4H), 2.00 - 1.47 (m, 4H), 1.39 (d, J = 6.4 Hz, 3H), 0.38 (s, 4H).

**Example 10: N-(4-(4,4-difluoropiperidin-1-yl)-1-ethyl-1-methyl-1,3-dihydrofuro[3,4-c]pyridin-6-yl)-4-(2-hydroxyethylsulfonamido)-2-(6-azaspiro[2.5]octan-6-yl)benzamide**

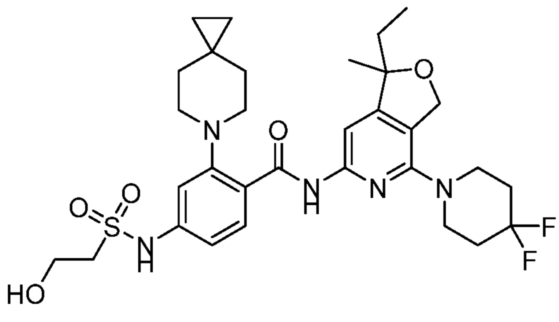

The preparations in **Example 10** follow the synthetic route of **Example 3:** LCMS (ESI): $[M+H]^+$ = 634.3. $^1H$ NMR (400 MHz, DMSO-*$d_6$*) δ ppm 12.96 (s, 1H), 11.06 - 9.21 (m, 1H), 8.06 (d, *J* = 8.8 Hz, 1H), 7.57 (s, 1H), 7.26 (d, *J* = 2.0 Hz, 1H), 7.12 (dd, *J* = 2.0, 8.4 Hz, 1H), 5.04 (d, *J* = 1.6 Hz, 3H), 3.76 (t, *J* = 6.8 Hz, 2H), 3.56 (br d, *J* = 4.0 Hz, 4H), 3.38 - 3.34 (m, 2H), 3.06 - 2.92 (m, 4H), 2.10 - 1.99 (m, 4H), 1.98 - 1.40 (m, 6H), 1.37 (s, 3H), 0.76 (t, *J* = 7.2 Hz, 3H), 0.38 (s, 4H)

**Example 11: N-(1-(4,4-difluoropiperidin-1-yl)pyrrolo[1,2-a]pyrazin-3-yl)-4-(2-hydroxyethylsul fonamido)-2-(6-azaspiro[2.5]octan-6-yl)benzamide**

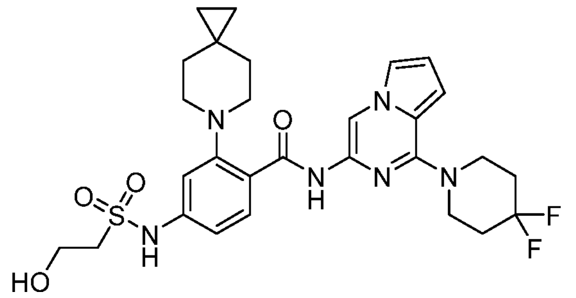

The preparations in **Example 11** follow the synthetic route of **Example 3:** LCMS (ESI): $[M+H]^+$ = 589.1. $^1H$ NMR (400 MHz, DMSO-$d_6$) δ ppm 12.62 - 12.88 (m, 1H), 9.98 -10.41 (m, 1H), 8.64 - 8.87 (m, 1H), 8.02 (s, 1H), 7.69 - 7.83 (m, 1H), 7.26 - 7.33 (m, 1H), 7.09 - 7.21 (m, 1H), 6.81 - 6.90 (m, 1H), 6.68 - 6.79 (m, 1H), 3.86 - 3.92 (m, 4H), 2.97 - 3.06 (m, 4H), 2.12 - 2.24 (m, 4H), 1.78 (br s, 4H), 1.26 (s, 4H), 0.34 - 0.48 (m, 4H)

**Example 12: N-(8-(4,4-difluoropiperidin-1-yl)-3-methylimidazo[1,5-a]pyrazin-6-yl)-4-(2-hydro xyethylsulfonamido)-2-(6-azaspiro[2.5]octan-6-yl)benzamide**

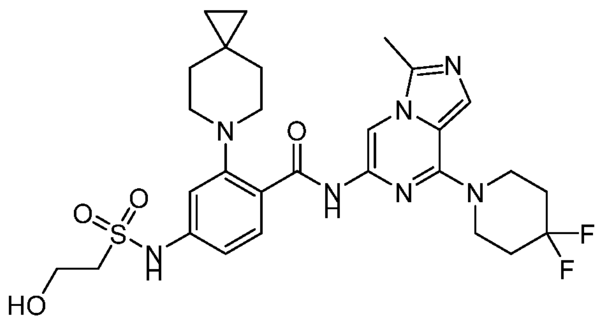

The preparations in **Example 12** follow the synthetic route of **Example 3** LCMS (ESI): $[M+H]^+$ = 604.4. $^1H$ NMR (400 MHz, DMSO-$d_6$) δ ppm 12.81 (s, 1H), 10.38 - 10.05 (m, 1H), 8.36 (s, 1H), 8.07 (d, *J = 8.8* Hz, 1H), 7.81 (s, 1H), 7.27 (d, *J* = 2.0 Hz, 1H), 7.13 (dd, *J* = 2.0, 8.8 Hz, 1H), 5.14 - 4.77 (m, 1H), 4.03 -3.88 (m, 4H), 3.76 (t, *J* = 6.4 Hz, 2H), 3.40 - 3.34 (m, 2H), 3.07-2.85 (m, 4H), 2.53 (s, 3H), 2.20 - 2.04 (m, 4H), 2.04 - 1.32 (m, 4H), 0.40 (s, 4H)

**Example 13: N-(8-(4,4-difluoropiperidin-1-yl)imidazolo[1,2-a]pyrazin-6-yl)-4-((2-hydroxyethyl) sulfonamide)-2-(6-azaspiro[2.5]octan-6-yl)benzamide**

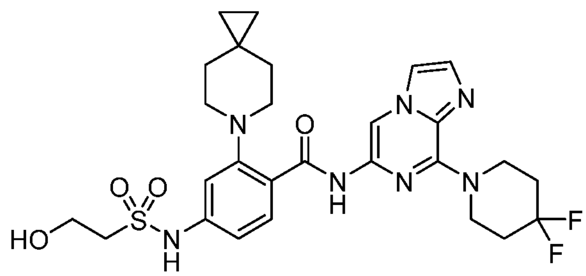

The preparations in **Example 13** follow the synthetic route of **Example 3:** LCMS (ESI): $[M+H]^+$ = 590.0. $^1H$NMR (400 MHz, DMSO) δ 12.89 (s, 1H), 10.21 (s, 1H), 8.84 (s, 1H), 8.08 (d, *J* = 8.8 Hz, 2H), 7.56 (d, *J* = 0.8 Hz, 1H), 7.28 (d, *J* = 2.0 Hz, 1H), 7.14 (dd, *J* = 8.6, 2.0 Hz, 1H), 4.43 (s, 5H), 3.76 (t, *J* = 6.6 Hz, 2H), 3.34 (s, 2H), 2.99 (s, 4H), 2.54 (s, 4H), 2.16 - 2.06 (m, 4H), 0.41 (s, 4H).

**Example 14: N-(8-(4,4-difluoropiperidin-1-yl)imidazo[1,2-a]pyridin-6-yl)-4-((2-hydroxyethyl)s ulfonamido)-2-(6-azaspiro[2.5]oct-6-yl)benzamide**

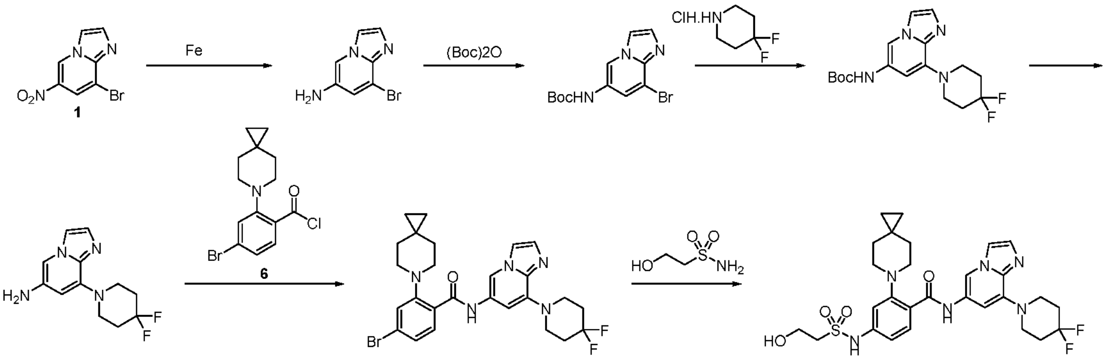


Step 1: To a stirred solution of iron powder (2.40 g, 43.38 mmol), ammonium chloride (2.30 g, 43.38 mmol) in ethanol (50 mL) and water (10 mL) was added 8-bromo-6-nitro-4-hydroimidazo[1,2-a]pyridine (3.50 g, 14.46 mmol). The mixture was stirred at 90°C for 6 h. the reaction solution was concentrated, extracted with ethyl acetate (50 mL × 3), and the organic layer was dried over anhydrous sodium sulfate, filtered and concentrated to give the residue, which was further purified by column chromatography (silica gel, 0-100% gradient of ethyl acetate/petroleum ether) to give 8-bromoimidazo [1,2-a]pyridine-6-amine (3.00 g, 14.15 mmol, yield: 97.8%) as a black solid compound. LCMS (ESI): $[M+H]^+$ =214.0

Step 2: To a stirred solution containing 8-bromo-4-hydroimidazo[1,2-a]pyridine-6-ylamine (3.00 g, 14.15 mmol) in N,N-diisopropylethylamine (5.60 g, 42.44 mmol) and dichloromethane (20 mL) was added di-tert-butyl dicarbonate (3.70 g, 16.98 mmol). The reaction was stirred at 50°C for 12 h, The reaction mixture was concentrated, and The residue was purified by column chromatography (silica gel, 0-50% gradient of ethyl acetate/petroleum ether) to give tert-butyl(8-bromoimidazo[1,2-a]pyridin-6-yl)carbamate (1.80 g, 5.77 mmol, yield: 40.8%) as a white solid compound. LCMS (ESI): [M+H]+ =312.1

Step 3 : To a stirred solution containing tert-butyl(8-bromoimidazo[1,2-a]pyridin-6-yl)carbamate (1.80 g, 5.77 mmol), 4,4-difluoropiperidine hydrochloride (1.40 g, 8.65 mmol), cesium carbonate (7.70 g, 23.07 mmol) in 1,4-dioxane (20 mL) was added 4,5-bis(diphenylphosphino)-9,9-dimethylxanthene (340 mg, 0.58 mmol) and tris(dibenzylideneacetone) dipalladium (539 mg, 0.58 mmol) under nitrogen atmosphere, and the reaction solution was stirred at 100°C for 12 h. The reaction mixture was poured into water (30 mL), extracted with ethyl acetate (30 mL×3), and the combined organic layer was dried over anhydrous sodium sulfate, filtered and concentrated to give the residue, which was further purified by column chromatography (silica gel, 0-30% gradient of ethyl acetate/petroleum ether) to give tert-butyl (8-(4,4-difluoropiperidin-1-yl)imidazo[1,2-a]pyridin-6-yl)carbamate (500 mg, 1.42 mmol, yield: 24.6%) as a yellow solid compound. LCMS (ESI): $[M+H]^+$ = 353.3

Step 4: To a stirred solution containing tert-butyl (8-(4,4-difluoropiperidin-1-yl)imidazo[1,2-a]pyridin-6-yl)carbamate (500 mg, 1.42 mmol) in dichloromethane (5 mL) was added trifluoroacetic acid (5 mL). The reaction was stirred at 20°C for 1 h, the reaction solution was concentrated. The pH was adjusted to be close to 8 by adding aqueous sodium bicarbonate solution and the mixture was extracted with ethyl acetate (30 mL × 3). the organic layer was dried over anhydrous sodium sulfate, filtered, and concentrated to give 8-(4,4-difluoropiperidin-1-yl)imidazo[1,2-a]pyridine-6-amine (360 mg, crude) as a brown solid compound. LCMS (ESI): $[M+H]^+$ = 253.1

Step 5: To the solution of 8-(4,4-difluoropiperidin-1-yl)imidazo[1,2-a]pyridine-6-amine (350 mg, 1.39 mmol), N,N-diisopropylethylamine (915 mg, 6.94 mmol) in THF (5 mL) was added 2-(6-azaspiro[2.5]oct-6-yl)-4-bromobenzoyl chloride (502 mg, 1.53 mmol). The reaction solution was stirred at 20°C for 30 min, The reaction solution was poured into water (30 mL), extracted with ethyl acetate (30 mL×3), and the combined organic layer was dried over anhydrous sodium sulfate, filtered and concentrated to give the residue, which was further purified by column chromatography (silica gel, 0-30% gradient of ethyl acetate/petroleum ether) to give 4-bromo-N-(8-(4,4-difluoropiperidin-1-yl)imidazo[1,2-a] pyridin-6-yl)-2-(6-azaspiro[2. 5]oct-6-yl) benzamide (390 mg, 0.72 mmol, yield: 51.6%) as a yellow solid compound. LCMS (ESI): $[M+H]^+$ = 544.3; $^1$HNMR (400 MHz, DMSO) δ 11.25 (s, 1H), 9.04 (d, J = 1.6 Hz, 1H), 8.00 (d, J = 1.2 Hz, 1H), 7.67 (d, J = 8.4 Hz, 1H), 7.46 (dd, J = 2.8, 1.6 Hz, 2H), 7.39 (dd, J = 8.4, 2.0 Hz, 1H), 6.59 (d, J = 1.6 Hz, 1H), 3.77 (t, J = 5.6 Hz, 4H), 3.03 (t, J = 5.2 Hz, 4H), 2.26-2.05 (m, 4H), 1.58-1.44 (m, 4H), 0.32 (s, 4H).

Step 6: To a stirred solution of 2-hydroxyethanesulfonamide (174 mg, 1.36 mmol), potassium phosphate (442 mg, 2.04 mmol), 2-di-tert-butylphosphino-2',4',6'-triisopropylbiphenyl (29 mg, 0.068 mmol), tris(dibenzylideneacetone) dipalladium (64 mg, 0.068 mmol) in 1,4-dioxane (5 mL) was added 4-bromo-N-(8-(4,4-difluoropiperidin-1-yl)imidazo[1,2-a]pyridin-6-yl)-2-(6-azaspiro[2. 5]oct-6-yl) benzamide (370 mg, 0.68 mmol).The reaction was stirred at 100°C for 12 h under nitrogen atmosphere. The reaction solution was poured into water (30 mL), extracted with ethyl acetate (30 mL × 3), and the combined organic layer was dried over anhydrous sodium sulfate, filtered and concentrated to give the residue, which was further purified by column chromatography (silica gel, 0-100% gradient of ethyl acetate/petroleum ether) to give N-(8-(4,4-difluoropiperidin-1-yl)imidazo[1,2-a]pyridin-6-yl)-4-((2-hydroxyethyl)sulf onamido)-2-(6-azaspiro[2.5]oct-6-yl) benzamide (50.04 mg, 0.085 mmol, yield: 12.5%) as a white solid compound. LCMS (ESI): $[M+H]^+$ = 589.4; $^1$HNMR (400 MHz, DMSO) δ 11.60 (s, 1H), 10.15 - 10.02 (m, 1H), 9.05 (d, J = 1.6 Hz, 1H), 7.98 (d, J = 1.2 Hz, 1H), 7.80 (d, J = 8.4 Hz, 1H), 7.46 (d, J = 1.2 Hz, 1H), 7.16 (d, J = 2.0 Hz, 1H), 7.04 (dd, J = 8.4, 2.0 Hz, 1H), 6.57 (d, J = 1.6 Hz, 1H), 4.96 (s, 1H), 3.86-3.66 (m, 6H), 3.37-3.32 (m, 2H), 2.98 (t, J = 5.2 Hz, 4H), 2.25-2.09 (m, 4H), 1.67-1.48 (m, 4H), 0.35 (s, 4H).

**Example 15: N-(8-(4,4-difluoropiperidin-1-yl)-2,3-dimethylimidazo[1,2-a]pyrazin-6-yl)-4-(2-hy droxyethyl)sulfonamide)-2-(6-azaspiro[2.5]octan-6-yl)benzamide**

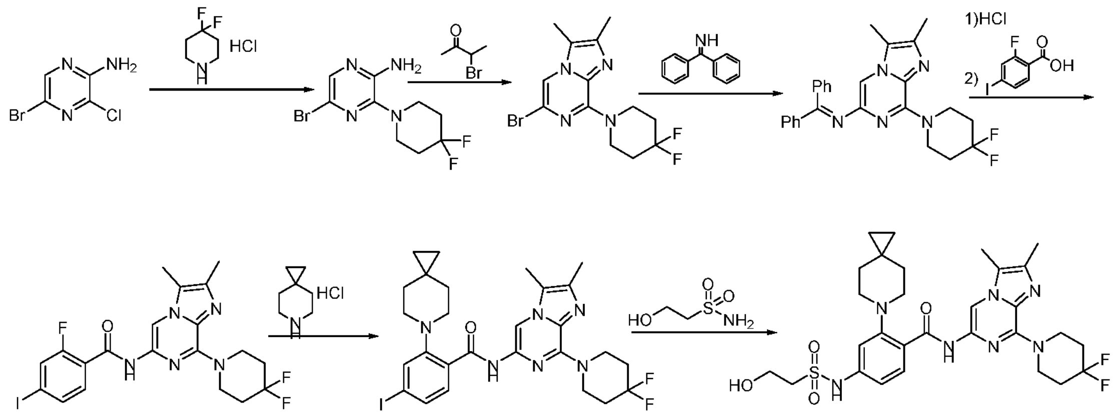


Step 1: To a stirres solution of 4,4-difluoropiperidine hydrochloride (2.27 g, 14.25 mmol), and potassium carbonate (4.02 g, 28.50 mmol) in N-methylpyrrolidone (5 mL) was added 5-bromo-3-chloropyrazin-2-amine (2.00 g, 9.50 mmol) , and the reaction solution was stirred at 130°C for 16 h. the reaction solution was filtered under reduced pressure. the filtrate was poured into the saturated ammonium chloride solution (50 mL), extracted with ethyl acetate (30 mL × 3),The combined organic layer was washed with the saturated aqueous sodium chloride solution (50 mL × 3), dried with anhydrous sodium sulfate, and concentrated to obtain the crude. the crude was purified by flash column chromatography (silica gel, 10-15% gradient of ethyl acetate/petroleum ether) to give 5-bromo-3-(4,4-difluoropiperidin-1-yl) pyrazine-2-amine (2.20 g, 67%) as a brown solid. LCMS (ESI): $[M+H]^+$ = 293.1, 295.1.

Step 2: 5-bromo-3-(4,4-difluoropiperidin-1-yl)pyrazine-2-amine (2.20 g, 6.38 mmol), 3-bromo-2-butanone (1.49 g, 9.57 mmol), and 1,4-dioxane (5 mL) were added into a 100 mL closed reactor, and the reaction solution was stirred at 120°C for 24 h. The reaction solution was poured into water (30 mL), extracted with ethyl acetate (30 mL × 3), The combined organic layer was dried over anhydrous sodium sulfate, and concentrated to obtain the crude. the crude was purified by flash column chromatography (silica gel, 0-10% gradient of ethyl acetate/petroleum ether) to give 6-bromo-8-(4,4-difluoropiperidin-1-yl)-2,3-dimethylimidazo[1,2-a]pyrazine (1.85 g, 85%) as a pale yellow solid. LCMS (ESI): $[M+H]^+$ = 345.1, 347.1.

Step 3: 6-bromo-8-(4,4-difluoropiperidin-1-yl)-2,3-dimethylimidazo[1,2-a]pyrazine (1.90 g, 4.13 mmol), benzophenonimine (1.53 g, 8.26 mmol), 2,2'-bis(diphenylphosphino)-1,1'-binaphthyl (262 mg, 0.41 mmol), tris(dibenzylideneacetone) dipalladium (193 mg, 0.21 mmol), and cesium carbonate (2.74 g, 8.26 mmol) were added to 1,4-dioxane (20 mL), and the reaction solution was stirred at 115°C for 16 h. the reaction solution was filtered under reduced pressure.the filtrate was poured into water (50 mL), extracted with ethyl acetate (30 mL × 3), The combined organic layer was dried over anhydrous sodium sulfate, and concentrated to obtain the crude. The crude was purified by flash column chromatography (silica gel, 0-6% gradient of ethyl acetate/petroleum ether) to give N-(8-(4,4-difluoropiperidin-1-yl)-2,3-dimethylimidazo [1,2-a]pyrazin-6-yl)-1,1-diphen ylmethanimine (990 mg, 54%) as a yellow solid.

Step 4: To a stirred solution of N-(8-(4,4-difluoropiperidin-1-yl)-2,3-dimethylimidazo[1,2-a]pyrazin-6-yl)-1,1-diphen ylmethanimine (500 mg, 1.12 mmol) in dichloromethane (5 mL), was slowly add 4 M HCl in dioxane (1.5 mL), and the reaction solution was stirred at 25°C for 30 min. the reaction solution was concentrated to give the crude. the crude was dissolved in acetonitrile (5 mL), and 2-fluoro-4-iodobenzoic acid (508 mg, 1.91 mmol), N,N-diisopropylethylamine (1.01 g, 7.63 mmol), and 50% solution of 1-propylphosphonic anhydride in ethyl acetate (2.43 g, 3.81 mmol) were added into the reaction solution, which was stirred at 85°C for 16 h. The reaction solution was poured into water (30 mL), extracted

with ethyl acetate (30 mL × 3), The combined organic layer was dried over anhydrous sodium sulfate, and concentrated to obtain the crude. The crude was purified by flash column chromatography (silica gel, 0-9% gradient of ethyl acetate/petroleum ether) to give N-(8-(4,4-difluoropiperidin-1-yl)-2,3-dimethylimidazo[1,2-a]pyrazin-6-yl)-2-fluoro-4 -iodobenzamide (320 mg, 48%). LCMS (ESI): $[M+H]^+$ =530.1. $^1H$ NMR (400 MHz, DMSO-d6) δ 10.44-10.40 (m, 1H), 8.34 (s, 1H), 7.80 (ddd, J = 9.2, 8.1, 1.5 Hz, 1H), 7.70 (ddd, J = 9.8, 8.1, 1.5 Hz, 1H), 7.48 - 7.32 (m, 1H), 4.36 (s, 4H), 2.33 (d, J = 12.4 Hz, 6H), 2.09 - 1.95 (m, 4H).

Step 5: N-(8-(4,4-difluoropiperidin-1-yl)-2,3-dimethylimidazo[1,2-a]pyrazin-6-yl)-2-fluoro-4 -iodobenzamide (320 mg, 0.60 mmol), 6-aza-spiro[2.5]octane hydrochloride (135 mg, 0.91 mmol), and potassium carbonate (256 mg, 1.81 mmol) were added to toluene (5 mL), and the reaction solution was stirred at 130°C for 18 h. The reaction solution was filtered under reduced pressure., The filtrate was poured into saturated ammonium chloride solution (30 mL), extracted with ethyl acetate (30 mL × 3), The combined organic layer was dried over anhydrous sodium sulfate, and concentrated to obtain the crude. The crude was purified by flash column chromatography (silica gel, 5-15% gradient of ethyl acetate/petroleum ether) to give N-(8-(4,4-difluoropiperidin-1-yl)-2,3-dimethylimidazo[1,2-a]pyrazin-6-yl)-4-iodo-2-( 6-azaspiro[2.5]octan-6-yl)benzamide (120 mg, 32%) as a pale yellow solid.

Step 6: N-(8-(4,4-difluoropiperidin-1-yl)-2,3-dimethylimidazo[1,2-a]pyrazin-6-yl)-4-iodo-2-( 6-azaspiro[2.5]octan-6-yl)benzamide (120 mg, 0.19 mmol), 2-hydroxy-1-sulfonamide (37 mg, 0.19 mmol), potassium phosphate (126 mg, 0.58 mmol), cuprous iodide (38 mg, 0.19 mmol) and (1S,2S)-(+)-N,N'-dimethyl-1,2-cyclohexanediamine (56 mg, 0.38 mmol) were added to N,N-dimethylformamide (2 mL). The reaction solution was replaced with nitrogen, and stirred at 115°C for 2 h. The reaction solution was filtered under reduced pressure. , The filtrate was poured into the saturated ammonium chloride solution (30 mL), extracted with ethyl acetate (30 mL × 3),The combined organic layer was washed with the saturated sodium chloride solution (30 mL × 3), dried with anhydrous sodium sulfate, and concentrated to obtain the crude. The crude was purified by preparative HPLC (C18, 25-55% gradient of acetonitrile/water) to give N-(8-(4,4-difluoropiperidin-1-yl)-2,3-dimethylimidazo[1,2-a]pyrazin-6-yl)-4-(2-hydro xyethyl)sulfonamide)-2-(6-azaspiro[2.5]octan-6-yl)benzamide as a white solid. LCMS (ESI): $[M+H]^+$ =618.3. $^1H$ NMR (400 MHz, DMSO-$d_6$) δ 12.90 (s, 1H), 10.21 (s, 1H), 8.46 (s, 1H), 8.09 (d, *J* = 8.7 Hz, 1H), 7.29 (d, *J* = 2.1 Hz, 1H), 7.14 (dd, *J* = 8.6, 2.1 Hz, 1H), 4.95 (s, 1H), 4.41 (t, *J* = 5.6 Hz, 4H), 3.77 (t, *J* = 6.5 Hz, 2H), 3.37 (d, *J* = 6.4 Hz, 2H), 2.99 (t, *J* = 5.3 Hz, 4H), 2.35 (d, *J* = 14.3 Hz, 6H), 2.16 - 2.01 (m, 4H), 1.60 (d, *J* = 111.7 Hz, 4H), 0.42 (s, 4H).

**Example 16: Azaspiro[2.5]octan-6-yl)-4-{(2-hydroxyethylsulfonyl]amino}phenyl)-*N*-[8-(4,4-dif luoropiperidinyl)(4-hydro-1,2,4-triazolo[1,5-a]pyrazin-6-yl)]formamide**

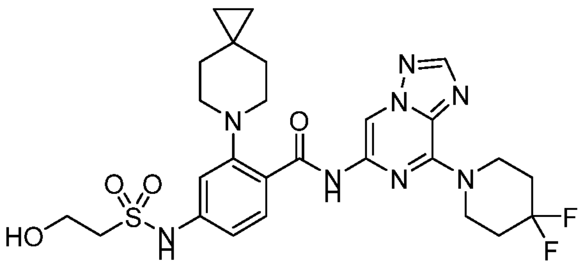

The preparations in **Example 16** follow the synthetic route of **Example 3:** LCMS (ESI): $[M+H]^+$ =591.3; $^1H$ NMR (400 MHz, DMSO) δ 13.39 (s, 1H), 8.91 (s, 1H), 8.42 (s, 1H), 7.90 (d, J = 8.7 Hz, 1H), 7.15 (s, 1H), 7.03 (s, 1H), 6.87 (d, J = 8.3 Hz, 1H), 6.61 (s, 1H), 6.06 (s, 1H), 5.26 (t, J = 4.8 Hz, 1H), 4.32 (m, 4H), 3.67 (t, J = 6.6 Hz, 2H), 3.31 (m, 2H), 3.09 (s, 2H), 2.91 (s, 4H), 2.09 (dd, J = 15.2, 9.5 Hz, 4H), 1.94 (dd, J = 14.4, 6.8 Hz, 4H), 0.35 (s, 4H).

**Example 17: N-(4-(4,4-difluoropiperidin-1-yl)pyrazolo[1,5-a]pyrazin-6-yl)-4-(2-hydroxyethyl)s ulfonamido)-2-(6-azaspiro[2.5]octan-6-yl)benzamide**

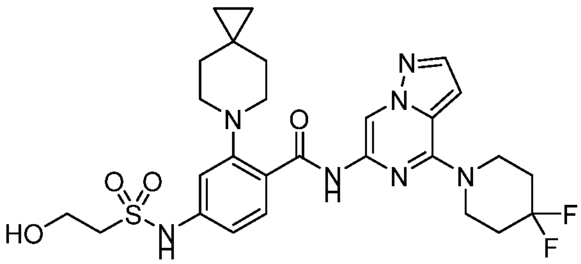

The preparations in **Example 17** follow the synthetic route of **Example 3:** LCMS (ESI): $[M+1]^+$ = 590.0. $^1H$ NMR (400 MHz, DMSO) δ 13.06 (s, 1H), 10.24 (s, 1H), 8.95 (d, *J* = 0.9 Hz, 1H), 8.09 (d, *J* = 8.6 Hz, 1H), 7.98 (d, *J* = 2.4 Hz, 1H), 7.30 (d, *J* = 2.2 Hz, 1H), 7.15 (dd, *J* = 8.7, 2.1 Hz, 1H), 7.06 (d, *J* = 2.7 Hz, 1H), 3.95 (dd, *J* = 7.1, 4.5 Hz, 4H), 3.77 (d, *J* = 6.5 Hz, 3H), 3.36 (t, *J* = 6.5 Hz, 2H), 2.99 (d, *J* = 10.5 Hz, 4H), 2.17 (td, *J* = 13.6, 6.6 Hz, 4H), 1.76 (s, 4H), 0.41 (s, 4H).

**Example 18: N-(8-(4,4-difluoropiperidin-1-yl)-[1,2,4]triazolo[1,5-a]pyridin-6-yl)-4-(2-hydroxye thyl)sulfonamido)-2-(6-azaspiro[2.5]octan-6-yl)benzamide**

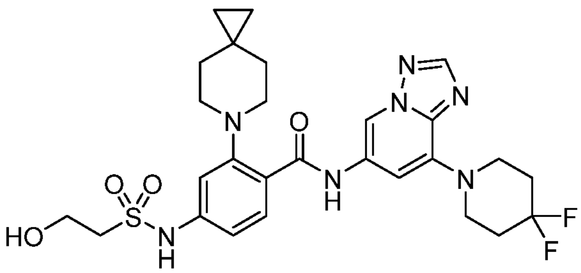

The preparations in **Example 18** follow the synthetic route of **Example 3:** LCMS (ESI): $[M+1]^+$ = 590.0. $^1H$ NMR (400 MHz, DMSO) δ 12.23 - 12.16 (m, 1H), 9.23 (d, *J= 1.6* Hz, 1H), 8.37 (s, 1H), 7.76 (d, *J* = 8.6 Hz, 1H), 7.03 (d, *J* = 2.0 Hz, 1H), 6.95 - 6.85 (m, 2H), 5.99 (s, 1H), 3.81 (t, *J* = 5.6 Hz, 4H), 3.74 (t, *J* = 6.8 Hz, 2H), 3.18 (d, *J* = 13.4 Hz, 3H), 2.97 (t, *J* = 5.2 Hz, 4H), 2.21 - 2.12 (m, 4H), 1.58 (t, *J* = 5.2 Hz, 4H), 0.36 (s, 4H).

**Example 19: N-(8-(4,4-difluoropiperidin-1-yl)-[1,2,4]triazolo[4,3-a]pyrazin-6-yl)-4-((2-hydroxy ethyl)sulfonamido)-2-(6-azaspiro[2.5]oct-6-yl)benzamide**

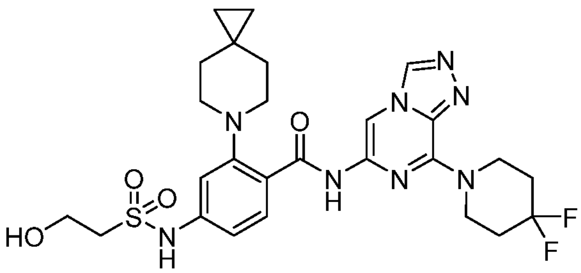

The preparations in **Example 19** follow the synthetic route of **Example 3:** LCMS (ESI): $[M+H]^+$ =591.1. $^1H$ NMR (400 MHz, DMSO-$d_6$) δ 13.08 (s, 1H), 10.20 (s, 1H), 9.37 (d, *J* = 1.1 Hz, 1H), 8.79 (d, *J* = 1.1 Hz, 1H), 8.08 (d, *J* = 8.6 Hz, 1H), 7.29 (d, *J* = 2.2 Hz, 1H), 7.15 (dd, *J* = 8.8, 1.9 Hz, 1H), 4.97 (s, 1H), 4.45 (s, 4H), 3.76 (t, *J* = 6.4 Hz, 2H), 3.17 (d, *J* = 5.1 Hz, 2H), 2.98 (t, *J* = 5.2 Hz, 4H), 2.22 - 2.11 (m, 4H), 1.74 (s, 4H), 0.42 (s, 4H).

**Example 20: N-(1-(4,4-difluoropiperidin-1-yl)pyrrolo[1,2-c]pyrimidin-3-yl)-4-(2-hydroxyethyl sulfonamido)-2-(6-azaspiro[2.5]octan-6-yl)benzamide**

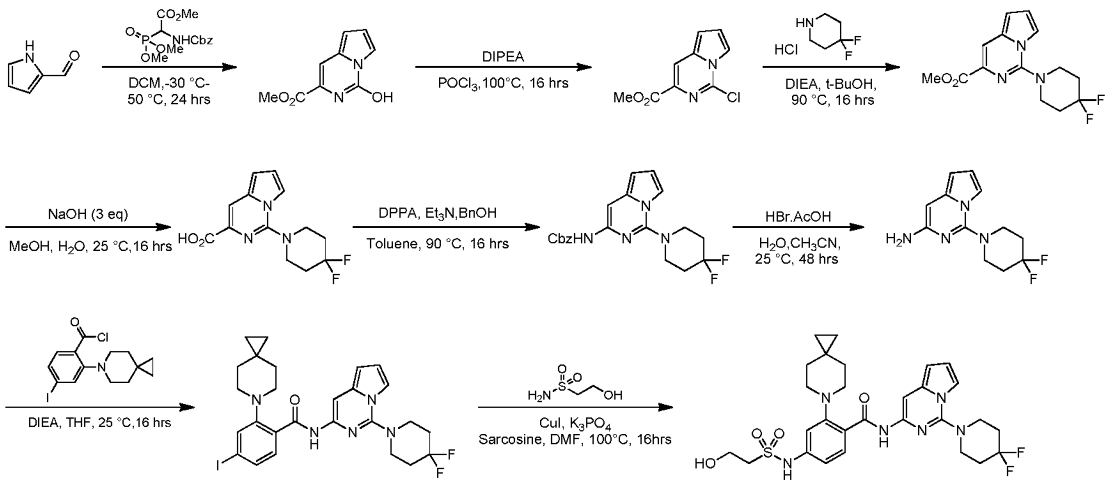


Step 1: To a solution of (±)-benzyloxycarbonyl-α-phosphonoglycine trimethyl ester (17.8 g, 53.7 mmol) in dichloromethane (170 mL) was added tetramethylguanidine (6.66 g, 57.8 mmol), and The mixture was stirred at 25°C for 15 min. Then, a solution of 2-pyrrolecarbaldehyde (5.00 g, 52.5 mmol) in dichloromethane (12 mL) was slowly added to the mixture at -30°C and stirred for 45 min. The mixture was heated to 50°C and stirred for 24 h. water (100 mL) was added to the reaction mixture, which was thenextracted with dichloromethane (100 mL × 2). The combined organic layer was dried over anhydrous sodium sulfate, filtered and concentrated under reduced pressure to give the residue. The residue was purified by flash column chromatography (silica gel, 0-15% gradient of ethyl acetate/petroleum ether) to give methyl 1-hydroxypyrrolo[1,2-c]pyrimidine-3-carboxylate (5.00 g, 52.5 mmol, yield: 49%) as a yellow solid. LCMS (ESI): $[M+H]^+$ =193.2.

Step 2: To a solution of methyl 1-hydroxypyrrolo[1,2-c]pyrimidine-3-carboxylate (5.00 g, 26.01 mmol) in phosphorus oxychloride (50 mL) was added N,N-diisopropylethylamine (10.1 g, 78.0 mmol), and the reactionmixture was stirred at 100°C for 16 h. the reaction solution was concentrated. water (100 mL) was added to the reaction mixture, which was thenextracted with ethyl acetate (100 mL × 3). The combined organic layer was dried over anhydrous sodium sulfate, filtered and concentrated under reduced pressure to give the residue. The residue was purified by flash column chromatography (silica gel, 0-15% gradient of ethyl acetate/petroleum ether) to give methyl 1-chloropyrrolo[1,2-c]pyrimidine-3-carboxylate (1.20 g, 26.0 mmol, yield: 21.9%) as a yellow solid. LCMS (ESI): $[M+H]^+$ = 210.9.

Step 3: To a solution of methyl 1-chloropyrrolo[1,2-c]pyrimidine-3-carboxylate (1.20 g, 5.70 mmol) and 4,4-difluoropiperidine hydrochloride (1.08 g, 6.84 mmol) in tert-butanol (24 mL) was added triethylamine (1.73 g, 17.1 mmol). The mixture was heated to 90°C and stirred for 16 h. water (20 mL) was added to the reaction solution, which was then extracted with ethyl acetate (20 mL × 3). The combined organic layer was dried over anhydrous sodium sulfate, filtered and concentrated under reduced pressure to give the residue. The residue was purified by flash column chromatography (silica gel, 0-10% gradient of ethyl acetate/petroleum ether) to give methyl 1-(4,4-difluoropiperidin-1-yl)pyrrolo[1,2-c]pyrimidine-3-carboxylate (1.70 g, 5.69 mmol, yield: 96%) as a yellow solid. LCMS (ESI): $[M+H]^+$ = 295.9.

Step 4: To a solution of methyl 1-(4,4-difluoropiperidin-1-yl)pyrrolo[1,2-c]pyrimidine-3-carboxylate (1.70 g, 5.76 mmol) in methanol (17 mL) and water (17 mL) was added sodium hydroxide (0.69 g, 17.2 mmol). The mixture was stirred at 25°C for 16 h. the reaction solution was concentrated, and the pH of the remaining aqueous phase was adjusted to 4 with 1 N HCl and extracted with ethyl acetate (20 mL×3). The combined organic layer was dried over anhydrous magnesium sulfate, filtered and concentrated under reduced pressure to give the residue 1-(4,4-difluoropiperidin-1-yl)pyrrolo[1,2-c]pyrimidine-3-carboxylic acid (1.70 g, 5.75 mmol, yield: 100%). LCMS (ESI): $[M+H]^+$ = 281.9.

Step 5: To a solution of 1-(4,4-difluoropiperidin-1-yl)pyrrolo[1,2-c]pyrimidine-3-carboxylic acid (1.00 g, 3.13 mmol), benzyl alcohol (0.52 g, 4.80 mmol) and triethylamine (0.65 g, 6.40 mmol) in toluene (9 mL) was added diphenylphosphoryl azide (1.32 g, 4.80 mmol). The mixture was heated to 90°C and stirred for 16 h. water (10 mL) was added to the reaction solution, which was thenextracted with ethyl acetate (10 mL × 2). The combined organic layer was dried over anhydrous sodium sulfate, filtered and concentrated under reduced pressure to give the residue. The residue was purified by flash column chromatography (silica gel, 0-10% gradient of ethyl acetate/petroleum ether) to give benzyl (1-(4,4-difluoropiperidin-1-yl)pyrrolo[1,2-c]pyrimidin-3-yl)carbamate (0.70 g, 3.19 mmol, yield: 56.6%) as a yellow solid. LCMS (ESI): $[M+H]^+$ = 387.0.

Step 6: To a solution of benzyl (1-(4,4-difluoropiperidin-1-yl)pyrrolo[1,2-c]pyrimidin-3-yl)carbamate (600 mg, 1.55 mmol) in acetonitrile (3 mL) and water (3 mL) was added a solution of hydrobromic acid in acetic acid (6 mL, 35%). The mixture was stirred at 25°C for 48 h. the reaction solution was concentrated. The residue was purified by preparative HPLC (C18, 0-60% gradient of acetonitrile/water) to give 1-(4,4-difluoropiperidin-1-yl)pyrrolo[1,2-c]pyrimidin-3-amine (200 mg, 1.55 mmol, yield: 51%) as a yellow solid compound. LCMS (ESI): $[M+H]^+$ =253.2.

Step 7: To a solution of 1-(4,4-difluoropiperidin-1-yl)pyrrolo[1,2-c]pyrimidin-3-amine (200 mg, 0.79 mmol) and 4-iodo-2-(6-azaspiro[2.5]octan-6-yl)benzoyl chloride (357 mg, 0.95 mmol) in THF (4 mL) was added the solution of triethylamine (0.65 g, 6.40 mmol) in toluene (9 mL) and N,N-diisopropylethylamine (313 mg, 2.38 mmol) . The mixture was stirred at 25°C for 16 h. water (10 mL) was added to the reaction solution, which was thenextracted with ethyl acetate (10 mL × 2). The combined organic layer was dried over anhydrous sodium sulfate, filtered and concentrated under reduced pressure to give the residue. The residue was purified by flash column chromatography (silica gel, 0-10% gradient of ethyl acetate/petroleum ether) to give N-(1-(4,4-difluoropiperidin-1-yl)pyrrolo[1,2-c]pyrimidin-3-yl)-4-iodo-2-(6-azaspiro[2. 5]octan-6-yl)benzamide (255 mg, 0.79 mmol, yield: 54.3%) as a yellow solid. LCMS (ESI): $[M+H]^+$ = 592.3.

Step 8: To a solution of 2-hydroxyethanesulfonamide (67.4 mg, 0.54 mmol), 2-(methylamino)acetic acid (36.9 mg, 0.41 mmol), and potassium phosphate (358 mg, 1.66 mmol) in N,N-dimethylformamide (5 mL) was added cuprous iodide (40.2 mg, 0.21 mmol) . The mixture was stirred at 50°C for 5 min. Then, N-(1-(4,4-difluoropiperidin-1-yl)pyrrolo[1,2-c]pyrimidin-3-yl)-4-iodo-2-(6-azaspiro[2. 5]octan-6-yl)benzamide (245 mg, 0.41 mmol) was added to the reaction mixture at 50°C, and the mixture was heated to 100°C and stirred at 100°C for 16 h under nitrogen atmosphere. water (10 mL) was added to the reaction solution, which was thenextracted with ethyl acetate (10 mL × 2). The combined organic layer was dried over anhydrous sodium sulfate, filtered and concentrated under reduced pressure to give the residue. The residue was purified by preparative HPLC (C18, 0-60% gradient of acetonitrile/water) to give N-(1-(4,4-difluoropiperidin-1-yl) pyrrolo[1,2-c]pyrimidin-3-yl)-4-(2-hydroxyethylsulf onamido)-2-(6-azaspiro[2.5]octan-6-yl)benzamide (29 mg, 0.41 mmol, yield: 11%) as a yellow solid compound. LCMS (ESI): $[M+H]^+$ = 589.5. $^1$H NMR (400 MHz, DMSO-$d_6$) δ ppm 13.15 - 12.87 (m, 1H), 10.39 - 9.98 (m, 1H), 8.16 - 8.02 (m, 1H), 7.98 - 7.82 (m, 1H), 7.46 - 7.36 (m, 1H), 7.31 - 7.23 (m, 1H), 7.20 - 7.05 (m, 1H), 6.94 - 6.74 (m, 1H), 6.49 - 6.28 (m, 1H), 5.05 - 4.81 (m, 1H), 3.81 - 3.70 (m, 2H), 3.57 - 3.45 (m, 4H), 3.42 - 3.35 (m, 2H), 3.06 - 2.91 (m, 4H), 2.35 - 2.19 (m, 4H), 2.05 - 1.49 (m, 4H), 0.49 - 0.32 (m, 4H)

**Example 21: N-(5-(4,4-difluoropiperidin-1-yl)imidazolo[1,2-c]pyrimidin-7-yl)-4-((2-hydroxyet hyl)sulfonamide)-2-(6-azaspiro[2.5]octan-6-yl)benzamide**

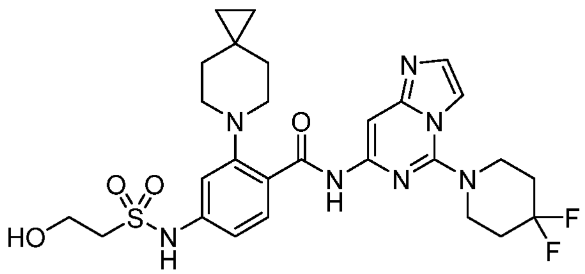

The preparations in **Example 21** follow the synthetic route of **Example 3:** LCMS (ESI): $[M+H]^+$ = 590.3; $^1$H NMR (400 MHz, DMSO) δ 13.86 (s, 1H), 10.37 (s, 1H), 8.13 (s, 1H), 8.11 (s, 2H), 8.03 (s, 1H), 7.32-7.36 (m, 1H), 7.16-7.22 (m, 1H), 3.77 (t, *J* = 6.4 Hz, 2H), 3.66-3.73 (m, 4H), 2.96-3.04 (m, 4H), 2.22-2.32 (m, 4H), 1.65-1.83 (s, 4H), 1.54 (d, *J* = 6.8 Hz, 1H), 1.20-1.34 (m, 2H), 0.42 (s, 4H).

**Example 22: N-(7-(4,4-difluoropiperidin-1-yl)imidazolo[1,2-c]pyrimidin-5-yl)-4-((2-hydroxyet hyl)sulfamoyl)-2-(6-azaspiro[2.5]octan-6-yl)benzamide**

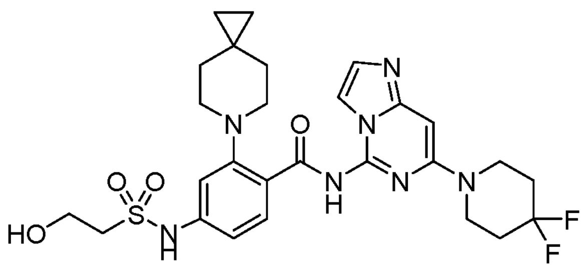

The preparations in **Example 22** follow the synthetic route of **Example 3:** LCMS(ESI): $[M+H]^+$=590.4. $^1$H-NMR (400 MHz, DMSO) δ 12.27 (s, 1H), 10.13 (s, 1H), 7.79 - 7.61 (m, 2H), 7.40 (d, *J* = 1.5 Hz, 1H), 7.05 (d, *J* = 7.2 Hz, 2H), 6.53 (s,

1H), 4.96 (s, 1H), 3.77 (t, *J* = 6.5 Hz, 2H), 3.46 - 3.32 (m, 6H), 2.80 (s, 4H), 1.89 (d, *J* = 16.2 Hz, 4H), 1.28 (s, 4H), 0.25 (s, 4H).

**Example 23: N-(5-(4,4-difluoropiperidin-1-yl)imidazo[1,2-c]pyrimidin-7-yl)-6-((2-hydroxyethy l)sulfonamido)-4-(6-azaspiro[2.5]octan-6-yl)nicotinamide**

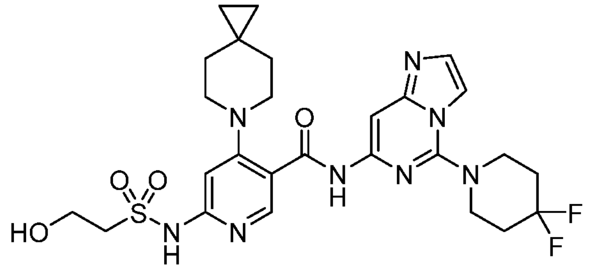

The preparations in **Example 23** follow the synthetic route of **Example 3:** LCMS (ESI): $[M+H]^+$=633.3.

**Example 24: N-(5-(4,4-difluoropiperidin-1-yl)-[1,2,4]triazolo[4,3-c]pyrimidin-7-yl)-4-(2-hydro xyethylsulfonamido)-2-(6-azaspiro[2.5]octan-6-yl)benzamide**

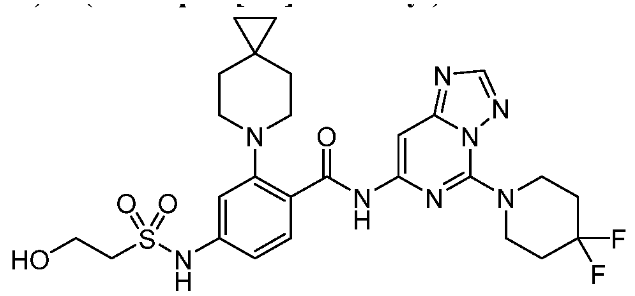

The preparations in **Example 24** follow the synthetic route of **Example 3:** LCMS (ESI): $[M+H]^+$ =591.5. $^1$H NMR (400 MHz, DMSO-d6) δ ppm 13.37 (s, 1H), 10.27 (br s, 1H), 8.42 (s, 1H), 8.11 (d, J = 8.6 Hz, 1H), 7.91 (s, 1H), 7.30 (d, J = 1.9 Hz, 1H), 7.16 (dd, J = 1.9, 8.7 Hz, 1H), 4.96 (br s, 1H), 4.23 (br t, J = 5.2 Hz, 4H), 3.77 (br s, 2H), 3.40 - 3.35 (m, 2H), 3.00 (br s, 4H), 2.31 - 2.11 (m, 4H), 2.01 - 1.30 (m, 4H), 0.41 (s, 4H)

**Example 25: (2-(6-azaspiro[2.5]oct-6-yl)-5-fluoro-4-{[(2-hydroxyethyl)sulphonyl]amino}pheny l)-N-[5-(4,4-difluoropiperidinyl)(4-hydro-1,2,4-triazolo[4,3-e]pyrimidin-7-yl)]for mamide**

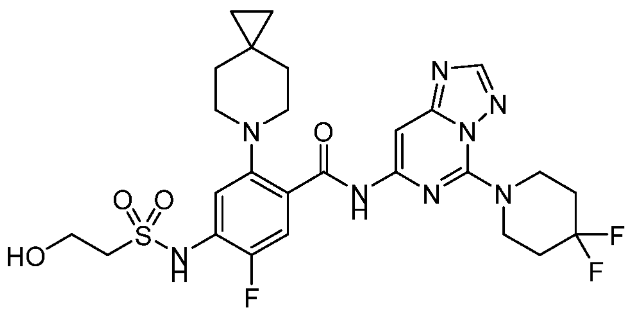

The preparations in **Example 25** follow the synthetic route of **Example 3:** LCMS (ESI): $[M+H]^+$ =609.2. $^1$H NMR (400 MHz, DMSO-d6) δ ppm 13.69 (s, 1H), 10.12 (br s, 1H), 8.44 (s, 1H), 7.97 - 7.84 (m, 2H), 7.61 (d, *J* = 7.2 Hz, 1H), 5.17 - 4.80 (m, 1H), 4.31 - 4.17 (m, 4H), 3.88 - 3.76 (m, 2H), 3.43 - 3.40 (m, 2H), 3.05 - 2.95 (m, 4H), 2.28 - 1.51 (m, 8H), 0.42 (s, 4H)

**Example 26: N-(5-(4,4-difluoropiperidin-1-yl)-8-fluoro-[1,2,4]triazolo[4,3-c]pyrimidin-7-yl)-4-( 2-hydroxyethylsulfonamido)-2-(6-azaspiro[2.5]octan-6-yl)benzamide**

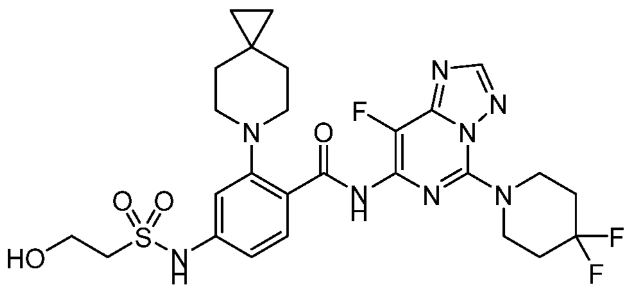

The preparations in **Example 26** follow the synthetic route of **Example 3:** LCMS (ESI): $[M+H]^+$ = 609.2. $^1H$ NMR (400 MHz, DMSO-$d_6$) δ ppm 12.68 (s, 1H), 10.12 (s, 1H), 8.55 (s, 1H), 7.94 (br d, *J* = 8.4 Hz, 1H), 7.24 (br s, 1H), 7.10 (dd, *J* = 1.6, 8.4 Hz, 1H), 5.11 (s, 1H), 4.10 (br s, 4H), 3.76 (t, *J* = 6.4 Hz, 2H), 3.33 - 3.31 (m, 2H), 3.03 - 2.91 (m, 4H), 2.26 - 2.08 (m, 4H), 1.80 - 1.55 (m, 4H), 0.39 (s, 4H)

**Example 27: N-(5-(4,4-difluoropiperidin-1-yl)-3-methyl-[1,2,4]triazolo[4,3-c]pyrimidin-7-yl)-4-(2-hydroxyethylsulfonamido)-2-(6-azaspiro[2.5]octan-6-yl)benzamide**

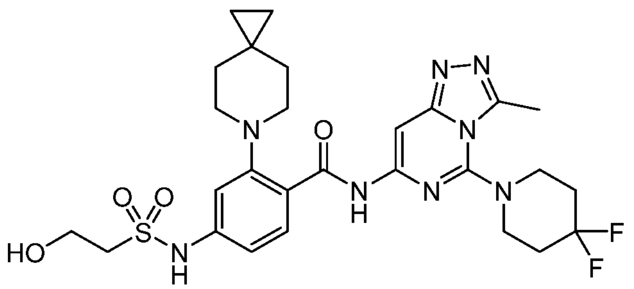

The preparations in **Example 27** follow the synthetic route of **Example 3:** LCMS (ESI): $[M+H]^+$ = 605.0. $^1H$ NMR (400 MHz, DMSO-$d_6$) δ ppm 13.34 (s, 1H), 10.31 (s, 1H), 8.10 (d, *J* = 8.4 Hz, 1H), 7.81 (s, 1H), 7.30 (s, 1H), 7.15 (br d, *J* = 8.4 Hz, 1H), 5.29 - 4.67 (m, 1H), 4.21 (br s, 4H), 3.77 (br t, *J* = 6.4 Hz, 2H), 3.39 - 3.38 (m, 2H), 2.99 (br s, 4H), 2.47 - 2.40 (m, 3H), 2.25 - 2.14 (m, 4H), 1.92 - 1.58 (m, 4H), 0.41 (s, 4H)

**Example 28: N-(7-(4,4-difluoropiperidin-1-yl)thieno[2,3-c]pyridin-5-yl)-4-(2-hydroxyethylsulf onamido)-2-(6-azaspiro[2.5]octan-6-yl)benzamide**

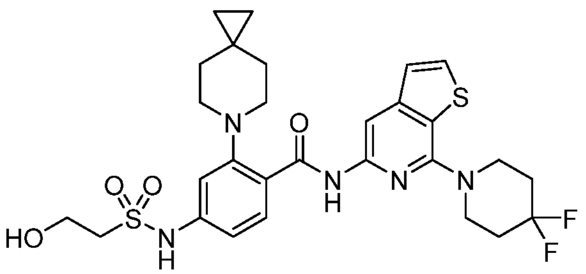

The preparations in **Example 28** follow the synthetic route of **Example 3:** LCMS (ESI): $[M+H]^+$ = 606.5. $^1H$ NMR (400 MHz, DMSO-d6) δ ppm 12.91 (s, 1H), 10.21 (s, 1H), 8.30 (s, 1H), 8.09 (d, J = 8.6 Hz, 1H), 8.01 (d, J = 5.4 Hz, 1H), 7.51 (d, J = 5.4 Hz, 1H), 7.27 (d, J = 1.9 Hz, 1H), 7.13 (dd, J = 2.0, 8.6 Hz, 1H), 3.82-3.80 (m, 4H), 3.76 (t, J = 6.5 Hz, 2H), 3.37 (br

d, J = 6.5 Hz, 2H), 3.05-2.95 (m, 4H), 2.25 - 2.10 (m, 4H), 2.06 - 1.40 (m, 4H), 0.47 - 0.35 (m, 4H)

**Example 29: N-(7-(4,4-difluoropiperidin-1-yl)furo[2,3-c]pyridin-5-yl)-4-(2-hydroxyethylsulfon amido)-2-(6-azaspiro[2.5]octan-6-yl)benzamide**

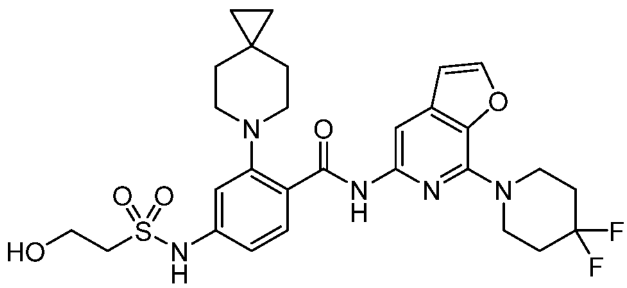

The preparations in **Example 29** follow the synthetic route of **Example 3:** LCMS (ESI): $[M+H]^+$ = 590.5. $^1H$ NMR (400 MHz, DMSO-$d_6$) δ ppm 12.82 (s, 1H), 10.18 (s, 1H), 8.12 - 8.05 (m, 2H), 7.99 (s, 1H), 7.27 (d, *J* = 1.9 Hz, 1H), 7.12 (dd, *J* = 1.8, 8.6 Hz, 1H), 6.99 (d, *J*= 2.0 Hz, 1H), 4.95 (br d, *J* = 1.4 Hz, 1H), 3.97 (br t, *J* = 5.6 Hz, 4H), 3.76 (br t, *J* = 5.9 Hz, 2H), 3.37 - 3.36 (m, 2H), 2.98 (br s, 4H), 2.22 - 2.03 (m, 4H), 1.99 - 1.37 (m, 4H), 0.40 (s, 4H)

**Example 30: N-(7-(4,4-difluoropiperidin-1-yl)benzofuran-5-yl)-4-(2-hydroxyethylsulfonamido) -2-(6-azaspiro [2.5]octan-6-yl)benzamide**

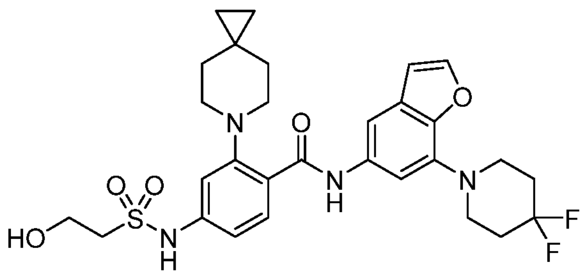

The preparations in **Example 30** follow the synthetic route of **Example 3:** LCMS (ESI): [M+H]+ = 589.3. $^1H$ NMR (400 MHz, DMSO-d6) δ ppm 11.68 (s, 1H), 10.09 (s, 1H), 7.96 (d, *J* = 2.0 Hz, 1H), 7.84 (d, *J* = 1.2 Hz, 2H), 7.17 (d, *J* = 2.0 Hz, 1H), 7.10 (d, *J* = 1.6 Hz, 1H), 7.04 (dd, *J* = 2.0, 8.4 Hz, 1H), 6.96 (d, *J* = 2.0 Hz, 1H), 5.33 - 4.50 (m, 1H), 3.80 - 3.72 (m, 2H), 3.48 - 3.44 (m, 4H), 3.33 - 3.29 (m, 2H), 3.01 - 2.95 (m, 4H), 2.23 - 2.13 (m, 4H), 1.60 - 1.53 (m, 4H), 0.35 (s, 4H)

**Example 31: N-(4-(4,4-difluoropiperidin-1-yl)furo[3,2-c]pyridin-6-yl)-4-(2-hydroxyethylsulfon amido)-2-(6-azaspiro[2.5]octan-6-yl)benzamide**

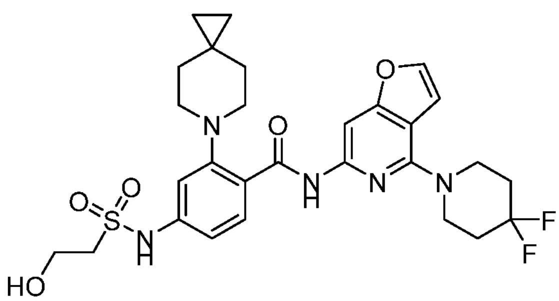

The preparations in **Example 31** follow the synthetic route of **Example 3:** LCMS (ESI): [M+H]+ =590.5. $^1H$ NMR

(400 MHz, DMSO-d6) δ ppm 12.98 (s, 1H), 10.22 (br s, 1H), 8.08 (d, J = 8.8 Hz, 1H), 7.95 (d, J= 0.8 Hz, 1H), 7.91 (d, J = 2.4 Hz, 1H), 7.27 (d, J = 2.0 Hz, 1H), 7.18 (d, J = 1.2 Hz, 1H), 7.12 (dd, J = 2.0, 8.6 Hz, 1H), 5.14 - 4.79 (m, 1H), 3.87 - 3.81 (m, 4H), 3.78 - 3.73 (m, 2H), 3.38 - 3.35 (m, 2H), 3.02 - 2.94 (m, 4H), 2.16 - 2.05 (m, 4H), 1.99 - 1.25 (m, 4H), 0.40 (s, 4H)

**Example 32: N-(4-(4,4-difluoropiperidin-1-yl)furo[3,2-d]pyrimidin-2-yl)-4-((2-hydroxyethyl)su lfanilamide)-2-(6-azaspiro[2.5]octan-6-yl)benzamide**

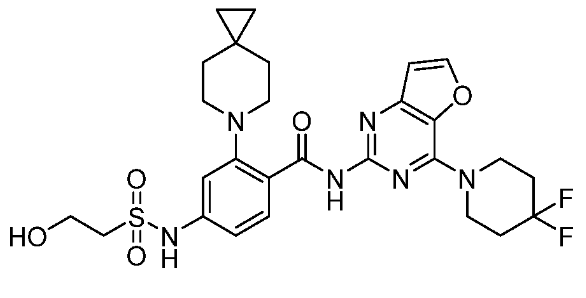

The preparations in **Example 32** follow the synthetic route of **Example 3:** LCMS (ESI): $[M+H]^+$ =591.4. $^1H$ NMR (400 MHz, DMSO-$d_6$) δ 12.69 (s, 1H), 9.90 (s, 1H), 8.24 (d, J = 2.2 Hz, 1H), 7.97 (d, J = 8.6 Hz, 1H), 7.22 (s, 1H), 7.09 (dd, J = 8.6, 2.1 Hz, 1H), 6.96 (d, J = 2.2 Hz, 1H), 5.47 (s, 1H), 4.07 (t, J = 5.7 Hz, 4H), 3.76 (t, J = 6.5 Hz, 2H), 3.35 (d, J = 6.7 Hz, 2H), 2.96 (d, J = 5.5 Hz, 4H), 2.13 (s, 4H), 1.85 - 1.43 (m, 4H), 0.38 (s, 4H).

**Example 33: N-(4-chloro-7-(4,4-difluoropiperidin-1-yl)furo[2,3-c]pyridin-5-yl)-4-(2-hydroxyet hylsulfonamido)-2-(6-azaspiro[2.5]octan-6-yl)benzamide**

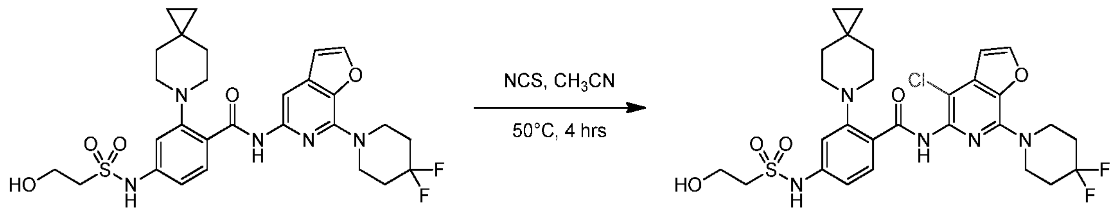


Step 1: To a solution of N-(7-(4,4-difluoropiperidin-1-yl)furo[2,3-c]pyridin-5-yl)-4-(2-hydroxyethylsulfonami do)-2-(6-azaspiro[2.5]octan-6-yl)benzamide (30.0 mg, 0.05 mmol) in acetonitrile (1.00 mL) was added N-chlorosuccinimide (8.15 mg, 0.06 mmol). The mixture was heated to 50°C and stirred for 4 h. water (5 mL) was added to the reaction mixture which was thenextracted with ethyl acetate (5 mL × 3). The combined organic layer was dried over anhydrous magnesium sulfate, filtered and concentrated under reduced pressure to give the residue, which was purified by preparative HPLC (C18, 18-58% gradient of water (formic acid)/acetonitrile) to give N-(4-chloro-7-(4,4-difluoropiperidin-1-yl)furo[2,3-c]pyridin-5-yl)-4-(2-hydroxyethyls ulfonamido)-2-(6-azaspiro[2.5]octan-6-yl)benzamide (8.00 mg, 0.05 mmol, yield: 25%) as a white solid. LCMS (ESI): $[M+H]^+$ = 624.2. $^1H$ NMR (400 MHz, DMSO-$d_6$) δ ppm 11.72 (s, 1H), 10.13 (br s, 1H), 8.26 (d, J = 2.1 Hz, 1H), 7.90 (br d, J = 8.3 Hz, 1H), 7.21 (br s, 1H), 7.14 - 6.99 (m, 2H), 4.95(br s, 1H), 4.08 - 3.85 (m, 4H), 3.82 - 3.72 (m, 2H), 3.43 - 3.37 (m, 2H), 3.07 - 2.95 (m, 4H), 2.18 - 2.02 (m, 4H), 1.76 - 1.55 (m, 4H), 0.37 (s, 4H)

**Example 34: 4-(4,4-Difluoropiperidinyl)-3-methyl-3H-imidazopyridine-6-yl-4-hydroxyethylsul fonamido-2-(2-hydroxyethyl)benzamide**

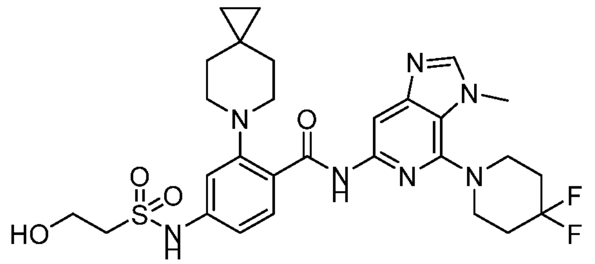

The preparations in **Example 34** follow the synthetic route of **Example 3:** LCMS (ESI) m/z: 604[M+H]$^+$; $^1$H NMR (400 MHz, DMSO) δ 13.05 (s, 1H), 10.18 (s, 1H), 8.24 (d, *J* = 18.6 Hz, 2H), 8.10 (d, *J* = 8.6 Hz, 1H), 7.27 (d, *J* = 2.2 Hz, 1H), 7.13 (dd, *J* = 8.6, 2.1 Hz, 1H), 4.95 (s, 1H), 4.04 (s, 3H), 3.76 (q, *J* = 5.4, 4.7 Hz, 2H), 3.38 - 3.33 (m, 5H), 3.17 (d, *J* = 5.2 Hz, 1H), 2.99 (t, *J* = 5.2 Hz, 5H), 2.22 (td, *J* = 13.6, 12.7, 6.6 Hz, 4H), 1.78 (s, 4H), 0.39 (s, 5H).

**Example 35: N-(4-(4,4-difluoropiperidin-1-yl)-1-methyl-1H-imidazolo[4,5-c]pyridin-6-yl)-4-((2 -hydroxyethyl)sulfonamido)-2-(6-azaspiro[2.5]octan-6-yl)benzamide**

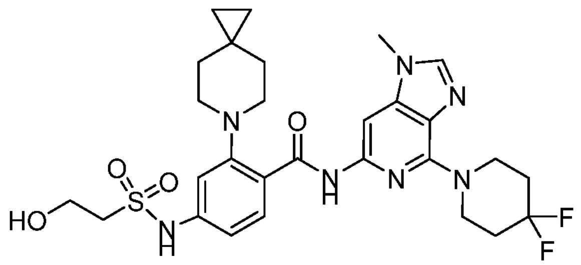

The preparations in **Example 35** follow the synthetic route of **Example 3:** LCMS (ESI): [M+H]$^+$ = 604.4. $^1$H NMR (400 MHz, DMSO) δ 12.71 (s, 1H), 10.18 (s, 1H), 8.10 - 8.02 (m, 2H), 7.86 (s, 1H), 7.26 (d, *J* = 2.2 Hz, 1H), 7.13 (dd, *J* = 8.6, 2.1 Hz, 1H), 4.94 (s, 1H), 4.29 (t, *J* = 5.7 Hz, 4H), 3.77 (s, 5H), 3.36 (t, *J* = 6.5 Hz, 2H), 2.99 (s, 4H), 2.12 - 1.98 (m, 4H), 1.74 (s, 4H), 0.40 (s, 4H).

**Example 36: 2-(4-Cyclopropylpiperazin-1-yl)-N-(2-(4,4-difluoropiperidin-1-yl)-6-methylpyrim idin-4-yl)-4-((2-hydroxyethyl)sulfonamido)benzamide**

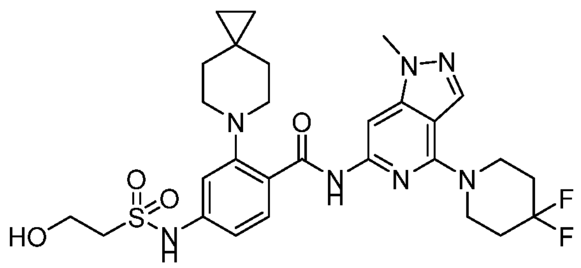

The preparations in **Example 36** follow the synthetic route of **Example 3:** LCMS (ESI) m/z: 604[M+H]$^+$; $^1$H NMR (400 MHz, DMSO) δ 12.80 (s, 1H), 8.24 (d, *J* = 1.0 Hz, 1H), 8.08 (d, *J* = 8.6 Hz, 1H), 7.79 (d, *J* = 0.8 Hz, 1H), 7.26 (d, *J* = 2.2 Hz, 1H), 7.13 (dd, *J* = 8.6, 2.1 Hz, 1H), 4.99 (s, 1H), 3.97 (dd, *J* = 6.8, 4.6 Hz, 4H), 3.92 (s, 3H), 3.77 (t, *J* = 6.4 Hz, 2H), 3.36 (t, *J* = 6.4 Hz, 2H), 2.98 (d, *J* = 5.6 Hz, 4H), 2.10 (dt, *J* = 14.8, 5.5 Hz, 4H), 1.23 (s, 1H), 0.39 (s, 4H).

**Example 37: N-(7-(4,4-difluoropiperidin-1-yl)-1-methyl-1H-pyrrolo[2,3-c]pyridin-5-yl)-4-(2-hy droxyethyl)sulfamoyl)-2-(6-azaspiro[2.5]octan-6-yl)benzamide**

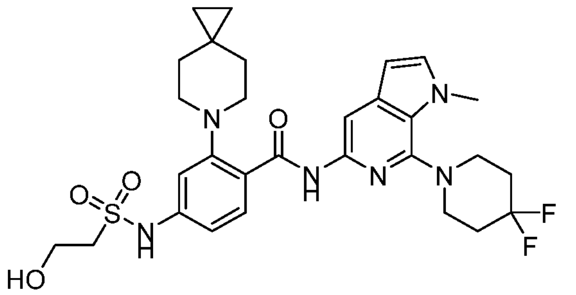

The preparations in **Example 37** follow the synthetic route of **Example 3:** LCMS (ESI): $[M+H]^+$ = 603.4. $^1$H NMR (400 MHz, DMSO) δ 12.86 (s, 1H), 10.15 (s, 1H), 8.16 (s, 1H), 8.08 (d, *J* = 8.4 Hz, 1H), 7.45 (d, *J* = 3.2 Hz, 1H), 7.26 (d, *J* = 2.4 Hz, 1H), 7.12 (dd, *J* = 8.4, 2.0 Hz, 1H), 6.46 (d, *J* = 3.2 Hz, 1H), 4.94 (s, 1H), 4.05 (s, 3H), 3.76 (t, *J* = 6.4 Hz, 2H), 3.35 (t, *J* = 6.4 Hz, 2H), 3.30 (d, *J* = 6.4 Hz, 4H), 2.98 (t, *J* = 5.2 Hz, 4H), 2.25-2.08 (m, 4H), 1.75 (s, 4H), 0.39 (s, 4H).

**Example 38: 4-(4,4-Difluoropiperidinyl)-1-methylpyrrole-3,2-pyridin-6-yl)-4-hydroxyethylsulf onamido-2-(6-azaspiro[2.5]octan-6-yl)benzamide**

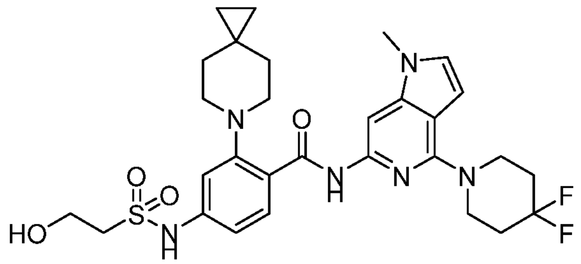

The preparations in **Example 38** follow the synthetic route of **Example 3:** LCMS (ESI): $[M+1]^+$ = 603. $^1$H NMR (400 MHz, DMSO) δ 12.79 (s, 1H), 10.27 (s, 1H), 7.96 (d, *J* = 8.6 Hz, 1H), 7.90 (d, *J* = 0.8 Hz, 1H), 7.20 (d, *J* = 3.2 Hz, 1H), 7.07 (d, *J* = 2.0 Hz, 1H), 6.93 (dd, *J* = 8.6, 2.0 Hz, 1H), 6.55 (dd, *J* = 3.2, 0.8 Hz, 1H), 5.80 (s, 2H), 3.87 - 3.77 (m, 4H), 3.72 (d, *J* = 9.8 Hz, 5H), 3.17 (t, *J* = 6.6 Hz, 3H), 2.97 (t, *J* = 5.2 Hz, 4H), 2.34 (s, 4H), 2.09 (ddd, *J* = 14.3, 7.6, 3.5 Hz, 4H), 0.38 (s, 4H).

**Example 39: N-(4-(4,4-difluoropiperidin-1-yl)-[1,2,5]thiadiazolo[3,4-c]pyridin-6-yl)-4-(2-hydro xyethyl)sulfamoyl)-2-(6-azaspiro[2.5]octan-6-ylbenzamide**

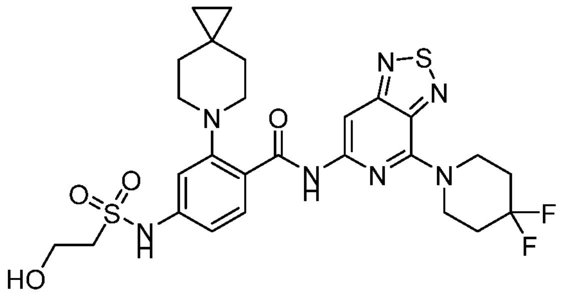

The preparations in **Example 39** follow the synthetic route of **Example 3:** LCMS (ESI): $[M+H]^+$ = 608.3. $^1$H NMR (400 MHz, DMSO) δ 12.92 (s, 1H), 10.23 (s, 1H), 8.10 (d, *J* = 8.4 Hz, 1H), 8.06 (s, 1H), 7.29 (d, *J* = 2.0 Hz, 1H), 7.14 (dd, *J* = 8.4, 2.0 Hz, 1H), 4.95 (t, *J* = 5.6 Hz, 1H), 4.43 (t, *J* = 5.6 Hz, 4H), 3.77 (q, *J* = 6.4 Hz, 2H), 3.37 (t, *J* = 6.4 Hz, 2H), 3.00 (t, *J* = 5.2 Hz, 4H), 2.24 - 2.14 (m, 4H), 1.74 (s, 4H), 0.41 (s, 4H).

**Example 40: 2-(6-Azaspiro[2.5]oct-6-yl)-4-(2-hydroxyethylsulfonyl)aminophenyl)-N-[4-(4,4-dif luoroieridinyl) benzo[3,4-c]1,2,5-thiadiazole-5-ylcarboxamide**

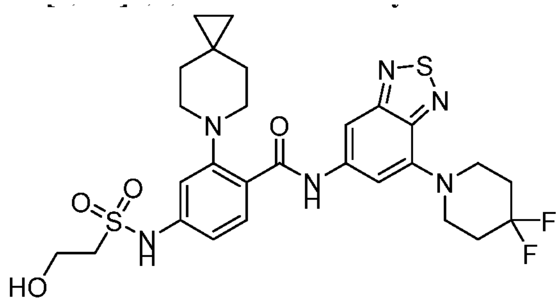

The preparations in **Example 40** follow the synthetic route of **Example 3:** LCMS (ESI): $[M+1]^+$ = 607. $^1H$ NMR (400 MHz, DMSO) δ 11.95 (s, 1H), 10.10 (s, 1H), 8.29 (d, *J* = 1.6 Hz, 1H), 7.85 (d, *J* = 8.4 Hz, 1H), 7.18 (d, *J* = 2.4 Hz, 1H), 7.10 - 7.02 (m, 2H), 4.99 (s, 1H), 3.78 (d, *J* = 6.4 Hz, 2H), 3.76 - 3.72 (m, 4H), 3.35 (d, *J* = 6.4 Hz, 2H), 3.00 (t, *J* = 5.2 Hz, 4H), 2.21 (tt, *J* = 13.6, 5.5 Hz, 4H), 1.57 (t, *J* = 5.2 Hz, 4H), 0.35 (s, 4H).

**Example 41: 2-{6-Azaspiro[2.5]octan-6-yl}-N-[4-(4,4-difluoropiperidin-1-yl)-1,3-benzothiazol-6-yl]-4-(2-hydroxyethanesulfonamido) benzamide**

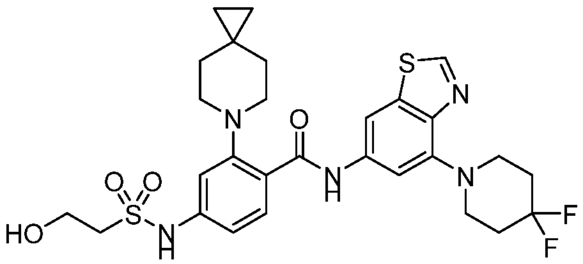

The preparations in **Example 41** follow the synthetic route of **Example 3:** LCMS (ESI): $[M+H]^+$ = 606.2, $^1H$ NMR (400 MHz, DMSO-d6) δ ppm 11.88 (s, 1H), 10.11 (s, 1H), 9.20 (s, 1H), 8.37 (d, *J* = 1.2 Hz, 1H), 7.85 (d, *J* = 8.4 Hz, 1H), 7.17 (dd, *J* = 1.6, 14.0 Hz, 2H), 7.05 (dd, *J* = 2.0, 8.4 Hz, 1H), 5.06 - 4.86 (m, 1H), 3.77 (br t, *J* = 6.4 Hz, 2H), 3.65 (br s, 4H), 3.36 (br s, 2H), 2.99 (br s, 4H), 2.26 - 2.11 (m, 4H), 1.58 (br s, 4H), 0.36 (s, 4H)

**Example 42: N-[2-amino-4-(4,4-difluoropiperidin-1-yl)-5-fluoro-1,3-benzothiazol-6-yl]-2-{6-az aspiro[2.5]octan-6-yl}-4-(2-hydroxyethanesulfonamido) benzamide**

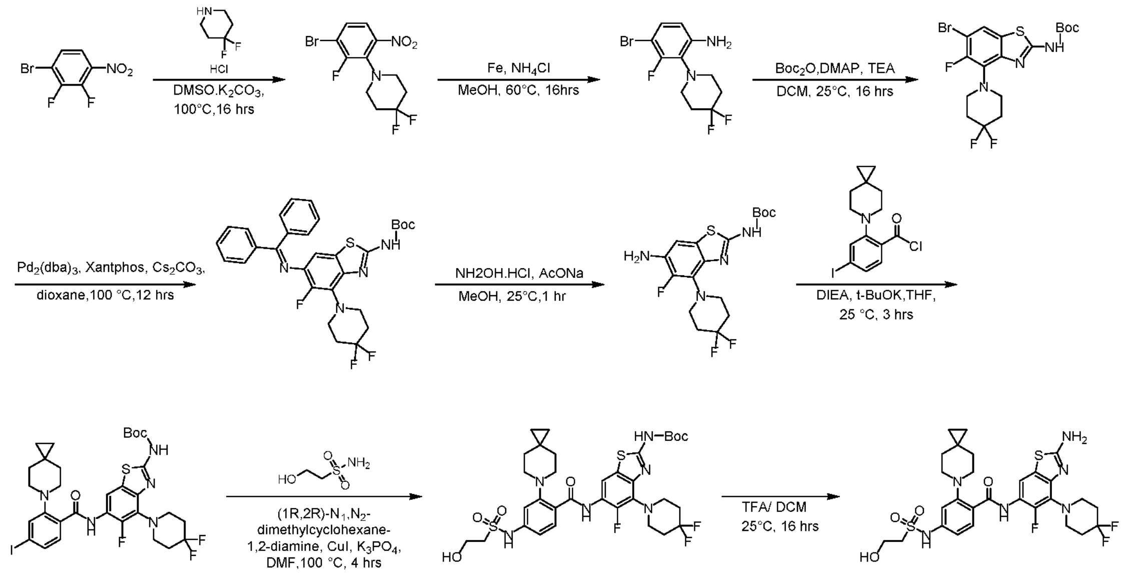


Step 1: To a solution of 1-bromo-2,3-difluoro-4-nitrobenzene (10.0 g, 42.0 mmol) and 4,4-difluoropiperidine hydrochloride (7.28 g, 46.2 mmol) in DMSO (200 mL) was added potassium carbonate (17.8 g, 126 mmol). The mixture was heated to 100°C and stirred for 16 h under nitrogen atmosphere. water (200 mL) was added to the reaction solution, which was thenextracted with ethyl acetate (200 mL × 3). The combined organic layer was dried over anhydrous sodium sulfate, filtered and concentrated under reduced pressure to give 1-(3-bromo-2-fluoro-6-nitrophenyl)-4,4-difluoropiperidine (12.0 g, 35.3 mmol, yield: 84%) as a yellow solid. LCMS (ESI): [M+H]+ =338.9; $^1$H NMR (400 MHz, DMSO-d6) δ 7.72 - 7.61 (m, 2H), 3.16 (br t, *J* = 5.3 Hz, 4H), 2.16 - 2.01 (m, 4H)

Step 2: To a solution of 1-(3-bromo-2-fluoro-6-nitrophenyl)-4,4-difluoropiperidine (12.0 g, 35.4 mmol) and ammonium chloride (7.64 g, 141 mmol) in methanol (240 mL) and water (80 mL) was added iron powder (13.3 g, 236 mmol) portionwise . The mixture was heated to 60°C and stirred for 16 h under nitrogen atmosphere. he reaction solution was filtered. The reaction solution was diluted with water (160 mL) and extracted with ethyl acetate (200 mL × 3). The combined organic layer was dried over anhydrous sodium sulfate, filtered and concentrated under reduced pressure to give 4-bromo-2-(4,4-difluoropiperidin-1-yl)-3-fluoroaniline (10.8 g, 34.9 mmol, yield: 98.7%) as a yellow solid. LCMS (ESI): $[M+H]^+$ = 308.9;

Step 3: To a solution of 4-bromo-2-(4,4-difluoropiperidin-1-yl)-3-fluoroaniline (1.00 g, 2.73 mmol) and di-tert-butyl dicarbonate (1.79 g, 8.19 mmol) in dichloromethane (20.0 mL) was added triethylamine (846 mg, 8.19 mmol) and 4-dimethylaminopyridine (34.0 mg, 0.27 mmol). The mixture was stirred at 25°C for 16 h. The reaction solution was diluted with water (20 mL) and extracted with ethyl acetate (20 mL × 3). The combined organic layer was dried over anhydrous sodium sulfate, filtered, concentrated to give the residue. The residue was purified by flash column chromatography (silica gel, 0-8% gradient of ethyl acetate/petroleum ether) to give tert-butyl N-[6-bromo-4-(4,4-difluoropiperidin-1-yl)-5-fluoro-1,3-benzothiazol-2-yl]carbamate (490 mg, 1.04 mmol, yield: 38%) as a yellow oil. LCMS (ESI): [M+H]+ =465.9; $^1$H NMR (400 MHz, DMSO-$d_6$) δ ppm 11.86 (s, 1H), 7.93 (d, *J* = 6.4 Hz, 1H), 3.44 (br t, *J* = 5.2 Hz, 4H), 2.18 - 2.01 (m, 4H), 1.50 (s, 9H)

Step 4: To a solution of tert-butyl N-[6-bromo-4-(4,4-difluoropiperidin-1-yl)-5-fluoro-1,3-benzothiazol-2-yl]carbamate (480 mg, 1.03 mmol), diphenylmethanimine (280 mg, 1.54 mmol) and cesium carbonate (1027 mg, 3.09 mmol) in dioxane (20.0 mL) were added 4,5-bis(diphenylphosphino)-9,9-dimethylxanthene (60.1 mg, 0.10 mmol) and (2-amino-1,1-biphenyl-2-yl) palladium (II) (96.2 mg, 0.10 mmol). the mixture was replaced with nitrogen 3 times. The mixture was heated to 100°C under nitrogen atmosphere, and stirred for 12 h. The reaction solution was diluted with water (5 mL), extracted with dichloromethane (15 mL × 2). the combined organic layer was washed with saturated saline (20 mL), dried over magnesium sulfate, filtered, and concentrated under reduced pressure to give the residue. The residue was purified by flash column chromatography (silica gel, 0-6% gradient of ethyl acetate/petroleum ether) to give tert-butyl N-[6-bromo-4-(4,4-difluoropiperidin-1-yl)-5-fluoro-1,3-benzothiazol-2-yl] carbamate (260 mg, 0.46 mmol, yield: 45%) as a yellow solid. LCMS (ESI): $[M+H]^+$ =567.1; $^1$H NMR (400 MHz, DMSO-$d_6$) δ ppm 11.61 (br s, 1H), 7.71 - 7.65 (m, 2H), 7.60 - 7.54 (m, 1H), 7.52 - 7.46 (m, 2H), 7.34 - 7.32 (m, 2H), 7.16 (dd, *J* = 2.8, 6.4 Hz, 2H), 6.99 (d, *J*= 7.2 Hz, 1H), 3.27 (br t, *J*= 5.6 Hz, 4H), 2.10 - 2.00 (m, 4H), 1.48 (s, 10H)

Step 5: To a mixed solution of tert-butyl N-[6-bromo-4-(4,4-difluoropiperidin-1-yl)-5-fluoro-1,3-benzothiazol-2-yl] carbamate (240 mg, 0.42 mmol) and methanol (2.00 mL) were added hydroxylamine hydrochloride (59.0 mg, 0.85 mmol) and sodium acetate (104 mg, 1.27 mmol) . The mixture was stirred at 25°C for 1 h. the reaction solution was diluted with water (5 mL), extracted with dichloromethane (15 mL × 2). the combined organic layer was washed with saturated saline

(20 mL), dried over magnesium sulfate, filtered, and concentrated under reduced pressure to give the residue. The residue was purified by flash column chromatography (silica gel, 0-50% gradient of ethyl acetate/petroleum ether) to give tert-butyl N-[6-amino-4-(4,4-difluoropiperidin-1-yl)-5-fluoro-1,3-benzothiazol-2-yl] carbamate (120 mg, 0.30 mmol, yield: 70%) as a yellow solid. LCMS (ESI): $[M+H]^+$ =403.0.

Step 6: To a solution of tert-butyl N-[6-amino-4-(4,4-difluoropiperidin-1-yl)-5-fluoro-1,3-benzothiazol-2-yl] carbamate (120 mg, 0.30 mmol) and 4-iodo-2-(6-azaspiro[2.5]octan-6-yl)benzoyl chloride (168 mg, 0.45 mmol) in THF (4.00 mL) was added N,N-diisopropylethylamine (118 mg, 0.89 mmol) , and the mixture was stirred at 25°C for 3 h. the reaction solution was diluted with water (5 mL), extracted with dichloromethane (15 mL × 2), the combined organic layer was washed with saturated saline (20 mL), dried over magnesium sulfate, filtered, and concentrated under reduced pressure to give the residue. The residue was purified by flash column chromatography (silica gel, 0-10% gradient of ethyl acetate/petroleum ether) to give tert-butyl N-[6-(2-{6-azaspiro[2.5]octan-6-yl}-4-iodobenzoylamino)-4-(4,4-difluoropiperidin-1-yl)-5-fluoro-1,3-benzothiazol-2-yl] carbamate (80 mg, 0.10 mmol, yield: 36%) as a yellow solid. LCMS (ESI): $[M+H]^+$ =742.2

Step 7: To a solution of 2-hydroxyethanesulfonamide (23.6 mg, 0.19 mmol), trans-N,N-dimethyl-1,2-cyclohexanediamine (13.6 mg, 0.09 mmol), potassium phosphate (80.1 mg, 0.38 mmol) in N,N-dimethylformamide (4.00 mL) was added cuprous iodide (9.17 mg, 0.05 mmol) . The mixture was stirred at 50°C for 5 min. Then, tert-butyl *N*-[6-(2-{6-azaspiro [2.5]octan-6-yl}-4-iodobenzoylamino)-4-(4,4-difluoropiperidin-1-yl)-5-fluoro-1,3-benzothiazol-2-yl]carbamate (70.0 mg, 0.09 mmol) was added to the reaction mixture at 50°C, and the mixture was heated to 100°C and stire at 100°C for 4 h under nitrogen atmosphere. The reaction solution was diluted with water (5 mL) and extracted with ethyl acetate (5 mL × 2).The combined organic layer was washed with liquor ammonia (5 mL × 2), driede over anhydrous magnesium sulfate, filterd and concentrated to give tert-butyl N-[6-(2-6-azaspiro[2.5]octan-6-yl}-4-(2-hydroxyethanesulfonamido)benzoylamino)-4-(4,4-difluoropiperidin-1-yl)-5-fluoro-1,3-benzothiazol-2-yl]carbamate (69 mg, crude) as a yellow solid compound. LCMS (ESI): $[M+H]^+$ =739.2.

Step 8: To a mixed solution of trifluoroacetic acid (1.00 mL) and dichloromethane (3.00 mL) was added tert-butyl N-[6-(2-{6-azaspiro[2.5]octan-6-yl}-4-(2-hydroxyethanesulfonamido)benzoylamino)-4-(4,4-difluoropiperidin-1-yl)-5-fluoro-1,3-benzothiazol-2-yl]carbamate (60.0 mg, 0.08 mmol). The mixture was stirred at 25°C for 16 h. the reaction solution was diluted with water (5 mL) and extracted with ethyl acetate (5 mL × 2). The combined the organic phases was washed with liquor ammonia (5 mL × 2), dried over anhydrous magnesium sulfate, filtered and concentrated to give a residue. The residue was purified by preparative HPLC (C18, 30%-70% gradient of water (formic acid)/acetonitrile) to give N-[2-amino-4-(4,4-difluoropiperidin-1-yl)-5-fluoro-1,3-benzothiazol-6-yl]-2-{6-azasp iro[2.5]octan-6-yl}-4-(2-hydroxyethanesulfonamido) benzamide (2.5 mg, 0.04 mmol, yield: 4.8%) as a white solid. LCMS (ESI): $[M+H]^+$ =639.2; $^1H$ NMR (400 MHz, DMSO-$d_6$) δ ppm 12.07 (s, 1H), 10.17 (s, 1H), 8.43 (d, *J* = 6.4 Hz, 1H), 8.23 - 7.44 (m, 3H), 7.27 (d, *J* = 1.6 Hz, 1H), 7.11 (dd, *J* = 2.0, 8.4 Hz, 1H), 3.77 - 3.74 (m, 2H), 3.41 - 3.34 (m, 6H), 3.03 - 2.94 (m, 4H), 2.24 - 2.05 (m, 4H), 1.67 - 1.50 (m, 4H), 0.37 (s, 4H)

**Example 43: N-(1-(4,4-difluoropiperidin-1-yl)-8-oxo-5,6,7,8-tetrahydro-2,7-naphthyridin-3-yl) -4-(2-hydroxyethylsulfonamido)-2-(6-azaspiro[2.5]octan-6-yl)benzamide**

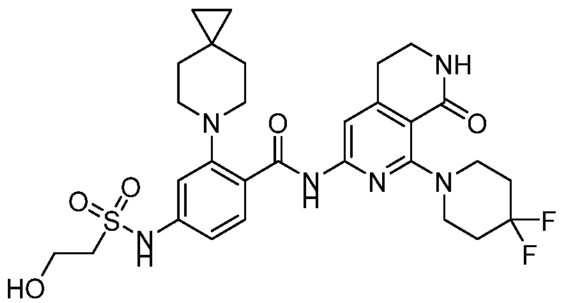

The preparations in **Example 43** follow the synthetic route of **Example 3:** LCMS (ESI): $[M+H]^+$ = 619.3. $^1H$ NMR (400 MHz, DMSO-d6) δ ppm 13.30 (s, 1H), 10.53 - 9.86 (m, 1H), 8.06 (d, *J* = 8.8 Hz, 1H), 7.83 (br s, 1H), 7.63 (s, 1H), 7.26 (d, *J* = 2.0 Hz, 1H), 7.13(dd, *J* = 2.0, 6.8 Hz, 1H), 5.14 - 4.76 (m, 1H), 3.76 (br t, *J* = 6.4 Hz, 2H), 3.56 - 3.46 (m, 4H), 3.30 (br d, *J* = 5.6 Hz, 4H), 2.90 - 3.00 (m, 4H), 2.84 (br t, *J* = 5.6 Hz, 2H), 2.20 - 1.35 (m, 8H), 0.39 (s, 4H)

**Example 44: N-(8-(4,4-difluoropiperidin-1-yl)-1,7-naphthyridin-6-yl)-4-(2-hydroxyethyl)sulfa moyl)-2-(6-azaspiro[2.5]octan-6-yl)benzamide**

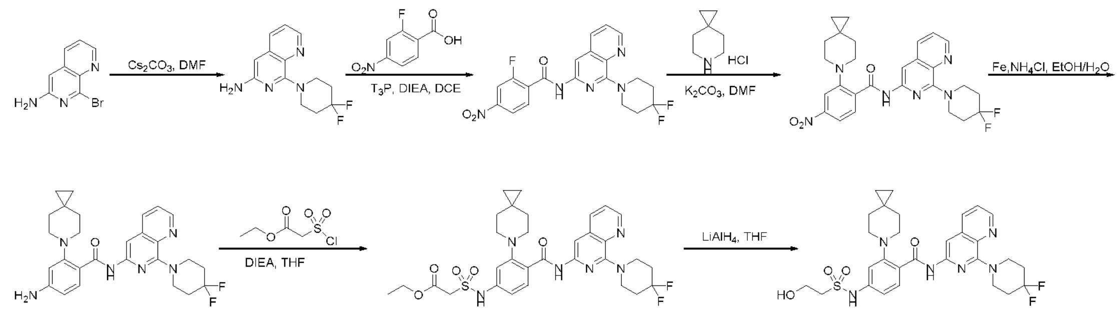


Step 1: To a stirred solution containing 4,4-difluoropiperidine hydrochloride (1.7 g, 10.71 mmol), cesium carbonate (3.6 g, 10.71 mmol) in DMF (20 mL) was added 8-bromo-1,7-naphthyridine-6-amine (2 g, 8.93 mmol). The mixture was stirred at 100°C for 12 h, The reaction solution was poured into water (50 mL), extracted with ethyl acetate (50 mL×3), and the combined organic layer was dried over anhydrous sodium sulfate, filtered and concentrated to give the residue, which was further purified by column chromatography (silica gel, 0-50% gradient of ethyl acetate/petroleum ether) to give 8-(4,4-difluoropiperidin-1-yl)-1,7-naphthyridin-6-amine (1.9 g) as a yellow solid compound. LCMS (ESI): $[M+H]^+$ = 265.1.

Step 2: To a stirred solution of 2-fluoro-4-nitrobenzoic acid (1.5 g, 7.91 mmol), propylphosphoric tricyclic anhydride (9.2 g, 14.38 mmol, 50% in EA), N,N-diisopropylethylamine (2.8 g, 21.57 mmol) in 1,2-dichloroethane (20 mL) was added 8-(4,4-difluoropiperidin-1-yl)-1,7-naphthyridin-6-amine (1.9 g, 7.19 mmol). The mixture was stirred at 80°C for 12 h. The reaction solution was poured into water (50 mL), extracted with ethyl acetate (50 mL × 3), and the combined organic layer was dried over anhydrous sodium sulfate, filtered and concentrated to give the residue, which was further purified by column chromatography (silica gel, 0-100% gradient of ethyl acetate/petroleum ether) to give N-(8-(4,4-difluoropiperidin-1-yl)-1,7-naphthyridin-6-yl)-2-fluoro-4-nitrobenzamide (1.7 g) as a yellow solid compound. LCMS (ESI): $[M+H]^+$ = 432.2.

Step 3: To a stirred solution of 6-aza-spiro[2.5]octane hydrochloride (373 mg, 2.50 mmol), potassium carbonate (883 mg, 6.26 mmol) in DMSO (10 mL) was added N-(8-(4,4-difluoropiperidin-1-yl)-1,7-naphthyridin-6-yl)-2-fluoro-4-nitrobenzamide (900 mg, 2.09 mmol). The mixture was stitted at 110°C for 12 h. The reaction solution was poured into water (30 mL), extracted with ethyl acetate (30 mL×3), and the combined organic layer was dried over anhydrous sodium sulfate, filtered and concentrated to give the residue, which was further purified by column chromatography (silica gel, 0-20% gradient of methanol/dichloromethane) to give N-(8-(4,4-difluoropiperidin-1-yl)-1,7-naphthyridin-6-yl)-4-nitro-2-(6-azaspiro[2.5]oct an-6-yl)benzamide (820 mg) as a yellow solid compound. LCMS (ESI): $[M+H]^+$ = 523.3.

Step 4: To a stirred solution of iron powder (756 mg, 13.40 mmol), ammonium chloride (724 mg, 13.40 mmol) in ethanol/water (5:1) (24 mL) was added N-(8-(4,4-difluoropiperidin-1-yl)-1,7-naphthyridin-6-yl)-4-nitro-2-(6-azaspiro[2.5]oct an-6-yl)benzamide (700 mg, 1.34 mmol). The mixture was stirred at 60°C for 30 min. The hot reaction solution was filtered and the filtrate was poured into water (30 mL), extracted with ethyl acetate (30 mL × 3), dried over anhydrous sodium sulfate, filtered and concentrated to give 4-amino-N-(8-(4,4-difluoropiperidin-1-yl)-1,7-naphthyridin-6-yl)-2-(6-azaspiro[2.5]o ctan-6-yl)benzamide (500 mg, yield: 88.5%) as a yellow solid compound. LCMS (ESI): $[M+H]^+$ = 493.4

Step 5: To a stirred solution containing 4-amino-N-(8-(4,4-difluoropiperidin-1-yl)-1,7-naphthyridin-6-yl)-2-(6-azaspiro[2.5]o ctan-6-yl)benzamide (500 mg, 1.02 mmol),N,N-diisopropylethylamine (402 mg, 3.05 mmol) in THF (5 mL) was added ethyl chlorosulfonylacetate (230 mg, 1.22 mmol) dropwise at 0°C. The reaction solution was stirred at 20°C for 1 h. The reaction solution was poured into water (30 mL), extracted with ethyl acetate (30 mL×3). The combined organic layer was dried over anhydrous sodium sulfate, filtered, and concentrated to give ethyl 2-(N-(4-(8-(4,4-difluoropiperidin-1-yl)-1,7-naphthyridin-6-yl)carbamoyl)-3-(6-azaspir o[2.5]octan-6-yl)phenyl)sulfamoyl)acetate (310 mg) as a yellow solid compound. LCMS (ESI): $[M+H]^+$ = 643.4.

Step 6: To a solution containing ethyl 2-(N-(4-(8-(4,4-difluoropiperidin-1-yl)-1,7-naphthyridin-6-yl)carbamoyl)-3-(6-azaspir o[2.5]octan-6-yl)phenyl)sulfamoyl)acetate (300 mg, 0.467 mmol) in THF (5 mL) was added lithium aluminum hydride (50 mg, 1.40 mmol) at 0°C in portions. The reaction solution was stirred at 20°C for 30 min, and sodium sulfate decahydrate to added the reaction solution. After stirring for 30 min, the reaction solution was filtered and concentrated, and then purified by column chromatography (silica gel, 0-100% gradient of ethyl acetate/petroleum ether) to give N-(8-(4,4-difluoropiperidin-1-yl)-1,7-naphthyridin-6-yl)-4-(2-hydroxyethyl)sulfamoyl) -2-(6-azaspiro[2.5]octan-6-yl)benzamide (35.77 mg) as a yellow solid compound. LCMS (ESI): $[M+H]^+$ = 601.4. $^1H$ NMR (400 MHz, DMSO) $\delta$ 12.90 (s,

1H), 10.65-9.58(m, 1H), 8.75 - 8.68 (m, 1H), 8.24 (dd, $J$ = 8.4, 2.0 Hz, 1H), 8.10 (t, $J$ = 4.4 Hz, 2H), 7.61 (dd, $J$ = 8.4, 4.0 Hz, 1H), 7.28 (d, $J$ = 2.4 Hz, 1H), 7.14 (dd, $J$ = 8.4, 2.0 Hz, 1H), 5.23-4.64 (m, 1H), 4.20 (t, $J$ = 5.6 Hz, 4H), 3.77 (t, $J$ = 6.4 Hz, 2H), 3.01 (d, $J$ = 5.2 Hz, 6H), 2.22 - 2.13 (m, 4H), 1.76 (s, 4H), 0.41 (s, 4H).

**Example 45: N-(8-(4,4-difluoropiperidin-1-yl)-1,7-naphthyridin-6-yl)-5-fluoro-4-((2-hydroxyet hyl)sulfonamide)-2-(6-azaspiro[2.5]octan-6-yl)benzamide**

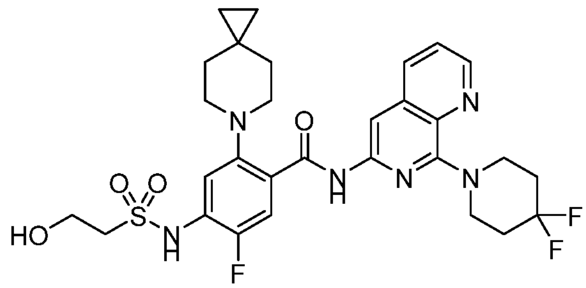

The preparations in **Example 45** follow the synthetic route of **Example 3:** LCMS (ESI): $[M+H]^+$ = 619.4. $^1$H NMR (400 MHz, DMSO) δ 13.22 (s, 1H), 10.07 (s, 1H), 8.73 (dd, $J$ = 4.0, 1.6 Hz, 1H), 8.25 (dd, $J$ = 8.4, 2.0 Hz, 1H), 8.10 (s, 1H), 7.91 (d, $J$ = 11.6 Hz, 1H), 7.62 (dd, $J$ = 8.4, 4.0 Hz, 1H), 7.58 (d, $J$ = 7.2 Hz, 1H), 5.01 (s, 1H), 4.27 - 4.11 (m, 4H), 3.81 (t, $J$ = 6.4 Hz, 2H), 3.42 (t, $J$ = 6.4 Hz, 2H), 3.00 (t, $J$ = 5.2 Hz, 4H), 2.23-2.12 (m, 4H), 1.76 (s, 4H), 0.42 (s, 4H).

**Example 46: N-(8-(4,4-difluoropiperidin-1-yl)-1,7-naphthyridin-6-yl)-2-fluoro-4-((2-hydroxyet hyl)sulfonamido)-6-(6-azaspiro[2.5]octan-6-yl)benzamide**

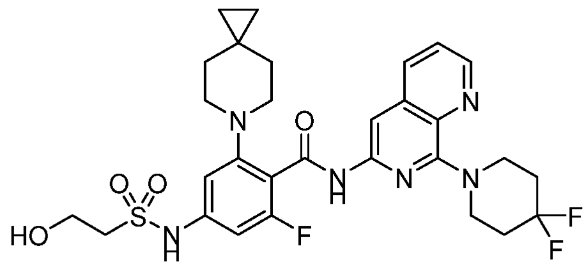

The preparations in **Example 46** follow the synthetic route of **Example 3:** LCMS (ESI): $[M+H]^+$ =310.30. $^1$H NMR (400 MHz, DMSO) δ 10.69 (s, 1H), 10.17 (s, 1H), 8.72 (dd, $J$ = 4.1, 1.7 Hz, 1H), 8.26 (dd, $J$ = 8.5, 1.7 Hz, 1H), 7.96 (s, 1H), 7.61 (dd, $J$ = 8.4, 4.1 Hz, 1H), 6.80 (d, $J$ = 2.0 Hz, 1H), 6.71 (dd, $J$ = 11.9, 1.9 Hz, 1H), 4.97 (s, 1H), 4.21 (t, $J$ = 5.7 Hz, 4H), 3.77 (d, $J$ = 6.9 Hz, 2H), 3.37 (t, $J$ = 6.5 Hz, 2H), 3.02 (t, $J$ = 5.2 Hz, 4H), 2.13 (td, $J$ = 14.2, 7.0 Hz, 4H), 1.44 (t, $J$ = 5.2 Hz, 4H), 0.27 (s, 4H).

**Example 47: (R)-4-(2-hydroxyethyl)sulfamoyl)-N-(8-(2-methylmorpholinyl)-1,7-naphthyridin-6-yl)-2-(6-azaspiro [2.5]octan-6-methyl)benzamide**

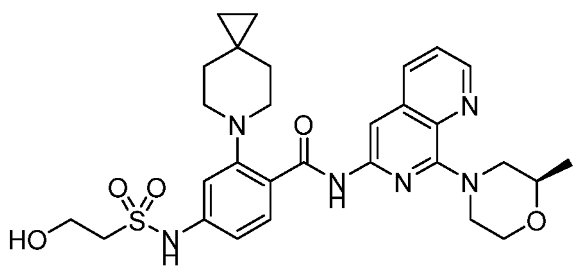

The preparations in **Example 47** follow the synthetic route of **Example 3:** LCMS (ESI): [M+H/2]$^+$ = 581.4. $^1$H NMR (400 MHz, DMSO) δ 12.81 (s, 1H), 10.21 (s, 1H), 8.70 (dd, J = 4.0, 2.0 Hz, 1H), 8.22 (dd, J = 8.4, 1.8 Hz, 1H), 8.14 -8.04 (m, 2H), 7.59 (dd, J = 8.4, 4.0 Hz, 1H), 7.29 (d, J = 2.0 Hz, 1H), 7.14 (dd, J = 8.4, 2.0 Hz, 1H), 5.00 - 4.80 (m, 3H), 3.99-3.91 (m, 1H), 3.85-3.73 (m, 4H), 3.37 (t, J = 6.4 Hz, 2H), 3.17-3.07 (m, 1H), 3.01 (d, J = 5.6 Hz, 4H), 2.83 (dd, J = 12.8, 10.2 Hz, 1H), 2.55-2.47 (m, 4H), 1.76 (s, 4H), 1.19 (d, J = 6.0 Hz, 3H), 0.41 (s, 4H).

**Example 48: 4-(2-hydroxyethyl)sulfamoyl)-N-(8-(4-methylpiperazin-1-yl)-1,7-naphthalidin-6-y 1)-2-(6-azaspiro [2.5] octan-6-methyl)benzamide**

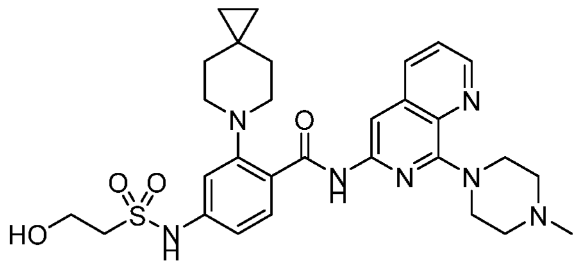

The preparations in **Example 48** follow the synthetic route of **Example 3:** LCMS (ESI): [M+H/2]$^+$ = 580.4. $^1$H NMR (400 MHz, DMSO) δ 12.95 (s, 1H), 10.74-9.50(m, 1H), 8.69 (d, J = 4.0 Hz, 1H), 8.20 (d, J = 8.0 Hz, 1H), 8.11 (d, J = 8.4 Hz, 1H), 8.04 (s, 1H), 7.58 (dd, J = 8.4, 4.0 Hz, 1H), 7.29 (d, J = 2.0 Hz, 1H), 7.14 (dd, J = 8.8, 2.0 Hz, 1H), 5.48-4.58(m, 1H), 4.07 (d, J = 6.0 Hz, 4H), 3.77 (t, J = 6.4 Hz, 2H), 3.37 (d, J = 6.4 Hz, 2H), 3.00 (t, J = 5.2 Hz, 4H), 2.52 - 2.48 (m, 4H), 2.28 (s, 3H), 1.77 (s, 4H), 0.41 (s, 4H).

**Example 49: 4-(2-hydroxyethylsulfonamido)-2-(6-azaspiro[2.5]octan-6-yl)-N-(8-(4-(2,2,2-triflu oroethyl)piperazin-1-yl)-1,7-naphthyridin-6-yl)benzamide**

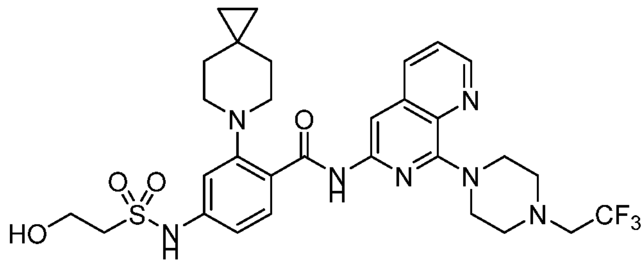

The preparations in **Example 49** follow the synthetic route of **Example 3:** LCMS (ESI): [M+H]+ = 648.3. $^1$H NMR (400 MHz, DMSO-d6) δ ppm 13.02 (s, 1H), 10.21 (br s, 1H), 8.69 (dd, J = 1.6, 4.0 Hz, 1H), 8.21 (dd, J = 1.6, 8.4 Hz, 1H), 8.11 (d, J = 8.8 Hz, 1H), 8.05 (s, 1H), 7.59 (dd, J = 4.0, 8.4 Hz, 1H), 7.29 (d, J = 2.0 Hz, 1H), 7.14 (dd, J = 2.0, 8.8 Hz, 1H), 5.23 - 4.71 (m, 1H), 4.16 - 3.99 (m, 4H), 3.83 - 3.70 (m, 2H), 3.33 - 3.11 (m, 4H), 3.08 - 2.93 (m, 4H), 2.91 - 2.77 (m, 4H), 2.31 - 1.27 (m, 4H), 0.40 (s, 4H)

**Example 50: 4-(2-hydroxyethylsulfonamido)-2-(6-azaspiro[2.5]octan-6-yl)-N-(8-(1-(2,2,2-triflu oroethyl)piperidin-4-yl)-1,7-naphthyridin-6-yl)benzamide**

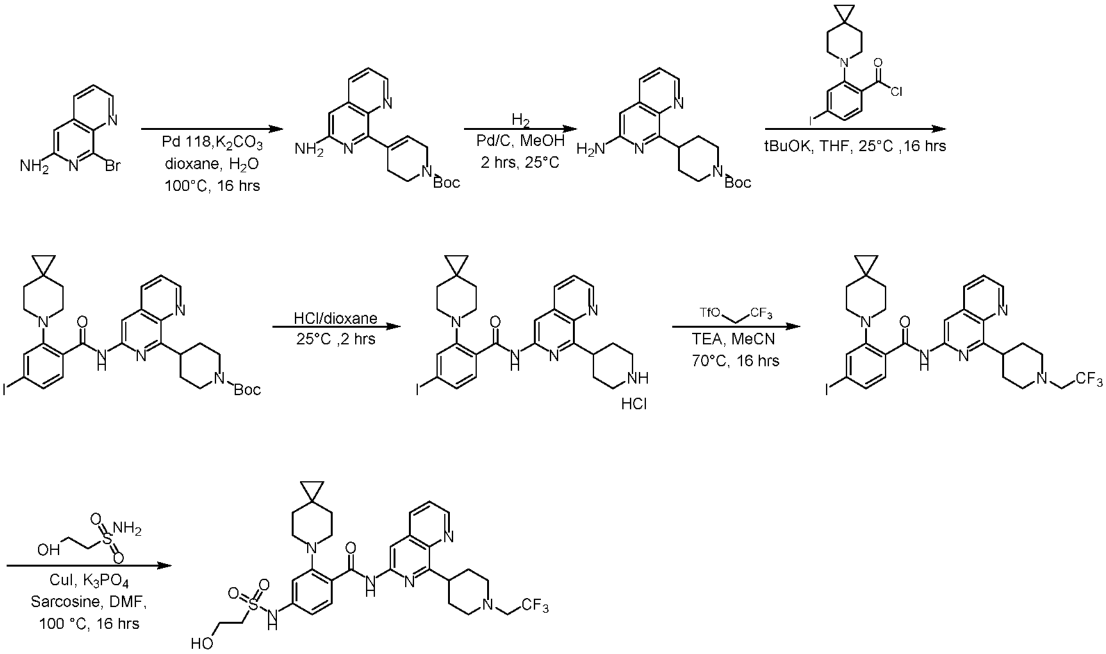


Step 1: To a solution of 8-bromo-1,7-naphthyridine-6-amine (2.00 g, 8.93 mmol) and tert-butyl 4-(4,4,5,5-tetramethyl-1,3,2-dioxolan-2-yl)-1,2,5,6-tetrahydropyridinyl ester (3.31 g, 10.7 mmol) in dioxane (16.0 mL)/water (4.00 mL) were added 1,1-bis(tert-butylphosphorus)ferrocene palladium chloride (0.60 g, 0.89 mmol) and potassium carbonate (3.78 g, 26.8 mmol) . Then the mixture was heated to 100°C and stirred at 100°C for 16 h under nitrogen atmosphere. the reaction solution was diluted with water (10 mL) and extracted with dichloromethane (10 mL × 3). The combined organic layer was dried over anhydrous sodium sulfate, filtered and concentrated under reduced pressure to give the residue. The residue was purified by flash column chromatography (silica gel, 0-25% gradient of ethyl acetate/petroleum ether) to give tert-butyl 4-(6-amino-1,7-naphthyridin-8-yl)-5,6-dihydropyridine-1(2H)-formate (1.90 g, 5.78 mmol, yield: 65%) as a yellow oil. LCMS (ESI): [M+H]+ = 327.1

Step 2: To a solution of tert-butyl 4-(6-amino-1,7-naphthyridin-8-yl)-5,6-dihydropyridine-1(2H)-formate (1.50 g, 4.60 mmol) in methanol (20.0 mL) was added wet palladium/carbon (0.50 g, 10% purity) and then hydrogen was introduced. The mixture was stirred at 25°C for 2 h. the solution was concentrated to give the residue. The residue was purified by preparative HPLC (C18, 18-58% gradient of water (formic acid)/acetonitrile) to give tert-butyl 4-(6-amino-1,7-naphthyridin-8-yl)piperidine-1-carboxylate (210 mg, 0.60 mmol, yield: 8%) as a yellow oil. LCMS (ESI): $[M+H]^+$ = 329.1

Step 3 : To a solution of tert-butyl 4-(6-amino-1,7-naphthyridin-8-yl)piperidine-1-carboxylate (200 mg, 0.61 mmol) and 2-(6-azaspiro[2.5]octan-6-yl)-4-iodobenzoyl chloride (343 mg, 0.91 mmol) in THF (4 mL) were added a solution of potassium tert-butoxide (1.83 mL, 1M) in THF and N,N-diisopropylethylamine (241 mg, 1.83 mmol) The mixture was stirred at 25°C for 16 h. The reaction solution was diluted with water (20 mL) and extracted with ethyl acetate (20 mL×3). The combined organic layer was washed with saturated saline (20 mL), dried over anhydrous sodium sulfate, filtered and concentrated to give a residue. The residue was purified by flash column chromatography (silica gel, 0-13% gradient of ethyl acetate/petroleum ether) to give tert-butyl 4-(6-(4-iodo-2-(6-azaspiro[2.5]octan-6-yl)benzoylamino)-1,7-naphthyridin-8-yl)piperi dine-1-carboxylate (250 mg, 0.23 mmol, yield: 61%) as a yellow solid. LCMS (ESI): [M+Na]+ = 690.1

Step 4: To a solution of tert-butyl 4-(6-(4-iodo-2-(6-azaspiro[2.5]octan-6-yl)benzoylamino)-1,7-naphthyridin-8-yl)piperi dine-1-carboxylate (230 mg, 0.34 mmol) in dioxane (2 mL) was added HCl in dioxane (2 mL). The mixture was stirred at 25°C for 2 h. the reaction solution was concentrated to give 4-iodo-N-(8-(piperidin-4-yl)-1,7-naphthyridin-6-yl)-2-(6-azaspiro[2.5]octan-6-yl)benz amide hydrochloride (200 mg, crude).

Step 5: To a solution of 4-iodo-N-(8-(piperidin-4-yl)-1,7-naphthyridin-6-yl)-2-(6-azaspiro[2.5]octan-6-yl)benz amide hydrochloride (200 mg, crude) (330 mg, 0.58 mmol) and 2,2,2-trifluoroethyl triflate (202 mg, 0.87 mmol) in acetonitrile (5.00 mL) was added triethylamine (180 mg, 1.74 mmol) . The mixture was stirred at 70°C for 16 h. the solution was concentrated to give the residue. The residue was purified by flash column chromatography (silica gel, 0-8% gradient of ethyl acetate/petroleum ether) to give 4-iodo-2-(6-azaspiro[2.5]octan-6-yl)-N-(8-(1-(2,2,2-trifluoroethyl)piperidin-4-yl)-1,7-naphthyridin-6-yl)benzamide (130 mg, 0.19 mmol, yield: 34%) as a white solid. LCMS (ESI): [M+H]+ = 650.1

Step 6: To a solution of potassium phosphate (133 mg, 0.62 mmol), 2-hydroxyethanesulfonamide (38.5 mg, 0.31 mmol), and sarcosine (14.0 mg, 0.15 mmol) in dimethylformamide (5.00 mL) was added cuprous iodide (29.9 mg, 0.15 mmol). The mixture was heated to 50°C and stirred for 5 min. 4-iodo-2-(6-azaspiro[2.5]octan-6-yl)-N-(8-(1-(2,2,2-trifluoroethyl)piperidin-4-yl)-1,7-naphthyridin-6-yl)benzamide (100 mg, 0.15 mmol) was added to the mixture, and then heated to 100°C with stirring for 16 h under nitrogen atmosphere. The reaction solution was diluted with water (10 mL) and extracted with dichloromethane (10 mL × 3). The combined organic layer was dried over anhydrous sodium sulfate, filtered and concentrated under reduced pressure to give the residue. The residue was purified by preparative HPLC (C18, 46-86% gradient of water (formic acid)/acetonitrile) to give 4-(2-hydroxyethylsulfonamido)-2-(6-azaspiro[2.5]octan-6-yl)-N-(8-(1-(2,2,2-trifluoro ethyl)piperidin-4-yl)-1,7-naphthyridin-6-yl)benzamide (33 mg, 0.05 mmol, yield: 33%) as a white solid. LCMS (ESI): $[M+H]^+$ = 647.3. $^1H$ NMR (400 MHz, DMSO-$d_6$) δ ppm 13.57 (s, 1H), 10.26 (br s, 1H), 8.89 (dd, *J* = 1.2 Hz, 1H), 8.53 (s, 1H), 8.39 (d, *J* = 8.0 Hz, 1H), 8.14 (d, *J* = 8.4 Hz, 1H), 7.71 (dd, *J* = 4.0, 8.4 Hz, 1H), 7.31 (s, 1H), 7.17 (br d, *J* = 8.4 Hz, 1H), 5.07 - 4.87 (m, 1H), 4.24 - 4.06 (m, 1H), 3.84 - 3.72 (m, 2H), 3.41 - 3.35 (m, 2H), 3.27 - 3.16 (m, 2H), 3.14 - 2.93 (m, 6H), 2.63 - 2.52 (m, 2H), 2.37 - 1.32 (m, 8H), 0.41 (s, 4H)

**Example 51: N-(8-(4,4-difluoropiperidin-1-yl)-2-methyl-1,7-naphthyridin-6-yl)-4-(2-hydroxyet hylsulfonamido)-2-(6-azaspiro[2.5]octan-6-yl)benzamide**

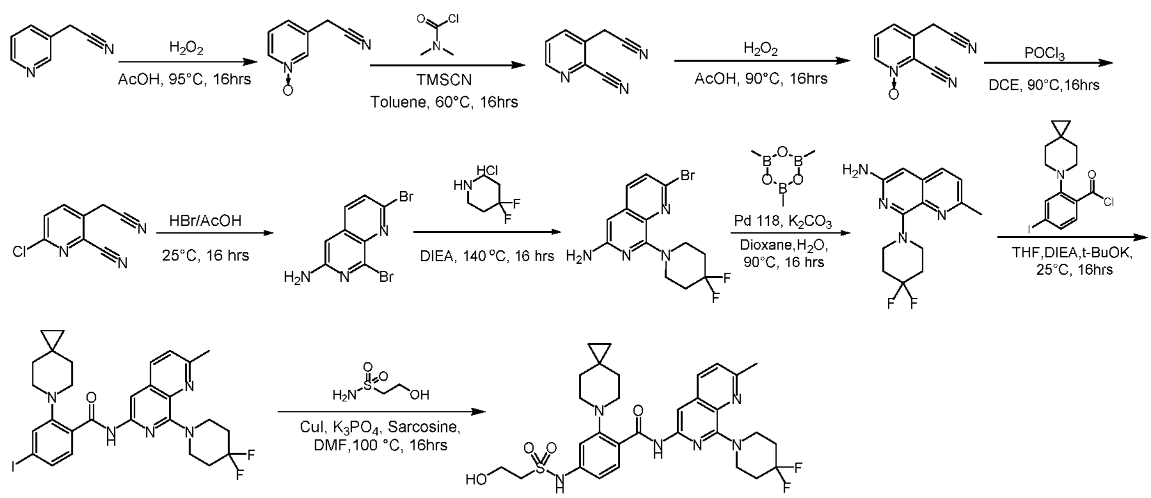


Step 1: Hydrogen peroxide (55.1 g, 584 mmol, 36% purity) was added to a solution of 2-(pyridine-3-yl)acetonitrile (23.0 g, 194 mmol) in acetic acid (200 mL). The mixture was stirred at 95°C for 16 h. The reaction solution was concentrated under reduced pressure to give 3-(cyanomethyl)pyridine 1-oxide (24.0 g, 178 mmol, yield: 91.9%) as a yellow solid.

Step 2: Dimethylcarbamic chloride (19.2 g, 178 mmol) and trimethylsilyl cyanide (21.7 g, 214 mmol) were added to a solution of 3-(cyanomethyl)pyridine 1-oxide (24.0 g, 178 mmol) in toluene (240 mL). The mixture was stirred at 60°C for 16 h. Quench the reaction solution with potassium carbonate solution (240 mL, 1M) and then extracted withethyl acetate (240 mL×2). The combined organic layer was dried over anhydrous sodium sulfate, filtered and concentrated under reduced pressure to give the residue. Separate the residue by column chromatography (silica gel, 0-45% gradient of ethyl acetate/petroleum ether) to give 3-(cyanomethyl)cyanopyridine (21.0 g, 146 mmol, yield: 81.9%).

Step 3: Add hydrogen peroxide (18.5 g, 196 mmol, 36% purity) to a solution of 3-(cyanomethyl)cyanopyridine (14.0 g, 97.8 mmol) in acetic acid (140 mL), and The mixture was stirred at 90°C for 16 h. The reaction solution was concentrated under reduced pressure to give 2-cyano-3-(cyanomethyl)pyridine 1-oxide (15.6 g, 75.4 mmol, yield: 77.1%). $^1H$ NMR (400 MHz, DMSO-d6) δ 8.46 (br d, *J* = 6.4 Hz, 1H), 7.73 (br t, *J* = 7.2 Hz, 1H), 7.54 (br d, *J* = 8.0 Hz, 1H), 4.31 (s, 2H)

Step 4: Phosphorus oxychloride (6 mL) was added to a solution of 2-cyano-3-(cyanomethyl)pyridine 1-oxide (6.00 g, 37.7 mmol) in dichloroethane (60 mL), and the mixture was stirred at 90°C for 16 h. The reaction solution was diluted with water (100 mL), extracted with dichloromethane (100 mL × 2). Then the combined organic layer was washed with saturated saline (100 mL × 2), dried over magnesium sulfate, filtered, and concentrated under reduced pressure to give the residue. The residue was separated by column chromatography (silica gel, 0-30% gradient of ethyl acetate/petroleum ether) to give 6-chloro-3-(cyanomethyl)cyanopyridine (3.50 g, 19.7 mmol, yield: 52.3%). $^1H$ NMR (400 MHz, DMSO-d6) δ 8.17 (d, *J* = 8.4 Hz, 1H), 7.96 (d, *J* = 8.4 Hz, 1H), 4.36 (s, 2H)

Step 5: 6-chloro-3-(cyanomethyl)cyanopyridine (3.50 g, 19.7 mmol) was added to a mixture of hydrobromic acid and acetic acid (30 mL). And the mixture was stirred at 25°C for 16 h. The reaction solution was poured into water (100 mL) and the pH was adjusted to 8 with sodium carbonate solution (240 mL, 1M), and extracted with ethyl acetate (500 mL×3). The combined organic layer was washed with saturated saline (100 mL×2), dried over magnesium sulfate, filtered, and concentrated under reduced pressure to give 2,8-Dibromo-1,7-naphthyridin-6-amine (3.70 g, 12.2 mmol, yield: 62.0%).

LCMS (ESI): $[M+H]^+$ =303.9

Step 6: 4,4-difluoropiperidine (3.33 g, 21.1 mmol) and N,N-diisopropylethylamine (6.97 g, 52.8 mmol) were successively added to a solution of 2,8-dibromo-1,7-naphthyridin-6-amine (3.20 g, 10.6 mmol) in tert-butanol (60 mL). The mixture was stirred at 140°C for 16 h. The reaction solution was diluted with water (100 mL), extracted with ethyl acetate (100 mL × 2) and the combined organic layer was washed with saturated saline (100 mL × 2), dried over magnesium sulfate, filtered, and concentrated under reduced pressure to give the residue. The residue was separated by column chromatography (silica gel, 0-15% gradient of ethyl acetate/petroleum ether) to give 2-bromo-8-(4,4-difluoropiperidin-1-yl)-1,7-naphthyridin-6-amine (1.50 g, 4.38 mmol, yield: 41.4%). LCMS (ESI): $[M+H]^+$ =342.9

Step 7: 1,1-bis(tert-butylphosphine)ferrocene palladium chloride (59.0 mg, 0.09 mmol) was added to a solution of 2-bromo-8-(4,4-difluoropiperidin-1-yl)-1,7-naphthyridin-6-amine (300 mg, 0.87 mmol), potassium carbonate (247 mg, 1.75 mmol), and trimethylboroxine (ca. 50% in THF) (0.37 mL, 1.31 mol) in dioxane (6 mL) and water (1.2 mL). The mixture was heated to 90°C and stirred for 16 h under nitrogen. Water (10 mL) was added to the reaction solution and extracted with ethyl acetate (10 mL × 2). The combined organic layer was dried over anhydrous sodium sulfate, filtered and concentrated under reduced pressure to give the residue. The residue was separated by column chromatography (silica gel, 0-20% gradient of ethyl acetate/petroleum ether) to give 8-(4,4-difluoropiperidin-1-yl)-2-methyl-1,7-naphthyridin-6-amine (110 mg, 0.39 mmol, yield: 45.2%). LCMS (ESI): $[M+H]^+$ =279.2

Step 8: Potassium tert-butoxide (1.08 mL, 1.08 mmol, 1M) and N,N-diisopropylethylamine (142 mg, 1.08 mol) were successively added to a solution of 8-(4,4-difluoropiperidin-1-yl)-2-methyl-1,7-naphthyridin-6-amine (100 mg, 0.36 mmol) and 4-iodo-2-(6-azaspiro[2.5]octan-6-yl)benzoyl chloride (162 mg, 0.43 mmol) in THF (1.00 mL). The mixture was stirred at 25°C for 16 h. Add water (10 mL) was added to the reaction solution and extracted withethyl acetate (10 mL × 2). The organic phases was combined and the combined organic layer was washed with saturated saline (200 mL), dried over anhydrous sodium sulfate, filtered and concentrated under reduced pressure to give a residue. The residue was purified by column chromatography (silica gel, 0-10% gradient of ethyl acetate/petroleum ether) to give N-(8-(4,4-difluoropiperidin-1-yl)-2-methyl-1,7-naphthyridin-6-yl)-4-iodo-2-(6-azaspir o[2.5]octan-6-yl)benzamide (120 mg, 0.19 mmol, yield: 54.1%). LCMS (ESI): $[M+H]^+$ =618.2

Step 9: Cuprous iodide (18.9 mg, 0.10 mmol) was added to a solution of 2-hydroxyethanesulfonamide (48.6 mg, 0.39 mmol), sarcosine (17.3 mg, 0.19 mmol), and potassium phosphate (168 mg, 0.78 mmol) in N,N-dimethylformamide (2 mL). The mixture was stirred at 100°C for 16 h. Then N-(8-(4,4-difluoropiperidin-1-yl)-2-methyl-1,7-naphthyridin-6-yl)-4-iodo-2-(6-azaspir o[2.5]octan-6-yl)benzamide (120 mg, 0.19 mmol) was added to the reaction mixture at 50°C, and the mixture was heated to 100°C with stirring for 16 h under nitrogen atmosphere. Add water (20 mL) was added to the reaction solution and extracted with ethyl acetate (20 mL × 2). The combined organic layer was dried over anhydrous sodium sulfate, filtered and concentrated under reduced pressure to give the residue. The residue was purified by preparative HPLC (C18, 50-90% gradient of water (formic acid)/acetonitrile) to give N-(8-(4,4-difluoropiperidin-1-yl)-2-methyl-1,7-naphthyridin-6-yl)-4-(2-hydroxyethyls ulfonamido)-2-(6-azaspiro[2.5]octan-6-yl)benzamide (51.0 mg, 0.08 mmol, yield: 42.7%) as a white solid compound. LCMS (ESI): $[M+H]^+$ =615.1. $^1H$ NMR (400 MHz, DMSO-d6) $\delta$ 12.85 (s, 1H), 10.22 (s, 1H), 8.22 - 8.01 (m, 3H), 7.52 (d, *J* = 8.4 Hz, 1H), 7.28 (d, *J* = 1.6 Hz, 1H), 7.14 (dd, *J* =2.0, 8.8 Hz, 1H), 5.06 - 4.85 (m, 1H), 4.31 - 4.05 (m, 4H), 3.77 (br t, *J* = 6.4 Hz, 2H), 3.40 - 3.36 (m, 2H), 3.12 - 2.88 (m, 4H), 2.63 (s, 3H), 2.28 - 2.10 (m, 4H), 1.98 - 1.42 (m, 4H), 0.41 (s, 4H)

### Example 52: N-(8-(4,4-difluoropiperidin-1-yl)-2-methoxy-1,7-naphthyridin-6-yl)-4-(2-hydroxy ethylsulfonamido)-2-(6-azaspiro[2.5]octan-6-yl)benzamide

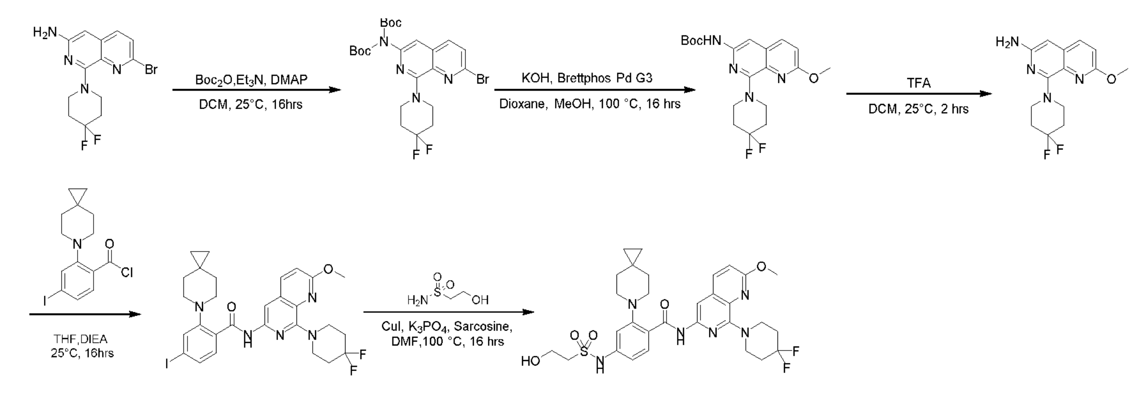


Step 1: Triethylamine (451 mg, 4.37 mmol), 4-dimethylaminopyridine (36.3 mg, 0.29 mmol) and di-tert-butyl

dicarbonate (954 mg, 4.37 mmol) were successively added to a solution of 2-bromo-8-(4,4-difluoropiperidin-1-yl)-1,7-naphthyridin-6-amine (500 mg, 1.46 mmol) in dichloromethane (10 mL). The mixture was stirred at 25°C for 16 h. Water (10 mL) was added to the reaction solution and extracted withethyl acetate (10 mL × 2).The combined organic layer was washed with saturated saline (10 mL), dried over magnesium sulfate, filtered, and concentrated under reduced pressure to give the residue. The residue was purified by column chromatography (silica gel, 0-20% gradient of ethyl acetate/petroleum ether) to give tert-butyl (2-bromo-8-(4,4-difluoropiperidin-1-yl)-1,7-naphthyridin-6-yl) tert-butoxycarbonylcarbamate (200 mg, 1.46 mmol, yield: 25.3%). LCMS (ESI): $[M+H]^+$ =543.1.

Step 2: Methanol (53.1 mg, 1.66 mmol), potassium hydroxide (93.9 mg, 1.66 mmol), and methanesulfonic acid (2-dicyclohexylphosphino-3,6-dimethoxy-2,4,6-triisopropyl-1,1-biphenyl)(2-amino-1, 1-biphenyl-2-yl) palladium (II) (50.5 mg, 0.06 mmol) were successively added to a solution of tert-butyl (2-bromo-8-(4,4-difluoropiperidin-1-yl)-1,7-naphthyridin-6-yl) tert-butoxycarbonylcarbamate (300 mg, 0.55 mmol) in dioxane (1 mL), and the mixture was heated to 100°C with stirring for 16 h under nitrogen atmosphere. The reaction solution washed with water (10 mL), extracted with ethyl acetate (10 mL × 2), the combined organic layer was washed with saturated saline (10 mL × 2), dried over magnesium sulfate, filtered, and concentrated under reduced pressure to give the residue. The residue was purified by column chromatography (silica gel, 0-20% gradient of ethyl acetate/petroleum ether) to give tert-butyl (8-(4,4-difluoropiperidin-1-yl)-2-methoxy-1,7-naphthyridin-6-yl)carbamate (200 mg, 0.55 mmol, yield: 91.9%) as a yellow solid. LCMS (ESI): $[M+H]^+$ =395.1.

Step 3: Trifluoroacetic acid (1 mL) was added to a solution of tert-butyl (8-(4,4-difluoropiperidin-1-yl)-2-methoxy-1,7-naphthyridin-6-yl)carbamate (200 mg, 0.51 mmol) in dichloromethane (4 mL). The mixture was stirred at 25°C for 2 h. Quench the reaction solution with sodium bicarbonate (5 mL), extracted with dichloromethane (10 mL × 2), the combined organic layer was washed with saturated saline (10 mL × 2), dried over magnesium sulfate, filtered, and concentrated under reduced pressure to give 8-(4,4-difluoropiperidin-1-yl)-2-methoxy-1,7-naphthyridin-6-amine (130 mg, 0.44 mmol, yield: 87.1%) as a yellow solid. LCMS (ESI): $[M+H]^+$ =295.1.

Step 4: Add 4-iodo-2-(6-azaspiro[2.5]octan-6-yl)benzoyl chloride (199 mg, 0.53 mmol) and N,N-diisopropylethylamine (174 mg, 132 mmol) to a solution of 8-(4,4-difluoropiperidin-1-yl)-2-methoxy-1,7-naphthyridin-6-amine (130 mg, 0.44 mmol) in THF (4 mL). The mixture was stirred at 25°C for 16 h. The reaction solution was cooled to ambient temperature and extracted with water (10 mL) and ethyl acetate (10 mL × 2), The organic phases were combined and washed with saturated saline (10 mL×2), dried over anhydrous magnesium sulfate, filtered and concentrated under reduced pressure to give a residue. The residue was purified by flash column chromatography (silica gel, 0-10% gradient of ethyl acetate/petroleum ether) to give N-(8-(4,4-difluoropiperidin-1-yl)-2-methoxy-1,7-naphthyridin-6-yl)-4-iodo-2-(6-azas piro[2.5]octan-6-yl)benzamide (200 mg, 0.31 mmol, yield: 71.5%) as a yellow solid. LCMS (ESI): $[M+H]^+$ =634.5.

Step 5: Cuprous iodide (15.3 mg, 0.08 mmol) was added to a solution of 2-hydroxyethanesulfonamide (39.5.0 mg, 0.32 mmol), sarcosine (22.7 mg, 0.16 mmol), and potassium phosphate (137 mg, 0.63 mmol) in N,N-dimethylformamide (2 mL). The mixture was stirred at 50°C for 5 min. Then N-(8-(4,4-difluoropiperidin-1-yl)-2-methoxy-1,7-naphthyridin-6-yl)-4-iodo-2-(6-azas piro[2.5]octan-6-yl)benzamide (100 mg, 0.16 mmol) was added to the reaction mixture at 50°C, and the mixture was heated to 100°C with stirring for 16 h under nitrogen atmosphere. Water (20 mL) was added to the reaction solution and extracted with ethyl acetate (20 mL × 2). The organic phases were combined and washed with liquor ammonia (20 mL × 2), dried over anhydrous magnesium sulfate, filtered and concentrated under reduced pressure to give a residue. The residue was purified by preparative HPLC (C18, 50-90% gradient of water (formic acid)/acetonitrile) to give N-(8-(4,4-difluoropiperidin-1-yl)-2-methoxy-1,7-naphthyridin-6-yl)-4-(2-hydroxyethy lsulfonamido)-2-(6-azaspiro[2.5]octan-6-yl)benzamide (43 mg, 0.07 mmol, yield: 43.2%) as a white solid compound. LCMS (ESI): $[M+H]^+$ =631.2. $^1H$ NMR (400 MHz, DMSO-d6) δ 12.90 (s, 1H), 10.22 (s, 1H), 8.18 (d, *J* = 9.2 Hz, 1H), 8.12 - 8.06 (m, 2H), 7.27 (d, *J* = *2.0* Hz, 1H), 7.19 - 7.10 (m, 2H), 4.97 (br s, 1H), 4.07 (br s, 4H), 3.96 (s, 3H), 3.76 (br t, *J* = 6.0 Hz, 2H), 3.44 (br s, 2H), 2.99 (br s, 4H), 2.27 - 2.15 (m, 4H), 2.01 - 1.40 (m, 4H), 0.40 (s, 4H)

**Example 53: N-(2-amino-8-(4,4-difluoropiperidin-1-yl)-1,7-naphthyridin-6-yl)-4-(2-hydroxyet hylsulfonamido)-2-(6-azaspiro[2.5]octan-6-yl)benzamide**

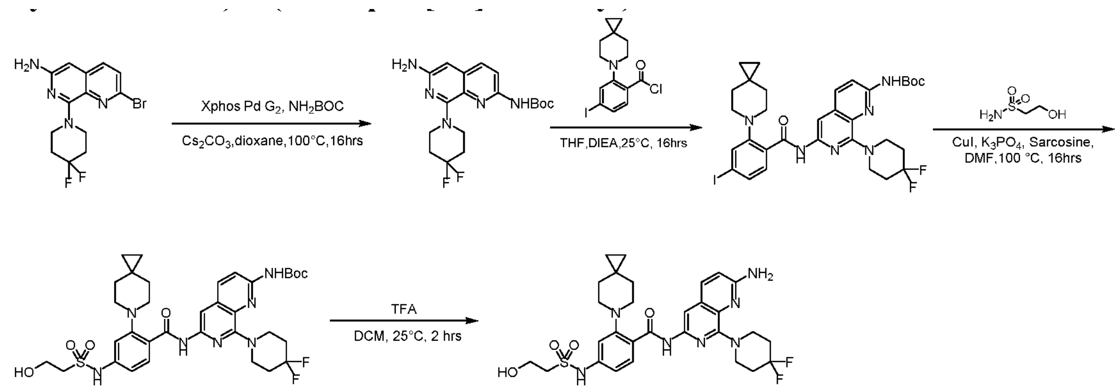


Step 1: Cesium carbonate (1709 mg, 5.25 mmol) and tert-butyl carbamate (310 mg, 2.62 mmol) were added successively to a solution of 2-bromo-8-(4,4-difluoropiperidin-1-yl)-1,7-naphthyridin-6-amine (600 mg, 1.75 mmol) in dioxane (12 mL), and then add chloro(2-dicyclohexylphosphino-2,4,6-triisopropyl-1,1-biphenyl)[2-(2-amino-1,1-bip henyl)] palladium (II) (137 mg, 0.17 mmol). Replace the mixture with nitrogen three times and stir at 100°C for 16 h. The reaction solution was cooled to ambient temperature and extracted with water (50 mL) and ethyl acetate (50 mL × 2), the organic phases were combined and washed with saturated saline (50 mL × 2), dried over anhydrous magnesium sulfate, filtered and concentrated under reduced pressure to give a residue. The residue was purified by flash column chromatography (silica gel, 0-10% gradient of ethyl acetate/petroleum ether) to give tert-butyl(6-amino-8-(4,4-difluoropiperidin-1-yl)-1,7-naphthyridin-2-yl)carbamate (200 mg, 0.52 mmol, yield: 30.2%) as a yellow solid. LCMS (ESI): $[M+H]^+$ =380.1.

Step 2: 4-iodo-2-(6-azaspiro[2.5]octan-6-yl)benzoyl chloride (238 mg, 0.63 mmol) and N,N-diisopropylethylamine (209 mg, 1.58 mmol) were added successively to a solution of tert-butyl(6-amino-8-(4,4-difluoropiperidin-1-yl)-1,7-naphthyridin-2-yl)carbamate (200 mg, 0.53 mmol) in THF (4 mL). The mixture was stirred at 25°C for 16 h. The reaction solution was cooled to ambient temperature and extracted withwater (10 mL) and ethyl acetate (10 mL × 2). The organic phases were combined and washed with saturated saline (10 mL×2), dried over anhydrous sodium sulfate, filtered and concentrated under reduced pressure to give a residue. The residue was purified by flash column chromatography (silica gel, 0-10% gradient of ethyl acetate/petroleum ether) to give tert-butyl (8-(4,4-difluoropiperidin-1-yl)-6-(4-iodo-2-(6-azaspiro[2.5]octan-6-yl)benzoylamino) -1,7-naphthyridin-2-yl)carbamate (110 mg, 0.15 mmol, yield: 29.0%). LCMS (ESI): $[M+H]^+$ =719.1

Step 3: Cuprous iodide (13.5 mg, 0.07 mmol) was added to a solution of 2-hydroxyethanesulfonamide (34.8 mg, 0.28 mmol), sarcosine (19.9 mg, 0.14 mmol), and potassium phosphate (121 mg, 0.56 mmol) in N,N-dimethylformamide (2 mL). The mixture was stirred at 50°C for 5 min. Then tert-butyl (8-(4,4-difluoropiperidin-1-yl)-6-(4-iodo-2-(6-azaspiro[2.5] octan-6-yl)benzoylamino) -1,7-naphthyridin-2-yl)carbamate (100 mg, 0.14 mmol) was added to the reaction mixture at 50°C, and the mixture was heated to 100°C with stirring for 16 h under nitrogen atmosphere. The reaction solution was cooled to ambient temperature, extracted with water (20 mL) and ethyl acetate (20 mL × 2). The combined organic layer was washed with liquor ammonia (20 mL × 2), dried over anhydrous magnesium sulfate, filtered and concentrated under reduced pressure to give tert-butyl (8-(4,4-difluoropiperidin-1-yl)-6-(4-(2-hydroxyethylsulfonamido)-2-(6-azaspiro[2.5]o ctan-6-yl)benzoylamino)-1,7-naphthyridin-2-yl)carbamate (80.0 mg, 0.11 mmol, yield: 80.0%) as the crude. LCMS (ESI): $[M+H]^+$ =716.2

Step 4: Trifluoroacetic acid (0.40 mL) was added to a solution of tert-butyl (8-(4,4-difluoropiperidin-1-yl)-6-(4-(2-hydroxyethylsulfonamido)-2-(6-azaspiro[2.5]o ctan-6-yl)benzoylamino)-1,7-naphthyridin-2-yl)carbamate (80 mg, 0.11 mmol) in dichloromethane (2 mL), and the mixture was stirred at 25°C for 2 h. The reaction solution was concentrated to give a residue, and purified by preparative HPLC (C18, 10-50% gradient of water (formic acid)/acetonitrile) to give N-(2-amino-8-(4,4-difluoropiperidin-1-yl)-1,7-naphthyridin-6-yl)-4-(2-hydroxyethyls ulfonamido)-2-(6-azaspiro[2.5]octan-6-yl) benzamide (19.0 mg, 0.03 mmol, yield: 28.0%) as a yellow solid. LCMS (ESI): $[M+H]^+$ =616.1. $^1$H NMR (400 MHz, DMSO-d6) δ 12.68 (s, 1H), 10.44 - 9.80 (m, 1H), 8.07 (br d, $J$ = 8.4 Hz, 1H), 7.94 (s, 1H), 7.83 (br d, $J$ = 8.9 Hz, 1H), 7.25 (br s, 1H), 7.11 (br d, $J$ = 8.5 Hz, 1H), 6.87 (br d, $J$ = 8.8 Hz, 1H), 6.43 (br s, 2H), 4.96 (br s, 1H), 4.01 (br s, 4H), 3.86 - 3.64 (m, 2H), 3.59 - 3.47 (m, 2H), 2.98 (br s, 4H), 2.24 - 2.05 (m, 4H), 1.96 - 1.38 (m, 4H), 0.39 (br s, 4H)

**Example 54: 2-{6-azaspiro[2.5]octan-6-yl}-N-[8-(4,4-difluoropiperidin-1-yl)-2-hydroxy-1,7-nap hthyridin-6-yl]-4-(2-hydroxyethanesulfonamido)benzamide**

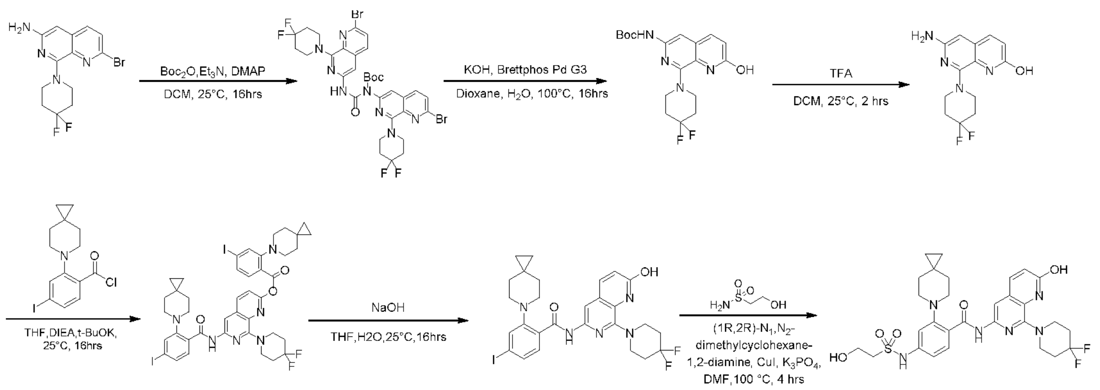


Step 1: Triethylamine (902 mg, 8.74 mmol), 4-dimethylaminopyridine (36.3 mg, 2.91 mmol) and di-tert-butyl dicarbonate (1.91 mg, 8.74 mmol) were successively added to a solution of 2-bromo-8-(4,4-difluoropiperidin-1-yl)-1,7-naphthyridin-6-amine (1.00 mg, 2.91 mmol) in dichloromethane (20 mL). The mixture was stirred at 25°C for 16 h. Water (10 mL) was added to the reaction solution and extracted withethyl acetate (10 mL × 2). The combined organic layer was washedthe combined organic layer was washed with saturated saline (10 mL), dried over magnesium sulfate, filtered, and concentrated under reduced pressure to give the residue. The residue was purified by column chromatography (silica gel, 0-20% gradient of ethyl acetate/petroleum ether) to give tert-butyl N-[2-bromo-8-(4,4-difluoropiperidin-1-yl)-1,7-naphthyridin-6-yl]-N-{[2-bromo-8-(4, 4-difluoropiperidin-1-yl)-1,7-naphthyridin-6-yl]aminocarbonyl}carbamate (1.00 g, 1.22 mmol, yield: 42%). LCMS (ESI): $[M+H]^+$ =812.9.

Step 2: Water (0.22 g, 12.3 mmol), potassium hydroxide (0.69 g, 12.3 mmol), and methanesulfonic acid (2-dicyclohexylphosphino-3,6-dimethoxy-2,4,6-triisopropyl-1,1-biphenyl)(2-amino-1, 1-biphenyl-2-yl) palladium (II) (0.11 g, 0.122 mmol) were successively added to a solution of tert-butyl N-[2-bromo-8-(4,4-difluoropiperidin-1-yl)-1,7-naphthyridin-6-yl]-N-{[2-bromo-8-(4, 4-difluoropiperidin-1-yl)-1,7-naphthyridin-6-yl]aminocarbonyl}carbamate (1.00 g, 1.23 mmol) in dioxane (20 mL). The mixture was heated to 100°C with stirring for 16 h under nitrogen atmosphere and the reaction solution was diluted with water (10 mL), extracted with ethyl acetate (50 mL × 2). Then the combined organic layer was washed with saturated saline (50 mL × 2), dried over magnesium sulfate, filtered, and concentrated under reduced pressure to give the residue. The residue was purified by column chromatography (silica gel, 0-10% gradient of ethyl acetate/petroleum ether) to give tert-butyl N-[8-(4,4-difluoropiperidin-1-yl)-2-hydroxy-1,7-naphthyridin-6-yl]carbamate (301 mg, 0.78 mmol, yield: 64.0%) as a yellow solid. LCMS (ESI): $[M+H]^+$ =381.1.

Step 3: Trifluoroacetic acid (1 mL) was added to a solution of tert-butyl N-[8-(4,4-difluoropiperidin-1-yl)-2-hydroxy-1,7-naphthyridin-6-yl]carbamate (300 mg, 0.78 mmol) in dichloromethane (6 mL). The mixture was stirred at 25°C for 2 h. The reaction solution was quenched with sodium bicarbonate (5 mL), extracted with dichloromethane (10 mL × 2), the combined organic layer was washed with saturated saline (10 mL × 2), dried over magnesium sulfate, filtered, and concentrated under reduced pressure to give 6-amino-8-(4,4-difluoropiperidin-1-yl)-1,7-naphthyridin-2-ol (452 mg, crude) as a yellow solid. LCMS (ESI): $[M+H]^+$ =281.0.

Step 4: 2-{6-azaspiro[2.5]octan-6-yl}-4-iodobenzoyl chloride (723 mg, 1.92 mmol) and N,N-diisopropylethylamine (635 mg, 4.81 mmol) were added successively to a solution of 6-amino-8-(4,4-difluoropiperidin-1-yl)-1,7-naphthyridin-2-ol (450 mg, 1.60 mmol) in THF (10 mL). The mixture was stirred at 25°C for 16 h. The reaction solution was cooled to ambient temperature and extracted with water (10 mL) and ethyl acetate (10 mL × 2). The organic phases were combined and washed with saturated saline (10 mL×2), dried over anhydrous magnesium sulfate, filtered and concentrated under reduced presurre to give a residue. The residue was purified by flash column chromatography (silica gel, 0-10% gradient of ethyl acetate/petroleum ether) to give 6-(2-{6-azaspiro[2.5]octan-6-yl}-4-iodobenzoylamino)-8-(4,4-difluoropiperidin-1-yl) -1,7-naphthyridin-2-yl 2-{6-azaspiro[2.5]octan-6-yl}-4-iodobenzoate (200 mg, 0.21 mmol, yield: 13.0%) as a yellow solid. LCMS (ESI): $[M+H]^+$ =959.0.

Step 5: Sodium hydroxide (25.3 mg, 0.60 mmol) was added to a solution of 6-(2-{6-azaspiro[2.5]octan-6-yl}-4-iodobenzoylamino)-8-(4,4-difluoropiperidin-1-yl) -1,7-naphthyridin-2-yl 2-{6-azaspiro[2.5]octan-6-yl}-4-iodobenzoate (200 mg, 0.20 mmol) in THF (2 mL) and water (2 mL). The mixture was stirred at 25°C for 16 h. The reaction solution was extracted with water (10 mL) and ethyl acetate (10 mL × 2). The combined organic layer was washed with saturated saline (10 mL × 2), dried over anhydrous magnesium sulfate, filtered and concentrated under reduced pressure to give 2-{6-azaspiro[2.5]octan-6-yl}-N-[8-(4,4-difluoropiperidin-1-yl)-2-hydroxy-1,7-napht hyridin-6-yl]-4-iodobenzamide (120 mg, 0.19 mmol, yield: 93.8%) as a yellow solid. LCMS (ESI): $[M+H]^+$ =620.1

Step 6: Cuprous iodide (36.9 mg, 0.19 mmol) was added to a solution of 2-hydroxyethanesulfonamide (48.5 mg, 0.38 mmol), trans-N,N-dimethyl-1,2-cyclohexanediamine (27.8 mg, 0.19 mmol), potassium phosphate (164 mg, 0.77 mmol) in N,N-dimethylformamide (3 mL). The mixture was stirred at 50°C for 5 min. Then 2-{6-Azaspiro[2.5]octan-6-yl}-*N*-[8-(4,4-difluoropiperidin-1-yl)-2-hydroxy-1,7-napht hyridin-6-yl]-4-iodobenzamide was added to the reaction mixture at

50°C, and the mixture was heated to 100°C with stirring for 4 h under nitrogen atmosphere. Water (20 mL) was added to the reaction solution and extracted withethyl acetate (20 mL × 2). The organic phases were combined and washed with liquor ammonia (20 mL × 2), dried over anhydrous magnesium sulfate, filtered and concentrated under reduced pressure to give a residue. The residue was purified by preparative HPLC (C18, 26-66% gradient of water (formic acid)/acetonitrile) to give 2-{6-azaspiro[2.5]octan-6-yl}-N-[8-(4,4-difluoropiperidin-1-yl)-2-hydroxy-1,7-napht hyridin-6-yl]-4-(2-hydroxyethanesulfonamido)benzamide (23 mg, 0.03 mmol, yield: 19.2 %) as a white solid compound. LCMS (ESI): $[M+H]^+$ =632.5. $^1H$ NMR (400 MHz, DMSO-d6) δ 13.15 (s, 1H), 11.53 - 10.45 (m, 1H), 10.16 (s, 1H), 8.12 (s, 1H), 8.01 (d, *J* = 8.8 Hz, 1H), 7.90 (d, *J* = 9.6 Hz, 1H), 7.19 (d, *J* = 1.6 Hz, 1H), 7.06 (dd, *J* = 1.6, 8.8 Hz, 1H), 6.69 (d, *J* = 9.6 Hz, 1H), 5.30 - 4.75 (m, 1H), 3.69 (br t, *J* = 6.4 Hz, 2H), 3.29 - 3.27 (m, 2H), 3.22 - 3.16 (m, 4H), 2.94 - 2.82 (m, 4H), 2.27 - 2.16 (m, 4H), 1.98 - 1.42 (m, 4H), 0.31 (s, 4H)

**Example 55: Synthesis of N-(1-(4,4-difluoropiperidin-1-yl)isoquinolin-3-yl)-4-(2-hydroxyethyl)sulfonamido) -2-(6-azaspiro[2.5]octan-6-yl)benzamide**

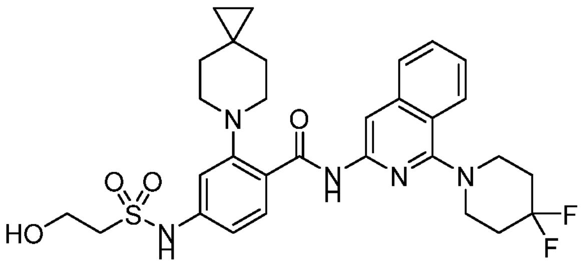

The preparations in **Example 55** follow the synthetic route of **Example 3:** LCMS (ESI) m/z: $[M+H]^+$ = 600.4. $^1H$-NMR (400 MHz, DMSO) δ 13.14 (s, 1H), 10.21 (s, 1H), 8.27 (s, 1H), 8.09 (dd, *J* = 18.6, 8.4 Hz, 2H), 7.85 (d, *J* = 8.2 Hz, 1H), 7.66 (ddd, *J* = 8.0, 6.8, 1.2 Hz, 1H), 7.47 (ddd, *J* = 8.4, 6.8, 1.2 Hz, 1H), 7.29 (d, *J* = 2.4 Hz, 1H), 7.15 (dd, *J*= 8.8, 2.0 Hz, 1H), 4.95 (s, 1H), 3.77 (s, 2H), 3.51 (t, *J* = 5.6 Hz, 4H), 3.37 (t, *J* = 6.4 Hz, 2H), 3.00 (s, 4H), 2.34 - 2.23 (m, 4H), 2.08 - 1.54 (m, 5H), 1.24 (s, 1H), 0.40 (s, 4H).

**Example 56: N-(1-(4,4-difluoropiperidin-1-yl)-2,7-naphthyridin-3-yl)-4-(2-hydroxyethyl)sulfon amide)-2-(6-azaspiro[2.5]octan-6-yl)benzamide**

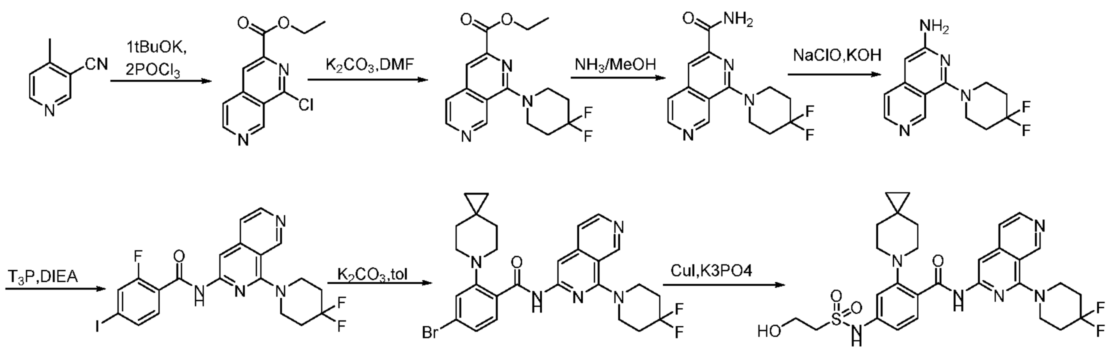


Step 1: 4-methylnicotinonitrile (5.00 g, 42.32 mmol) and diethyl oxalate (9.37 g, 63.48 mmol) were added successively to toluene (50 mL), and the reaction solution was stirred at 25°C for 5 min. Potassium tert-butoxide (19.18 g, 169.30 mmol) was then added to the reaction solution within half an hour in 3 portions and stir at 25°C for 48 h. The reaction solution was filtered under reduced pressure, then the solid obtained was dissolved by filtration in water (2 L), the pH was adjusted to 3 with 1 M hydrochloric acid, and stir for 24 h at 25°C. The solution was extracted with dichloromethane (300 mL × 4). The combined organic layer was dried over anhydrous sodium sulfate, and concentrated under reduced pressure to give the crude. The crude residue was added to phosphorus oxychloride (10 mL), and the reaction solution was stirred at 95°C for 2 h. The reaction solution was concentrated and slowly added to ice water (100 mL) while stirring. The pH of the resulting solution was adjusted to 7 with sodium bicarbonate solids, extracted with dichloromethane (100 mL × 3). The combined organic layer was dried over anhydrous sodium sulfate, and concentrate to give the crude. The crude was

purified by flash column chromatography (silica gel, 0-10% gradient of ethyl acetate/petroleum ether) to give ethyl 1-chloro-2,7-naphthyridine-3-carboxylate (2.40 g, 37%) as a white solid. LCMS (ESI): $[M+H]^+$ =237.1, 239.1. $^1$H NMR (400 MHz, DMSO-$d_6$) δ 9.73 (d, *J* = 1.0 Hz, 1H), 8.99 (d, *J* = 5.6 Hz, 1H), 8.72 (d, *J* = 0.9 Hz, 1H), 8.23 (dd, *J* = 5.7, 1.1 Hz, 1H), 4.43 (q, *J*= 7.1 Hz, 2H), 1.39 (t, *J* = 7.1 Hz, 3H).

Step 2: Ethyl 1-chloro-2,7-naphthyridine-3-carboxylate (2.40 g, 10.14 mmol), 4,4-difluoropiperidine hydrochloride (1.94 g, 12.17 mmol), and potassium carbonate (5.00 g, 35.49 mmol) were successively added to N-methylpyrrolidone (5 mL), and the reaction solution was stirred at 95°C for 24 h. The reaction solution was poured into the saturated ammonium chloride solution (50 mL), extracted with ethyl acetate (50 mL × 3). The combined organic layer was washed with the saturated sodium chloride solution (30 mL × 3), the combined organic layer was dried over anhydrous sodium sulfate, and concentrated to obtain the crude. The crude was purified by flash column chromatography (silica gel, 0-10% gradient of ethyl acetate/petroleum ether) to give ethyl 1-(4,4-difluoropiperidin-1-yl)-2,7-naphthyridine-3-carboxylate (2.20 g, 68%) as a pale yellow solid. LCMS (ESI): $[M+H]^+$ = 322.2.

Step 3: Ethyl 1-(4,4-difluoropiperidin-1-yl)-2,7-naphthyridine-3-carboxylate (1.00 g, 3.11 mmol) was added to a 100 mL sealed tube, and 7 M ammonia methanol solution (5 mL) was added to a reaction tube, then the tube was sealed and the reaction system was stirred at 85°C for 16 h. The reaction solution was cooled to ambient temperature and concentrated to give 1-(4,4-difluoropiperidin-1-yl)-2,7-naphthyridine-3-carboxamide (850 mg, 93%) as a pale yellow solid. LCMS (ESI): $[M+H]^+$ = 293.1.

Step 4: 1-(4,4-difluoropiperidin-1-yl)-2,7-naphthyridine-3-carboxamide (600 mg, 2.05 mmol) was added to 1,4-dioxane (60 mL) and potassium hydroxide (291 mg, 5.13 mmol) was added to the reaction solution. The reaction solution was stirred at 25°C for 5 min, and 8% sodium hypochlorite solution (4.25 g) was added dropwise to the reaction solution. The reaction solution was stirred at ambient temperature for 6 h. Saturated sodium thiosulfate solution (30 mL) was added to the reaction solution, stirred at ambient temperature for 15 min, extracted withethyl acetate (30 mL×3), The combined organic layer was dried over anhydrous sodium sulfate, and concentrate to give the crude. The crude was purified by flash column chromatography (silica gel, 10-33% gradient of ethyl acetate/dichloromethane) to give 1-(4,4-difluoropiperidin-1-yl)-2,7-naphthyridin-3-amine (420 mg, 77%) as a yellow solid. LCMS (ESI): $[M+H]^+$= 265.1.

Step 5: 1-(4,4-difluoropiperidin-1-yl)-2,7-naphthyridin-3-amine (420 mg, 1.59 mmol), 2-fluoro-4-iodobenzoic acid (635 mg, 2.38 mmol), N,N-diisopropylethylamine (1.05 g, 7.95 mmol), and 50% solution of 1-propylphosphonic anhydride in ethyl acetate (3.03 g, 4.77 mmol) were successively added to acetonitrile (5 mL), and the reaction solution was stirred at 85°C for 16 h. The reaction solution was poured into water (30 mL), extracted with ethyl acetate (30 mL × 3). The combined organic layer was dried over anhydrous sodium sulfate, and concentrated to obtain the crude. The crude was purified by flash column chromatography (silica gel, 30-50% gradient of ethyl acetate/petroleum ether) to give N-(1-(4,4-difluoropiperidin-1-yl)-2,7-naphthyridin-3-yl)-2-fluoro-4-iodobenzamide (560 mg, 69%) as a pale yellow solid. LCMS (ESI): $[M+H]^+$ = 513.1.

Step 6: N-(1-(4,4-difluoropiperidin-1-yl)-2,7-naphthyridin-3-yl)-2-fluoro-4-iodobenzamide (190 mg, 0.37 mmol), 6-aza-spiro[2.5]octane hydrochloride (83 mg, 0.56 mmol), and potassium carbonate (131 mg, 0.93 mmol) were added successively to toluene (5 mL), and the reaction solution was stirred at 130°C for 16 h. The reaction solution was poured into saturated aqueous ammonium chloride solution (30 mL), extracted with ethyl acetate (30 mL × 3). The combined organic layer was dried over anhydrous sodium sulfate, and concentrate to obtain the crude. The crude was purified by flash column chromatography (silica gel, 20-50% gradient of ethyl acetate/dichloromethane) to give N-(1-(4,4-difluoropiperidin-1-yl)-2,7-naphthyridin-3-yl)-4-iodo-2-(6-azaspiro[2.5]oct an-6-yl)benzamide (200 mg, 89%) as a pale yellow solid. LCMS (ESI): $[M+H]^+$ = 604.3.

Step 7: *N*-(1-(4,4-difluoropiperidin-1-yl)-2,7-naphthyridin-3-yl)-4-iodo-2-(6-azaspiro[2.5]oct an-6-yl)benzamide (200 mg, 0.33 mmol), 2-hydroxyethanesulfonamide (63 mg, 0.50 mmol), cuprous iodide (64 mg, 0.33 mmol), (1*S*,2*S*)-(+)-*N,N'*-dimethyl-1,2-cyclohexanediamine (48 mg, 0.33 mmol), and potassium phosphate (179 mg, 0.83 mmol) were successively added to *N,N*-dimethylformamide (2 mL), and the reaction solution was stirred at 115°C for 4 h under nitrogen atmosphere. The reaction solution was poured into the saturated aqueous ammonium chloride solution (30 mL), extracted with ethyl acetate (30 mL×3). The combined organic layer was washed with the saturated aqueous sodium chloride solution (30 mL × 3), the combined organic layer was dried over anhydrous sodium sulfate, and concentrate to obtain the crude. The crude was purified by flash column chromatography (silica gel, 10-50% gradient of ethyl acetate/dichloromethane) to give N-(1-(4,4-difluoropiperidin-1-yl)-2,7-naphthyridin-3-yl)-4-(2-hydroxyethyl)sulfonami de)-2-(6-azaspiro[2.5]octan-6-yl)benzamide (93 mg, 47%) as a white solid. LCMS (ESI): $[M+H]^+$ =601.4. $^1$HNMR(400MHz, DMSO-$d_6$) δ13.32 (s, 1H),10.49(s, 1H), 9.33 (s, 1H), 8.53 (d, *J* = 5.8 Hz, 1H), 8.18 (s, 1H), 8.10 (d, *J* = 8.6 Hz, 1H), 7.72 (d, *J* = 5.8 Hz, 1H), 7.30 (d, *J* = 2.2 Hz, 1H), 7.15 (dd, *J* = 8.6, 2.1 Hz, 1H), 5.76 (s, 1H), 3.77 (t, *J* = 6.4 Hz, 2H), 3.65 (dd, *J* = 7.2, 4.2 Hz, 4H), 3.40-3.32 (m, 2H), 3.00 (t, *J* = 5.3 Hz, 4H), 2.29 (tt, *J* = 14.3, 5.6 Hz, 4H), 1.89 (d, *J* = 84.2 Hz, 4H), 0.41 (s, 4H).

**Example 57: N-(5-(4,4-difluoropiperidin-1-yl)-1,6-naphthyridin-7-yl)-4-(2-hydroxyethyl)sulfa moyl)-2-(6-azaspiro[2.5]octan-6-yl)benzamide**

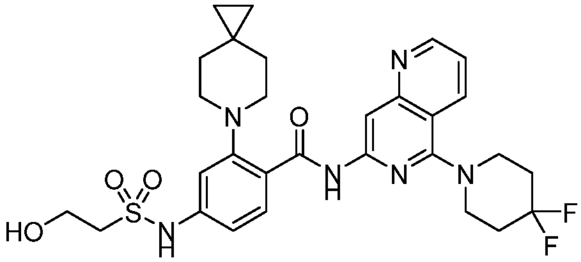

The preparations in **Example 57** follow the synthetic route of **Example 3:** LCMS (ESI): $[M+H]^+$ = 601.3. $^1$H NMR (400 MHz, DMSO) δ 13.29 (s, 1H), 10.24 (s, 1H), 8.95 (d, *J* = 4.0 Hz, 1H), 8.44 (d, *J* = 8.4 Hz, 1H), 8.32 (s, 1H), 8.13 (d, *J* = 8.8 Hz, 1H), 7.44 (dd, *J* = 8.4, 4.4 Hz, 1H), 7.30 (s, 1H), 7.15 (d, *J* = 8.8 Hz, 1H), 5.16 - 4.84 (m, 1H), 3.77 (q, *J* = 6.0 Hz, 2H), 3.56 (t, *J* = 5.6 Hz, 4H), 3.17 (s, 2H), 3.01 (s, 4H), 2.47-2.10 (m, 4H), 1.78 (s, 4H), 0.41 (s, 4H).

**Example 58: N-(8-(4,4-difluoropiperidin-1-yl)pyrido[3,4-d]pyrimidin-6-yl)-4-((2-hydroxyethyl) sulfonamide)-2-(6-azaspiro[2.5]octan-6-methyl)benzamide**

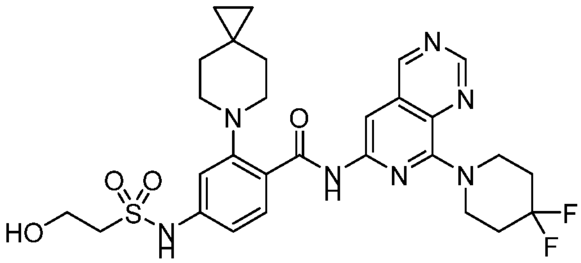

The preparations in **Example 58** follow the synthetic route of **Example 3:** LCMS (ESI): [M+H]+ = 602.10. $^1$H NMR (400 MHz, CDCl3) δ 12.99 (s, 1H), 9.30 (d, J = 2.0 Hz, 1H), 9.14 (s, 1H), 8.28 (d, J = 8.4 Hz, 1H), 8.20 (s, 1H), 7.35 (d, J = 7.2 Hz, 1H), 7.11 (s, 2H), 5.46 - 5.29 (m, 1H), 4.30 (t, J = 6.0 Hz, 4H), 4.15 (t, J = 5.2 Hz, 2H), 3.35 (t, J = 5.2 Hz, 2H), 3.11 (s, 4H), 2.21 (dd, J = 12.8, 6.8 Hz, 4H), 1.43 (s, 4H), 0.44 (s, 4H).

**Example 59: N-(5-(4,4-difluoropiperidin-1-yl)pyrido[3,4-b]pyrazin-7-yl)-4-(2-hydroxyethyl)sul fonamido)-2-(6-azaspiro[2.5]octan-6-yl)benzamide**

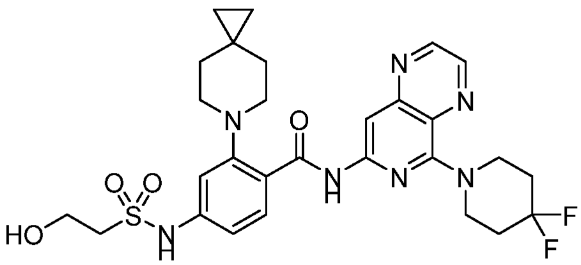

The preparations in **Example 59** follow the synthetic route of **Example 3:** LCMS (ESI) m/z: $[M+H]^+$ = 602.4. $^1$H NMR (400 MHz, DMSO) δ 13.07 (s, 1H), 10.24 (s, 1H), 8.93 (d, *J* = 1.6 Hz, 1H), 8.69 (d, *J* = 2.0 Hz, 1H), 8.17 - 8.08 (m, 2H), 7.30 (d, *J* = 2.0 Hz, 1H), 7.15 (d, *J* = 8.4, 2.1 Hz, 1H), 4.95 (s, 1H), 4.25-4.21 (m, 4H), 3.78 (t, *J* = 5.6 Hz, 2H), 3.37 (t, *J* = 6.4 Hz, 2H), 3.06-2.96 (m, 4H), 2.22 - 2.15 (m, 4H), 1.77-1.73 (m, 4H), 0.41 (s, 4H).

**Example 60: N-(8-(4,4-difluoropiperidin-1-yl)-3-methoxypyrido[3,4-c]pyridazin-6-yl)-4-(2-hyd roxyethylsulfonamido)-2-(6-azaspiro[2.5]octan-6-yl)benzamide**

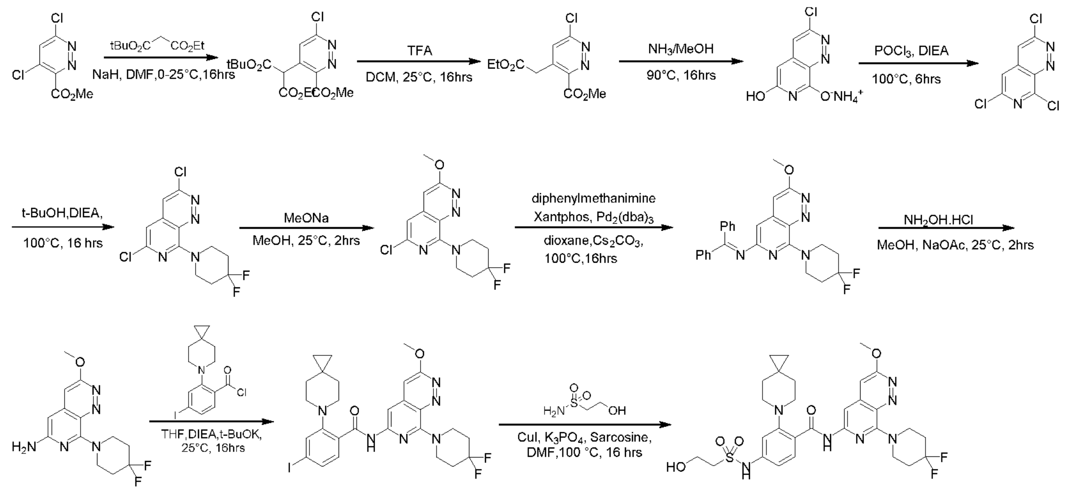


Step 1: Sodium hydrogen (4.25 g, 106 mmol, 60% purity) was added to a solution of tert-butyl ethyl malonate (21.8 g, 116 mmol) in N,N-dimethylformamide (220 mL). The mixture was stirred at 0°C for 1 h, then methyl 4,6-dichloropyridazine-3-carboxylate (20.0 g, 96.6 mmol) was added to the reaction mixture at 0°C. And the mixture was stirred at 25°C for 16 h under nitrogen atmosphere. The reaction solution was slowly poured into water (50 mL), extracted with ethyl acetate (100 mL×2), dried over magnesium sulfate, filtered, and concentrated under reduced pressure to give the residue. The residue was purified by column chromatography (silica gel, 0-15% gradient of ethyl acetate/petroleum ether) to give 1-tert-butyl 3-ethyl 2-(6-chloro-3-(methylester)pyridazin-4-yl)malonate (32.0 g, 89.2 mmol, yield: 92.3%) as a yellow oil. LCMS (ESI): $[M+H]^+$ =359.1

Step 2: Trifluoroacetic acid (160 mL) was added to a solution of 1-tert-butyl 3-ethyl 2-(6-chloro-3-(methylester) pyridazin-4-yl)malonate (32.0 g, 89.2 mmol) in dichloromethane (320 mL), and the mixture was stirred at 25°C for 16 h. The pH of the reaction solution was adjusted to 7 with saturated sodium bicarbonate, extracted with dichloromethane (500 mL×2), the combined organic layer was washed with saturated saline (500 mL×2), dried over magnesium sulfate, filtered, and concentrated under reduced pressure to give methyl 6-chloro-4-(2-ethoxy-2-oxoethylidene)pyridazine-3-carboxylate (22.0 g, 85.1 mmol, yield: 95.4%) as a white solid. LCMS (ESI): $[M+H]^+$ =259.0.

Step 3: Methyl 6-chloro-4-(2-ethoxy-2-oxoethylidene)pyridazine-3-carboxylate (20.0 g, 77.3 mmol) was dissolved in ammonia methanol solution (200 mL). The mixture was stirred at 90°C for 16 h. The reaction solution was concentrated under reduced pressure to give ammonium 3-chloro-6-hydroxypyrido[3,4-c]pyridazin-8-ol anion (15.0 g, 75.9 mmol, yield: 98.2%) as a yellow solid. LCMS (ESI): $[M+H]^+$ =198.0. $^1H$ NMR (400 MHz, DMSO-d6) δ 10.67 - 9.72 (m, 1H), 7.64 - 6.86 (m, 4H), 6.81 (s, 1H), 4.65 (s, 1H)

Step 4: N,N-diisopropylethylamine (10.0 g, 75.9 mol) was added to a solution of ammonium 3-chloro-6-hydroxypyrido[3,4-c]pyridazin-8-ol anion (7.50 g, 38.0 mmol) in phosphorus oxychloride (150 mL), and the mixture was stirred at 100°C for 6 h. The reaction solution was concentrated under reduced pressure to give the residue. The residue was purified by column chromatography (silica gel, 0-15% gradient of ethyl acetate/petroleum ether) to give 3,6,8-trichloropyrido[3,4-c]pyridazine (6.50 g, 27.7 mmol, yield: 73.0%). LCMS (ESI): $[M+H]^+$ =233.9.

Step 5: 4,4-difluoropiperidine (4.37 g, 27.7 mmol) and N,N-diisopropylethylamine (36.6 g, 277 mol) were successively added to a solution of 3,6,8-trichloropyrido[3,4-c]pyridazine (6.50 g, 27.7 mmol) in tert-butanol (130 mL). The mixture was stirred at 100°C for 16 h. The reaction solution was diluted with water (100 mL), extracted with ethyl acetate (100 mL × 2), the combined organic layer was washed with saturated saline (100 mL × 2), dried over magnesium sulfate, filtered, and concentrated under reduced pressure to give the residue. The residue was purified by column chromatography (silica gel, 0-20% gradient of ethyl acetate/petroleum ether) to give 3,6-dichloro-8-(4,4-difluoropiperidin-1-yl)pyrido[3,4-c]pyridazine (6.10 g, 19.1 mmol, yield: 69.0%) as a yellow solid. LCMS (ESI): $[M+H]^+$ =319.0. $^1H$ NMR (400 MHz, DMSO-d6) δ 8.21 (s, 1H), 7.10 (s, 1H), 4.40 - 4.30 (m, 4H), 2.27 - 2.14 (m, 4H)

Step 6: 3,6-dichloro-8-(4,4-difluoropiperidin-1-yl)pyrido[3,4-c]pyridazine (150 mg, 0.47 mmol) was dissolved in a solution of sodium methoxide (3 mL). The mixture was stirred at 25°C for 16 h. The reaction solution was poured into ice water (10 mL), extracted with ethyl acetate (10 mL×3), and the combined organic layer was washed with saturated saline (10 mL×2), dried over magnesium sulfate, filtered, and concentrated under reduced pressure to give 6-chloro-8-(4,4-difluoropiperidin-1-yl)-3-methoxypyrido[3,4-c]pyridazine (125 mg, 0.39 mmol, yield: 84.5%) as a yellow solid. LCMS (ESI): $[M+H]^+$ =315.0. $^1H$ NMR (400 MHz, DMSO-d6) δ 7.37 (s, 1H), 7.04 (s, 1H), 4.47 - 4.34 (m, 4H), 4.16 (s, 3H), 2.25 - 2.15 (m, 4H)

Step 7: Cesium carbonate (311 mg, 0.95 mmol), chloro(2-dicyclohexylphosphino-2,4,6-triisopropyl-1,1-biphenyl)[2-(2-amino-1,1-bip henyl)] palladium (II) (29.1 mg, 0.03 mmol), 5-bis(diphenylphosphine)-9,9-dimethoxyanthracene (36.7 mg, 0.06 mmol) and benzophenonimine (70.5 mg, 0.38 mmol) were successively added to a solution of 6-chloro-8-(4,4-difluoropiperidin-1-yl)-3-methoxypyrido[3,4-c]pyridazine (100 mg, 0.31 mmol) in dioxane (2 mL). The mixture was exchanged with nitrogen three times and stirred at 100°C for 16 h. The reaction solution was cooled to ambient temperature and extracted with water (10 mL) and ethyl acetate (10 mL × 2). The organic phases were combined and washed with saturated saline (10 mL× 2), dried over anhydrous magnesium sulfate, filtered and concentrated under reduced pressure to give a residue. The residue was purified by flash column chromatography (silica gel, 0-15% gradient of ethyl acetate/petroleum ether) to give 8-(4,4-difluoropiperidin-1-yl)-N-(diphenylmethylene)-3-methoxypyrido[3,4-c]pyridaz in-6-amine (110 mg, 0.24 mmol, yield: 75.3%) as a white solid. LCMS (ESI): $[M+H]^+$ =460.2

Step 8: Sodium acetate (53.6 mg, 0.65 mmol) and hydroxylamine hydrochloride (30.3 mg, 0.44 mol) were added successively to a solution of 8-(4,4-difluoropiperidin-1-yl)-N-(diphenylmethylene)-3-methoxypyrido[3,4-c]pyridaz in-6-amine (100 mg, 0.22 mmol) in methanol (2 mL). The mixture was stirred at 25°C for 2 h. The reaction solution was diluted with water (10 mL), extracted with ethyl acetate (10 mL × 2), the combined organic layer was washed with saturated saline (10 mL × 2), dred over magnesium sulfate, filtered, and concentrated under reduced pressure to give the residue. The residue was purified by column chromatography (silica gel, 0-10% gradient of ethyl acetate/petroleum ether) to give 8-(4,4-difluoropiperidin-1-yl)-3-methoxypyrido[3,4-c]pyridazine-6-amine (50.0 mg, 0.17 mmol, yield: 77.8%) as a yellow solid. LCMS (ESI): $[M+H]^+$ =296.1

Step 9: The solution of N,N-diisopropylethylamine (60.3 mg, 0.46 mmol) and potassium tert-butoxide (0.45 mL, 0.18 mmol, 1M) in THF was added to a solution of 8-(4,4-difluoropiperidin-1-yl)-3-methoxypyrido[3,4-c]pyridazine-6-amine (45 mg, 0.15 mmol) and 4-iodo-2-(6-azaspiro[2.5]octan-6-yl)benzoyl chloride (68.7 mg, 0.18 mmol) in THF (2 mL). The mixture was stirred at 25°C for 16 h. The reaction solution was cooled to ambient temperature and extracted with water (10 mL) and ethyl acetate (10 mL × 2), the organic phases were combined and washed with saturated saline (10 mL×2), dried over anhydrous magnesium sulfate, filtered and concentrated under reduced pressure to give a residue. The residue was purified by flash column chromatography (silica gel, 0-10% gradient of ethyl acetate/petroleum ether) to give N-(8-(4,4-difluoropiperidin-1-yl)-3-methoxypyrido[3,4-c]pyridazin-6-yl)-4-iodo-2-(6-azaspiro[2.5]octan-6-yl)benzamide (80.0 mg, 0.124 mmol, yield: 82.7%) as a yellow solid. LCMS (ESI): $[M+H]^+$ =635.1

Step 10: Cuprous iodide (12.3 mg, 0.06 mmol) was added to a solution of 2-hydroxyethanesulfonamide (31.6 mg, 0.25 mmol), sarcosine (11.2 mg, 0.13 mmol), and potassium phosphate (109 mg, 0.50 mmol) in N,N-dimethylformamide (2 mL). The mixture was stirred at 50°C for 5 min. Then N-(8-(4,4-difluoropiperidin-1-yl)-3-methoxypyrido[3,4-c]pyridazin-6-yl)-4-iodo-2-(6-azaspiro[2.5]octan-6-yl)benzamide (80 mg, 0.13 mmol) was added to the reaction mixture at 50°C, and the mixture was heated to 100°C with stirring for 16 h under nitrogen atmosphere. The reaction solution was cooled to ambient temperature and extracted with water (20 mL) and ethyl acetate (20 mL×2). The organic phases were combined and wash with ammonia (20 mL×2), dried over anhydrous magnesium sulfate, filtered and concentrated under reduced pressure to give a residue. The residue was purified by preparative HPLC (C18, 42-82% gradient of water (formic acid)/acetonitrile) to give N-(8-(4,4-difluoropiperidin-1-yl)-3-methoxypyrido[3,4-c]pyridazin-6-yl)-4-(2-hydrox yethylsulfonamido)-2-(6-azaspiro[2.5]octan-6-yl)benzamide (31.0 mg, 0.05 mmol, yield: 38.9%) as a yellow solid. LCMS (ESI): $[M+H]^+$ =632.5. $^1H$ NMR (400 MHz, DMSO-d6) δ 12.99 (s, 1H), 10.26 (s, 1H), 8.09 (d, *J* = 8.8 Hz, 1H), 7.87 (d, *J* = 1.2 Hz, 1H), 7.39 (d, *J* = 2.0 Hz, 1H), 7.29 (d, *J* = 2.0 Hz, 1H), 7.15 (dd, *J* = 2.0, 8.4 Hz, 1H), 4.97 (br t, *J* = 5.6 Hz, 1H), 4.50 - 4.28 (m, 4H), 4.14 (s, 3H), 3.85 - 3.67 (m, 2H), 3.45 - 3.37 (m, 2H), 3.12 - 2.91 (m, 4H), 2.30 - 2.12 (m, 4H), 2.02 - 1.47 (m, 4H), 0.41 (s, 4H)

**Example 61: 2-{6-Azaspiro[2.5]octan-6-yl}-N-[8-(4,4-difluoropiperidin-1-yl)-3-hydroxypyrido[ 3,4-c]pyridazin-6-yl]-4-(2-hydroxyethanesulfonamido)benzamide**

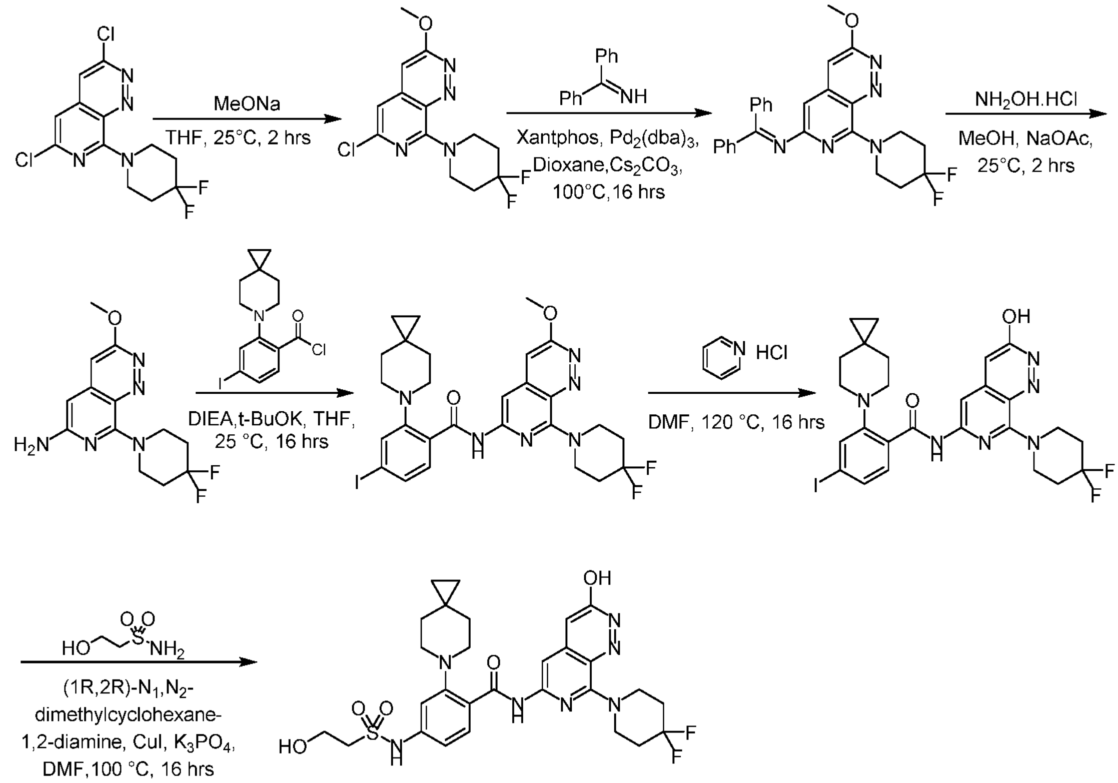


Step 1: The solution of sodium methoxide (6 mL, 5.4 M) was added to the solution of 1-{3,6-dichloropyrido[3,4-c]pyridazin-8-yl}-4,4-difluoropiperidine (600 mg, 1.88 mmol) in THF (6 mL). The mixture was stirred at 25°C for 2 h. The reaction solution was poured into ice water (20 mL) and extracted withethyl acetate (20 mL × 3). The combined organic layer was dried over anhydrous sodium sulfate, filtered and concentrated under reduced pressure to give 1-{6-chloro-3-methoxypyrido[3,4-c]pyridazin-8-yl}-4,4-difluoropiperidine (250 mg, 0.79 mmol, yield: 42%) as a yellow solid. LCMS (ESI): [M+H]+ = 315.0. $^{1}$H NMR (400 MHz, DMSO-d6) δ ppm 7.37 (s, 1H), 7.04 (s, 1H), 4.46 - 4.27 (m, 4H), 4.16 (s, 3H), 2.26 - 2.12 (m, 4H)

Step 2: Benzophenonimine (151 mg, 0.83 mmol), cesium carbonate (528 mg, 1.59 mmol), 4,5-bis(diphenylphosphino)-9,9-dimethylxanthene (93.8 mg, 0.16 mmol) and tris(dibenzylideneacetone) dipalladium (74.2 mg, 0.08 mmol) were successively added to a solution of 1-{6-chloro-3-methoxypyrido[3,4-c]pyridazin-8-yl}-4,4-difluoropiperidine (250 mg, 0.79 mmol) in dioxane (5 mL). Replace the reaction solution with nitrogen three times, then the reaction system was heated to 100°C under nitrogen atmosphere and stirred for 16 h. The reaction solution was cooled to ambient temperature and extracted withwater (20 mL) and ethyl acetate (20 mL × 3). The organic phases were combined and washed with saturated saline (20 mL), dried over anhydrous sodium sulfate, filtered and concentrated under reduced pressure to give a residue. The residue was purified by flash column chromatography (silica gel, 0-9% gradient of THF/petroleum ether) to give N-[8-(4,4-difluoropiperidin-1-yl)-3-methoxypyrido[3,4-c]pyridazin-6-yl]-1,1-dipheny lmethanimine (230 mg, 0.50 mmol, yield: 63%) as a yellow solid. LCMS (ESI): [M+H]+ = 460.2

Step 3: Sodium acetate (112 mg, 1.37 mmol) and hydroxylamine hydrochloride (63.7 mg, 0.91 mmol) were successively added to a solution of N-[8-(4,4-difluoropiperidin-1-yl)-3-methoxypyrido[3,4-c]pyridazin-6-yl]-1,1-dipheny lmethanimine (210 mg, 0.46 mmol) in methanol (4 mL). The mixture was stirred at 25°C for 2 h. The reaction solution was poured into ice water (20 mL) and extracted with ethyl acetate (20 mL × 3). The combined organic layer was dried over anhydrous sodium sulfate, filtered and concentrated under reduced pressure to give the residue. The residue was purified by flash column chromatography (silica gel, 0-12% gradient of THF/petroleum ether) to give 8-(4,4-difluoropiperidin-1-yl)-3-methoxypyrido[3,4-c]pyridazine-6-amine (130 mg, 0.44 mmol, yield: 96%) as a yellow solid. LCMS (ESI): [M+H]+ = 296.0. $^{1}$H NMR (400 MHz, DMSO-d6) δ ppm 6.77 (s, 1H), 6.42 (br s, 2H), 5.70 (s, 1H), 4.29 - 4.18 (m, 4H), 4.02 (s, 3H), 2.21 - 2.04 (m, 4H)

Step 4: Potassium tert-butoxide (1.02 mL, 1M) in THF and N,N-diisopropylethylamine (134 mg, 1.02 mmol) was added to a solution of 8-(4,4-difluoropiperidin-1-yl)-3-methoxypyrido[3,4-c]pyridazine-6-amine (100 mg, 0.34 mmol) and 2-(6-azaspiro[2.5]octan-6-yl)-4-iodobenzoyl chloride (165 mg, 0.44 mmol) in THF (2 mL). The mixture was stirred at 25°C for 16 h. The reaction solution was extracted with water (20 mL) and ethyl acetate (20 mL×3). The organic phases were combined and washed with saturated saline (20 mL), dried over anhydrous sodium sulfate, filtered and concentrated under reduced pressure to give a residue. The residue was purified by flash column chromatography (silica gel, 0-10%

gradient of ethyl acetate/petroleum ether) to give 2-{6-azaspiro[2.5]octan-6-yl}-N-[8-(4,4-difluoropiperidin-1-yl)-3-methoxypyrido[3,4 -c]pyridazin-6-yl]-4-iodobenzamide (110 mg, 0.17 mmol, yield: 51%) as a yellow solid. LCMS (ESI): [M+H]+ = 635.1

Step 5: Pyridine hydrochloride (200 mg, 1.73 mmol) was added to a solution of 2-{6-azaspiro[2.5]octan-6-yl}-N-[8-(4,4-difluoropiperidin-1-yl)-3-methoxypyrido[3,4 -c]pyridazin-6-yl]-4-iodobenzamide (110 mg, 0.17 mmol) in N,N-dimethylformamide (5.00 mL). The mixture was stirred at 120°C for 16 h. The reaction solution was cooled to ambient temperature and extracted with water (20 mL) and ethyl acetate (20 mL × 3). The organic phases were combined and washed with saturated saline (20 mL), dried over anhydrous sodium sulfate, filtered and concentrated under reduced pressure to give a residue. The residue was purified by flash column chromatography (silica gel, 0-7% gradient of methanol/dichloromethane) to give 2-{6-azaspiro[2.5]octan-6-yl}-N-[8-(4,4-difluoropiperidin-1-yl)-3-hydroxypyrido[3,4-c]pyridazin-6-yl]-4-iodobenzamide (100 mg, 0.16 mmol, yield: 93%) as an orange solid. LCMS (ESI): [M+H]+ = 621.4

Step 6: Cuprous iodide (12.5 mg, 0.06 mmol) and potassium phosphate (112 mg, 0.52 mmol) were successively added to a solution of 2-hydroxyethanesulfonamide (21.0 mg, 0.17 mmol) and (1R,2R)-(-)-N,N-dimethylcyclohexane-1,2-diamine (18.5 mg, 0.13 mmol) in N,N-dimethylformamide (2 mL). The mixture was heated to 50°C and stir for 5 min. 2-{6-Azaspiro[2.5]octan-6-yl}-N-[8-(4,4-difluoropiperidin-1-yl)-3-hydroxypyrido[3,4 -c]pyridazin-6-yl]-4-iodobenzamide (80.0 mg, 0.13 mmol) was added to the mixture and then heat to 100°C with stirring for 16 h under nitrogen atmosphere. The reaction solution was cooled to ambient temperature and extracted with water (20 mL) and ethyl acetate (20 mL × 3). The organic phases were combined and washed with saturated saline (20 mL), dried over anhydrous sodium sulfate, filtered and concentrated under reduced pressure to give a residue. The residue was purified by preparative HPLC (C18, 22-62% gradient of water (formic acid)/acetonitrile) to give 2-{6-azaspiro[2.5]octan-6-yl}-N-[8-(4,4-difluoropiperidin-1-yl)-3-hydroxypyrido[3,4-c]pyridazin-6-yl]-4-(2-hydroxyethanesulfonamido)benzamide (2.50 mg, 0.005 mmol, yield: 3%) as an orange solid. LCMS (ESI): [M+H]+ = 618.4. $^1$H NMR (400 MHz, DMSO-d6) δ ppm 13.82 (s, 1H), 12.81 (s, 1H), 10.27 (s, 1H), 8.05 (d, *J* = 8.8 Hz, 1H), 7.52 (s, 1H), 7.24 (s, 1H), 7.11 (br d, *J* = 8.8 Hz, 1H), 6.79 (s, 1H), 5.01 (s, 1H), 4.18 (br s, 4H), 3.76 (t, *J* = 6.4 Hz, 2H), 3.31 - 3.30 (m, 2H), 2.98 (br s, 4H), 2.22 - 2.11 (m, 4H), 1.88 - 1.55 (m, 4H), 0.40 (s, 4H)

### Example 62: N-[3-amino-8-(4,4-difluoropiperidin-1-yl)pyrido[3,4-c]pyridazin-6-yl]-2-{6-azaspi ro[2.5]octan-6-yl}-4-(2-hydroxyethanesulfonamido)benzamide

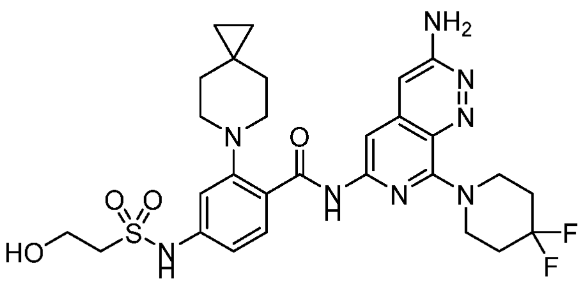

The preparations in **Example 62** follow the synthetic route of **Example 3:** LCMS (ESI): $[M+H]^+$ =617.3, $^1$H NMR (400 MHz, DMSO-d6) δ 12.80 (s, 1H), 10.94 - 9.71 (m, 1H), 8.07 (d, *J* = 8.4 Hz, 1H), 7.63 (s, 1H), 7.27 (d, *J* = 1.6 Hz, 1H), 7.13 (dd, *J* = 1.8, 8.8 Hz, 1H), 6.71 (s, 2H), 6.62 (s, 1H), 5.32 - 4.67 (m, 1H), 4.30 (br s, 4H), 3.76 (t, *J* = 6.4 Hz, 2H), 3.52 - 3.41 (m, 2H), 2.99 (br s, 4H), 2.24 - 2.10 (m, 4H), 1.92 - 1.49 (m, 4H), 0.41 (s, 4H)

### Example 63: N-(8-(4,4-difluoropiperidin-1-yl)quinolin-6-yl)-4-((2-hydroxyethyl)sulfamoyl)-2-( 6-azaspiro[2.5]octan-6-yl)benzamide

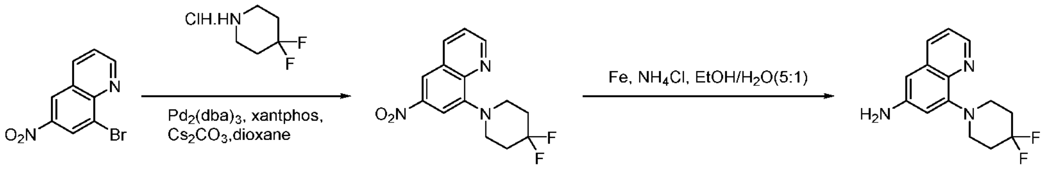


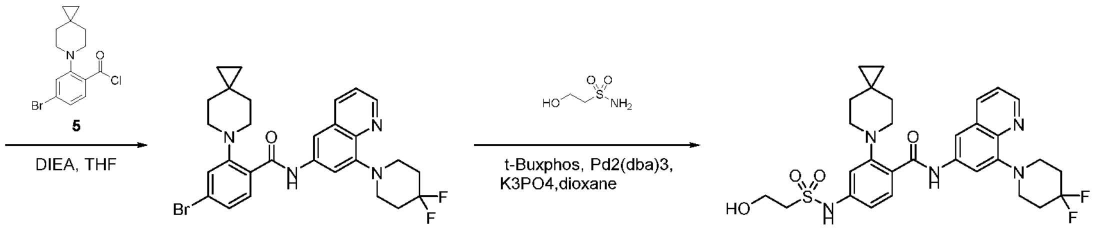


Step 1: 4,5-bis(diphenylphosphino)-9,9-dimethylxanthene (420 mg, 0.711 mmol) and tris(dibenzylideneacetone) dipalladium (665 mg, 0.81 mmol) under nitrogen atmosphere were successively added to a mixed solution containing 8-bromo-6-nitroquinoline (1.8 g, 7.11 mmol), 4,4-difluoropiperidine hydrochloride (1.7 g, 10.67 mmol), cesium carbonate (9.9 g, 29.64 mmol) and dioxane (20 mL). The resulting mixture was stirred at 120°C for 12 h. The reaction solution was poured into water (50 mL), extracted with ethyl acetate (50 mL×3), and the combined organic layer was dried over anhydrous sodium sulfate, filtered and concentrated to give the residue, which was further purified by column chromatography (silica gel, 0-50% gradient of ethyl acetate/petroleum ether) to give 8-(4,4-difluoropiperidin-1-yl)-6-nitroquinoline (1.4 g, 4.77 mmol, yield: 67%) as a yellow solid compound. LCMS (ESI): $[M+H]^+$ = 294.1.

Step 2: The mixed solution containing 8-(4,4-difluoropropidine)-6-nitroquinoline (500 mg, 1.70 mmol), iron powder (962 mg, 17.05 mmol), ammonium chloride (921 mg, 17.05 mmol), ethanol (10 mL) and water (2 mL) was stirred at 60°C for 1 h. The reaction solution was filtered while hot then the filtrate was poured into water (30 mL). The mixture was extrated with ethyl acetate (30 mL), the combined organic layer was dried over anhydrous sodium sulfate, filtered, and concentrated to give 8-(4,4-difluoropiperidin-1-yl)quinolin-6-amine (420 mg, 1.60 mmol, yield: 93.6%) as a yellow solid compound. LCMS (ESI): $[M+H]^+$ = 264.2.

Step 3: 2-(6-azaspiro[2.5]octan-6-yl)-4-bromobenzoyl chloride (599 mg, 1.82 mmol) was added to a mixed solution containing 8-(4,4-difluoropiperidinyl)-6-quinolinamine (400 mg, 1.52 mmol), N,N-diisopropylethylamine (601 mg, 4.56 mmol) and THF (10 mL). Stir the resulting mixture at 20°C for 1 h, The reaction solution was poured into water (30 mL), extracted with ethyl acetate (30 mL×3), and the combined organic layer was dried over anhydrous sodium sulfate, filtered and concentrated to give the residue, which was further purified by column chromatography (silica gel, 0-50% gradient of ethyl acetate/petroleum ether) to give the compound 4-bromo-N-(8-(4,4-difluoropiperidin-1-yl)quinolin-6-yl)-2-(6-azaspiro[2.5]octan-6-yl) benzamide (700 mg, 1.26 mmol, yield: 83%). LCMS (ESI): $[M+H]^+$ = 555.3.

Step 4: 2-di-tert-butylphosphino-2',4',6'-triisopropylbiphenyl (23 mg, 0.054 mmol) and tris(dibenzylideneacetone) dipalladium (50 mg, 0.054 mmol) under nitrogen atmosphere were successively added to a mixed solution containing [2-(6-azaspiro[2.5]octan-6-yl)-4-bromophenyl]-N-[8-(4.4-difluoropropiperidinyl)(6-quinolinyl)] carboxamide (300 mg. 0.54 mmol), 2-hydroxyethanesulfonamide (83 mg, 0.648 mmol), potassium phosphate (351 mg, 1.62 mmol) and dioxane (5 mL). The resulting mixture was stirred at 100°C for 12 h. The reaction solution was poured into water (30 mL), extracted withethyl acetate (30 mL×3), and the combined organic layer was dried over anhydrous sodium sulfate, filtered and concentrated to give the residue, which was further purified by column chromatography (silica gel, 0-50% gradient of ethyl acetate/petroleum ether) to give N-(8-(4,4-difluoropiperidin-1-yl)quinolin-6-yl)-4-((2-hydroxyethyl)sulfamoyl)-2-(6-a zaspiro[2.5]octan-6-yl)benzamide (115.74 mg, 0.19 mmol, yield: 35.7%) as a white solid compound. LCMS (ESI): $[M+H/2]^+$ = 300.8. $^1$H NMR (400 MHz, DMSO) δ 11.95 (s, 1H), 10.12 (s, 1H), 8.76 (dd, *J* = 4.0, 2.0 Hz, 1H), 8.30 - 8.20 (m, 2H), 7.87 (d, *J* = 8.4 Hz, 1H), 7.48 (dd, *J* = 8.4, 4.0 Hz, 1H), 7.33 (d, *J* = 2.0 Hz, 1H), 7.19 (d, *J* = 2.0 Hz, 1H), 7.07 (dd, *J* = 8.4, 2.0 Hz, 1H), 4.97 (s, 1H), 3.76 (d, *J* = 6.8 Hz, 2H), 3.54 (t, *J* = 5.6 Hz, 4H), 3.36 (d, *J* = 6.4 Hz, 2H), 3.00 (t, *J* = 5.2 Hz, 4H), 2.30 - 2.17 (m, 4H), 1.67 - 1.51 (m, 4H), 0.35 (s, 4H).

**Example 64: N-(8-(4,4-difluoropiperidin-1-yl)-2-methylquinolin-6-yl)-4-(2-hydroxyethyl)sulfo namide)-2-(6-azaspiro[2.5]octan-6-yl)benzamide**

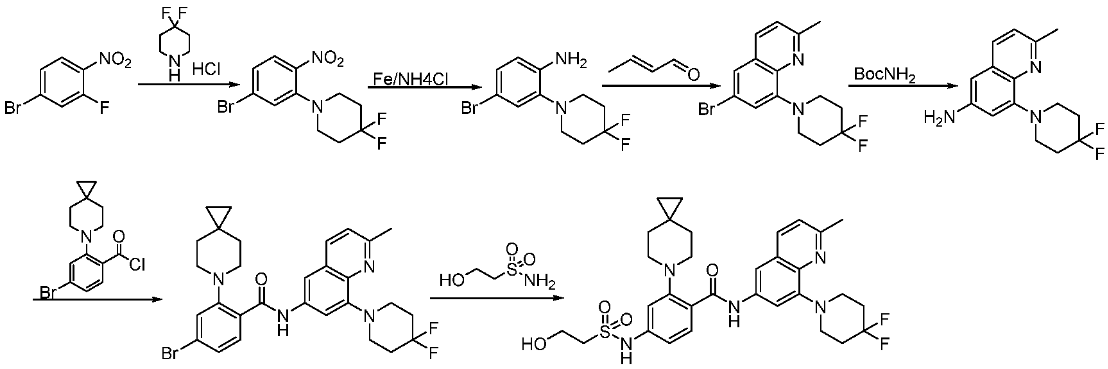


Step 1: 4-bromo-2-fluoro-1-nitrobenzene (10.00 g, 45.45 mmol), 4,4-difluoropiperidine hydrochloride (8.59 g, 54.55 mmol), and potassium carbonate (19.23 g, 136.36 mmol) were successively added to N,N-dimethylformamide (100 mL), and the reaction solution was stirred at 120°C for 6 h. The reaction solution was filtered under reduced pressure. The filtrate was poured into the saturated ammonium chloride solution (200 mL), extracted with ethyl acetate (100 mL × 3). The combined organic layer was washed with the saturated sodium chloride solution (100 mL × 3), dried over anhydrous sodium sulfate, and concentrated to obtain the crude. The crude was purified by flash column chromatography (silica gel, 0-20% gradient of ethyl acetate/petroleum ether) to give 1-(5-bromo-2-nitrophenyl)-4,4-difluoropiperidine (22.00 g, 75%) as a yellow solid. LCMS (ESI): $[M+H]^+$ =321.0

Step 2: 1-(5-bromo-2-nitrophenyl)-4,4-difluoropiperidine (4.00 g, 12.46 mmol), iron powder (3.51 g, 62.28 mmol) and ammonium chloride (1.01 g, 18.69 mmol) were successively added to water (4 mL), ethanol (20 mL) and THF (20 mL), and the reaction solution was stirred at 85°C for 16 h. The reaction solution was filtered under reduced pressure. The filtrate was poured into water (50 mL), extracted with ethyl acetate (50 mL × 3). The combined organic layer was dried over anhydrous sodium sulfate, and concentrated to obtain the crude. The crude was purified by flash column chromatography (silica gel, 10-15% gradient of ethyl acetate/petroleum ether) to give 4-bromo-2-(4,4-difluoropiperidin-1-yl)aniline (3.50 g, 96%) as a yellow solid. LCMS (ESI): $[M+H]^+$ =291.0

Step 3: 4-bromo-2-(4,4-difluoropiperidin-1-yl)aniline (3.50 g, 12.02 mmol) and crotonaldehyde (4.26 g, 60.11 mmol) were successively added to hydrochloric acid (6 M, 20 mL), and the reaction solution was stirred under reflux at 100°C for 16 h. Slowly The reaction solution was poured into water (100 mL), the pH was adjusted to 7 with sodium hydroxide solid, extracted with ethyl acetate (50 mL × 3). The combined organic layer was dried over anhydrous sodium sulfate, and concentrate to give the crude. The crude was purified by flash column chromatography (silica gel, 0-10% gradient of ethyl acetate/petroleum ether) to give 6-bromo-8-(4,4-difluoropiperidin-1-yl)-2-methylquinoline (1.20 g, 29%) as a pale yellow solid. LCMS (ESI): $[M+H]^+$ =341.0

Step 4: 6-bromo-8-(4,4-difluoropiperidin-1-yl)-2-methylquinoline (1.10 g, 3.22 mmol), tert-butyl carbamate (572 mg, 4.84 mmol), 4,5-bis(diphenylphosphino)-9,9-dimethylxanthene (190 mg, 0.32 mmol), tris(dibenzylideneacetone) dipalladium (301 mg, 0.32 mmol), and cesium carbonate (2.14 g, 6.45 mmol) were successively added to 1,4-dioxane (10 mL), and the reaction solution was stirred at 100°C for 16 h. The reaction solution was filtered under reduced pressure. The filtrate was poured into water (50 mL), extracted with ethyl acetate (30 mL × 3). The combined organic layer was dried over anhydrous sodium sulfate, and concentrated to obtain the crude. The crude was added to dichloromethane (10 mL) and trifluoroacetic acid (5 mL) was added to the reaction solution, and the reaction solution was stirred at 50°C for 2 h. Slowly The reaction solution was poured into water (50 mL), the pH was adjusted to 7 with sodium bicarbonate solid, extracted with ethyl acetate (50 mL × 3), The combined organic layer was dried over anhydrous sodium sulfate, and concentrate to give the crude. The crude was purified by flash column chromatography (silica gel, 10-25% gradient of ethyl acetate/dichloromethane) to give 8-(4,4-difluoropiperidin-1-yl)-2-methylquinolin-6-amine (450 mg, 50%) as a brown solid. LCMS (ESI): $[M+H]^+$ =278.2

Step 5: 8-(4,4-difluoropiperidin-1-yl)-2-methylquinolin-6-amine (200 mg, 0.72 mmol) and N,N-diisopropylethylamine (285 mg, 2.16 mmol) to THF (10 mL), and 4-bromo-2-(6-azaspiro[2.5]octan-6-yl) benzoyl chloride (355 mg, 1.08 mmol) was added to the reaction solution, and the reaction solution was stirred at 25°C for 2 h. The reaction solution was poured into saturated aqueous ammonium chloride solution (30 mL), extracted with ethyl acetate (30 mL × 3). The combined organic layer was dried over anhydrous sodium sulfate, and concentrated to obtain the crude. The crude was purified by flash column chromatography (silica gel, 10-25% gradient of ethyl acetate/dichloromethane) to give 4-bromo-N-(8-(4,4-difluoropiperidin-1-yl)-2-methylquinolin-6-yl)-2-(6-azaspiro[2.5] octan-6-yl) benzamide (300 mg, 73%) as a pale yellow solid. LCMS (ESI): $[M+H]^+$ =569.3

Step 6: 4-bromo-N-(8-(4,4-difluoropiperidin-1-yl)-2-methylquinolin-6-yl)-2-(6-azaspiro[2.5] octan-6-yl) benzamide (300 mg, 0.53 mmol), 2-hydroxyethanesulfonamide (101 mg, 0.79 mmol), cuprous iodide (102 mg, 0.53 mmol), (1S,2S)-(+)-N,N'-dimethyl-1,2-cyclohexanediamine (113 mg, 0.79 mmol) and potassium phosphate (342 mg, 1.58 mmol)

were successively added to N,N-dimethylformamide (4 mL), and the reaction solution was stirred at 115°C for 4 h under nitrogen atmosphere. The reaction solution was poured into the saturated aqueous ammonium chloride solution (30 mL), extracted with ethyl acetate (30 mL×3). The combined organic layer was washed with the saturated aqueous sodium chloride solution (30 mL × 3), the combined organic layer was dried over anhydrous sodium sulfate, and concentrated to obtain the crude. The crude was purified by flash column chromatography (silica gel, 30-50% gradient of ethyl acetate/dichloromethane) to give N-(8-(4,4-difluoropiperidin-1-yl)-2-methylquinolin-6-yl)-4-(2-hydroxyethyl)sulfonam ide)-2-(6-azaspiro[2.5]octan-6-yl)benzamide (165 mg, 51%). LCMS (ESI): $[M+H]^+$ =614.4. $^1$H NMR (400 MHz, DMSO-$d_6$) δ 11.92 (s, 1H), 10.11 (s, 1H), 8.17 (d, *J* = 2.0 Hz, 1H), 8.13 (d, *J* = 8.4 Hz, 1H), 7.87 (d, *J* = 8.5 Hz, 1H), 7.37 (d, *J* = 8.4 Hz, 1H), 7.29 (d, *J* = 2.2 Hz, 1H), 7.19 (d, *J* = 2.1 Hz, 1H), 7.07 (dd, *J* = 8.5, 2.1 Hz, 1H), 4.96 (s, 1H), 3.77 (q, *J* = 5.6, 5.0 Hz, 2H), 3.53 (t, *J* = 5.5 Hz, 4H), 3.36 (d, *J* = 6.5 Hz, 2H), 3.00 (t, *J* = 5.2 Hz, 4H), 2.64 (s, 3H), 2.30 - 2.19 (m, 4H), 1.59 (t, *J* = 5.0 Hz, 4H), 0.35 (s, 4H).

**Example 65: N-(8-(4,4-difluoropiperidin-1-yl)-2-methoxyquinolin-6-yl)-4-(2-hydroxyethylsulfo namide)-2-(6-azaspiro[2.5]octan-6-yl)benzamide**

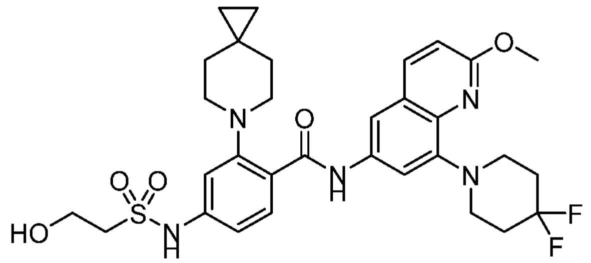

The preparations in **Example 65** follow the synthetic route of **Example 3:** LCMS (ESI): $[M+1]^+$ = 630.3. $^1$H NMR (400 MHz, DMSO) δ 11.89 (s, 1H), 10.06 (s, 1H), 8.18 - 8.11 (m, 2H), 7.86 (d, J = 8.4 Hz, 1H), 7.32 (d, J = 2.0 Hz, 1H), 7.19 (d, J = 2.0 Hz, 1H), 7.06 (dd, J = 8.4, 2.0 Hz, 1H), 6.99 (d, J = 8.8 Hz, 1H), 5.04 (s, 1H), 3.98 (s, 3H), 3.77 (t, J = 6.4 Hz, 2H), 3.49 (t, J = 5.6 Hz, 4H), 3.35 (d, J = 6.4 Hz, 2H), 3.00 (t, J = 5.2 Hz, 4H), 2.26 (td, J = 14.4, 13.0, 6.9 Hz, 4H), 1.59 (t, J = 5.2 Hz, 4H), 0.36 (s, 4H).

**Example 66: N-(8-(4,4-difluoropiperidin-1-yl)-2-hydroxyquinolin-6-yl)-4-(2-hydroxyethyl)sulf onamide)-2-(6-azaspiro[2.5]octan-6-yl)benzamide**

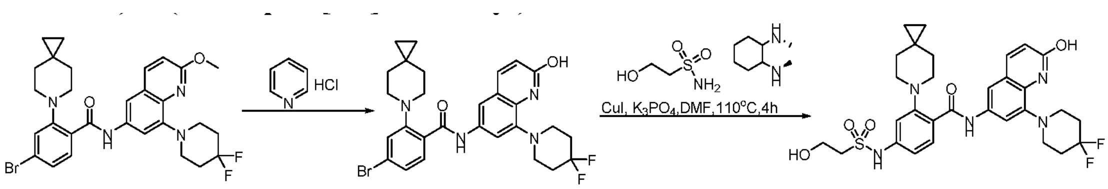


Step 1: The solution of pyridine hydrochloride (276 mg, 2.39 mmol) was added to the solution of 4-bromo-N-(8-(4,4-difluoropiperidin-1-yl)-2-methoxyquinolin-6-yl)-2-(6-azaspiro[2.5] octan-6-yl) benzamide (200 mg, 0.34 mmol) in THF (4 mL), and The mixture was stirred at 105°C for 8 h. Stirring is stopped once solid compounds were precipitated, then the system was diluted with water (8 mL), filtered out the solids, the cake was rinsed with methanol (1 mL), the cake was collected, and dried to give 2-(6-azaspiro[2.5]oct-6-yl)-4-bromophenyl]-N-[8-(4,4-difluoropiperidinyl)-2-hydroxy (6-quinolinyl)]formamide (150 mg, 77%) as a white solid. LCMS (ESI): $[M+H]^+$ = 571.3. $^1$H NMR (400 MHz, DMSO) δ 11.30 (s, 1H), 10.61 (s, 1H), 8.02 (d, J = 2.0 Hz, 1H), 7.96 - 7.92 (m, 1H), 7.71 - 7.64 (m, 2H), 7.44 (d, J = 2.0 Hz, 1H), 7.38 (dd, J = 8.4, 1.9 Hz, 1H), 6.55 (d, J = 9.6 Hz, 1H), 3.03 (t, J = 5.2 Hz, 4H), 2.89 (s, 6H), 2.73 (s, 2H), 1.49 (t, J = 5.2 Hz, 4H), 0.31 (s, 4H).

Step 2: 2-(6-Azaspiro[2.5]oct-6-yl)-4-bromophenyl]-N-[8-(4,4-difluoropiperidinyl)-2-hydrox y(6-quinolinyl)]formamide (100 mg, 0.17 mmol), 2- hydroxyethanesulfonamide (44 mg, 0.30 mmol), cuprous iodide (34 mg, 0.17 mmol), potassium phosphate (150 mg, 0.70 mmol), and N,N'-dimethyl-1,2-cyclohexanediamine (100 mg, 0.70 mmol) were successively added to N,N-dimethylformamide (3 mL). After replacing nitrogen for three times, the reaction mixture was heated to 110°C and stirred for 2 h. The reaction solution was quenched with water (25 mL), extracted with ethyl acetate (25 mL×3),The combined organic layer was washed with saturated aqueous sodium chloride solution (50 mL), dried over

anhydrous sodium sulfate, concentrated under reduced pressure and The crude was purified with preparative liquid phase (C18, 30-55% gradient of acetonitrile/water) to give N-(8-(4,4-difluoropiperidin-1-yl)-2-hydroxyquinolin-6-yl)-4-(2-hydroxyethyl)sulfona mide)-2-(6-azaspiro[2.5]octan-6-yl)benzamide (32 mg, 29%) as a white solid. LCMS (ESI): $[M+1]^+$ = 616. $^1H$ NMR (400 MHz, DMSO) δ 11.73 (s, 1H), 10.60 (s, 1H), 9.76 (s, 1H), 8.03 (d, *J* = 2.1 Hz, 1H), 7.93 (d, *J* = 9.6 Hz, 1H), 7.81 (d, *J* = 8.4 Hz, 1H), 7.67 (d, *J* = 2.0 Hz, 1H), 7.15 (d, *J* = 2.0 Hz, 1H), 7.02 (dd, *J* = 8.4, 2.1 Hz, 1H), 6.55 (d, *J* = 9.6 Hz, 1H), 5.07 (s, 1H), 3.76 (t, *J* = 6.4 Hz, 2H), 3.30 (d, *J* = 6.4 Hz, 2H), 3.08 - 2.80 (m, 8H), 2.01 (s, 4H), 1.55 (t, *J* = 5.2 Hz, 4H), 0.34 (s, 4H).

**Example 67: N-(2-amino-8-(4,4-difluoropiperidin-1-yl)quinolin-6-yl)-4-(2-hydroxyethyl)sulfon amide)-2-(6-azaspiro[2.5]octan-6-yl)benzamide**

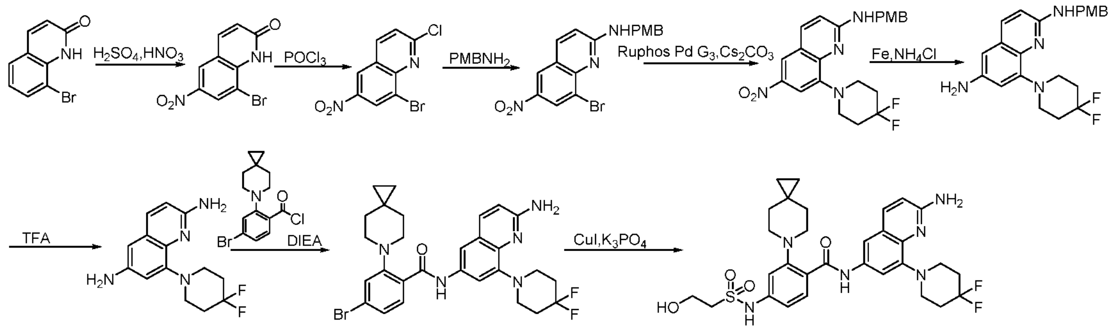


Step 1: 8-bromoquinoline-2(1H)-one (5.00 g, 22.32 mmol) was added to concentrated sulfuric acid (20 mL), and the reaction solution was stirred for 15 min in an ice bath. Concentrated nitric acid (65%, 12 mL) was slowly added dropwise to the reaction solution, and the temperature of the reaction solution was kept below 5°C. After dropwise addition, the reaction solution was warmed to 25°C and stirred for 16 h. Slowly the reaction solution was poured into ice water (200 mL), stirring vigorously, and perform suction filtration. The solid obtained by filtration was washed with water (200 mL), saturated sodium bicarbonate solution (200 mL), and water (200 mL), and dried to give 8-bromo-6-nitroquinoline-2(1H)-one (50% purity, 5.77 g, 47%) as a pale yellow solid crude. LCMS (ESI): $[M+H]^+$ = 269.0, 271.0.

Step 2: 8-bromo-6-nitroquinoline-2(1H)-one (50% purity, 5.70 g, 10.59 mmol) was added to phosphorus oxychloride (10 mL), and the reaction solution was stirred at 90°C for 4 h. The reaction solution was slowly poured into ice water (200 mL). After stirring, the pH was adjusted to 7 with saturated sodium bicarbonate solution, extracted with ethyl acetate (50 mL×3), The combined organic layer was dried over anhydrous sodium sulfate, and concentrated to give the crude. The crude was purified by flash column chromatography (silica gel, 0-10% gradient of ethyl acetate/petroleum ether) to give 8-bromo-2-chloro-6-nitroquinoline (2.60 g, 73%) as a pale yellow solid. LCMS(ESI): $[M+H]^+$ =286.9. $^1H$ NMR (400 MHz, DMSO-$d_6$) δ 9.15 (d, *J* = 2.5 Hz, 1H), 8.83 (d, *J* = 8.6 Hz, 1H), 8.80 (d, *J* = 2.5 Hz, 1H), 7.91 (d, *J* = 8.6 Hz, 1H).

Step 3: 8-bromo-2-chloro-6-nitroquinoline (2.60 g, 9.04 mmol), 4-methoxybenzylamine (1.88 g, 13.56 mmol), and cesium carbonate (4.51 g, 13.56 mmol) were successively added to N,N-dimethylformamide (5 mL), and the reaction solution was stirred at 95°C for 16 h. The reaction solution was poured into the saturated aqueous ammonium chloride solution (50 mL), extracted with ethyl acetate (50 mL × 3),The combined organic layer was washed with saturated sodium chloride solution (30 mL × 3), the combined organic layer was dried with anhydrous sodium sulfate, and concentrated to obtain the crude. The crude was purified by flash column chromatography (silica gel, 0-15% gradient of ethyl acetate/petroleum ether) to give 8-bromo-N-(4-methoxybenzyl)-6-nitroquinoline-2-amine (2.90 g, 83%) as a brown solid. LCMS(ESI): $[M+H]^+$ =388.0

Step 4: 8-bromo-N-(4-methoxybenzyl)-6-nitroquinoline-2-amine (2.90 g, 7.47 mmol), 4,4-difluoropiperidine hydrochloride (1.78 g, 11.20 mmol), cesium carbonate (7.45 g, 22.41 mmol), and methanesulfonic acid (2-dicyclohexylphosphino-2',6'-diisopropoxy-1,1'-biphenyl)(2-amino-1,1'-biphenyl-2-yl) palladium (II) (319 mg, 0.37 mmol) were successively added to 1,4-dioxane (10 mL). After nitrogen replacement, the reaction solution was stirred at 100°C for 16 h. The reaction solution was poured into saturated aqueous ammonium chloride solution (50 mL), extracted with ethyl acetate (50 mL × 3), The combined organic layer was dried over anhydrous sodium sulfate, and concentrated to obtain the crude. The crude was purified by flash column chromatography (silica gel, 10-25% gradient of ethyl acetate/petroleum ether) to give 8-(4,4-difluoropiperidin-1-yl)-N-(4-methoxybenzyl)-6-nitroquinoline-2-amine (1.70 g, 53%) as a red solid. LCMS (ESI): $[M+H]^+$ = 429.3.

Step 5: 8-(4,4-difluoropiperidin-1-yl)-N-(4-methoxybenzyl)-6-nitroquinoline-2-amine (800 mg, 1.87 mmol), iron powder (527 mg, 9.34 mmol), and ammonium chloride (151 mg, 2.80 mmol) were successively added to a solution of water

(1 mL), ethanol (5 mL), and THF (5 mL), and the reaction solution was stirred at 90°C for 16 h. The reaction solution was filtered under reduced pressure, then the filtrate was concentrated, poured into water (30 mL), extracted with ethyl acetate (30 mL × 3), The combined organic layer was dried over anhydrous sodium sulfate, and concentrated to give 8-(4,4-difluoropiperidin-1-yl)-N2-(4-methoxybenzyl)quinoline-2,6-diamine (700 mg, 94%) as the crude. LCMS (ESI): $[M+H]^+$ = 399.3.

Step 6: 8-(4,4-difluoropiperidin-1-yl)-N2-(4-methoxybenzyl)quinoline-2,6-diamine (700 mg, 1.76 mmol) was added to trifluoroacetic acid (4 mL), and the reaction solution was stirred at 50°C for 16 h. The reaction solution was poured slowly into saturated ammonium chloride solution (50 mL), extracted with dichloromethane (30 mL × 3), The combined organic layer was dried over anhydrous sodium sulfate, and concentrated to obtain the crude. The crude was purified by flash column chromatography (silica gel, 0-10% gradient of methanol/dichloromethane) to give 8-(4,4-difluoropiperidin-1-yl)quinoline-2,6-diamine (220 mg, 45%). LCMS (ESI): $[M+H]^+$ = 279.1.

Step 7: 8-(4,4-difluoropiperidin-1-yl)quinoline-2,6-diamine (140 mg, 0.50 mmol) and N,N-diisopropylethylamine (133 mg, 1.01 mmol) were successively added to THF (2 mL), the reaction solution was stirred at 25°C for 10 min, then 4-bromo-2-(6-azaspiro[2.5]octan-6-yl) benzoyl chloride (174 mg, 0.53 mmol) was added to the reaction solution, and the reaction solution was stirred at 25°C for 2 h. The reaction solution was poured into saturated ammonium chloride solution (30 mL), extracted withdichloromethane (30 mL × 3), The combined organic layer was dried over anhydrous sodium sulfate, and concentrate to obtain the crude. The crude was purified by flash column chromatography (silica gel, 0-10% gradient of methanol/dichloromethane) to give N-(2-amino-8-(4,4-difluoropiperidin-1-yl)quinolin-6-yl)-4-bromo-2-(6-azaspiro[2.5]o ctan-6-yl) benzamide (140 mg, 49%) as a brown solid. LCMS(ESI): $[M+H]^+$ =570.3

Step 8: N-(2-amino-8-(4,4-difluoropiperidin-1-yl)quinolin-6-yl)-4-bromo-2-(6-azaspiro[2.5]o ctan-6-yl) benzamide (140 mg, 0.25 mmol), potassium phosphate (159 mg, 0.74 mmol), cuprous iodide (48 mg, 0.25 mmol), (1S,2S)-(+)-N,N'-dimethyl-1,2-cyclohexanediamine (35 mg, 0.25 mmol), and 2-hydroxyethanesulfonamide (47 mg, 0.37 mmol) were successively added to N,N-dimethylformamide (1 mL). After nitrogen replacement, the reaction solution was stirred at 110°C for 16 h. The reaction solution was poured into the saturated ammonium chloride solution (30 mL), extracted with ethyl acetate (30 mL × 3),The combined organic layer was washed with the saturated sodium chloride solution (30 mL × 3), the combined organic layer was dried with anhydrous sodium sulfate, and concentrate to obtain the crude. The crude was purified by flash column chromatography (C18, 10-40% gradient of acetonitrile/water) to give N-(2-amino-8-(4,4-difluoropiperidin-1-yl)quinolin-6-yl)-4-(2-hydroxyethyl)sulfonami de)-2-(6-azaspiro[2.5]octan-6-yl)benzamide (27 mg, 18%) as a pale yellow solid. LCMS(ESI): $[M+H]^+$ =615.4. $^1$H NMR (400 MHz, DMSO-$d_6$) δ 11.81 (d, *J* = 4.2 Hz, 1H), 10.09 (s,1H), 7.94 (q, *J* = 2.1 Hz, 1H), 7.85 (ddd, *J* = 18.0, 8.7, 4.3 Hz, 2H), 7.18 (dt, *J* = 6.0, 2.9 Hz, 2H), 7.06 (dq, *J* = 7.3, 2.1 Hz, 1H), 6.75 (dd, *J* = 8.8, 4.2 Hz, 1H), 6.25 (s, 2H), 4.98 (s, 1H), 3.77 (q, *J* = 5.9 Hz, 2H), 3.46 - 3.40 (m, 6H), 2.99 (q, *J* = 4.9 Hz, 4H), 2.23 (t, *J* = 10.4 Hz, 4H), 1.59 (d, *J* = 6.6 Hz, 4H), 0.36 (d, *J* = 4.2 Hz, 4H).

**Example 68: N-(2-cyano-8-(4,4-difluoropiperidin-1-yl)quinolin-6-yl)-4-((2-hydroxyethyl)sulfon amido)-2-(6-azaspiro[2.5]oct-6-yl)benzamide**

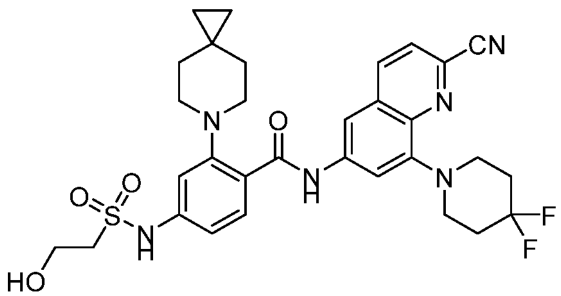

The preparations in **Example 68** follow the synthetic route of **Example 3:** LCMS (ESI): $[M+H]^+$ =625.4, $^1$H NMR (400 MHz, DMSO-$d_6$) δ 12.01 (s, 1H), 10.13 (s, 1H), 8.52 (d, *J* = 8.6 Hz, 1H), 8.34 (d, *J* = 2.0 Hz, 1H), 7.95 (d, *J* = 8.4 Hz, 1H), 7.84 (d, *J* = 8.5 Hz, 1H), 7.43 (d, *J* = 2.2 Hz, 1H), 7.18 (d, *J* = 2.1 Hz, 1H), 7.06 (dd, *J* = 8.5, 2.1 Hz, 1H), 4.97 (s, 1H), 3.77 (s, 2H), 3.55 (d, *J* = 6.2 Hz, 4H), 3.36(m, 2H), 3.00 (t, *J* = 5.2 Hz, 4H), 2.23 (d, *J* = 15.1 Hz, 4H), 1.56 (s, 4H), 0.34 (s, 4H).

**Example 69: (2-(6-Azaspiro[2.5]octan-6-yl)-4-{[(2-hydroxyethyl)sulfonyl]amino}phenyl)-N-[8-(4,4-difluoropiperidinyl)-2-(trifluoromethyl)(6-quinolinyl)formamide**

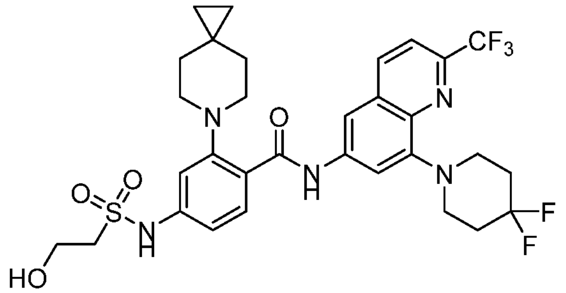

The preparations in **Example 69** follow the synthetic route of **Example 3:** LCMS (ESI): $[M+H]^+$ =668.4, $^1H$ NMR (400 MHz, DMSO) δ 11.99 (s, 1H), 10.13 (s, 1H), 8.57 (d, *J* = 8.6 Hz, 1H), 8.35 (d, *J* = 2.0 Hz, 1H), 7.87 (dd, *J* = 13.1, 8.5 Hz, 2H), 7.43 (d, *J* = 2.1 Hz, 1H), 7.19 (d, *J* = 2.1 Hz, 1H), 7.06 (dd, *J* = 8.5, 2.1 Hz, 1H), 4.97 (s, 1H), 3.77 (t, *J* = 5.1 Hz, 2H), 3.58 (t, *J* = 5.5 Hz, 4H), 3.35 (t, *J* = 6.6 Hz, 2H), 3.00 (t, *J* = 5.2 Hz, 4H), 2.25 (dt, *J* = 14.4, 8.5 Hz, 4H), 1.57 (s, 4H), 0.35 (s, 4H).

**Example 70: N-(8-(4,4-difluoropiperidin-1-yl)quinolin-6-yl-7-d)-4-((2-hydroxyethyl)sulphona mino)-2-(6-azaspiro[2.5]octan-6-yl)benzamide**

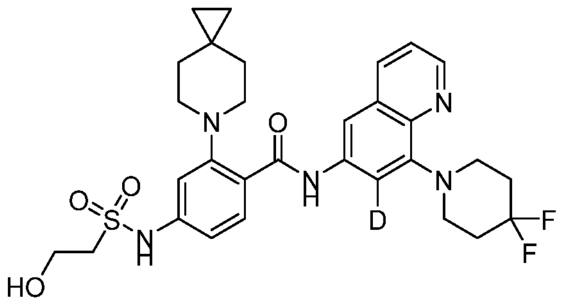

The preparations in **Example 70** follow the synthetic route of **Example 3:** LCMS (ESI): $[M+H]^+$ = 601.2; $^1H$ NMR (400 MHz, DMSO-$d_6$) δ ppm 11.95 (s, 1H), 10.53 - 9.68 (m, 1H), 8.75 (dd, *J* = 1.2, 4.0 Hz, 1H), 8.35 - 8.16 (m, 2H), 7.87 (d, *J* = 8.8 Hz, 1H), 7.47 (dd, *J* = 4.0, 8.0 Hz, 1H), 7.19 (s, 1H), 7.07 (dd, *J* = 1.6, 8.4 Hz, 1H), 5.16 - 4.76 (m, 1H), 3.77 (t, *J* = 6.4 Hz, 2H), 3.57 - 3.49 (m, 4H), 3.35 (br t, *J* = 6.4 Hz, 2H), 3.00 (br t, *J* = 4.4 Hz, 4H), 2.31 - 2.16 (m, 4H), 1.63 - 1.53 (m, 4H), 0.35 (s, 4H)

**Example 71: N-(8-(4,4-difluoropiperidin-1-yl)-7-fluoroquinolin-6-yl)-4-((2-hydroxyethyl)sulfo namido)-2-(6-azaspiro[2.5]octan-6-yl]benzamide**

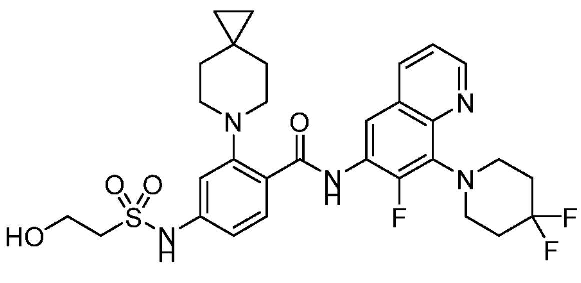

The preparations in **Example 71** follow the synthetic route of **Example 3:** LCMS (ESI): [M+H]+ = 618.4. $^1H$ NMR (400 MHz, DMSO) δ 12.48 (d, J = 2.7 Hz, 1H), 10.22 (s, 1H), 8.82 (dd, J = 4.2, 1.7 Hz, 1H), 8.74 (d, J = 7.9 Hz, 1H), 8.32 (dd, J = 8.4, 1.7 Hz, 1H), 8.05 (d, J = 8.5 Hz, 1H), 7.49 (dd, J = 8.3, 4.2 Hz, 1H), 7.31 (d, J = 2.2 Hz, 1H), 7.14 (dd, J = 8.6, 2.1 Hz, 1H), 4.95 (t, J = 5.7 Hz, 1H), 3.77 (q, J = 5.8 Hz, 2H), 3.58 (t, J = 5.6 Hz, 4H), 3.38 (t, J = 6.5 Hz, 2H), 2.99 (t, J = 5.3 Hz, 4H), 2.19 (tt, J = 14.1, 5.6 Hz, 4H), 1.60 (s, 4H), 0.38 (s, 4H).

**Example 72: N-(8-(4,4-difluoropiperidin-1-yl)-7-(trifluoromethyl)quinolin-6-yl)-4-((2-hydroxy ethyl)sulfonamido)-2-(6-azaspiro[2.5]octan-6-yl)**

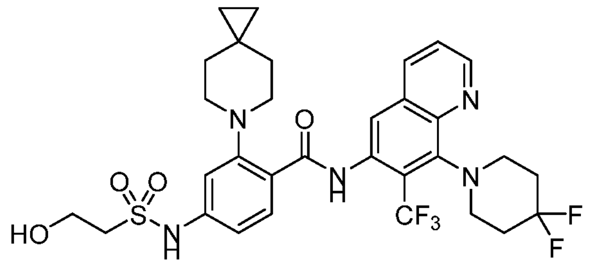

The preparations in **Example 72** follow the synthetic route of **Example 3:** LCMS (ESI): $[M+H]^+$ =668.4. $^1H$ NMR (400 MHz, DMSO) δ 12.00 (s, 1H), 10.18 (s, 1H), 9.02 (dd, *J* = 4.1, 1.7 Hz, 1H), 8.47 (dd, *J* = 8.5, 1.7 Hz, 1H), 8.30 (s, 1H), 7.98 (d, *J* = 8.5 Hz, 1H), 7.68 (dd, *J* = 8.4, 4.1 Hz, 1H), 7.27 (d, *J* = 2.1 Hz, 1H), 7.10 (dd, *J* = 8.6, 2.1 Hz, 1H), 4.95 (s, 1H), 4.18 - 3.96 (m, 2H), 3.78 (s, 2H), 3.37 (t, *J* = 6.5 Hz, 2H), 3.02 (t, *J* = 5.4 Hz, 6H), 2.08 (d, *J* = 35.4 Hz, 4H), 1.48 (s, 4H), 0.33 (s, 4H).

**Example 73: N-(7-chloro-8-(4,4-difluoropiperidin-1-yl)quinolin-6-yl)-4-((2-hydroxyethyl)sulfo namido)-2-(6-azaspiro[2.5]oct-6-yl)benzamide**

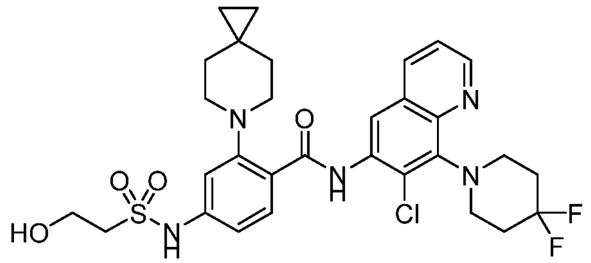

The preparations in Example 73 follow the synthetic route of Example 3: LCMS (ESI): $[M+H]^+$ = 634.6; $^1H$ NMR (400 MHz, DMSO) δ 11.66 (s, 1H), 10.20 (s, 1H), 8.90 - 8.83 (m, 1H), 8.78 (s, 1H), 8.38 (d, *J* = 8.4 Hz, 1H), 7.96 (d, *J* = 8.4 Hz, 1H), 7.54 (dd, *J* = 8.3, 4.1 Hz, 1H), 7.26 (d, *J* = 2.1 Hz, 1H), 7.09 (dd, *J* = 8.4, 2.0 Hz, 1H), 4.96 (t, *J* = 5.6 Hz, 1H), 3.98 -3.62 (m, 4H), 3.47 - 3.30 (m, 4H), 3.04 (t, *J* = 5.2 Hz, 4H), 2.27 - 2.03 (m, 4H), 1.65 - 1.47 (m, 4H), 0.34 (s, 4H).

**Example 74: N-(7-cyano-8-(4,4-difluoropiperidin-1-yl)quinolin-6-yl)-4-((2-hydroxyethyl)sulfon amido)-2-(6-azaspiro[2.5]oct-6-yl)benzamide**

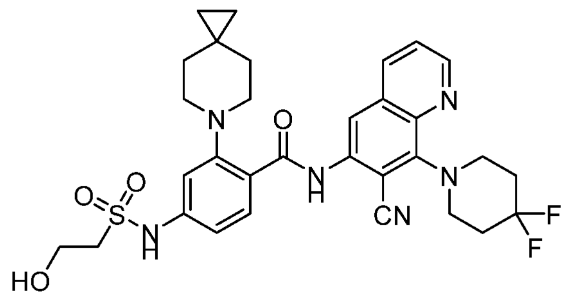

The preparations in **Example 74** follow the synthetic route of **Example 3:** LCMS (ESI): $[M+H]^+$ = 625.4; $^1H$ NMR (400 MHz, DMSO) δ 11.71 (s, 1H), 10.19 (s, 1H), 8.91 (dd, J = 4.0, 1.6 Hz, 1H), 8.40 (dd, J = 8.4, 2.0 Hz, 1H), 8.26 (s, 1H), 7.92 (d, J = 8.4 Hz, 1H), 7.64 (dd, J = 8.4, 4.0 Hz, 1H), 7.24 (d, J = 2.0 Hz, 1H), 7.08 (dd, J = 8.4, 2.0 Hz, 1H), 4.96 (d, J = 5.6

Hz, 1H), 3.79-3.62 (m, 6H), 3.38 (t, J = 6.4 Hz, 2H), 3.04 (t, J = 5.2 Hz, 4H), 2.28-2.14 (m, 4H), 1.58-1.47 (m, 4H), 0.34 (s, 4H).

**Example 75: N-(8-(4,4-difluoropiperidin-1-yl)-7-fluoro-2-methylquinolin-6-yl)-4-((2-hydroxyet hyl)sulfonamido)-2-(6-azaspiro[2.5]octan-6-yl)benzamide**

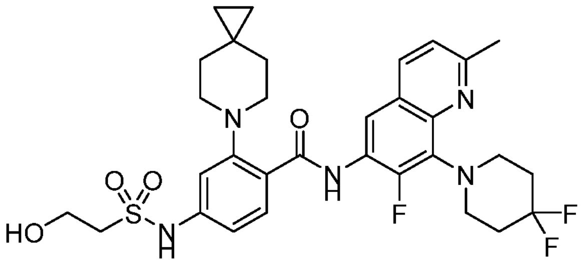

The preparations in **Example 75** follow the synthetic route of **Example 3:** LCMS (ESI): $[M+H]^+$ =632.4. $^1$H NMR (400 MHz, DMSO) δ 12.40 (d, *J* = 2.5 Hz, 1H), 10.21 (s, 1H), 8.66 (d, *J* = 7.9 Hz, 1H), 8.19 (d, *J* = 8.4 Hz, 1H), 8.04 (d, *J* = 8.6 Hz, 1H), 7.37 (d, *J* = 8.3 Hz, 1H), 7.30 (d, *J* = 2.1 Hz, 1H), 7.14 (dd, *J* = 8.6, 2.1 Hz, 1H), 4.95 (s, 1H), 3.77 (t, *J* = 6.5 Hz, 2H), 3.57 (t, *J* = 5.9 Hz, 4H), 3.37 (t, *J* = 6.5 Hz, 2H), 2.99 (t, *J* = 5.2 Hz, 4H), 2.66 (s, 3H), 2.20 (tt, *J* = 14.3, 5.6 Hz, 4H), 1.60 (s, 4H), 0.38 (s, 4H).

**Example 76: N-(8-(4,4-difluoropiperidin-1-yl)-7-fluoro-2-hydroxyquinolin-6-yl)-4-((2-hydroxy ethyl)sulfonamido)-2-(6-azaspiro[2.5]oct-6-yl)benzamide**

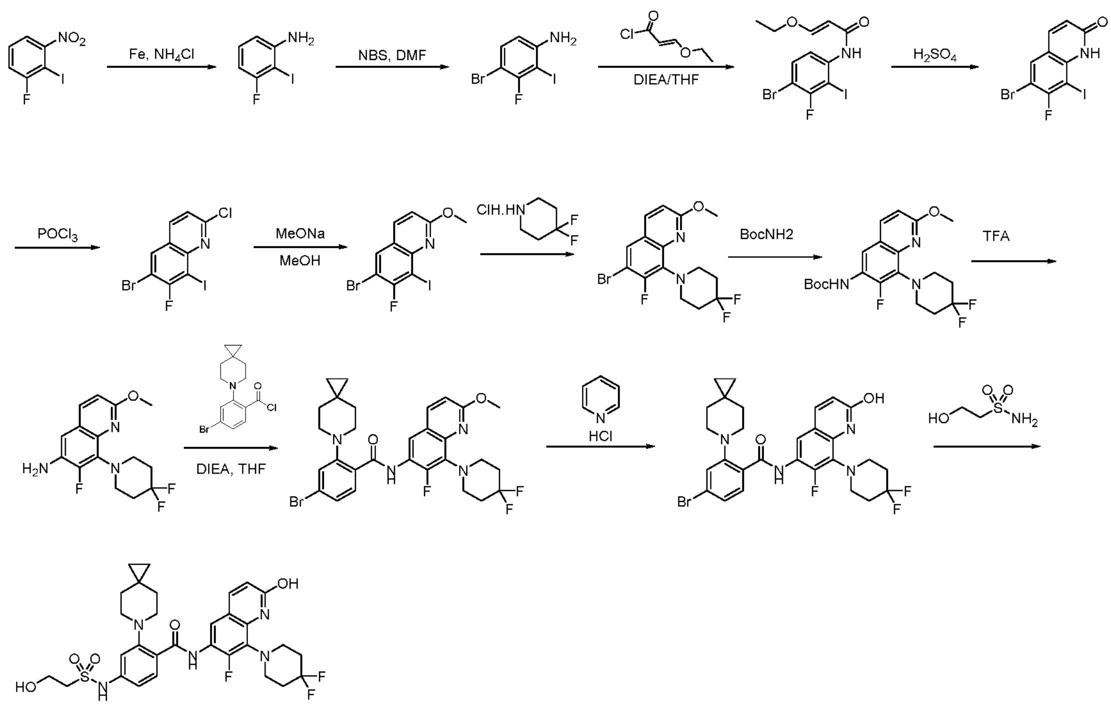


Step 1: The solution containing 3-fluoro-2-iodo-1-nitrobenzene (25.00 g, 93.63 mmol), iron powder (10.00 g, 177.90 mmol), ammonium chloride (9.60 g, 177.90 mmol), ethanol (100 mL) and water (20 mL) was stirred at 60°C for 1 h. The reaction solution was filtered while hot, then concentrated, extracted with ethyl acetate (100 mL × 3), and the organic layer was dried over anhydrous sodium sulfate, filtered and concentrated to give the residue, which was further purified by column chromatography (silica gel, 0-50% gradient of ethyl acetate/petroleum ether) to give 3-fluoro-2-iodoaniline (22.00 g, 92.82 mmol, yield: 99.1%) as a white solid compound. LCMS (ESI): $[M+H]^+$ = 238.0

Step 2: Add N-bromosuccinimide (16.50 g, 92.82 mmol) to a solution containing 3-fluoro-2-iodoaniline (22.00 g, 92.82 mmol) and N,N-dimethylformamide (200 mL), the reaction solution was stirred at 20°C for 1 h, The reaction solution was poured into water (300 mL), extracted withethyl acetate (100 mL×3), and the combined organic layer was dried s over anhydrous sodium sulfate, filtered and concentrated to give the residue, which was further purified by column chromatography (silica gel, 0-20% gradient of ethyl acetate/petroleum ether) to give 4-bromo-3-fluoro-2-iodoaniline (21.50 g, 68.06 mmol, yield: 73.3%) as a gray solid compound. LCMS (ESI): $[M+H]^+$ = 317.9

Step 3: 3-ethoxyacryloyl chloride (9.00 g, 66.48 mmol) was added to a solution containing 4-bromo-3-fluoro-2-iodoaniline (21.00 g, 66.48 mmol), N,N-diisopropylethylamine (26.30 g, 199.42 mmol) and THF (210 mL), and the reaction solution was stirred at 20°C for 12 h. The reaction mixture was poured into water (100 mL), extracted with ethyl acetate (100 mL×3), and the combined organic layer was dried over anhydrous sodium sulfate, filtered and concentrated to give the residue, which was further purified by column chromatography (silica gel, 0-50% gradient of ethyl acetate/petroleum ether) to give (E)-N-(4-bromo-3-fluoro-2-iodophenyl)-3-ethoxyacrylamide (15.00 g, 36.23 mmol, yield: 54.5%) as a yellow solid compound. LCMS (ESI): $[M+H]^+$ = 416.1

Step 4: The solution containing (2E)-N-(4-bromo-2-fluoro-2-iodophenyl)-3-methoxypropyl-2-enamide (15.00 g, 36.23 mmol) and concentrated sulfuric acid (100 mL) was stirred at 20°C for 12 h, and The reaction mixture was poured into ice water and filtered to give 6-bromo-7-fluoro-8-iodoquinolin-2(1H)-one (12.00 g, 32.61 mmol, yield: 90.0%) as a yellow solid compound. LCMS (ESI): $[M+H]^+$ = 369.9

Step 5: The reaction solution containing 6-bromo-7-fluoro-8-iodoquinolin-2(1H)-one (12.00 g, 32.61 mmol) and phosphorus oxychloride (80 mL) was stirred at 25°C for 2 h, the reaction solution was concentrated, poured into water (30 mL), and filtered to give 6-bromo-2-chloro-7-fluoro-8-iodoquinoline (9.00 g, 23.29 mmol, yield: 71.4%) as a yellow solid compound. LCMS (ESI): $[M+H]^+$ = 385.9

Step 6: The reaction solution containing 6-bromo-2-chloro-7-fluoro-8-iodoquinoline (1.00 g, 2.59 mmol), sodium methoxide (30%) (2.30 g, 12.94 mmol), and methanol (10 mL) was stirred at 20°C for 12 h. The reaction solution was poured into water (30 mL), extracted withethyl acetate (30 mL×3), and the combined organic layer was dried over anhydrous sodium sulfate, filtered and concentrated to give the residue, which was further purified by column chromatography (silica gel, 0-20% gradient of ethyl acetate/petroleum ether) to give 6-bromo-7-fluoro-8-iodo-2-methoxyquinoline (800 mg, 2.09 mmol, yield: 80.9%) as a yellow solid compound. LCMS (ESI): $[M+H]^+$ = 383.9

Step 7: 4,5-bis(diphenylphosphino)-9,9-dimethylxanthene (124 mg, 0.21 mmol) and tris(dibenzylideneacetone) dipalladium (196 mg, 0.21 mmol) were successively added to a mixed solution containing 6-bromo-7-fluoro-8-iodo-2-methoxyquinoline (800 mg, 2.09 mmol), 4,4-difluoropiperidine hydrochloride (367 mg, 2.30 mmol), cesium carbonate (2.10 g, 6.28 mmol) and 1,4-dioxane (10 mL) under nitrogen atmosphere, and the reaction solution was stirred at 100°C for 12 h. The reaction mixture was poured into water (30 mL), extracted with ethyl acetate (30 mL × 3), and the combined organic layer was dried over anhydrous sodium sulfate, filtered and concentrated to give the residue, which was further purified by column chromatography (silica gel, 0-20% gradient of ethyl acetate/petroleum ether) to give 6-bromo-8-(4,4-difluoropiperidin-1-yl)-7-fluoro-2-methoxyquinoline (600 mg, 1.60 mmol, yield: 76.4%) as a yellow solid compound. LCMS (ESI): $[M+H]^+$ = 377.1

Step 8: 4,5-bis(diphenylphosphino)-9,9-dimethylxanthene (94 mg, 0.16 mmol) and tris(dibenzylideneacetone) dipalladium (149 mg, 0.16 mmol) were successively added to a solution of 6-bromo-8-(4,4-difluoropiperidin-1-yl)-7-fluoro-2-methoxyquinoline (600 mg, 1.60 mmol), tert-butyl carbamate (227 mg, 1.92 mmol), cesium carbonate (1.60 g, 4.80 mmol) and 1,4-dioxane (10 mL) under nitrogen atmosphere, and the reaction solution was stirred at 100°C for 12 h. The reaction solution was poured into water (30 mL), extracted withethyl acetate (30 mL × 3), and the combined organic layer was dried over anhydrous sodium sulfate, filtered and concentrated to give the residue, which was further purified by column chromatography (silica gel, 0-20% gradient of ethyl acetate/petroleum ether) to give N-[8-(4,4-difluoropiperidinyl)-7-fluoro-2-methoxy(6-quinolinyl)](tert-butoxy)formam ide (400 mg, 0.97 mmol, yield: 60.8%) as a yellow solid compound. LCMS (ESI): $[M+H]^+$ = 412.3

Step 9: The reaction solution containing N-[8-(4,4-difluoropiperidinyl)-7-fluoro-2-methoxy(6-quinolinyl)](tert-butoxy)formam ide (400 mg, 0.97 mmol) and trifluoroacetic acid (2 mL) was stirred at 20°C for 1 h. After the concentration of the reaction solution, add the saturated aqueous sodium bicarbonate solution to the pH was adjusted of the reaction solution to be close to 8, then extracted with ethyl acetate (30 mL×3). The combined organic layer was dried The combined organic layer was dried over anhydrous sodium sulfate, filtered, and concentrated to give 8-(4,4-difluoropiperidinyl)-7-fluoro-2-methoxy-6-quinolinamine (300 mg, crude) as a yellow solid compound. LCMS (ESI): $[M+H]^+$ = 312.1

Step 10: 2-(6-azaspiro[2.5]oct-6-yl)-4-bromobenzoyl chloride (380 mg, 1.16 mmol) was added to a solution containing 8-(4,4-difluoropiperidinyl)-7-fluoro-2-methoxy-6-quinolinamine (300 mg, 0.96 mmol), N,N-diisopropylethylamine (635 mg, 4.82 mmol) and THF (5 mL). The reaction solution was stirred at 20°C for 12 h, poured into water (30 mL), extracted with ethyl acetate (30 mL×3), and the combined organic layer was dried over anhydrous sodium sulfate, filtered and concentrated to give the residue, which was further purified by column chromatography (silica gel, 0-20% gradient of ethyl acetate/petroleum ether) to give [2-(6-azaspiro[2.5]oct-6-yl)-4-bromophenyl]-N-[8-(4,4-difluoropiperidinyl)-7-fluoro-2-methoxy(6-quinolinyl)]formamide (260 mg, 0.43 mmol, yield: 44.7%) as a yellow solid compound. LCMS

(ESI): $[M+H]^+$ = 603.3

Step 11: The solution containing [2-(6-azaspiro[2.5]oct-6-yl)-4-bromophenyl]-N-[8-(4,4-difluoropiperidinyl)-7-fluoro-2-methoxy(6-quinolinyl)]formamide (200 mg, 0.33 mmol), pyridine hydrochloride (391 mg, 3.31 mmol) and N,N-dimethylformamide (5 mL) was stirred at 100°C for 12 h. The reaction solution was filtered to give 4-bromo-N-(8-(4,4-difluoropiperidin-1-yl)-7-fluoro-2-hydroxyquinolin-6-yl)-2-(6-aza spiro[2.5]octan-6-yl) benzamide (140 mg, 0.24 mmol, yield: 71.7%) as a white solid compound. LCMS (ESI): $[M+H]^+$ = 589.1

Step 12: The reaction solution containing [2-(6-azaspiro[2.5]oct-6-yl)-4-bromophenyl]-N-[8-(4,4-difluoropiperidinyl)-7-fluoro-2-hydroxy(6-quinolinyl)]formamide (140 mg, 0.24 mmol), 2-hydroxyethanesulfonamide (45 mg, 0.36 mmol), potassium phosphate (154 mg, 0.71 mmol), cuprous iodide (46 mg, 0.24 mmol), trans-N,N'-dimethylcyclohexane-1,2-diamine (34 mg, 0.24 mmol) and 1,4-dioxane (5 mL) was stirred at 100°C for 12 h under nitrogen atmosphere. The reaction solution was poured into water (20 mL), extracted withethyl acetate (20 mL×3), and the combined organic layer was dried over anhydrous sodium sulfate, filtered and concentrated to give the residue, which was further purified by flash column chromatography (C18, 0-100% gradient of acetonitrile/water) to give N-(8-(4,4-difluoropiperidin-1-yl)-7-fluoro-2-hydroxyquinolin-6-yl)-4-((2-hydroxyeth yl)sulfonamido)-2-(6-azaspiro[2.5]oct-6-yl)benzamide (22.2 mg, 0.035 mmol, yield: 14.8%) as a white solid compound. LCMS (ESI): $[M+H]^+$ = 634.4, $^1H$ NMR (400 MHz, DMSO) δ 12.30 (s, 1H), 10.90 (s, 1H), 8.62 (s, 1H), 8.04 - 7.97 (m, 1H), 7.95 (d, *J* = 9.6 Hz, 1H), 7.26-6.98 (m, 2H), 7.08 (s, 1H), 6.52 (d, *J* = 9.6 Hz, 1H), 3.76 (t, *J* = 6.0 Hz, 2H), 3.23-2.97 (m, 4H), 2.87-2.76 (m, 4H), 2.72-2.58 (m, 2H), 2.06 - 1.97 (m, 4H), 1.57 (s, 4H), 0.37 (s, 4H).

**Example 77: N-(8-(4,4-difluoropiperidin-1-yl)-7-fluoro-2-methoxyquinolin-6-yl)-4-((2-hydroxy ethyl)sulfonamido)-2-(6-azaspiro[2.5]oct-6-yl)benzamide**

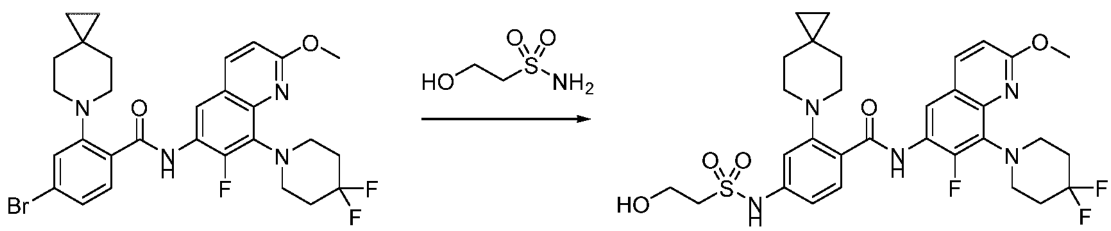

Step 1: The reaction solution containing [2-(6-azaspiro[2.5]oct-6-yl)-4-bromophenyl]-N-[8-(4,4-difluoropiperidinyl)-7-fluoro-2-methoxy(6-quinolinyl)]formamide (40 mg, 0.07 mmol), 2-hydroxyethanesulfonamide (13 mg, 0.10 mmol), potassium phosphate (43 mg, 0.20 mmol), cuprous iodide (13 mg, 0.066 mmol), trans-N,N'-dimethylcyclohexane-1,2-diamine (10 mg, 0.066 mmol) and 1,4-dioxane (5 mL) was stirred for 12 h under nitrogen atmosphere. The reaction solution was poured into water (20 mL), extracted with ethyl acetate (20 mL×3), and the combined organic layer was dried over anhydrous sodium sulfate, filtered and concentrated to give the residue, which was further purified by flash column chromatography (C18, 0-100% gradient of acetonitrile/water) to give N-(8-(4,4-difluoropiperidin-1-yl)-7-fluoro-2-methoxyquinolin-6-yl)-4-((2-hydroxyeth yl)sulfonamido)-2-(6-azaspiro[2.5]oct-6-yl)benzamide (4.0 mg, 6.2 umol, yield: 9.3%) as a white solid compound. LCMS (ESI): $[M+H]^+$ = 648.4; $^1H$ NMR (400 MHz, DMSO) δ 12.38 - 12.32 (m, 1H), 10.21 (s, 1H), 8.67 (d, *J* = 8.0 Hz, 1H), 8.23 (d, *J* = 8.8 Hz, 1H), 8.05 (d, *J* = 8.4 Hz, 1H), 7.31 (d, *J = 2.0* Hz, 1H), 7.15 (dd, *J* = 8.4, 2.0 Hz, 1H), 7.01 (d, *J* = 8.8 Hz, 1H), 4.99 (s, 1H), 4.02 (s, 3H), 3.79 (t, *J* = 6.4 Hz, 2H), 3.58 (t, *J* = 5.6 Hz, 4H), 3.37 (s, 2H), 3.01 (t, *J* = 5.2 Hz, 4H), 2.26-2.15 (m, 4H), 1.61 (s, 4H), 0.40 (s, 4H).

**Example 78: N-(2-amino-8-(4,4-difluoropiperidin-1-yl)-7-fluoroquinolin-6-yl)-4-((2-hydroxyet hyl)sulfonamido)-2-(6-azaspiro[2.5]octan-6-yl)benzamide**

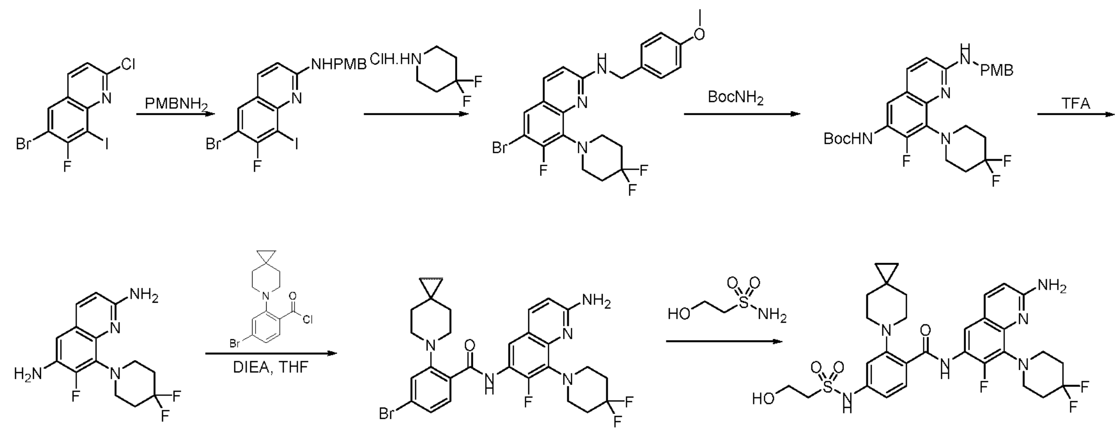


Step 1: The reaction solution containing 6-bromo-2-chloro-7-fluoro-8-iodoquinoline (1.00 g, 2.59 mmol), p-methoxybenzylamine (2.30 g, 12.94 mmol), N,N-diisopropylethylamine (3.10 g, 23.29 mmol) and acetonitrile (30 mL) was stirred at 60°C for 12 h. The reaction solution was poured into water (20 mL), extracted with ethyl acetate (20 mL×3), and the combined organic layer was dried over anhydrous sodium sulfate, filtered and concentrated to give the residue, which was further purified by column chromatography (silica gel, 0-50% gradient of ethyl acetate/petroleum ether) to give 6-bromo-7-fluoro-8-iodo-N-(4-methoxybenzyl)quinolin-2-amine (1.20 g, 2.46 mmol, yield: 31.7%) as a yellow solid compound. LCMS (ESI): $[M+H]^+$ = 487.0

Step 2: 4,5-bis(diphenylphosphino)-9,9-dimethylxanthene (145 mg, 0.25 mmol) and tris(dibenzylideneacetone) dipalladium (230 mg, 0.25 mmol) were successively added to a mixed solution containing 6-bromo-7-fluoro-8-iodo-N-(4-methoxybenzyl)quinolin-2-amine (1.20 g, 2.46 mmol), 4,4-difluoropiperidine hydrochloride (431 mg, 2.71 mmol), cesium carbonate (2.50 g, 7.39 mmol) and 1,4-dioxane (20 mL) under nitrogen atmosphere, and the reaction solution was stirred at 100°C for 12 h. The reaction mixture was poured into water (30 mL), extracted with ethyl acetate (30 mL×3), and the combined organic layer was dried over anhydrous sodium sulfate, filtered and concentrated to give the residue, which was further purified by column chromatography (silica gel, 0-40% gradient of ethyl acetate/petroleum ether) to give 6-bromo-8-(4,4-difluoropiperidin-1-yl)-7-fluoro-N-(4-methoxybenzyl)quinolin-2-ami ne (400 mg, 0.83 mmol, yield: 33.8%) as a yellow solid compound. LCMS (ESI): $[M+H]^+$ = 480.2

Step 3: 4,5-bis(diphenylphosphino)-9,9-dimethylxanthene (43 mg, 0.073 mmol) and tris(dibenzylideneacetone) dipalladium (68 mg, 0.073 mmol) were successively added to a solution of 6-bromo-8-(4,4-difluoropiperidin-1-yl)-7-fluoro-N-(4-methoxybenzyl)quinolin-2-ami ne (350 mg. 0.73 mmol), tert-butyl carbamate (103 mg, 0.87 mmol), cesium carbonate (727 mg, 2.19 mmol) and 1,4-dioxane (5 mL) under nitrogen atmosphere, and the reaction solution was stirred at 100°C for 12 h. The reaction solution was poured into water (30 mL), extracted with ethyl acetate (30 mL × 3), and the combined organic layer was dried over anhydrous sodium sulfate, filtered and concentrated to give the residue, which was further purified by column chromatography (silica gel, 0-20% gradient of ethyl acetate/petroleum ether) to give tert-butyl (8-(4,4-difluoropiperidin-1-yl)-7-fluoro-2-((4-methoxybenzyl)amino)quinolin-6-yl)ca rbamate (240 mg, 0.46 mmol, yield: 63.8%) as a yellow solid compound. LCMS (ESI): $[M+H]^+$ = 517.3

Step 4: The reaction solution containing tert-butyl (8-(4,4-difluoropiperidin-1-yl)-7-fluoro-2-((4-methoxybenzyl) amino)quinolin-6-yl)ca rbamate (240 mg, 0.46 mmol) and trifluoroacetic acid (2 mL) was stirred at 20°C for 1 h. After the concentration of the reaction solution, saturated aqueous sodium bicarbonate solution was added to and the pH of the reaction solution was adjusted to be close to 8. Then extracted with ethyl acetate (30 mL×3), the combined organic layer was dried. The combined organic layer was dried over anhydrous sodium sulfate, filtered, and concentrated to give 8-(4,4-difluoropiperidin-1-yl)-7-fluoroquinoline-2,6-diamine (140 mg, crude) as a yellow solid compound. LCMS (ESI): $[M+H]^+$ = 297.1

Step 5: 2-(6-azaspiro[2.5]oct-6-yl)-4-bromobenzoyl chloride (186 mg, 0.57 mmol) was added to a solution containing 8-(4,4-difluoropiperidin-1-yl)-7-fluoroquinoline-2,6-diamine (140 mg, 0.47 mmol), N,N-diisopropylethylamine (312 mg, 2.36 mmol) and THF (5 mL). The reaction solution was stirred at 20°C for 12 h, poured into water (30 mL), extracted with ethyl acetate (30 mL×3), and the combined organic layer was dried over anhydrous sodium sulfate, filtered and concentrated to give the residue, which was further purified by column chromatography (silica gel, 0-100% gradient of ethyl acetate/petroleum ether) to give N-(2-amino-8-(4,4-difluoropiperidin-1-yl)-7-fluoroquinolin-6-yl)-4-bromo-2-(6-azasp iro[2.5]octan-6-yl) benzamide (80 mg, 0.14 mmol, yield: 28.8%) as a yellow solid compound. LCMS (ESI): $[M+H]^+$ = 590.3

Step 6: The reaction solution containing N-(2-amino-8-(4,4-difluoropiperidin-1-yl)-7-fluoroquinolin-6-yl)-4-bromo-2-(6-azasp iro[2.5]octan-6-yl) benzamide (80 mg, 0.14 mmol), 2-hydroxyethanesulfonamide (26 mg, 0.20 mmol), potassium phosphate (88 mg, 0.41 mmol), cuprous iodide (26 mg, 0.14 mmol), trans-N,N'-dimethylcyclohexane-1,2-diamine (20 mg, 0.14 mmol) and 1,4-dioxane (5 mL) was stirred at 100°C for 12 h under nitrogen atmosphere. The reaction solution was poured into water (20 mL), extracted withethyl acetate (20 mL × 3), and the combined organic layer was dried over anhydrous sodium sulfate, filtered and concentrated to give the residue, which was further purified by flash column chromatography (C18, 0-100% gradient of acetonitrile/water) to give N-(2-amino-8-(4,4-difluoropiperidin-1-yl)-7-fluoroquinolin-6-yl)-4-((2-hydroxyethyl) sulfonamido)-2-(6-azaspiro[2.5]octan-6-yl)benzamide (3.60 mg, 5.7 umol, yield: 4.2%) as a yellow solid compound. LCMS (ESI): $[M+H]^+$ = 633.4, $^1H$ NMR (400 MHz, DMSO) δ 12.19 (s, 1H), 10.21 (s, 1H), 8.43 (s, 1H), 8.01 (d, *J* = 8.4 Hz, 1H), 7.29 (d, *J* = 2.0 Hz, 1H), 7.13 (dd, *J* = 8.4, 2.0 Hz, 1H), 6.76 (s, 3H), 6.40 (s, 1H), 4.94 (t, *J* = 5.6 Hz, 1H), 3.78 - 3.76 (m, 2H), 3.32-3.27(m, 4H), 3.15 (t, *J* = 6.8 Hz, 2H), 2.97 (d, *J* = 5.6 Hz, 4H), 2.17 (s, 4H), 1.58 (s, 4H), 0.37 (s, 4H).

Example 79: N-(8-(4,4-difluoropiperidin-1-yl)-7-fluoro-2-(methylamino)quinolin-6-yl)-4-((2-hydroxyethyl)sulfonamido)-2-(6-azaspiro[2.5] oct-6-yl)benzamide

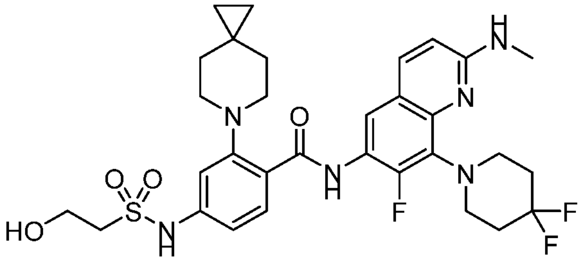

The preparations in Example 79 follow the synthetic route of Example 3: LCMS (ESI): [M+H]+ = 647.4; 1HNMR (400MHz, DMSO) δ 12.10 (s, 1H), 10.16 (s, 1H), 8.40 (d, J = 8.0 Hz, 1H), 8.00 (d, J = 8.4 Hz, 1H), 7.80 (d, J = 8.8 Hz, 1H), 7.27 (d, J = 2.4 Hz, 1H), 7.11 (dd, J = 8.4, 2.0 Hz, 1H), 7.06 (d, J = 4.8 Hz, 1H), 6.71 (d, J = 8.8 Hz, 1H), 4.94 (t, J = 5.6 Hz, 1H), 3.76 (q, J = 6.4 Hz, 2H), 3.54 (d, J = 5.6 Hz, 4H), 3.36 (d, J = 6.4 Hz, 2H), 2.98 (d, J = 5.2 Hz, 4H), 2.91 (d, J = 4.8 Hz, 3H), 2.16 (q, J = 15.2, 13.6 Hz, 4H), 1.58 (s, 4H), 0.37 (s, 4H).

**Example 80: N-(8-(4,4-difluoropiperidin-1-yl)-7-fluoroquinolin-6-yl)-6-((2-hydroxyethyl)sulfo namido)-4-(6-azaspiro[2.5]oct-6-yl)nicotinamide**

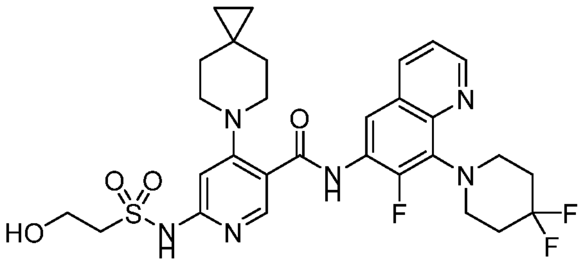

The preparations in **Example 80** follow the synthetic route of **Example 3:** LCMS (ESI): $[M+H]^+$ = 619.4, $^1H$ NMR (400 MHz, DMSO) δ 10.69 (s, 1H), 8.84 (dd, J = 4.0, 1.6 Hz, 1H), 8.43 (d, J = 7.6 Hz, 1H), 8.38 - 8.30 (m, 1H), 8.23 (s, 1H), 7.50 (dd, J = 8.4, 4.0 Hz, 1H), 6.66 (s, 1H), 3.78 (t, J = 6.8 Hz, 2H), 3.55 (t, J = 5.6 Hz, 4H), 3.42 (s, 2H), 3.23 - 3.05 (m, 4H), 2.26 - 2.08 (m, 4H), 1.52 - 1.44 (m, 4H), 0.34 (s, 4H).

**Example 81: N-(8-(4,4-difluoropiperidin-1-yl)isoquinolin-6-yl)-4-(2-hydroxyethylsulfonamido) -2-(6-azaspiro [2.5]octan-6-yl)benzamide**

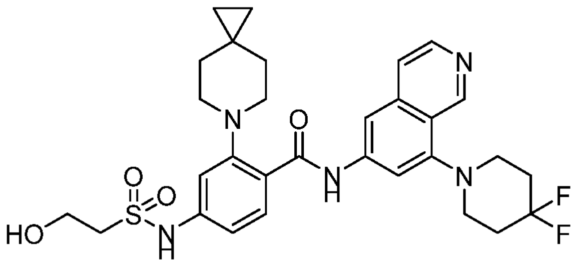

The preparations in **Example 81** follow the synthetic route of **Example 3:** LCMS (ESI): $[M+H]^+$ = 600.4, $^1H$ NMR (400 MHz, DMSO) δ 11.96 (s, 1H), 10.18 (s, 1H), 9.18 (s, 1H), 8.26 (d, J = 5.8 Hz, 1H), 8.11 (d, J = 1.6 Hz, 1H), 7.68 (d, J = 8.4 Hz, 1H), 7.56 (d, J = 5.6 Hz, 1H), 7.22 (d, J = 1.8 Hz, 1H), 6.99 (d, J = 2.0 Hz, 1H), 6.86 (dd, J = 8.4, 2.1 Hz, 1H), 5.28 - 4.88 (m, 1H), 3.60 (t, J = 6.4 Hz, 2H), 3.17 (s, 2H), 3.10 (dd, J = 26.0, 6.2 Hz, 4H), 2.83 (t, J = 5.2 Hz, 4H), 2.15 (tt, J = 13.6, 11.6, 5.4 Hz, 4H), 1.41 (t, J = 5.2 Hz, 4H), 0.18 (s, 4H).

**Example 82: 2-{6-azaspiro[2.5]octan-6-yl}-N-[8-(4,4-difluoropiperidin-1-yl)misonin-6-yl]-4-(2-hydroxyethane-sulfonamido)benzamide**

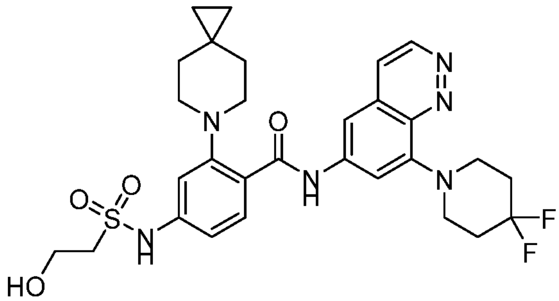

The preparations in **Example 82** follow the synthetic route of **Example 3:** LCMS (ESI): [M+H]+ = 601.2, $^1H$ NMR (400 MHz, DMSO-d6) δ ppm 12.06 (s, 1H), 10.15 (s, 1H), 9.24 (br s, 1H), 8.24 (s, 1H), 8.12 (br s, 1H), 7.84 (d, *J* = 8.4 Hz, 1H), 7.35 (s, 1H), 7.18 (s, 1H), 7.06 (dd, *J* = 1.6, 8.4 Hz, 1H), 5.53 - 4.23 (m, 1H), 3.77 (t, *J* = 6.4 Hz, 2H), 3.72 - 3.64 (m, 4H), 3.42 - 3.39 (m, 2H), 3.07 - 2.94 (m, 4H), 2.33 - 2.22 (m, 4H), 1.64 - 1.52 (m, 4H), 0.35 (s, 4H)

**Example 83: N-(8-(4,4-difluoropiperidin-1-yl)quinoxalin-6-yl)-4-((2-hydroxyethyl)sulfonamido) -2-(6-azaspiro [2.5]octan-6-yl)benzamide**

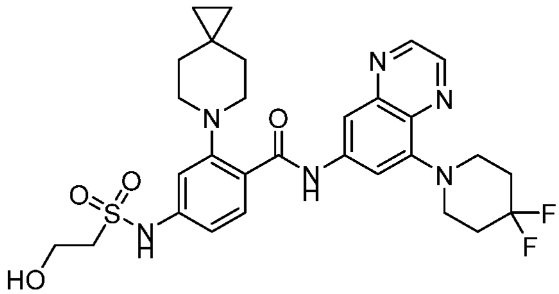

The preparations in **Example 83** follow the synthetic route of **Example 3:** LCMS (ESI): $[M+H]^+$ =601.4. $^1H$ NMR (400 MHz, CDCl3) δ 12.95 (s, 1H), 8.80 (d, *J* = 1.9 Hz, 1H), 8.69 (d, *J* = 1.9 Hz, 1H), 8.21 (d, *J* = 8.6 Hz, 1H), 7.98 (d, *J* = 2.1 Hz, 1H), 7.91 (d, *J* = 2.2 Hz, 1H), 7.39 (d, *J* = 2.2 Hz, 1H), 7.15 (dd, *J* = 8.6, 2.1 Hz, 1H), 4.02 (t, *J* = 5.6 Hz, 2H), 3.59 (t, *J* = 5.7 Hz, 4H), 3.33 (s, 2H), 3.11 (t, *J* = 5.4 Hz, 4H), 2.31 (tt, *J* = 12.9, 5.5 Hz, 4H), 1.71 (s, 4H), 0.45 (s, 4H).

**Example 84: N-(2-amino-8-(4,4-difluoropiperidin-1-yl)quinazolin-6-yl)-4-((2-hydroxyethyl)sulf onamido)-2-(6-azaspiro[2.5]oct-6-yl)benzamide**

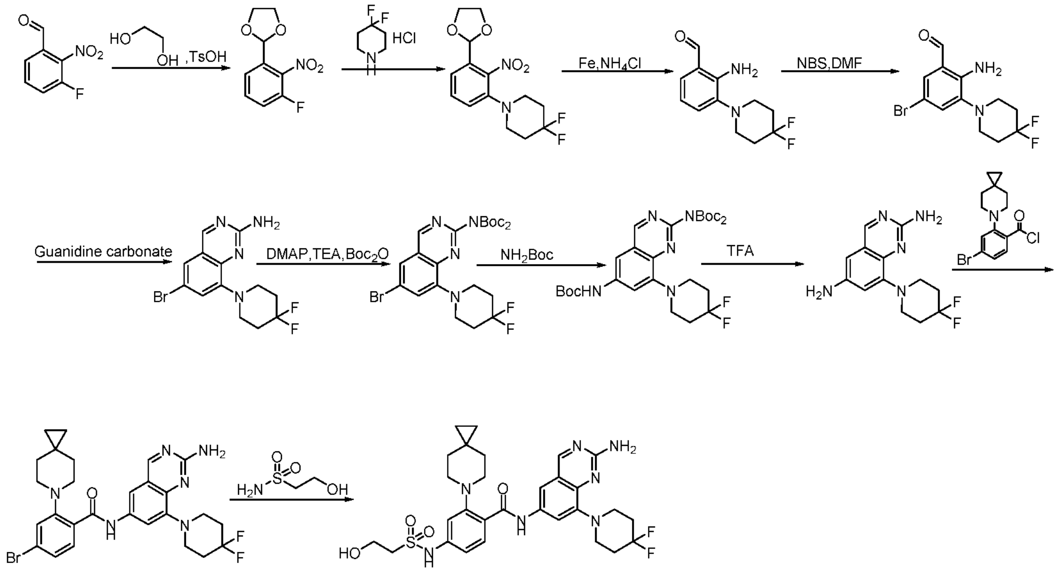


Step 1: 3-fluoro-2-nitrobenzaldehyde (5.00 g, 28.68 mmol), ethylene glycol (2.68 g, 43.01 mmol) and p-toluenesulfonic acid monohydrate (278 mg, 1.43 mmol) were successively added to toluene (40 mL), and the reaction solution was stirred at 100°C for 4 h. The reaction solution was poured into water (50 mL), extracted with ethyl acetate (50 mL × 3), The combined organic layer was dried over anhydrous sodium sulfate, and concentrate to obtain the crude. The crude was purified by flash column chromatography (silica gel, 10-25% gradient of ethyl acetate/petroleum ether) to give 2-(3-fluoro-2-nitrophenyl)-1,3-dioxolane (6.40 g, crude) as a pale yellow oily liquid. LCMS (ESI): $[M+H]^+$ =214.1

Step 2: 2-(3-Fluoro-2-nitrophenyl)-1,3-dioxolane (5.10 g, 23.93 mmol), 4,4-difluoropiperidine hydrochloride (5.71 g, 35.89 mmol) and potassium carbonate (8.43 g, 59.81 mmol) were successively added to DMSO (40 mL), and the reaction solution was stirred at 130°C for 16 h. The reaction solution was poured into water (50 mL), extracted with ethyl acetate (50 mL×3),The combined organic layer was washed with saturated sodium chloride solution (30 mL×3), dried over anhydrous sodium sulfate, and concentrated to give 1-(3-(1,3-dioxolan-2-yl)-2-nitrophenyl)-4,4-difluoropiperidine (6.00 g, crude). LCMS (ESI): $[M+H]^+$ =315.2

Step 3: 1-(3-(1,3-dioxolan-2-yl)-2-nitrophenyl)-4,4-difluoropiperidine (5.00 g, 15.91 mmol), ammonium chloride (1.72 g, 31.82 mmol), and iron powder (4.49 g, 79.54 mmol) were successively added to a solution of THF (20 mL), ethanol (20 mL), and water (5 mL), and the reaction solution was heated to 80°C and reacted for 16 h. The reaction solution was filtered under reduced pressure, , The filtrate was poured into water (30 mL), extracted with ethyl acetate (30 mL × 3), The combined organic layer was dried over anhydrous sodium sulfate, and concentrate to obtain the crude. The crude was purified by flash column chromatography (silica gel, 0-9% gradient of ethyl acetate/petroleum ether) to give 2-amino-3-(4,4-difluoropiperidin-1-yl)benzaldehyde (2.10 g, 8.75 mmol, yield: 54.9%) as a yellow solid crude. LCMS (ESI): $[M+H]^+$ =241.1

Step 4: 2-amino-3-(4,4-difluoropiperidin-1-yl)benzaldehyde (1.80 g, 7.49 mmol) was added to N,N-dimethylformamide (15 mL), then N-bromosuccinimide (1.47 g, 8.24 mmol) was added to the reaction solution, and the reaction solution was stirred at 20°C for 12 h. The reaction solution was poured into water (30 mL), extracted with ethyl acetate (30 mL × 3), The combined organic layer was washed with the saturated aqueous sodium chloride solution (30 mL × 3), dried over anhydrous sodium sulfate, and concentrated to obtain the crude. The crude was purified by flash column chromatography (silica gel, 0-9% gradient of ethyl acetate/petroleum ether) to give 2-amino-5-bromo-3-(4,4-difluoropiperidin-1-yl)benzaldehyde (1.60 g, 5.01 mmol, yield: 66.9%) as a yellow solid crude. LCMS (ESI): $[M+H]^+$ =319.1

Step 5: 2-amino-5-bromo-3-(4,4-difluoropiperidin-1-yl) benzaldehyde (1.60 g, 5.01 mmol) and guanidine carbonate (9.12 g, 50.13 mmol) were successively added to N,N-dimethylacetamide (10 mL), and the reaction solution was heated to 130°C and stirred for 2 h. The reaction solution was cooled to ambient temperature, poured into water (30 mL), suction filtration, and dried the solid obtained by filtration to give 6-bromo-8-(4,4-difluoropiperidin-1-yl)quinazoline-2-amine (1.60 g, 4.66 mmol, yield: 93.0%) as a yellow solid. LCMS (ESI): $[M+H]^+$ =343.1; $^1H$ NMR (400 MHz, DMSO-$d_6$) δ 9.82 (s, 1H), 7.59 (d, *J* = 2.2 Hz, 1H), 7.30 (d, *J* = 2.3 Hz, 1H), 6.98 (s, 2H), 2.91 (s, 4H), 2.29 - 2.06 (m, 4H).

Step 6: 6-bromo-8-(4,4-difluoropiperidin-1-yl)quinazoline-2-amine (1.45 g, 4.22 mmol), di-tert-butyl dicarbonate

(4.61 g, 21.13 mmol), 4-dimethylaminopyridine (105 mg, 0.85 mmol), and triethylamine (2.18 g, 21.13 mmol) were successively added to dichloromethane (20 mL), and the reaction solution was stirred at 50°C for 4 h. The reaction solution was poured into water (30 mL), extracted with dichloromethane (30 mL × 3), The combined organic layer was dried over anhydrous sodium sulfate, and concentrated to obtain the crude. The crude was purified by flash column chromatography (silica gel, 0-9% gradient of ethyl acetate/petroleum ether) to give tert-butyl(6-bromo-8-(4,4-difluoropiperidin-1-yl)quinazolin-2-yl)(tert-butoxycarbonyl) carbamate (1.72 g, 3.16 mmol, yield: 74.9%) as a pale yellow solid crude. LCMS (ESI): $[M+H]^+$ =369.1; $^1H$ NMR (400 MHz, Chloroform-d) δ 9.22 (s, 1H), 7.64 (d, *J* = 2.0 Hz, 1H), 7.28 (d, *J* = 2.0 Hz, 1H), 3.54 (t, *J* = 5.5 Hz, 4H), 2.24 (tt, *J* = 13.2, 5.3 Hz, 4H), 1.50 (s, 18H).

Step 7: tert-butyl(6-bromo-8-(4,4-difluoropiperidin-1-yl)quinazolin-2-yl)(tert-butoxycarbonyl) carbamate (1.00 g, 1.84 mmol), tert-butyl carbamate (653 mg, 5.52 mmol), methanesulfonic acid [9,9-dimethyl-4,5-bis(diphenylphosphine) xanthracene][2'-amino-1,1'- biphenyl] palladium (II) dichloromethane adduct (178 mg, 0.18 mmol), and cesium carbonate (1.23 g, 3.68 mmol) were successively added to 1,4-dioxane (10 mL) under nitrogen atmosphere. After nitrogen replacement, the reaction solution was heated to 110°C and stir for 3 h. The reaction solution was poured into water (30 mL), extracted withethyl acetate (30 mL × 3), The combined organic layer was dried over anhydrous sodium sulfate, and concentrate to obtain the crude. The crude was purifiedby flash column chromatography (silica gel, 10-25% gradient of ethyl acetate/petroleum ether) to give tert-butyl(tert-butoxycarbonyl)(6-(tert-butoxycarbonyl)amino)-8-(4,4-difluoropiperidi n-1-yl)quinazolin-2-yl)carbamate (800 mg, 1.38 mmol, yield: 75.0%) as a yellow solid. LCMS (ESI): $[M+H]^+$ =580.6

Step 8: tert-butyl(tert-butoxycarbonyl)(6-(tert-butoxycarbonyl)amino)-8-(4,4-difluoropiperidi n-1-yl)quinazolin-2-yl)carbamate (1.00 g, 1.72 mmol) was added to a mixed solution of dichloromethane (5 mL) and trifluoroacetic acid (5 mL), and the reaction solution was stirred at 25°C for 4 h. The reaction solution was concentrated to give the crude, the pH of the crude was adjusted to 7 with saturated aqueous sodium bicarbonate solution, extracted withdichloromethane (20 mL × 3), The combined organic layer was dried over anhydrous sodium sulfate, and concentrated to give 8-(4,4-difluoropiperidin-1-yl)quinazoline-2,6-diamine (200 mg, 0.72 mmol, yield: 41.5%) as a green solid. LCMS (ESI): $[M+H]^+$ =280.4; $^1H$ NMR (400 MHz, DMSO-$d_6$) δ 8.73 (s, 1H), 6.66 (d, *J* = 2.3 Hz, 1H), 6.37 (d, *J* = 2.2 Hz, 1H), 6.14 (s, 2H), 5.05 (s, 2H), 3.34 (s, 4H), 2.17 (td, *J* = 14.1, 13.5, 6.4 Hz, 4H).

Step 9: 8-(4,4-difluoropiperidin-1-yl)quinazoline-2,6-diamine (200 mg, 0.72 mmol), N,N-diisopropylethylamine (236 mg, 1.79 mmol), and 4-bromo-2-(6-azaspiro[2.5]octan-6-yl) benzoyl chloride (471 mg, 1.43 mmol) were successively added to dichloromethane (5 mL), and the reaction solution was stirred at 25°C for 2 h. The reaction solution was poured into water (20 mL), extracted with dichloromethane (20 mL × 3), The combined organic layer was dried over anhydrous sodium sulfate, and concentrated to obtain the crude. The crude was purified by flash column chromatography (silica gel, 25-50% gradient of ethyl acetate/dichloromethane) to give N-(2-amino-8-(4,4-difluoropiperidin-1-yl)quinazolin-6-yl)-4-bromo-2-(6-azaspiro[2.5] octan-6-yl) benzamide (190 mg, 0.33 mmol, yield: 46.4%) as a blue-green solid. LCMS (ESI): $[M+H]^+$ =573.3

Step 10: N-(2-amino-8-(4,4-difluoropiperidin-1-yl)quinazolin-6-yl)-4-bromo-2-(6-azaspiro[2.5] octan-6-yl) benzamide (60 mg, 0.10 mmol), 2-hydroxyethanesulfonamide (27 mg, 0.21 mmol), potassium phosphate (91 mg, 0.42 mmol), cuprous iodide (41 mg, 0.21 mmol) and (1S,2S)-(+)-N,N'-dimethyl-1,2-cyclohexanediamine (60 mg, 0.42 mmol) were successively added to N,N-dimethylformamide (1 mL) under nitrogen atmosphere. After nitrogen replacement, the reaction solution was stirred at 115°C for 2 h. The reaction solution was filtered under reduced pressure, , The filtrate was poured into the saturated ammonium chloride solution (30 mL), extracted with ethyl acetate (30 mL × 3), The combined organic layer was washed with the saturated sodium chloride solution (30 mL × 3), dried with anhydrous sodium sulfate, and concentrated to obtain the crude. The crude was purified by flash column chromatography (C18, 25-45% gradient of acetonitrile/water) to give N-(2-amino-8-(4,4-difluoropiperidin-1-yl)quinazolin-6-yl)-4-((2-hydroxyethyl)sulfon amido)-2-(6-azaspiro[2.5]oct-6-yl)benzamide (15 mg, 0.105 mmol, yield: 23.2%) as a grayish green solid. LCMS (ESI): $[M+H]^+$ =616.6; $^1H$ NMR (400 MHz, DMSO-$d_6$) δ 11.89 (s, 1H), 9.02 (s, 1H), 8.08 (s, 1H), 7.85 (d, *J* = 7.2 Hz, 1H), 7.14 (d, *J* = 95.5 Hz, 3H), 6.64 (s, 2H), 3.75 (d, *J* = 6.0 Hz, 2H), 3.45 (d, *J* = 5.5 Hz, 6H), 2.98 (t, *J* = 5.0 Hz, 4H), 2.21 (dd, *J* = 16.6, 10.2 Hz, 4H), 1.58 (s, 4H), 0.35 (s, 4H).

**Example 85: N-(8-(4,4-difluoropiperidin-1-yl)-3-(dimethylphosphoryl)quinolin-6-yl)-4-(2-hydr oxyethylsulfonamido)-2-(6-azaspiro[2.5]octan-6-yl)benzamide**

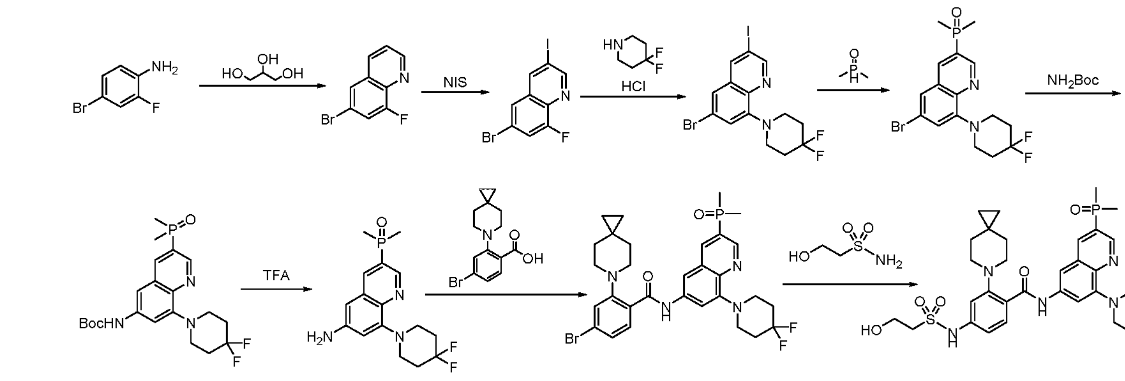


Step 1: Glycerol (63.00 g, 684.17 mmol) was added to a mixed solution of concentrated sulfuric acid (20 mL) and water (15 mL), and the mixture was heated to 100°C and stirred for 15 min. The mixture was cooled to 40°C then 4-bromo-2-fluoroaniline (10.00 g, 52.63 mmol) and sodium 3-nitrobenzenesulfonate (11.96 g, 52.63 mmol) were added thereto, and the mixture was heated to 100°C and stirred for 15 h. After completion of the reaction, water (100 mL) was added to the reaction mixture and extracted with ethyl acetate (100 mL × 3). The combined organic layer was dried over anhydrous sodium sulfate, filtered and concentrated under reduced pressure to give the residue. The residue was purified by flash column chromatography (silica gel, 0-25% gradient of ethyl acetate/petroleum ether) to give 6-bromo-8-fluoroquinoline (10.00 g, 44.25 mmol, yield: 84.0%) as a yellow solid. LCMS (ESI): $[M+H]^+$ = 226.0

Step 2: N-iodosuccinimide (11.94 g, 53.08 mmol) was added to a solution of 6-bromo-8-fluoroquinoline (8.00 g, 35.39 mmol) in acetic acid (80 mL), and the mixture was heated to 120°C with stirring for 36 h. After completion of the reaction, The reaction solution was poured into saturated aqueous sodium bicarbonate solution (200 mL) and extracted with ethyl acetate (200 mL×3). The combined organic layer was washed with saturated aqueous sodium chloride solution (100 mL), dried over anhydrous sodium sulfate, concentrated under reduced pressure, and The crude was purifiedby flash column chromatography (silica gel, 0-20% gradient of ethyl acetate/petroleum ether) to give 6-bromo-8-fluoro-3-iodoquinoline (4.20 g, 11.93 mmol, yield: 33.7%) as a yellow solid. LCMS (ESI): $[M+H]^+$ = 351.9; $^1$HNMR (400 MHz, DMSO) δ 9.12 (d, J = 2.0 Hz, 1H), 8.92 (t, J = 1.7 Hz, 1H), 8.07 (t, J = 1.6 Hz, 1H), 7.92 (dd, J = 10.2, 2.1 Hz, 1H).

Step 3: Potassium carbonate (4.81 g, 34.09 mmol) was added to the solution of 6-bromo-8-fluoro-3-iodoquinoline (4.00 g, 11.36 mmol) and 4,4-difluoropiperidine hydrochloride (2.71 g, 17.05 mmol) in N,N-dimethylformamide (40 mL), and The mixture was stirred at 100°C for 24 h. After completion of the reaction, water (40 mL) was added to the reaction mixture and extracted with ethyl acetate (100 mL×3). The combined organic layer was dried over anhydrous sodium sulfate, filtered and concentrated under reduced pressure to give the residue. The residue was purified by flash column chromatography (silica gel, 0-50% gradient of ethyl acetate/petroleum ether) to give 8-(4,4-difluoropiperidinyl)-6-bromo-3-iodoquinoline (4.20 g, 9.27 mmol, yield: 81.6%) as a yellow solid. LCMS (ESI): $[M+H]^+$ = 453.0

Step 4: The solution of triphenylphosphine (231 mg, 0.88 mmol), palladium acetate (101 mg, 0.44 mmol) and triethylamine (1367 mg, 13.24 mmol) were successively added to the solution of 8-(4,4-difluoropiperidinyl)-6-bromo-3-iodoquinoline (2.00 g, 4.41 mmol) and dimethylphosphine oxide (344 mg, 4.41 mmol) in ethanol (20 mL) under nitrogen atmosphere, and The mixture was stirred at 80°C for 6 h. After completion of the reaction, water (40 mL) was added to the reaction mixture and extracted with dichloromethane (50 mL × 3). The combined organic layer was dried over anhydrous sodium sulfate, filtered and concentrated under reduced pressure to give the residue. The residue was purified by flash column chromatography (silica gel, 0-20% gradient of methanol/dichloromethane) to give 6-bromo-8-(4,4-difluoropiperidin-1-yl)quinolin-3-yl)dimethylphosphine oxide (1.10 g, 2.73 mmol, yield: 61.8%) as a yellow solid. LCMS (ESI): $[M+H]^+$ = 403.1; $^1$HNMR (400 MHz, DMSO) δ 9.16 (dd, J = 4.2, 2.0 Hz, 1H), 8.71 (dd, J = 12.5, 2.1 Hz, 1H), 7.94 (d, J = 2.0 Hz, 1H), 7.34 (d, J = 2.1 Hz, 1H), 3.53 (t, J = 5.7 Hz, 4H), 2.20 (td, J = 14.0, 6.9 Hz, 4H), 1.80 (d, J = 13.6 Hz, 6H).

Step 5: 6-bromo-8-(4,4-difluoropiperidin-1-yl)quinolin-3-yl)dimethylphosphine oxide (1.00 g, 2.48 mmol), tert-butyl carbamate (435 mg, 3.72 mmol), tris(dibenzylideneacetone) dipalladium (227 mg, 0.25 mmol), 4,5-bis(diphenylphosphino)-9,9-dimethylxanthene (287 mg, 0.50 mmol), and cesium carbonate (2.42 g, 7.44 mmol) were successively added in a vial under nitrogen atmosphere. Then 1,4-dioxane (10 mL) was added to the reaction vial, and The mixture was stirred at 110°C for 8 h. After the completion of reaction, the reaction was cooled to ambient temperature, diluted the mixture with water (30 mL), extracted withethyl acetate (50 mL×3), and the combined organic layer was dried with anhydrous sodium sulfate, filtered and concentrated to give the residue, which was further purified by column chromatography (silica gel, 0-10% gradient of methanol/dichloromethane) to give tert-butyl (8-(4,4-difluoropiperidin-1-yl)-3-(dimethylphosphoryl) quinolin-6-yl)carbamate (600 mg, 1.36 mmol, yield: 55.0%) as a yellow solid. LCMS (ESI): $[M+H]^+$ = 440.3

Step 6: Dissolve tert-butyl (8-(4,4-difluoropiperidin-1-yl)-3-(dimethylphosphoryl)quinolin-6-yl)carbamate (500 mg, 1.13 mmol) in dichloromethane (5 mL), add trifluoroacetic acid (2.5 mL) dropwise, and The reactionmixture was stirred at 25°C for 5 h. Remove the solvent under reduced pressure, dissolve the residue in water (10 mL), the pH was adjusted of

the aqueous layer to 8 with saturated aqueous sodium bicarbonate solution (40 mL), and extracted withdichloromethane (50 mL×3).The combined organic layer was washedthe combined organic layer was washed with saturated aqueous sodium chloride solution (50 mL), dry over anhydrous sodium sulfate, and concentrate under reduced pressure to give 6-amino-8-(4,4-difluoropiperidin-1-yl)quinolin-3-yl)dimethylphosphine oxide (400 mg, 1.17 mmol, yield: 93.2%) as a green solid. LCMS (ESI): $[M+H]^+$ = 340.2

Step 7: N,N,N',N'-tetramethyl-O-(7-azabenzotriazol-1-yl) hexafluorophosphate urea (686 mg, 1.77 mmol) and N,N-diisopropylethylamine (466 mg, 3.54 mmol) were successively added to the solution of 6-amino-8-(4,4-difluoropiperidin-1-yl)quinolin-3-yl)dimethylphosphine oxide (400 mg, 1.17 mmol) and 2-(6-azaspiro[2.5]oct-6-yl)-4-bromobenzoic acid (402 mg, 1.30 mmol) in dichloromethane (8 mL), and The mixture was stirred at 100°C for 24 h. After completion of the reaction, water (10 mL) was added to the reaction mixture and extracted with dichloromethane (20 mL × 3). The combined organic layer was dried over anhydrous sodium sulfate, filtered and concentrated under reduced pressure to give the residue. The residue was purified by flash column chromatography (silica gel, 0-20% gradient of methanol/dichloromethane) to give 4-bromo-N-(8-(4,4-difluoropiperidin-1-yl)-3-(dimethylphosphoryl)quinolin-6-yl)-2-(6 -azaspiro[2.5]octan-6-yl) benzamide (500 mg, 0.792 mmol, yield: 67.2%) as a yellow solid. LCMS (ESI): $[M+H]^+$ = 631.3

Step 8: 2-hydroxyethanesulfonamide (61 mg, 0.47 mmol), cuprous iodide (61 mg, 0.32 mmol), potassium phosphate (274 mg, 1.27 mmol), and trans-N,N'-dimethylcyclohexane-1,2-diamine (91 mg, 0.63 mmol) were successively added in a vial under nitrogen atmosphere. N,N-dimethylformamide (4 mL) was added to the reaction vial, The mixture was stirred at 50°C for 0.5 h, then 4-bromo-N-(8-(4,4-difluoropiperidin-1-yl)-3-(dimethylphosphoryl)quinolin-6-yl)-2-(6 -azaspiro[2.5]octan-6-yl) benzamide (200 mg, 0.32 mmol) was added to the vial, and The mixture was stirred at 50°C for 5 min. The reaction system was heated to 120°C and stirred for 5 h. After completion of the reaction, the system was cooled to ambient temperature, The reaction solution was poured into water (10 mL), extracted with dichloromethane (30 mL × 3), and the combined organic layer was dried with anhydrous sodium sulfate. After filtration and concentration and purified by HPLC purification (C18, 0-100% gradient of acetonitrile/water) to give N-(8-(4,4-difluoropiperidin-1-yl)-3-(dimethylphosphoryl)quinolin-6-yl)-4-(2-hydroxy ethylsulfonamido)-2-(6-azaspiro[2.5]octan-6-yl)benzamide (105 mg, 0.15 mmol, yield: 49.0%) as a white solid compound. LCMS(ESI)m/z: $[M+H]^+$ =676.4; $^1$H NMR (400 MHz, DMSO) δ 11.94 (s, 1H), 10.12 (s, 1H), 9.02 (dd, J = 4.3, 2.0 Hz, 1H), 8.64 (dd, J = 12.7, 2.0 Hz, 1H), 8.29 (d, J = 2.0 Hz, 1H), 7.86 (d, J = 8.5 Hz, 1H), 7.50 (d, J = 2.2 Hz, 1H), 7.19 (d, J = 2.1 Hz, 1H), 7.06 (dd, J = 8.5, 2.0 Hz, 1H), 4.97 (s, 1H), 3.77 (ddt, J = 8.0, 6.1, 2.0 Hz, 2H), 3.55 (t, J = 5.6 Hz, 4H), 3.35 (t, J = 6.7 Hz, 2H), 3.01 (t, J = 5.3 Hz, 4H), 2.24 (s, 4H), 1.80 (d, J = 13.5 Hz, 6H), 1.58 (s, 4H), 0.35 (s, 4H).

**Example 86: Ethyl 6-(2-{6-azaspiro[2.5]octan-6-yl}-4-(2-hydroxyethanesulfonamido)benzoylamino)-8-(4,4-difluoropiperidin-1-yl)quinoline-3-carboxylate**

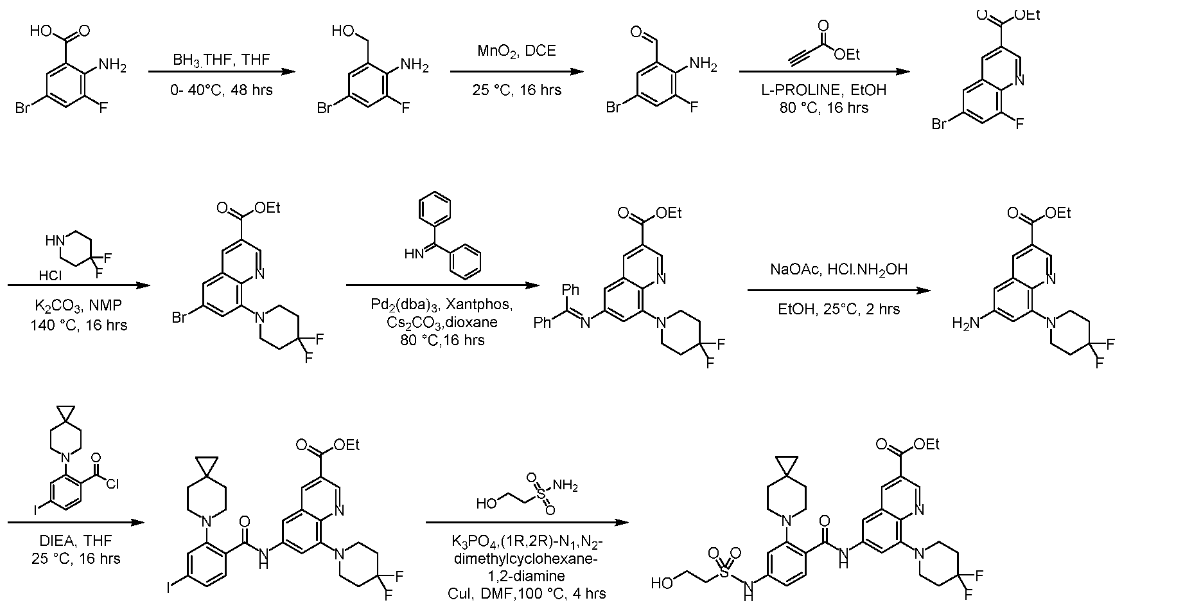


Step 1: 2-amino-5-bromo-3-fluorobenzoic acid (5.00 g, 21.3 mmol) was dissolved in THF (50.0 mL), then borane THF (64.1 mL, 64.1 mmol) was added at 0°C under nitrogen atmosphere, and stirred at 20-40°C for 48 h. The reaction solution was quenched with methanol (100 mL) at 0°C and concentrated under reduced pressure. The residue was

purified by flash column chromatography (silica gel, 0-10% gradient of THF/petroleum ether) to give (2-amino-5-bromo-3-fluorophenyl)methanol (4.48 g, 14.9 mmol, yield: 70.0%) as a white solid. LCMS (ESI): $[M+H]^+$ = 221.9; $^1H$ NMR (400 MHz, DMSO-$d_6$) δ ppm 7.19 (dd, *J* = 2.4, 10.8 Hz, 1H), 7.13 (s, 1H), 5.27 (t, *J* = 5.6 Hz, 1H), 5.07 (br s, 2H), 4.41 (d, *J* = 5.6 Hz, 2H)

Step 2: (2-amino-5-bromo-3-fluorophenyl)methanol (4.28 g, 19.4 mmol) was dissolved in 1,2-dichloroethane (84.0 mL), and manganese dioxide (19.7 g, 194 mmol) was added, and stirred at 25°C for 16 h. The reaction solution was filter and concentrated under reduced pressure to give 2-amino-5-bromo-3-fluorobenzene (formaldehyde) (4.00 g, 16.5 mmol, yield: 85.0%) as a brown solid. LCMS (ESI): $[M+H]^+$ = 220.0; $^1H$ NMR (400 MHz, DMSO-$d_6$) δ ppm 9.85 (s, 1H), 7.67 (br s, 1H), 7.58 (br d, *J* = 10.8 Hz, 1H), 7.14 (br s, 2H)

Step 3: 2-amino-5-bromo-3-fluorobenzene (formaldehyde) (4.00 g, 18.3 mmol) and ethyl prop-2-ynyl ester (2.16 g, 22.0 mmol) were successively dissolved in ethanol (80.0 mL), then L-proline (1.07 g, 9.17 mmol) was added, and stirred at 80°C for 16 h. Water (100 mL) was added into the reaction solution, extracted with ethyl acetate (200 mL×2), the combined organic layer was dried , filtered, and concentrated. The residue was purified by flash column chromatography (silica gel, 0-9% gradient of THF/petroleum ether) to give ethyl 6-bromo-8-fluoroquinoline-3-carboxylate (4.77 g, 14.3 mmol, yield: 78.0%) as an orange solid. LCMS (ESI): $[M+H]^+$ = 297.9; $^1H$ NMR (400 MHz, DMSO-$d_6$) δ ppm 9.31 (d, *J* = 2.0 Hz, 1H), 8.99 - 8.96 (m, 1H), 8.36 - 8.32 (m, 1H), 8.01 (dd, *J* = 2.0, 10.0 Hz, 1H), 4.43 (q, *J* = 7.2 Hz, 2H), 1.39 (t, *J* = 7.2 Hz, 3H)

Step 4: Ethyl 6-bromo-8-fluoroquinoline-3-carboxylate (4.67 g, 15.6 mmol) was dissolved in N-methylpyrrolidone (90.0 mL), 4,4-difluoropiperidine hydrochloride (3.21 g, 20.3 mmol) and potassium carbonate (6.50 g, 47.0 mmol) were successively added, and stirred at 140°C for 16 h. Water (200 mL) was added into the reaction solution, extracted with ethyl acetate (200 mL × 2), the combined organic layer was dried, filtered, and concentrated. The residue was purified by flash column chromatography (silica gel, 0-5% gradient of THF/petroleum ether) to give ethyl 6-bromo-8-(4,4-difluoropiperidin-1-yl)quinoline-3-carboxylate (2.50 g, 5.95 mmol, yield: 38.0%) as a yellow solid. LCMS (ESI): $[M+H]^+$ = 399.0; $^1H$ NMR (400 MHz, CHLOROFORM-d) δ ppm 9.33 (d, *J* = 1.6 Hz, 1H), 8.60 (d, *J* = 2.0 Hz, 1H), 7.63 (d, *J* = 1.6 Hz, 1H), 7.21 (d, *J* = 1.6 Hz, 1H), 4.40 (q, *J* = 7.2 Hz, 2H), 3.46 (br t, *J* = 4.8 Hz, 4H), 2.34 - 2.13 (m, 4H), 1.38 (t, *J* = 7.2 Hz, 3H)

Step 5: Ethyl 6-bromo-8-(4,4-difluoropiperidin-1-yl)quinolin-3-carboxyl ester (2.68 g, 8.17 mmol) and diphenylmethanimine (1.78 g, 9.80 mmol) were successively added in dioxane (50.0 mL), then cesium carbonate (7.98 g, 24.5 mmol), 4,5-bis(diphenylphosphino)-9,9-dimethylxanthene (0.95 g, 1.63 mmol), and tris(dibenzylideneacetone) dipalladium (0.75 g, 0.82 mmol) were successively added, and stirred for 16 h at 80°C under nitrogen atmosphere. Water (100 mL) was added into the reaction solution, extracted withethyl acetate (200 mL×2), the combined organic layer was dried , filtered, and concentrated. The residue was purified by flash column chromatography (silica gel, 0-9% gradient of THF/petroleum ether) to give ethyl 8-(4,4-difluoropiperidin-1-yl)-6-[(diphenylmethyl anionene)amino]quinoline-3-carboxylate (3.20 g, 6.28 mmol, yield: 77.0%) as a yellow solid. LCMS (ESI): $[M+H]^+$ = 429.1; $^1H$ NMR (400 MHz, DMSO-$d_6$) δ ppm 8.89 - 8.56 (m, 1H), 8.07 (br d, *J* = 8.0 Hz, 1H), 7.72 (br d, *J* = 7.2 Hz, 2H), 7.56 (br d, *J* = 7.2 Hz, 1H), 7.53 - 7.47 (m, 2H), 7.39 (dd, *J* = 4.0, 8.0 Hz, 1H), 7.34 - 7.27 (m, 3H), 7.27 - 7.19 (m, 2H), 6.85 (s, 1H), 3.31 - 3.25 (m, 4H), 2.18 - 2.02 (m, 4H)

Step 6: Ethyl 8-(4,4-difluoropiperidin-1-yl)-6-[(diphenylmethyl anionene)amino]quinoline-3-carboxylate (3.10 g, 6.21 mmol) was dissolved in ethanol (60.0 mL), then sodium acetate (1.53 g, 18.6 mmol) and hydroxylamine hydrochloride (0.86 g, 12.4 mmol) were successively added, and stirred for 2 h at 25°C. Water (100 mL) was added into the reaction solution, extracted withethyl acetate (200 mL×2), the combined organic layer was dried, filtered, and concentrated. The residue was purified by flash column chromatography (silica gel, 0-27% gradient of THF/petroleum ether) to give ethyl 6-amino-8-(4,4-difluoropiperidin-1-yl)quinoline-3-carboxylate (1.70 g, 4.90 mmol, yield: 79.0%) as a yellow solid. LCMS (ESI): $[M+H]^+$ = 336.1; $^1H$ NMR (400 MHz, CHLOROFORM-d) δ ppm 9.16 (d, *J* = 2.0 Hz, 1H), 8.53 (d, *J* = 2.0 Hz, 1H), 6.76 - 6.63 (m, 2H), 4.46 (q, *J* = 7.2 Hz, 2H), 4.01 (br s, 2H), 3.49 (br t, *J* = 5.6 Hz, 4H), 2.39 - 2.26 (m, 4H), 1.48 - 1.43 (m, 3H)

Step 7: Ethyl 6-amino-8-(4,4-difluoropiperidin-1-yl)quinoline-3-carboxylate (1.70 g, 5.07 mmol) was dissolved in THF (34.0 mL), then diisopropylethylamine (6.55 g, 50.6 mmol) and 4-iodo-2-(6-azaspiro[2.5]octan-6-yl)benzoyl chloride (2.86 g, 7.60 mmol) were successively added, and stirred at 25°C for 16 h. Water (100 mL) was added into the reaction solution, extracted with ethyl acetate (200 mL×2), the combined organic layer was dried, filtered, and concentrated. The residue was purified by flash column chromatography (silica gel, 0-17% gradient of THF/petroleum ether) to give ethyl 6-(2-{6-azaspiro[2.5]octan-6-yl}-4-iodobenzoylamino)-8-(4,4-difluoropiperidin-1-yl) quinoline-3-carboxylate (3.50 g, 4.46 mmol, yield: 88.0%) as a yellow solid. LCMS (ESI): $[M+H]^+$ = 675.1; $^1H$ NMR (400 MHz, CHLOROFORM-d) δ ppm 12.93 (s, 1H), 9.34 (d, *J* = 2.0 Hz, 1H), 8.79 (d, *J* = 2.0 Hz, 1H), 8.21 (d, *J* = 1.6 Hz, 1H), 8.03 (d, *J* = 8.8 Hz, 1H), 7.74 - 7.68 (m, 2H), 7.57 (d, *J* = 1.6 Hz, 1H), 4.49 (q, *J* = 7.2 Hz, 2H), 3.64 - 3.54 (m, 4H), 3.14 (br t, *J* = 5.2 Hz, 4H), 2.42 - 2.29 (m, 4H), 1.87 - 1.60 (m, 4H), 1.48 (t, *J* = 6.8 Hz, 3H), 0.49 (s, 4H)

Step 8: Ethyl 6-(2-{6-azaspiro[2.5]octan-6-yl}-4-iodobenzoylamino)-8-(4,4-difluoropiperidin-1-yl) quinoline-3-carboxylate (600 mg, 0.89 mmol) and 2-hydroxyethanesulfonamide (144 mg, 1.16 mmol) were successively added in N,N-dimethylformamide (6.00 mL), then potassium phosphate (566 mg, 2.67 mmol), (1R,2R)-N1,N2-dimethylcyclohexane-1,2-diamine (127 mg, 0.89 mmol), and cuprous iodide (169 mg, 0.89 mmol) were added successively, and stirred at 100°C for 4 h under nitrogen atmosphere. Water (50.0 mL + 0.50 mL liquor ammonia) was added into the reaction solution, and extracted withethyl acetate (100 mL × 2). The combined organic layer was dried , filtered, and concentrated.

1/3 of the residue was purified by preparative HPLC (C18, 32%-72% gradient of water (liquor ammonia + ammonium bicarbonate)/acetonitrile) to give ethyl 6-(2-{6-azaspiro[2.5]octan-6-yl}-4-(2-hydroxyethanesulfonamido)benzoylamino)-8-( 4,4-difluoropiperidin-1-yl)quinoline-3-carboxylate (24.19 mg, 0.04 mmol, yield: 6.00%) as a white solid. LCMS (ESI): $[M+H]^+$ = 672.3; $^1H$ NMR (400 MHz, DMSO-$d_6$) δ ppm 12.01 (s, 1H), 10.62 - 9.65 (m, 1H), 9.16 (d, *J* = 2.4 Hz, 1H), 8.83 (d, *J* = 2.0 Hz, 1H), 8.34 (d, *J* = 1.6 Hz, 1H), 7.88 (d, *J* = 8.8 Hz, 1H), 7.53 (d, *J* = 1.6 Hz, 1H), 7.20 (d, *J* = 1.6 Hz, 1H), 7.07 (dd, *J* = 2.0, 8.4 Hz, 1H), 5.38 - 4.72 (m, 1H), 4.42 (q, *J* = 7.2 Hz, 2H), 3.78 (t, *J* = 6.4 Hz, 2H), 3.62-3.48 (m, 4H), 3.35 (t, *J* = 6.4 Hz, 2H), 3.01 (br t, *J* = 4.4 Hz, 4H), 2.32 - 2.15 (m, 4H), 1.65-1.50 (m, 4H), 1.40 (t, *J* = 7.2 Hz, 3H), 0.36 (s, 4H)

**Example 87: 6-(2-{6-azaspiro[2.5]octan-6-yl}-4-(2-hydroxyethanesulfonamido)benzoylamino)-8-(4,4-difluoropiperidin-1-yl)quinoline-3-carboxylic acid**

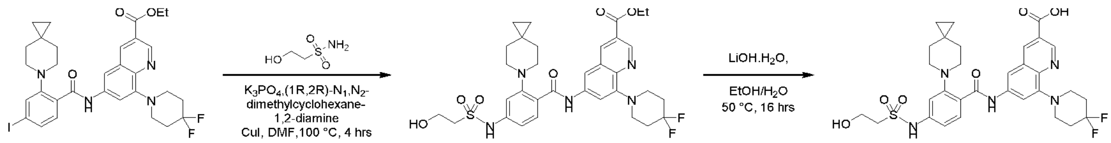


Step 1: Ethyl 6-(2-{6-azaspiro[2.5]octan-6-yl}-4-iodobenzoylamino)-8-(4,4-difluoropiperidin-1-yl) quinoline-3-carboxylate (600 mg, 0.89 mmol) and 2-hydroxyethanesulfonamide (144 mg, 1.16 mmol) were successively dissolved in *N,N*-dimethylformamide (6.00 mL), then potassium phosphate (566 mg, 2.67 mmol), (1*R*,2*R*)-*N*1,*N*2-dimethylcyclohexane-1,2-diamine (127 mg, 0.89 mmol), and cuprous iodide (169 mg, 0.89 mmol) were successively added, and stirred at 100°C for 4 h under nitrogen atmosphere. Water (50.0 mL + 0.50 mL of liquor ammonia) was added into the reaction solution, extracted withethyl acetate (100 mL × 2), the combined organic layer was dried, filtered, and concentrated. 2/3 of the residue was purified by flash column chromatography (silica gel, 0-17% gradient of THF/petroleum ether) to give ethyl 6-(2-{6-azaspiro[2.5]octan-6-yl}-4-(2-hydroxyethanesulfonamido)benzoylamino)-8-( 4,4-difluoropiperidin-1-yl)quinoline-3-carboxylate (270 mg, 0.39 mmol, yield: 66.0%) as a yellow solid. LCMS (ESI): $[M+H]^+$ = 672.1

Step 2: Ethyl 6-(2-{6-azaspiro[2.5]octan-6-yl}-4-(2-hydroxyethanesulfonamido)benzoylamino)-8-( 4,4-difluoropiperidin-1-yl)quinoline-3-carboxylate (220 mg, 0.33 mmol) was dissolved in ethanol (2.00 mL), and lithium hydroxide monohydrate (41.2 mg, 0.98 mmol) was added in pure water (2.00 mL), and the reaction system was stirred at 50°C for 16 h. Water (10.0 mL) was added into the reaction solution, the pH was adjusted to 4 with diluted hydrochloric acid, and extracted withethyl acetate (50.0 mL × 2). The combined organic layer was dried , filtered, and concentrated. The residue was purified by preparative HPLC (C18, 24%-64% gradient of water (formic acid)/acetonitrile) to give 6-(2-{6-azaspiro[2.5]octan-6-yl}-4-(2-hydroxyethanesulfonamido)benzoylamino)-8-( 4,4-difluoropiperidin-1-yl)quinoline-3-carboxylic acid (172 mg, 0.27 mmol, yield: 82.0%) as a yellow solid. LCMS (ESI): $[M+H]^+$ = 644.3; $^1H$ NMR (400 MHz, DMSO-$d_6$) δ ppm 14.08 - 12.87 (m, 1H), 12.01 (s, 1H), 10.14 (br s, 1H), 9.14 (d, *J* = 2.0 Hz, 1H), 8.77 (d, *J* = 2.0 Hz, 1H), 8.30 (s, 1H), 7.88 (d, *J* = 8.4 Hz, 1H), 7.51 (s, 1H), 7.19 (d, *J* = 1.6 Hz, 1H), 7.07 (dd, *J* = 1.6, 8.4 Hz, 1H), 4.97 (br s, 1H), 3.77 (br t, *J* = 6.4 Hz, 2H), 3.59-3.49 (m, 4H), 3.40-3.35 (m, 2H), 3.00 (br d, *J* = 4.4 Hz, 4H), 2.32 - 2.15 (m, 4H), 1.68 - 1.50 (m, 4H), 0.35 (s, 4H)

**Example 88: 6-(2-{6-azaspiro[2.5]octan-6-yl}-4-(2-hydroxyethanesulfonamido)benzoylamino)-8-(4,4-difluoropiperidin-1-yl)quinoline-3-carboxamide**

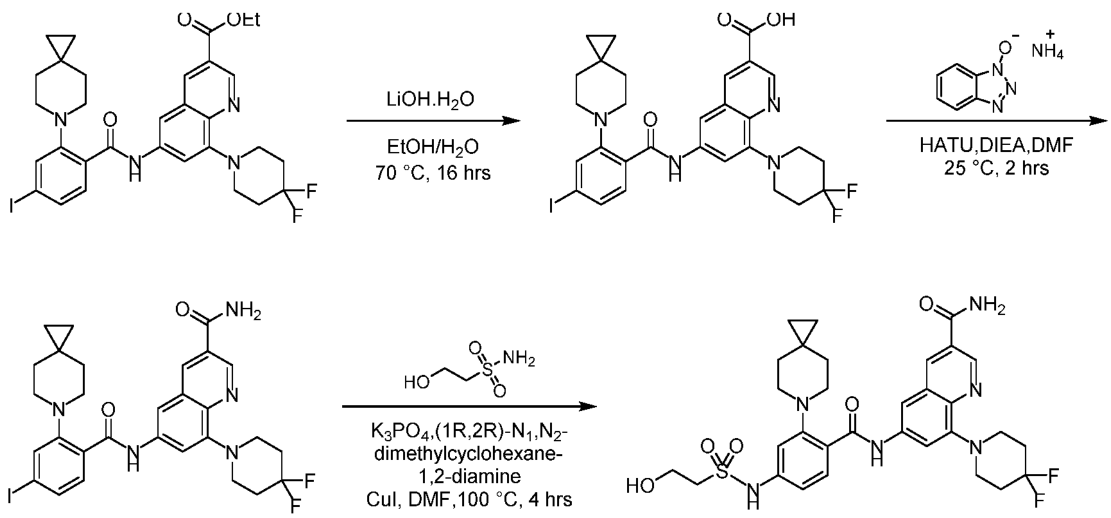


Step 1: Ethyl 6-(2-{6-azaspiro[2.5]octan-6-yl}-4-iodobenzoylamino)-8-(4,4-difluoropiperidin-1-yl) quinoline-3-carboxylate (220 mg, 0.33 mmol) was added in ethanol (2.00 mL), and lithium hydroxide monohydrate (37.3 mg, 0.89 mmol) was added in pure water (2.00 mL), and stirred at 70°C for 16 h. Water (5.00 mL) was added into the reaction solution, the pH was adjusted to 4 with diluted hydrochloric acid (1 M), and extracted with ethyl acetate (20.0 mL × 2). The combined organic layer was dried, filtered, and concentrated. The residue was purified by flash column chromatography (silica gel, 0-20-100% gradient of THF/petroleum ether) to give 6-(2-{6-azaspiro[2.5]octan-6-yl}-4-iodobenzoylamino)-8-(4,4-difluoropiperidin-1-yl) quinoline-3-carboxylic acid (140 mg, 0.19 mmol, yield: 66.0%) as a yellow solid. LCMS (ESI): $[M+H]^+$ = 647.1

Step 2: 6-(2-{6-azaspiro[2.5]octan-6-yl}-4-iodobenzoylamino)-8-(4,4-difluoropiperidin-1-yl) quinoline-3-carboxylic acid (170 mg, 0.26 mmol) was dissolved in N,N-dimethylformamide (4.00 mL), then 1-hydroxy-1H-benzotriazole ammonium salt (240 mg, 1.58 mmol), N,N-diisopropylethylamine (271 mg, 2.10 mmol), and O-(7-azabenzotriazol-1-yl)-N,N,N,N-tetramethylurea hexafluorophosphonate (299 mg, 0.79 mmol) were successively added into the reaction system and stirred at 25°C for 2 h. Water (10.0 mL) was added into the reaction solution, extracted with ethyl acetate (50 mL × 2) Then the combined organic layer was dried , filtered, and concentrated. The residue was purified by flash column chromatography (silica gel, 0-43% gradient of THF/petroleum ether) to give 6-(2-{6-azaspiro[2.5]octan-6-yl}-4-iodobenzoylamino)-8-(4,4-difluoropiperidin-1-yl) quinoline-3-carboxamide (100 g, 0.16 mmol, yield: 44.0%) as a yellow oil. LCMS (ESI): $[M+H]^+$ = 646.1; $^1$H NMR (400 MHz, DMSO-$d_6$) δ ppm 11.70 (s, 2H), 9.14 (s, 1H), 8.72 (s, 1H), 8.33 - 8.16 (m, 2H), 7.68 (br s, 1H), 7.65 (s, 1H), 7.59 (br d, *J* = 8.4 Hz, 1H), 7.46 (s, 1H), 3.54 (br s, 4H), 3.05 (br s, 4H), 2.30 - 2.20 (m, 4H), 1.56 - 1.49 (m, 4H), 0.32 (s, 4H)

Step 3: 6-(2-{6-azaspiro[2.5]octan-6-yl}-4-iodobenzoylamino)-8-(4,4-difluoropiperidin-1-yl) quinoline-3-carboxamide (90.0 mg, 0.14 mmol) and 2-hydroxyethanesulfonamide (22.6 mg, 0.18 mmol) were successively dissolved in N,N-dimethylformamide (3.60 mL) and potassium phosphate (88.7 mg, 0.42 mmol), (1R,2R)-N1,N2-dimethylcyclohexane-1,2-diamine (20.0 mg, 0.14 mmol), and cuprous iodide (26.5 mg, 0.14 mmol) were successively added, and stirred at 100°C for 4 h under nitrogen atmosphere. Water (50.0 mL + 0.50 mL liquor ammonia) was added into the reaction solution, and extracted with ethyl acetate (100 mL × 2). The combined organic layer was dried , filtered, and concentrated. The residue was purified by preparative HPLC (C18, 14%-54% gradient of water (liquor ammonia + ammonium bicarbonate)/acetonitrile) to give 6-(2-{6-azaspiro[2.5]octan-6-yl}-4-(2-hydroxyethanesulfonamido)benzoylamino)-8-( 4,4-difluoropiperidin-1-yl)quinoline-3-carboxamide (33.18 mg, 0.05 mmol, yield: 37.0%) as a white solid. LCMS (ESI): $[M+H]^+$ = 643.3; $^1$H NMR (400 MHz, DMSO-$d_6$) δ ppm 12.51 (s, 1H), 9.12 (d, *J* = 2.0 Hz, 1H), 8.69 (d, *J* = 2.0 Hz, 1H), 8.33 - 8.18 (m, 2H), 7.82 (d, *J* = 8.4 Hz, 1H), 7.66 (s, 1H), 7.42 (s, 1H), 7.03 (d, *J* = 1.6 Hz, 1H), 6.89 (dd, *J* = 1.6, 8.4 Hz, 1H), 6.10 - 5.77 (m, 2H), 3.74 (t, *J* = 6.4 Hz, 2H), 3.55 (br s, 4H), 3.15 (t, *J* = 6.8 Hz, 2H), 3.00 (br t, *J* = 4.4 Hz, 4H), 2.31 - 2.18 (m, 4H), 1.68 - 1.58 (m, 4H), 0.38 (s, 4H)

**Example 89: N-[2-Amino-8-(4,4-difluoropiperidinyl)-3-methyl(6-quinolinyl)](2-(6-azaspiro[2.5] octan-6-yl)-4-{[(2-hydroxyethyl)sulfonyl]amino}phenyl)formamide**

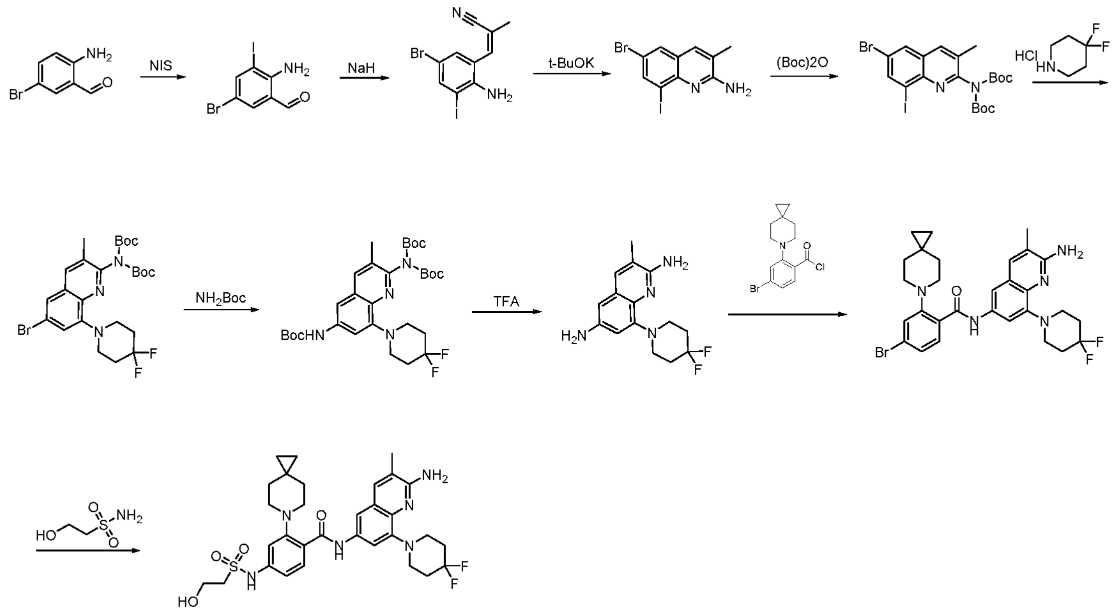


Step 1: Trifluoroacetic acid was added to the solution of 2-amino-5-bromobenzaldehyde (5.00 g, 24.97 mmol) and iodosuccinimide (6.72 g, 30.04 mmol) in acetonitrile (50 mL) at 25°C, and the resulting reaction mixture was stirred at 25°C for 2 h. The reaction mixture was diluted with ethyl acetate (100 mL) and washed with saturated saline (50 mL×3). The organic phase was concentrated, dried, filtered to give the residue, and purified by flash column chromatography (silica gel, 0-30% gradient of ethyl acetate/petroleum ether) to give 2-amino-5-bromo-3-iodobenzaldehyde (6.00 g, 18.46 mmol, yield: 73.6%) as a violet solid. LCMS (ESI): $[M+H]^+$ =325.9, $^1H$ NMR (400 MHz, DMSO) δ 9.70 (s, 1H), 8.03 (d, *J* = 2.3 Hz, 1H), 7.89 (d, *J* = 2.3 Hz, 1H), 7.15 (s, 2H).

Step 2: Sodium hydride (480 mg, 12.13 mmol, 60 % in mineral oil) was added to the solution of 2-(diethoxycarbonylphosphate)propionitrile (2.10 g, 11.00 mmol) in THF (40 mL) at 0°C. The resulting reaction mixture was stirred for 30 min at 25°C and then the solution of 2-amino-5-bromo-3-iodobenzaldehyde (2.50 g, 7.73 mmol) in THF (10 mL) was added. The reactionmixture was stirred at 25°C for 3 h. The reaction system was quenched with saturated ammonium chloride solution (100 mL) and extracted with ethyl acetate (200 mL×2). The combined organic layer was dried with anhydrous sodium sulfate, filtered, concentrated to give the crude, and purified by flash column chromatography (silica gel, 0-30% gradient of ethyl acetate/petroleum ether) to give (2E)-3-(2-amino-5-bromo-3-iodophenyl)-2-methylprop-2-enenitrile (2.90 g, 7.99 mmol, yield: 72.7%) as a yellow solid. LCMS (ESI): $[M+H]^+$ =363.0

Step 3: Potassium tert-butoxide (1.81 g, 15.98 mmol) was added to the solution of (2E)-3-(2-amino-5-bromo-3-iodophenyl)-2-methylprop-2-enenitrile (2.90 g, 7.99 mmol) in THF (50 mL) at 25°C, and the resulting reaction mixture was stirred for 16 h at 50°C. The reaction mixture was diluted with ethyl acetate (100 mL) and washed with saturated saline (50 mL×3). The combined organic layer was dried with anhydrous sodium sulfate, filtered, and concentrated to give the residue. The residue was purified by flash column chromatography (silica gel, 0-30% gradient of ethyl acetate/petroleum ether) to give 6-bromo-8-iodo-3-methyl-2-quinolinamine (1.80 g, 4.96 mmol, yield: 62.1%) as a pale brown solid. LCMS (ESI): $[M+H]^+$ =363.0

Step 4: tert-butyl carbamate (1.92 g, 8.82 mmol) was added to the solution of 6-bromo-8-iodo-3-methyl-2-quinolinamine (1.60 g, 4.41 mmol), 4-dimethylaminopyridine (110 mg, 0.88 mmol), and N,N-diisopropylethylamine (1.16 mL, 8.81 mmol) in dichloromethane (20 mL) at 25°C. The reaction mixture was stirred at 50°C for 3 h. The reaction mixture was concentrated to give the residue and purified by flash column chromatography (silica gel, 0-10% gradient of ethyl acetate/petroleum ether) to give tert-butyl(6-bromo-8-iodo-3-methylquinolin-2-yl)(tert-butoxycarbonyl)carbamate (2.00 g, 3.55 mmol, yield: 80.6%) as a pale yellow solid. LCMS (ESI): $[M+H]^+$ =565.1. $^1H$ NMR (400 MHz, DMSO) δ 8.45 (d, *J* = 2.1 Hz, 1H), 8.30 (t, *J* = 1.9 Hz, 2H), 2.35 (d, *J* = 1.0 Hz, 3H), 1.37 (s, 18H).

Step 5: 1,4-dioxane (10 mL) was added to the mixture of tert-butyl(6-bromo-8-iodo-3-methylquinolin-2-yl)(tert-butoxycarbonyl)carbamate (1.00 g, 1.77 mmol), 4,4-difluoropiperidine hydrochloride (0.31 g, 1.95 mmol), tris(dibenzylideneacetone) dipalladium (170 mg, 0.18 mmol), 2,2'-bis(diphenylphosphino)-1,1'-binaphthyl (210 mg, 0.36 mmol) and cesium carbonate (1.77 g, 5.33 mmol) at 25°C. Nitrogen was bubbled through the mixture for 5 minutes, and then the resulting reaction mixture was stirred at 100°C for 3 hours. The reaction mixture was filtered and washed with ethyl acetate (30 mL). The filtrate was washed with saturated sodium chloride solution (15 mL×3). The combined organic layer was dried with anhydrous sodium sulfate, filtered, and concentrated to give the residue. The residue was purified by flash column chromatography (silica gel, 0-20% gradient of ethyl acetate/petroleum ether) to give tert-butyl (6-bromo-8-(4,4-difluoropiperidin-1-yl)-3-methylquinolin-2-yl)(tert-butoxy)carbamat e (800 mg, 1.44 mmol, yield: 81.0%) as a pale yellow oil. LCMS (ESI): $[M+H]^+$ =558.3.

Step 6: 1,4-dioxane (10 mL) was added to the mixture of tert-butyl(6-bromo-8-(4,4-difluoropiperidin-1-yl)-3-methylquinolin-2-yl)(tert-butoxy) carbamate (1.50 g, 3.29 mmol), tert-butyl carbamate (580 mg, 4.93 mmol), tris(dibenzylideneacetone) dipalladium (310 mg, 0.33 mmol), 2,2'-bis(diphenylphosphino)-1,1'-binaphthyl (390 mg, 0.66 mmol), and cesium carbonate (3.28 g, 9.86 mmol) at 25°C, perform nitrogen bubbling for 5 min, and the resulting reaction mixture was stirredat 100°C for 3 h. Filter the reaction mixture and wash the cake with ethyl acetate (30 mL). Wash the filtrate with saturated sodium chloride solution (15 mL×3). The combined organic layer was dried with anhydrous sodium sulfate, filter, and concentrate to give the residue. The residue was purified by flash column chromatography (silica gel, 0-40% gradient ethyl acetate/petroleum ether) to give tert-butyl (tert-butoxycarbonyl)(6-(tert-butoxycarbonyl)amino)-8-(4,4-difluoropiperidin-1-yl)-3-methylquinolin-2-yl)carbamate (650 mg, 1.31 mmol, yield: 40.2%) as a pale yellow solid. LCMS (ESI): $[M+H]^+$ =593.5. $^1$H NMR (400 MHz, DMSO) δ 9.61 (s, 1H), 8.14 (s, 1H), 7.74 (d, *J* = 2.0 Hz, 1H), 7.31 (d, *J* = 2.1 Hz, 1H), 3.45 (t, *J* = 5.6 Hz, 4H), 2.32 (s, 3H), 2.28 - 2.15 (m, 4H), 1.57 (s, 9H), 1.44 (s, 18H).

Step 7: Trifluoroacetic acid (0.50 mL) was added to the solution of N-{8-(4,4-difluoropiperidinyl)-6-[(tert-butoxy) carbonylamino]-3-methyl(2-quinolinyl) }(tert-butoxy)formamide (50.00 mg, 0.08 mmol) in dichloromethane (1 mL) at 25°C, and The reaction mixture was stirred for 3 h at 25°C. The reaction solution was concentrated to give the residue, then the residue was diluted with ethyl acetate (10 mL), and washed with saturated sodium bicarbonate solution (10 mL × 2). The combined organic layer was dried with anhydrous sodium sulfate, filtered, and concentrated to give 8-(4,4-difluoropiperidyl)-3-methylquinoline-2,6-diamine (44 mg, crude) as a brown solid for the next step without purification. LCMS (ESI): $[M+H]^+$ =293.2

Step 8: N,N-diisopropylethylamine (300 μL, 1.64 mmol) was added to the solution of 2-(6-azaspiro[2.5]octan-6-yl)-4-bromobenzoyl chloride (580 mg, 1.70 mmol) and 8-(4,4-difluoropiperidinyl)-3-methylquinoline-2,6-amine (160 mg, 0.55 mmol) in THF (5 mL) at 20°C, and the resulting reaction mixture was stirred at 20°C for 1 h. The reaction solution was diluted with ethyl acetate (10 mL) and diluted with saturated sodium chloride solution (10 mL×3). The combined organic layer was dried over anhydrous sodium sulfate, filtered, and concentrated to give N-[2-amino-8-(4,4-difluoropiperidinyl)-3-methyl(6-quinolinyl)] [2-(6-azaspiro[2.5]octan-6-yl)-4-bromophenyl]formamide (70 mg, 0.12 mmol, yield: 21.7%) as a pale brown solid crude. LCMS (ESI): $[M+H]^+$=586.3

Step 9: 1,4-dioxane (2 mL) was added to the mixture of N-[2-amino-8-(4,4-difluoropiperidinyl)-3-methyl(6-quinolinyl)] [2-(6-azaspiro[2.5]octan-6-yl)-4-bromophenyl]formamide (60 mg, 0.10 mmol), 2-hydroxyethylsulfonamide (26 mg, 0.21 mmol), tris(dibenzylideneacetone) dipalladium (9 mg, 0.01 mmol), 2-di-tert-butylphosphine-2',4',6'-triisopropylbiphenyl (9 mg, 0.02 mmol), and potassium phosphate (67 mg, 0.15 mmol) at 25°C. Perform nitrogen bubbling for 1 min, and the resulting reaction mixture was stirred at 100°C for 6 h. The reaction mixture was purified by column chromatography (silica gel, 50% gradient of ethyl acetate/petroleum ether, 5-15% gradient of methanol/dichloromethane containing 3% liquor ammonia) to give the crude. Diluted with ethyl acetate (20 mL), washed with water (20 mL × 2), dried, filtered, and concentrated to give N-[2-amino-8-(4,4-difluoropiperidinyl)-3-methyl(6-quinolinyl)](2-(6-azaspiro[2.5]octan-6-yl)-4-{[(2-hydroxyethyl)sulfonyl]amino}phenyl)formamide (34 mg, 0.054 mmol, yield: 54.0%) as a pale yellow solid. LCMS (ESI): $[M+H]^+$ =629.4, $^1$H NMR (400 MHz, DMSO) δ 11.83 (s, 1H), 10.05 (s, 1H), 7.91 - 7.84 (m, 2H), 7.65 (s, 1H), 7.18 (d, *J* = 2.1 Hz, 1H), 7.14 (d, *J* = 2.2 Hz, 1H), 7.05 (dd, *J* = 8.5, 2.0 Hz, 1H), 6.05 (s, 2H), 4.95 (s, 1H), 3.76 (t, *J* = 6.5 Hz, 2H), 3.43 (t, *J* = 5.4 Hz, 4H), 3.34 (d, *J* = 6.8 Hz, 2H), 2.99 (t, *J* = 5.3 Hz, 4H), 2.21 (s, 7H), 1.60 (s, 4H), 0.36 (s, 4H).

**Example 90: 2-{6-azaspiro[2.5]octan-6-yl}-N-[8-(4,4-difluoropiperidin-1-yl)-7-fluoro-3-methyl quinolin-6-yl]-4-(2-hydroxyethanesulfonamido)benzamide**

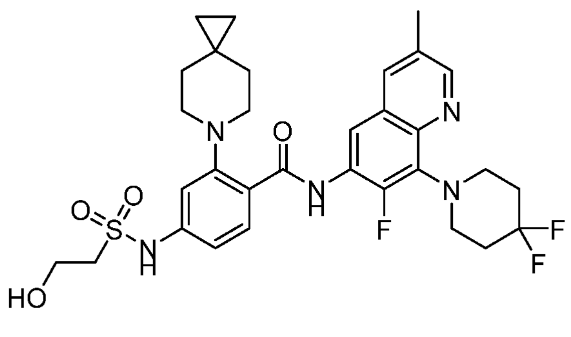

The preparations in **Example 90** follow the synthetic route of **Example 3:** LCMS (ESI): $[M+H]^+$ = 632.3; $^1$H NMR (400 MHz, DMSO-$d_6$) δ ppm 10.06 (br s, 1H), 8.91 (d, *J* = 3.6 Hz, 1H), 7.52 (d, *J* = 8.4 Hz, 1H), 7.48 (d, *J* = 3.6 Hz, 1H), 7.14

(d, $J$ = 2.0 Hz, 1H), 7.03 (dd, $J$ = 1.6, 8.4 Hz, 1H), 5.40 - 4.58 (m, 1H), 3.78 (t, $J$ = 6.4 Hz, 2H), 3.65 - 3.50 (m, 4H), 3.42 - 3.33 (m, 2H), 2.66 (br d, $J$ = 4.8 Hz, 4H), 2.42 (s, 3H), 2.20 - 2.04 (m, 4H), 1.35 - 1.15 (m, 4H), 0.24 (s, 4H)

**Example 91: N-[3-amino-8-(4,4-difluoropiperidin-1-yl)-7-fluoroquinolin-6-yl]-2-{6-azaspiro[2. 5]octan-6-yl}-4-(2-hydroxyethanesulfonamido)benzamid**e

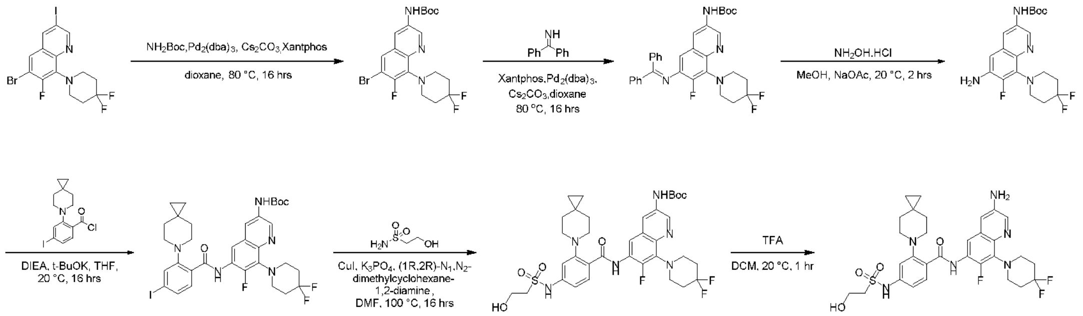


Step 1: 6-bromo-8-(4,4-difluoropiperidin-1-yl)-7-fluoro-3-iodoquinoline (1.50 g, 3.18 mmol) and tert-butyl carbamate (0.17 g, 1.45 mmol) were dissolved successively in dioxane (30.0 mL), and cesium carbonate (1.59 g, 4.87 mmol), 4,5-bis(diphenylphosphino)-9,9-dimethylxanthene (0.19 g, 0.32 mmol), and tris(dibenzylideneacetone) dipalladium (0.15 g, 0.16 mmol) were added successively, and stirred for 16 h at 80°C under nitrogen atmosphere. The reaction solution was diluted with water (100 mL), extracted with ethyl acetate (400 mL, 200 mL × 2), the combined organic layer was dried , filtered, and concentrated. The residue was purified by flash column chromatography (silica gel, 0-6% gradient of THF/petroleum ether) to give tert-butyl N-[6-bromo-8-(4,4-difluoropiperidin-1-yl)-7-fluoroquinolin-3-yl]carbamate (0.60 g, 1.23 mmol, yield: 76.0%) as a yellow solid. LCMS (ESI): $[M+H]^+$ = 462.0; $^1H$ NMR (400 MHz, CHLOROFORM-d) δ ppm 8.54 (br s, 1H), 8.28 (br s, 1H), 7.58 (br d, $J$ = 6.4 Hz, 1H), 6.64 (br s, 1H), 3.51 (br s, 4H), 2.20 - 2.08 (m, 4H), 1.49 (s, 9H)

Step 2: tert-butyl N-[6-bromo-8-(4,4-difluoropiperidin-1-yl)-7-fluoroquinolin-3-yl]carbamate (580 mg, 1.26 mmol) and diphenylmethanimine (274 mg, 1.51 mmol) were successively dissolved in dioxane (15.0 mL), then cesium carbonate (1.23 g, 3.78 mmol), 4,5-bis(diphenylphosphino)-9,9-dimethylxanthene (145 mg, 0.25 mmol), and tris(dibenzylideneacetone) dipalladium (0.15 g, 0.16 mmol) were successively added, and stirred for 16 h at 80°C under nitrogen atmosphere. The reaction solution was diluted with water (50.0 mL), extracted with ethyl acetate (200 mL, 100 mL × 2), the combined organic layer was dried , filtered, and concentrated. The residue was purified by flash column chromatography (silica gel, 0-8% gradient of THF/petroleum ether) to give tert-butyl N-[8-(4,4-difluoropiperidin-1-yl)-6-[(diphenylmethyl anion)amino]-7-fluoroquinolin-3-yl]carbamate (800 mg, 1.20 mmol, yield: 95.0%) as a yellow solid. LCMS (ESI): $[M+H]^+$ = 562.0; $^1H$ NMR (400 MHz, CHLOROFORM-d) δ ppm 8.43 (d, $J$ = 2.4 Hz, 1H), 8.25 (br s, 1H), 7.85 - 7.77 (m, 2H), 7.60 (br d, $J$ = 6.8 Hz, 1H), 7.53 - 7.50 (m, 1H), 7.47 - 7.44 (m, 2H), 7.23 (br d, $J$ = 6.4 Hz, 2H), 7.20 - 7.15 (m, 2H), 6.77 (d, $J$ = 8.0 Hz, 1H), 6.71 (s, 1H), 3.40 (br d, $J$ = 4.4 Hz, 4H), 2.21 - 2.09 (m, 4H), 1.62 - 1.50 (m, 9H)

Step 3: tert-butyl N-[8-(4,4-difluoropiperidin-1-yl)-6-[(diphenylmethyl anion)amino]-7-fluoroquinolin-3-yl]carbamate (780 mg, 1.39 mmol) was dissolved in methanol (16.0 mL), then sodium acetate (342 mg, 4.17 mmol) and hydroxylamine hydrochloride (193 mg, 2.78 mmol) were successively added, and stirred at 20°C for 2 h. Water (50.0 mL) was added into the reaction solution, extracted with ethyl acetate (100 mL × 2), the combined organic layer was dried , filtered, and concentrated. The residue was purified by flash column chromatography (silica gel, 0-18% gradient of THF/petroleum ether) to give tert-butyl N-[6-amino-8-(4,4-difluoropiperidin-1-yl)-7-fluoroquinolin-3-yl]carbamate (0.78 g, 0.92 mmol, yield: 66.0%) as a yellow solid. LCMS (ESI): $[M+H]^+$ = 397.1; $^1H$ NMR (400 MHz, CHLOROFORM-d) δ ppm 8.36 (d, $J$ = 2.4 Hz, 1H), 8.22 (br s, 1H), 6.66 (br d, $J$ = 8.8 Hz, 2H), 4.04 (s, 2H), 3.58 (br t, $J$ = 4.4 Hz, 4H), 2.29 - 2.16 (m, 4H), 1.57 (s, 9H)

Step 4: Dissolve tert-butyl N-[6-amino-8-(4,4-difluoropiperidin-1-yl)-7-fluoroquinolin-3-yl]carbamate (340 mg, 0.86 mmol) in THF (15.0 mL), add diisopropylethylamine (332 mg, 2.57 mmol) and 4-iodo-2-(6-azaspiro[2.5]octan-6-yl) benzoyl chloride (690 mg, 1.29 mmol), and stir at 20°C for 16 h. Add water (50.0 mL) into the reaction solution, extracted withethyl acetate (100 mL × 2), the combined organic layer was dried , filter, and concentrate. The residue was purified by flash column chromatography (silica gel, 0-12% gradient of THF/petroleum ether) to give tert-butyl N-[6-(2-{6-azaspiro [2.5]octan-6-yl}-4-iodobenzoylamino)-8-(4,4-difluoropiperidin-1-yl)-7-fluoroquinolin-3-yl]carbamate (340 mg, 0.41 mmol, yield: 48.0%) as a yellow solid. LCMS (ESI): $[M+H]^+$ = 736.1; $^1H$ NMR (400 MHz, CHLOROFORM-d) δ ppm 12.40 (br s, 1H), 8.74 (d, $J$ = 8.0 Hz, 1H), 8.65 (br d, $J$ = 2.4 Hz, 1H), 8.31 (br s, 1H), 7.97 (d, $J$ = 8.4 Hz, 1H), 7.69 (s, 1H), 7.66 (d, $J$ = 8.4 Hz, 1H), 6.65 (br s, 1H), 3.63 (br s, 4H), 3.08 (br t, $J$ = 4.8 Hz, 4H), 2.30 - 2.19 (m, 4H), 1.70 - 1.60 (m, 4H), 1.57 (s, 9H), 0.41 (s, 4H)

Step 5: tert-butyl N-[6-(2-{6-azaspiro[2.5]octan-6-yl}-4-iodobenzoylamino)-8-(4,4-difluoropiperidin-1-yl)-7-fluoroquinolin-3-yl]carbamate (300 mg, 0.41 mmol) and 2-hydroxyethanesulfonamide (102 mg, 0.82 mmol) were dissolved in N,N-dimethylformamide (15.0 mL), then potassium phosphate (259 mg, 1.22 mmol), (1R,2R)-N1,N2-dimethylcyclohexane-1,2-diamine (58.6 mg, 0.41 mmol) and cuprous iodide (77.6 mg, 0.41 mmol) were successively added, and stirred at 100°C for 16 h. 50 mL of water (0.5 mL of liquor ammonia) was added into the reaction solution, extracted with ethyl acetate (200 mL × 2), the combined organic layer was dried , filtered, and concentrated. The residue was purified by flash column chromatography (silica gel, 0-37% gradient of THF/petroleum ether) to give tert-butyl N-[6-(2-{6-azaspiro[2.5]octan-6-yl}-4-(2-hydroxyethanesulfonamido)benzoylamino)-8-(4,4-difluoropiperidin-1-yl)-7-fluoroquinolin-3-yl]carbamate (300 mg, 0.36 mmol, yield: 89.0%) as a yellow solid. LCMS (ESI): $[M+H]^+$ = 733.2; $^1H$ NMR (400 MHz, CHLOROFORM-d) δ ppm 12.44 (br d, *J = 2.0* Hz, 1H), 8.71 (d, *J* = 8.0 Hz, 1H), 8.67 (d, *J* = 2.4 Hz, 1H), 8.28 (br s, 1H), 8.19 (d, *J* = 8.4 Hz, 1H), 8.03 (s, 1H), 7.62 (br s, 1H), 7.35 (d, *J* = 1.6 Hz, 1H), 7.07 (dd, *J* = 2.0, 8.4 Hz, 1H), 6.82 (s, 1H), 4.14 (br t, *J* = 4.8 Hz, 2H), 3.66 - 3.58 (m, 4H), 3.36 (t, *J* = 5.6 Hz, 2H), 3.06 (br t, *J* = 4.8 Hz, 4H), 2.28 - 2.16 (m, 4H), 1.72 - 1.59 (m, 4H), 1.57 (s, 9H), 0.39 (s, 4H)

Step 6: Tert-butyl N-[6-(2-{6-azaspiro[2.5]octan-6-yl}-4-(2-hydroxyethanesulfonamido)benzoylamino)-8-(4,4-difluoropiperidin-1-yl)-7-fluoroquinolin-3-yl]carbamate (260 mg, 0.35 mmol) was dissolved in dichloromethane (2.40 mL) and trifluoroacetic acid (0.60 mL), and stirred for 1 h at 20°C. The reaction solution was quenched with 50 mL of saturated sodium bicarbonate solution, extracted withethyl acetate (100 mL × 2), the combined organic layer was dried , filtered, and concentrated. The residue was purified by preparative HPLC (C18, 12%-52% gradient of water (formic acid)/acetonitrile) to give N-[3-amino-8-(4,4-difluoropiperidin-1-yl)-7-fluoroquinolin-6-yl]-2-{6-azaspiro[2.5]o ctan-6-yl}-4-(2-hydroxyethanesulfonamido)benzamide (132 mg, 0.21 mmol, yield: 59.0%) as a yellow solid. LCMS (ESI): $[M+H]^+$ = 633.3; $^1H$ NMR (400 MHz, DMSO-$d_6$) δ ppm 12.29 (br s, 1H), 11.13 - 9.36 (m, 1H), 8.48 - 8.28 (m, 2H), 8.15 (s, 1H), 8.03 (d, *J* = 8.8 Hz, 1H), 7.28 (s, 1H), 7.13 (br d, *J* = 8.4 Hz, 1H), 7.08 (br d, *J* = 2.0 Hz, 1H), 5.60 (br s, 2H), 5.27 - 4.55 (m, 1H), 3.77 (br t, *J* = 6.4 Hz, 2H), 3.56 - 3.48 (m, 4H), 3.37 (br t, *J* = 6.4 Hz, 2H), 2.98 (br s, 4H), 2.25 - 2.07 (m, 4H), 1.65 - 1.50 (m, 4H), 0.37 (s, 4H)

**Example 92: N-(4-(4,4-difluoropiperidin-1-yl)-1,5-naphthyridin-2-yl)-4-(2-hydroxyethylsulfon amido)-2-(6-azaspiro[2.5]octan-6-yl)benzamide**

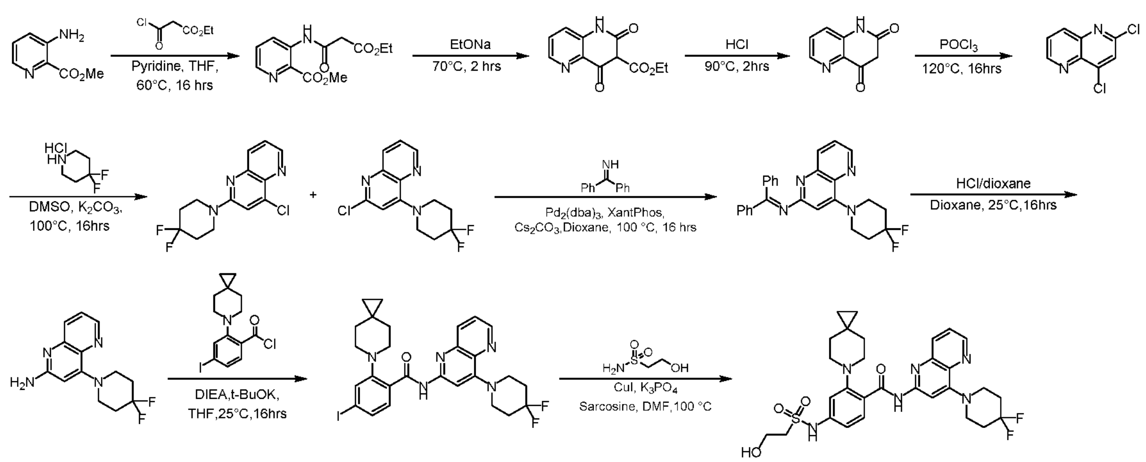


Step 1: Ethyl 3-chloro-3-oxopropanoate (3.56 g, 23.6 mmol) was added to the solution of methyl 3-aminomethylpyridine ester (3.00 g, 53.7 mmol) and pyridine (4.92 g, 59.1 mmol) in THF (30 mL), and the mixture was stirred at 60°C for 16 h. Water (60 mL) was added to the reaction mixture and extracted with ethyl acetate (60 mL × 2). The combined organic layer was dried over anhydrous sodium sulfate, filtered and concentrated under reduced pressure to give the residue. The residue was purified by flash column chromatography (silica gel, 0-30% gradient of ethyl acetate/petroleum ether) to give methyl 3-(3-ethoxy-3-oxopropionylamino)methylpyridinyl ester (1.50 g, 5.63 mmol, yield: 28.5%) as a yellow solid. LCMS (ESI): $[M+H]^+$ =266.9.

Step 2: Heat the solution of methyl 3-(3-ethoxy-3-oxopropionylamino)methylpyridinyl ester (1.00 g, 3.75 mmol) in sodium ethylate (1.91 g, 5.63 mmol) to 70°C and stir for 2 h. Concentrate and spin dry the mixture to give ethyl 2,4-dioxylidene-1,2,3,4-tetrahydro-1,5-naphthyridine-3-carboxylate (1.40 g, crude) as a yellow solid. LCMS (ESI): $[M+H]^+$ = 234.9.

Step 3 : The solution of ethyl 2,4-dioxylidene-1,2,3,4-tetrahydro-1,5-naphthyridine-3-carboxylate (1.00 g, 4.85

mmol) in hydrochloric acid (20 mL, 30%) was heated to 90°C and stir for 2 h. The reaction solution was cooled to ambient temperature, filtered and the cake was collected and dried under reduced pressure to give 1,5-naphthyridine-2,4(1H,3H)-dione (0.50 g, 3.08 mmol, yield: 63%) as a yellow solid. LCMS (ESI): $[M+H]^+$ = 162.8.

Step 4: Heat the solution of 1,5-naphthyridine-2,4(1H,3H)-dione (7.00 g, 5.76 mmol) in phosphorus oxychloride (80 mL) to 120°C and stir for 16 h. Concentrate the reaction solution and spin dry to give the residue. Pour the residue into water (100 mL) and extracted withethyl acetate (100 mL × 3). The combined organic layer was dried over anhydrous magnesium sulfate, filtered and concentrated under reduced pressure to give the residue 2,4-dichloro-1,5-naphthyridine (2.60 g, 12.9 mmol, yield: 30%). LCMS (ESI): $[M+H]^+$ = 198.9.

Step 5: 4,4-difluoropiperidine hydrochloride (2.18 g, 13.8 mmol) was added to the solution of 2,4-dichloro-1,5-naphthyridine (2.50 g, 12.5 mmol) and N,N-diisopropylethylamine (4.96 g, 37.7 mmol) in DMSO (50 mL). The mixture was heated to 100°C with stirring for 16 h. Water (100 mL) was added to the reaction solution and extracted with ethyl acetate (100 mL × 2). The combined organic layer was dried over anhydrous sodium sulfate, filtered and concentrated under reduced pressure to give the residue. The residue was purified by flash column chromatography (silica gel, 0-20% gradient of ethyl acetate/petroleum ether) to give the mixture of yellow solids 2-chloro-4-(4,4-difluoropiperidin-1-yl)-1,5-naphthyridine and 4-chloro-2-(4,4-difluoropiperidin-1-yl)-1,5-naphthyridine (2.80 g, 9.86 mmol, yield: 78%). LCMS (ESI): $[M+H]^+$ = 283.9.

Step 6: 4,5-bis(diphenylphosphino)-9,9-dimethylxanthene (1.30 g, 2.25 mmol) and tris(dibenzylideneacetone) dipalladium (1.03 g, 1.12 mmol) were added successively to the mixture of 2-chloro-4-(4,4-difluoropiperidin-1-yl)-1,5-naphthyridine and 4-chloro-2-(4,4-difluoropiperidin-1-yl)-1,5-naphthyridine (3.20 g, 11.3 mmol) and the solution of diphenylmethanimine (2.50 g, 13.5 mmol) and cesium carbonate (10.3 g, 31.7 mmol) in dioxane (60 mL). The mixture was heated to 100°C with stirring for 16 h under nitrogen atmosphere Add water (100 mL) to the reaction solution and extracted withethyl acetate (100 mL × 2). The combined organic layer was dried over anhydrous sodium sulfate, filtered and concentrated under reduced pressure to give the residue. The residue was purified by flash column chromatography (silica gel, 0-15% gradient of ethyl acetate/petroleum ether) to give 4-(4,4-difluoropiperidin-1-yl)-N-(diphenylmethylene)-1,5-naphthyridin-2-amine (3.40 g, 7.93 mmol, yield: 70%) as a yellow solid. LCMS (ESI): $[M+H]^+$ = 429.3.

Step 7: HCl in dioxane (34 mL, 4M) was added to the solution of 4-(4,4-difluoropiperidin-1-yl)-N-(diphenylmethylene)-1,5-naphthyridin-2-amine (3.40 g, 7.93 mmol) in dioxane (34 mL). The mixture was stirred at 25°C for 16 h. Then, filter the reaction solution, and concentrate and spin dry the solids to give 4-(4,4-difluoropiperidin-1-yl)-1,5-naphthyridin-2-amine (2.20 g, 7.48 mmol, yield: 94.4%). LCMS (ESI): $[M+H]^+$ = 265.1.

Step 8: The solution of potassium tert-butoxide in THF (5.67 mL, 5.67 mmol, 1 mol/L) and N,N-diisopropylethylamine (748 mg, 5.67 mmol) were successively added to the solution of 4-(4,4-difluoropiperidin-1-yl)-1,5-naphthyridin-2-amine (0.50 g, 1.89 mmol) and 4-iodo-2-(6-azaspiro[2.5]octan-6-yl)benzoyl chloride (852 mg, 2.27 mmol) in THF (10 mL). The reactionmixture was stirred at 25°C for 16 h. Water (50 mL) was added to the reaction mixture and extracted withethyl acetate (50 mL×2). The combined organic layer was dried over anhydrous sodium sulfate, filtered and concentrated under reduced pressure to give the residue. The residue was purified by flash column chromatography (silica gel, 0-5% gradient of ethyl acetate/petroleum ether) to give N-(4-(4,4-difluoropiperidin-1-yl)-1,5-naphthyridin-2-yl)-4-iodo-2-(6-azaspiro[2.5]oct an-6-yl)benzamide (519 mg, 0.87 mmol, yield: 46.1%) as a yellow solid. LCMS (ESI):

[M+H]+ = 604.2

Step 9: Cuprous iodide (80.5 mg, 0.41 mmol) was added to a solution of 2-hydroxyethanesulfonamide (207 mg, 1.65 mmol), 2-(methylamino)acetic acid (73.8 mg, 0.82 mmol), and potassium phosphate (717 mg, 3.31 mmol) in N,N-dimethylformamide (10 mL). The mixture was stirred at 50°C for 5 min. Then N-(4-(4,4-difluoropiperidin-1-yl)-1,5-naphthyridin-2-yl)-4-iodo-2-(6-azaspiro[2.5]oct an-6-yl)benzamide (500 mg, 0.83 mmol) was added to the reaction mixture at 50°C, and the mixture was heated to 100°C with stirring for 16 h under nitrogen atmosphere. And water (10 mL) was added to the reaction solution and extracted withethyl acetate (10 mL × 2). The combined organic layer was dried over anhydrous sodium sulfate, filtered and concentrated under reduced pressure to give the residue. The residue was purified by preparative HPLC (C18, 36-76% gradient of water (formic acid)/acetonitrile) to give N-(4-(4,4-difluoropiperidin-1-yl)-1,5-naphthyridin-2-yl)-4-(2-hydroxyethylsulfonami do)-2-(6-azaspiro[2.5]octan-6-yl)benzamide (188 mg, 0.31 mmol, yield: 37.7%) as a yellow solid compound. LCMS (ESI): $[M+H]^+$ = 601.3. $^1H$ NMR (400 MHz, DMSO-$d_6$) δ 13.16 (s, 1H), 10.25 (s, 1H), 8.76 (dd, *J* = 1.6, 4.0 Hz, 1H), 8.21 (s, 1H), 8.09 (d, *J* = 8.8 Hz, 1H), 8.03 (dd, *J* = 1.2, 8.4 Hz, 1H), 7.69 (dd, *J* = 4.0, 8.4 Hz, 1H), 7.30 (d, *J* = 2.0 Hz, 1H), 7.14 (dd, *J* = 2.0, 8.4 Hz, 1H), 5.09 - 4.82 (m, 1H), 3.92 - 3.83 (m, 4H), 3.78 (br t, *J* = 6.4 Hz, 2H), 3.38 (br t, *J* = 6.4 Hz, 2H), 3.08 - 2.95 (m, 4H), 2.31 - 2.14 (m, 4H), 1.96 - 1.50 (m, 4H), 0.41 (s, 4H)

**Example 93: N-(2-(4,4-difluoropiperidin-1-yl)-1,5-naphthyridin-4-yl)-4-(2-hydroxyethylsulfon amido)-2-(6-azaspiro[2.5]octan-6-yl)benzamide**

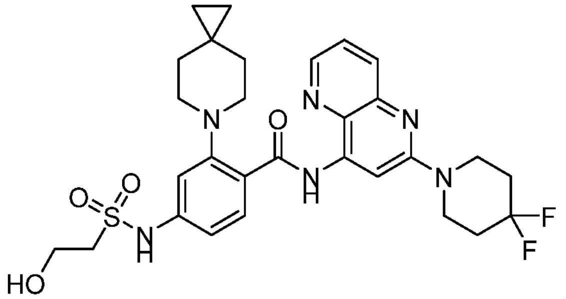

The preparations in **Example 93** follow the synthetic route of **Example 3:** LCMS (ESI): $[M+H]^+ = 601.3$. $^1H$ NMR (400 MHz, DMSO-$d_6$) δ 12.90 (s, 1H), 10.52 - 9.97 (m, 1H), 8.68 (s, 1H), 8.61 (dd, *J* = 1.6, 4.4 Hz, 1H), 8.03 - 7.97 (m, 2H), 7.69 (dd, *J* = 4.0, 8.4 Hz, 1H), 7.27 (d, *J* = 2.0 Hz, 1H), 7.09 (dd, *J* = 2.0, 8.4 Hz, 1H), 5.10 - 4.81 (m, 1H), 3.96 - 3.82 (m, 4H), 3.77 (t, *J* = 6.4 Hz, 2H), 3.38 (t, *J* = 6.4 Hz, 2H), 3.13 - 2.93 (m, 4H), 2.20 - 1.99 (m, 4H), 1.90 - 1.43 (m, 4H), 0.30 (s, 4H)

**Example 94: N-[2-amino-4-(4,4-difluoropiperidin-1-yl)-5-fluoro-1,3-benzothiazol-6-yl]-2-{6-az aspiro[2.5]octan-6-yl}-4-[(2R)-1-hydroxypropane-2-sulfonamido]benzamide**

**Example 95: N-[2-amino-4-(4,4-difluoropiperidin-1-yl)-5-fluoro-1,3-benzothiazol-6-yl]-2-{6-az aspiro[2.5]octan-6-yl}-4-[(2S)-1-hydroxypropane-2-sulfonamido]benzamide**

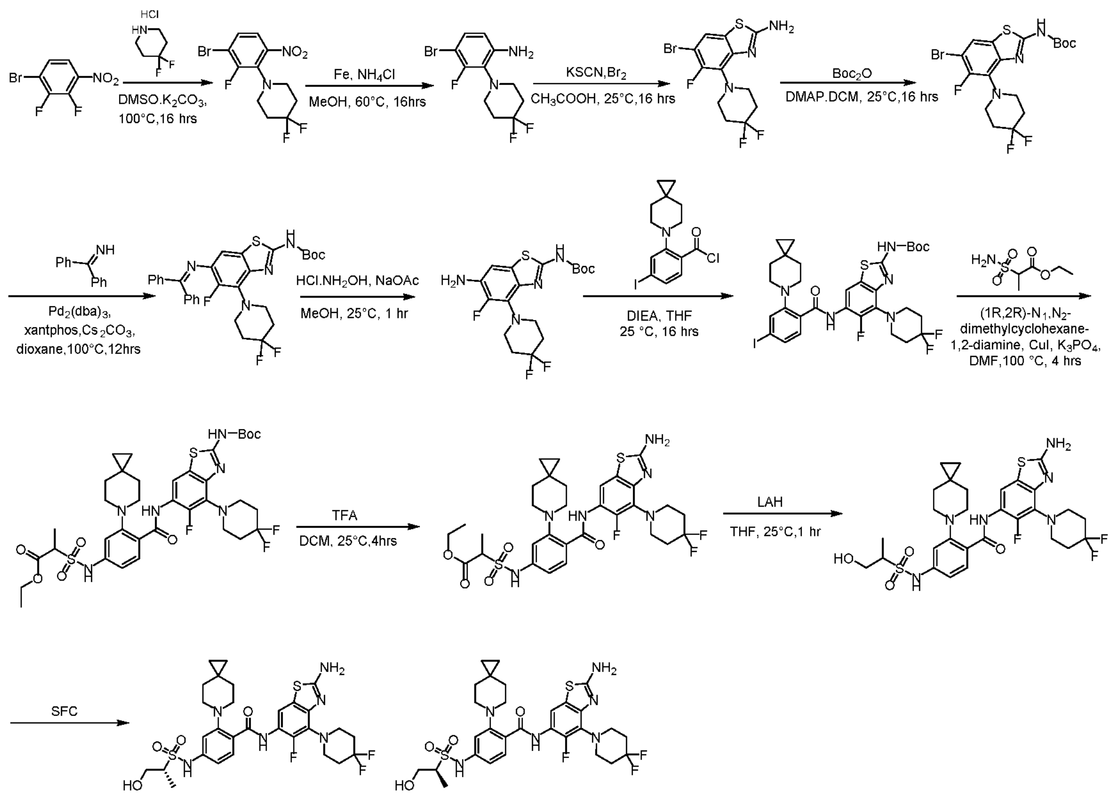


Step 1: Potassium carbonate (17.8 g, 126 mmol) was added to a solution of 1-bromo-2,3-difluoro-4-nitrobenzene (10.0 g, 42.0 mmol) and 4,4-difluoropiperidine hydrochloride (7.28 g, 46.2 mmol) in DMSO (200 mL). The mixture was heated to 100°C and stirred for 16 h under nitrogen atmosphere. And water (200 mL) was added to the reaction solution and extracted withethyl acetate (200 mL × 3). The combined organic layer was dried over anhydrous sodium sulfate, filtered and concentrated under reduced pressure to give 1-(3-bromo-2-fluoro-6-nitrophenyl)-4,4-difluoropiperidine (12.0 g, 35.3 mmol, yield: 84%) as a yellow solid. LCMS (ESI): [M+H]+ =338.9; $^1H$ NMR (400 MHz, DMSO-d6) δ 7.72 - 7.61 (m, 2H), 3.16 (br t, *J* = 5.3 Hz, 4H), 2.16 - 2.01 (m, 4H) Step 2: Iron powder (13.3 g, 236 mmol) was added portionwise to a solution of 1-(3-bromo-2-fluoro-6-nitrophenyl)-4,4-difluoropiperidine (12.0 g, 35.4 mmol) and ammonium chloride (7.64 g,

141 mmol) in methanol (240 mL) and water (80 mL). The mixture was heated to 60°C and stir for 16 h under nitrogen atmosphere. Filter the reaction solution. Add water (160 mL) to the reaction solution and extracted withethyl acetate (200 mL × 3). The combined organic layer was dried over anhydrous sodium sulfate, filtered and concentrated under reduced pressure to give 4-bromo-2-(4,4-difluoropiperidin-1-yl)-3-fluoroaniline (10.8 g, 34.9 mmol, yield: 98.7%) as a yellow solid. LCMS (ESI): [M+H]+ =308.9; $^{1}$H NMR (400 MHz, DMSO-d6) δ 7.09 (br t, *J* = 8.0 Hz, 1H), 6.47 (br d, *J* = 8.8 Hz, 1H), 5.54 (br s, 2H), 3.13 - 2.90 (m, 4H), 2.27 - 1.98 (m, 4H)

Step 3: Potassium thiocyanate (15.7 g, 161 mmol) was added to the solution of 4-bromo-2-(4,4-difluoropiperidin-1-yl)-3-fluoroaniline (10.0 g, 32.3 mmol) in acetic acid (200 mL). Then liquid bromine (5.17 g, 32.3 mmol) was added to the reaction solution. The mixture was heated to 25°C and stir for 16 h under nitrogen. The reaction solution was filtered and the cake was washed with THF (100 mL×5). And water (100 mL) was added to the filtrate and the pH was adjusted to 10 with liquor ammonia, and extracted with ethyl acetate (200 mL×3). The combined organic layer was dried over anhydrous sodium sulfate, filtered and concentrated under reduced pressure to give the residue. The residue was purified by flash column chromatography (silica gel, 0-30% gradient of THF/petroleum ether) to give 6-bromo-4-(4,4-difluoropiperidin-1-yl)-5-fluoro-1,3-benzothiazole-2-amine (5.00 g, 13.5 mmol, yield: 42%) as a yellow solid. LCMS (ESI): [M+H]+ =367.8; $^{1}$H NMR (400 MHz, DMSO-d6) δ 11.47 - 9.30 (m, 2H), 7.84 (br d, *J* = 5.8 Hz, 1H), 3.36 - 3.27 (m, 4H), 2.19 - 2.05 (m, 4H)

Step 4: Triethylamine (846 mg, 8.19 mmol) and 4-dimethylaminopyridine (34.0 mg, 0.27 mmol) were successively added to a solution of 6-bromo-4-(4,4-difluoropiperidin-1-yl)-5-fluoro-1,3-benzothiazole-2-amine (1.00 g, 2.73 mmol) and di-tert-butyl dicarbonate (1.79 g, 8.19 mmol) in dichloromethane (20.0 mL). The mixture was stirred at 25°C for 16 h. Add water (20 mL) to the reaction solution and extracted withethyl acetate (20 mL × 3). The combined organic layer was dried over anhydrous sodium sulfate, filter, spin dry the filtrate, and the residue was purified by flash column chromatography (silica gel, 0-8% gradient of ethyl acetate/petroleum ether) to give tert-butyl N-[6-bromo-4-(4,4-difluoropiperidin-1-yl)-5-fluoro-1,3-benzothiazol-2-yl]carbamate (490 mg, 1.04 mmol, yield: 38%) as a yellow oil. LCMS (ESI): [M+H]+ =465.9; $^{1}$H NMR (400 MHz, DMSO-$d_6$) δ ppm 11.86 (s, 1H), 7.93 (d, *J* = 6.4 Hz, 1H), 3.44 (br t, *J* = 5.2 Hz, 4H), 2.18 - 2.01 (m, 4H), 1.50 (s, 9H)

Step 5: 4,5-bis(diphenylphosphino)-9,9-dimethylxanthene (60.1 mg, 0.10 mmol) and (2-amino-1,1-biphenyl-2-yl) palladium (II) (96.2 mg, 0.10 mmol) were successively added to a solution of tert-butyl N-[6-bromo-4-(4,4-difluoropiperidin-1-yl)-5-fluoro-1,3-benzothiazol-2-yl]carbamate (480 mg, 1.03 mmol), diphenylmethanimine (280 mg, 1.54 mmol) and cesium carbonate (1027 mg, 3.09 mmol) in dioxane (20.0 mL). Replace the mixture 3 times with nitrogen, then the mixture was heated to 100°C under nitrogen atmosphere, and stirred for 12 h. The reaction solution was diluted with water (5 mL), extracted with dichloromethane (15 mL × 2), the combined organic layer was washed with saturated saline (20 mL), dried over magnesium sulfate, filtered, and concentrated under reduced pressure to give the residue. The residue was purified by flash column chromatography (silica gel, 0-6% gradient of ethyl acetate/petroleum ether) to give tert-butyl N-[6-bromo-4-(4,4-difluoropiperidin-1-yl)-5-fluoro-1,3-benzothiazol-2-yl] carbamate (260 mg, 0.46 mmol, yield: 45%) as a yellow solid. LCMS (ESI): $[M+H]^+$ =567.1; $^{1}$H NMR (400 MHz, DMSO-$d_6$) δ ppm 11.61 (br s, 1H), 7.71 - 7.65 (m, 2H), 7.60 - 7.54 (m, 1H), 7.52 - 7.46 (m, 2H), 7.34 - 7.32 (m, 2H), 7.16 (dd, *J* = 2.8, 6.4 Hz, 2H), 6.99 (d, *J* = 7.2 Hz, 1H), 3.27 (br t, *J* = 5.6 Hz, 4H), 2.10 - 2.00 (m, 4H), 1.48 (s, 9H)

Step 6: Hydroxylamine hydrochloride (59.0 mg, 0.85 mmol) and sodium acetate (104 mg, 1.27 mmol) were succesilly added to the mixed solution of tert-butyl (4-(4,4-difluoropiperidin-1-yl)-6-((diphenylmethylene)amino)-5-fluorobenzo[d]thiazo l-2-yl)carbamate (240 mg, 0.42 mmol) and methanol (2.00 mL). The mixture was stirred at 25°C for 1 h. The reaction solution was diluted with water (5 mL), extracted with dichloromethane (15 mL × 2), the combined organic layer was washed with saturated saline (20 mL), dried over magnesium sulfate, filtered, and concentrated under reduced pressure to give the residue. The residue was purified by flash column chromatography (silica gel, 0-50% gradient of ethyl acetate/petroleum ether) to give tert-butyl N-[6-amino-4-(4,4-difluoropiperidin-1-yl)-5-fluoro-1,3-benzothiazol-2-yl]carbamate (120 mg, 0.30 mmol, yield: 70%) as a yellow solid. LCMS (ESI): $[M+H]^+$ =403.0.

Step 7: N,N-diisopropylethylamine (118 mg, 0.89 mmol) was added to a solution of tert-butyl N-[6-amino-4-(4,4-difluoropiperidin-1-yl)-5-fluoro-1,3-benzothiazol-2-yl]carbamate (120 mg, 0.30 mmol) and 4-iodo-2-(6-azaspiro[2.5]octan-6-yl)benzoyl chloride (168 mg, 0.45 mmol) in THF (4.00 mL), and the mixture was stirred at 25°C for 16 h. The reaction solution was diluted with water (5 mL), extracted with dichloromethane (15 mL × 2), the combined organic layer was washed with saturated saline (20 mL), dried over magnesium sulfate, filtered, and concentrated under reduced pressure. The residue was purified by flash column chromatography (silica gel, 0-10% gradient of ethyl acetate/petroleum ether) to give tert-butyl N-[6-(2-{6-azaspiro[2.5]octan-6-yl}-4-iodobenzoylamino)-4-(4,4-difluoropiperidin-1-yl)-5-fluoro -1,3-benzothiazol-2-yl]carbamate (80 mg, 0.10 mmol, yield: 36%) as a yellow solid. LCMS (ESI): $[M+H]^+$ =742.2

Step 8: Cuprous iodide (249 mg, 1.30 mmol) was added to a solution of ethyl 2-sulfamoylpropyl ester (307 mg, 1.69 mmol), trans-N,N-dimethyl-1,2-cyclohexanediamine (187 mg, 1.30 mmol), potassium phosphate (832 mg, 3.90 mmol) in N,N-dimethylformamide (20 mL). The mixture was stirred at 50°C for 5 min. Then, tert-butyl N-[6-(2-{6-azaspiro [2.5]octan-6-yl}-4-iodobenzoylamino)-4-(4,4-difluoropiperidin-1-yl)-5-fluoro-1,3-benzothiazol-2-yl]carbamate (1100 mg, 1.30 mmol) was add to the reaction mixture at 50°C, and the mixture was heated to 100°C with stirring for 4 h under nitrogen

atmosphere. And water (50 mL) was added to the reaction solution and extracted with ethyl acetate (50 mL × 2). The combined organic layer was dried over anhydrous magnesium sulfate, filtered, and the filtrate was concentrated to dryness. The residue was purified by flash column chromatography (silica gel, 0-30% gradient of ethyl acetate/petroleum ether) to give ethyl 2-[(3-{6-azaspiro[2.5]octan-6-yl}-4-[(2-{[(tert-butoxy)carbonyl]amino}-4-(4,4-difluo ropiperidin-1-yl)-5-fluoro-1,3-benzothiazol-6-yl)aminocarbonyl]phenyl)sulfamoyl]pr opyl ester (1100 mg, crude) as a yellow solid compound. LCMS (ESI): $[M+H]^+$ =795.2.

Step 9: Ethyl 2-[(3-{6-azaspiro[2.5]octan-6-yl}-4-[(2-{[(tert-butoxy)carbonyl]amino}-4-(4,4-difluo ropiperidin-1-yl)-5-fluoro-1,3-benzothiazol-6-yl)aminocarbonyl]phenyl)sulfamoyl]pr opyl ester (1000 mg, 1.12 mmol) was added to the mixed solution of trifluoroacetic acid (5.00 mL) and dichloromethane (20.0 mL). The mixture was stirred at 25°C for 4 h. And saturated aqueous sodium bicarbonate solution (20 mL) was added to the reaction solution and extracted with dichloromethane (20 mL×2). The combined organic layer was dried over anhydrous magnesium sulfate, filtered and concentrated under reduced pressure to give ethyl 2-[(4-{[2-amino-4-(4,4-difluoropiperidin-1-yl)-5-fluoro-1,3-benzothiazol-6-yl]aminoc arbonyl}-3-{6-azaspiro[2.5]octan-6-yl}phenyl)sulfamoyl]propyl ester (700 mg, 1.01 mmol. yield: 90.2%) as a white solid. LCMS (ESI): $[M+H]^+$ =695.2.

Step 10: Ethyl 2-[(4-{[2-amino-4-(4,4-difluoropiperidin-1-yl)-5-fluoro-1,3-benzothiazol-6-yl]aminoc arbonyl}-3-{6-azaspiro[2.5]octan-6-yl}phenyl)sulfamoyl]propyl ester (700 mg, 1.01 mmol) was added to a solution of THF (14 mL), then lithium tetrahydridoaluminate (54.0 mg, 1.51 mmol) was added portionwise at 25°C, and The mixture was stirred at 25°C for 1 h. The reaction solution was quenched by successively adding water (0.05 mL) and 15% aqueous sodium hydroxide solution (0.05 mL), then water (0.1 mL), then filtered and concentrated under reduced pressure to give the residue. The residue was purified by flash column chromatography (silica gel, 0-30% gradient of THF/petroleum ether) to give a yellow solid compound, and then perform SFC separation (IG, system: carbon dioxide/ethanol (0.1% liquor ammonia)) to give N-[2-amino-4-(4,4-difluoropiperidin-1-yl)-5-fluoro-1,3-benzothiazol-6-yl]-2-{6-azasp iro[2.5]octan-6-yl}-4-[(2R)-1-hydroxypropane-2-sulfonamido]benzamide (103 mg, 0.15 mmol, yield: 15.6%) and N-[2-amino-4-(4,4-difluoropiperidin-1-yl)-5-fluoro-1,3-benzothiazol-6-yl]-2-{6-azasp iro[2.5]octan-6-yl}-4-[(2S)-1-hydroxypropane-2-sulfonamido]benzamide (142 mg, 0.22 mmol, yield: 21.5%) as white solid compounds.

**Example 94: L**CMS (ESI): [M+H]+ = 653.2; $^1$H NMR (400 MHz, DMSO-d6) δ ppm 12.01 (s, 1H), 10.37 - 9.92 (m, 1H), 8.37 (d, *J* = 6.8 Hz, 1H), 7.97 (d, *J* = 8.4 Hz, 1H), 7.52 (s, 2H), 7.28 (d, *J* = 1.6 Hz, 1H), 7.12 (dd, *J* = 1.6, 8.8 Hz, 1H), 5.24 - 4.84 (m, 1H), 3.85 (dd, *J* = 4.4, 10.8 Hz, 1H), 3.49 (br dd, *J* = 8.0, 10.8 Hz, 1H), 3.41 (br t, *J* = 5.2 Hz, 4H), 3.28 - 3.22 (m, 1H), 3.06 - 2.84 (m, 4H), 2.20 - 2.03 (m, 4H), 1.72 - 1.43 (m, 4H), 1.30 (d, *J* = 6.8 Hz, 3H), 0.37 (s, 4H)

**Example 95: L**CMS (ESI): [M+H]+ = 653.3; $^1$H NMR (400 MHz, DMSO-d6) δ ppm 12.01 (s, 1H), 10.38 - 9.92 (m, 1H), 8.37 (d, *J* = 6.8 Hz, 1H), 7.97 (d, *J* = 8.4 Hz, 1H), 7.52 (s, 2H), 7.28 (d, *J* = 1.6 Hz, 1H), 7.12 (dd, *J* = 1.6, 8.8 Hz, 1H), 5.23 - 4.92 (m, 1H), 3.85 (dd, *J* = 4.4, 10.8 Hz, 1H), 3.49 (dd, *J* = 8.0, 10.8 Hz, 1H), 3.41 (br t, *J* = 5.2 Hz, 4H), 3.29 - 3.25 (m, 1H), 3.08 - 2.79 (m, 4H), 2.19 - 2.04 (m, 4H), 1.68 - 1.44 (m, 4H), 1.30 (d, *J* = 6.8 Hz, 3H), 0.37 (s, 4H)

**Example 96: 2-16-Azaspiro[2.5]octan-6-yl)-N-[8-(4,4-difluoropiperidin-1-yl)-1,7-naphthyridin-6-yl]-4-[(2R)-1-hydroxypropane-2-sulfonamido]benzamide**

**Example 97: 2-{6-azaspiro[2.5]octan-6-yl}-N-[8-(4,4-difluoropiperidin-1-yl)-1,7-naphthyridin-6-yl]-4-[(2S)-1-hydroxypropane-2-sulfonamido]benzamide**

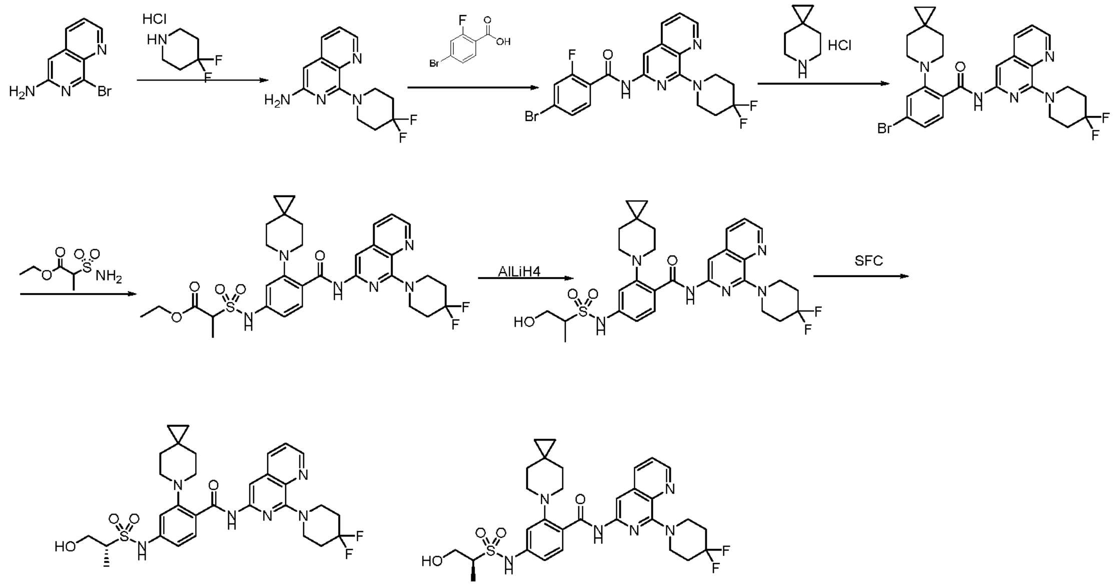


Step 1: The solution of 8-bromo-1,7-naphthyridine-6-amine (4.50 g, 20.08 mmol), 4,4-difluoropiperidine hydrochloride (3.84 g, 24.10 mmol), and cesium carbonate (13.36 g, 40.17 mmol) in N,N-dimethylformamide (50.00 mL) was stirred at 100°C for 5 h. The reaction mixture was filtered, and the cake was washed with ethyl acetate (200 mL), and the organic phase was concentrated to give 8-(4,4-difluoropiperidinyl)pyridyl[2,3-c]pyridine-6-ylamine (4.00 g, 15.10 mmol, yield: 75.1%) as a yellow oil. LCMS (ESI): $[M+H]^+$ =265.2; $^1$HNMR (400 MHz, DMSO) δ 8.34 (dd, J = 4.0, 1.7 Hz, 1H), 7.79 (dd, J = 8.5, 1.7 Hz, 1H), 7.32 (dd, J = 8.4, 4.0 Hz, 1H), 6.04 (s, 1H), 5.75 (s, 2H), 4.05 (dt, J = 6.7, 2.5 Hz, 4H), 2.09 (tt, J = 14.3, 5.6 Hz, 4H).

Step 2: 1-propylphosphoric cyclic anhydride (726 mg, 1.14 mmol) and N,N-diisopropylethylamine (180 mg, 1.37 mmol) was added to a solution of 4-bromo-2-fluorobenzoic acid (2.60 g, 11.87 mmol) and 8-(4,4-difluoropiperidinyl)pyridyl [2,3-c]pyridine-6-ylamine (3.73 g, 13.98 mmol) in N,N-dimethylformamide (10.00 mL) at 20°C. The reactionmixture was stirred at 70°C for 16 h. The reaction mixture was poured into water, extracted with ethyl acetate (100 mL×2), and the organic phases was washed with water (100 mL×2) and saturated sodium chloride solution (100 mL). The organic phase was concentrated, dried, and filtered to give the residue and purified by flash column chromatography (silica gel, 0-30% gradient of ethyl acetate/petroleum ether) to give N-[8-(4,4-difluoropiperidinyl)pyridylo[3,2-d]pyridin-6-yl](2-fluoro-4-iodophenyl)car boxylamide (2.60 g, 5.07 mmol, yield: 43.5%) as a yellow solid. LCMS (ESI): $[M+H]^+$ =513.2

Step 3: Potassium carbonate (1.43 g, 10.15 mmol) was added in a solution of N-[8-(4,4-difluoropiperidinyl) pyridylo[3,2-d]pyridin-6-yl](2-fluoro-4-iodophenyl)car boxylamide (1.30 g, 2.54 mmol) and 6-azaspiro[2.5]octane hydrochloride (760 mg, 5.08 mmol) in DMSO (15 mL) at 20°C, and The reactionmixture was stirred at 130°C for 7 h. The reaction mixture was filtered and the cake was washed with ethyl acetate (50 mL). Filter and dry the turbid organic phases to give [2-(6-azaspiro[2.5]octan-6-yl)-4-iodophenyl]-N-[8-(4,4-difluoropiperidinyl)pyridyl[3, 2-d]pyridin-6-yl]ammonium carboxylate (990 mg, 1.64 mmol, yield: 64.6%) as a yellow solid. LCMS (ESI): $[M+H]^+$ =604.3; $^1$HNMR (400 MHz, DMSO) δ 12.92 (s, 1H), 8.73 (dd, J = 4.1, 1.7 Hz, 1H), 8.26 (dd, J = 8.5, 1.8 Hz, 1H), 8.10 (s, 1H), 7.88 (d, J = 8.3 Hz, 1H), 7.82 (d, J = 1.7 Hz, 1H), 7.74 (dd, J = 8.3, 1.6 Hz, 1H), 7.62 (dd, J = 8.3, 4.1 Hz, 1H), 4.20 (t, J = 5.8 Hz, 4H), 3.05 (t, J = 5.3 Hz, 4H), 2.16 (dq, J = 13.8, 7.1, 5.8 Hz, 4H), 1.83 - 1.60 (m, 4H), 0.40 (s, 4H).

Step 4: Cuprous iodide (257 mg, 1.32 mmol) and (1R, 2R)-(-)-N,N'-dimethyl-1,2-cyclohexanediamine (190 mg, 1.32 mmol) were successively added to a solution of [2-(6-azaspiro[2.5]octan-6-yl)-4-iodophenyl]-N-[8-(4,4-difluoropiperidinyl)pyridyl[3, 2-d]pyridin-6-yl]ammonium carboxylate (800 mg, 1.33 mmol), ethyl 2-aminosulfonyl propionate (720.70 mg, 3.98 mmol), and potassium phosphate (861 mg, 3.98 mmol) in N,N-dimethylformamide (12 mL) at 20°C, perform nitrogen bubbling for 2 min and the resulting reaction mixture was stirred at 100°C for 6 h. The reaction mixture was poured into water (50 mL), extracted with ethyl acetate (30 mL × 3), the combined organic layer was dried The combined organic layer was dried over anhydrous sodium sulfate, filtered, and concentrated to give the residue. Purified by flash column chromatography (silica, 30-60% gradient of ethyl acetate/petroleum ether) to give ethyl 2-{[(3-(6-azaspiro[2.5] octan-6-yl)-4-{N-[8-(4,4-difluoropiperidinyl)pyridyl[3,2-d]pyr idin-6-yl]carbamoyl}phenyl)amino]sulfonyl}propionate (730 mg, 1.11 mmol, yield: 84%) as a yellow solid. LCMS (ESI): $[M+H]^+$ =657.4; $^1$H NMR (400 MHz, DMSO) δ 12.87 (s, 1H), 10.66 (s, 1H), 8.72 (dd, J = 4.1, 1.7 Hz, 1H), 8.24 (dd, J = 8.5, 1.8 Hz, 1H), 8.12 (d, J = 8.8 Hz, 2H), 7.61 (dd, J = 8.4, 4.1 Hz, 1H), 7.32 (d, J = 2.2 Hz, 1H), 7.20 - 7.09 (m, 2H), 4.32 (q, J = 7.0 Hz, 1H), 4.19 (q, J = 4.9, 4.1 Hz, 4H), 4.12 - 4.05 (m, 2H), 3.01 (t, J = 5.2 Hz, 4H), 2.17 (td, J = 13.9, 6.4 Hz, 4H), 1.67 (s, 3H), 1.51 - 1.43 (m, 4H), 1.16 (d, J = 7.1 Hz, 3H), 0.41 (s, 4H).

Step 5: Lithium aluminum hydride (114 mg, 3.20 mmol) was added to a solution of ethyl 2-{[(3-(6-azaspiro[2.5]

octan-6-yl)-4-{N-[8-(4,4-difluoropiperidinyl)pyridyl[3,2-d]pyr idin-6-yl]carbamoyl}phenyl)amino]sulfonyl}propionate (700 mg, 1.06 mmol) in THF (10 mL) at 0°C, and the reaction solution was stirred at 0°C for 1.5 h. Ethyl acetate (50 mL) and saturated ammonium chloride solution (50 mL) was added to the reaction solution. The mixture was stirred at ambient temperature for 3 h. The combined organic layer was dried, filtered, and concentrated to give the residue. Purified by flash column chromatography (silica, 0-5% gradient of methanol/dichloromethane) to give 330 mg of yellow solid product. Purify 100 mg of the product by preparative HPLC (C18, 40-90% gradient of acetonitrile/water) to give nitrogen-(8-(4,4-difluoropiperidin-1-yl)-1,7-naphthopyridin-6-yl)-4-((2-hydroxyethyl-1-methylethyl)sulfonamido)-2-(6-azaspiro[2.5]octan-6-yl)benzamide (45 mg, 0.073 mmol, yield: 7%) as a pale yellow solid. LCMS (ESI): $[M+H]^+$ =615.4; $^1H$ NMR (400 MHz, DMSO) δ 12.90 (s, 1H), 10.23 (s, 1H), 8.74 (dd, J = 4.1, 1.7 Hz, 1H), 8.26 (dd, J = 8.5, 1.7 Hz, 1H), 8.12 (d, J = 8.1 Hz, 2H), 7.64 (dd, J = 8.3, 4.1 Hz, 1H), 7.33 (d, J = 2.1 Hz, 1H), 7.18 (dd, J = 8.6, 2.1 Hz, 1H), 5.05 (s, 1H), 4.22 (t, J = 5.8 Hz, 4H), 3.87 (dd, J = 11.1, 4.4 Hz, 1H), 3.52 (dd, J = 11.1, 7.6 Hz, 1H), 3.30 (dt, J = 7.3, 3.6 Hz, 1H), 3.03 (d, J = 5.4 Hz, 4H), 2.19 (dq, J = 13.8, 6.7, 5.6 Hz, 4H), 1.78 (s, 4H), 1.32 (d, J = 6.9 Hz, 3H), 0.43 (s, 4H).

Step 6: Perform SFC separation (IG, system: carbon dioxide/ethanol (0.1% liquor ammonia)) for the compound 2-{6-azaspiro[2.5]octan-6-yl}-N-[8-(4,4-difluoropiperidin-1-yl)-1,7-naphthyridin-6-yl] -4-(1-hydroxypropane-2-sulfonamido)benzamide (230 mg, 0.37 mmol) to give 2-{6-azaspiro[2.5]octan-6-yl}-N-[8-(4,4-difluoropiperidin-1-yl)-1,7-naphthyridin-6-yl] -4-[(2R)-1-hydroxypropane-2-sulfonamido]benzamide (100 mg, 0.16 mmol, yield: 43.5%) and 2-{6-azaspiro[2.5]octan-6-yl}-N-[8-(4,4-difluoropiperidin-1-yl)-1,7-naphthyridin-6-yl] -4-[(2S)-1-hydroxypropane-2-sulfonamido]benzamide (100 mg, 0.16 mmol, yield: 43.5%) as yellow solid compounds.

**Example 96:** LCMS (ESI): [M+H]+ = 615.1; $^1H$ NMR (400 MHz, DMSO-d6) δ ppm 12.88 (s, 1H), 10.54 - 9.91 (m, 1H), 8.72 (dd, *J* = 1.6, 4.0 Hz, 1H), 8.24 (dd, *J* = 1.6, 8.4 Hz, 1H), 8.15 - 8.05 (m, 2H), 7.61 (dd, *J* = 4.0, 8.4 Hz, 1H), 7.30 (d, *J* = 2.0 Hz, 1H), 7.16 (dd, *J* = 2.0, 8.8 Hz, 1H), 5.30 - 4.71 (m, 1H), 4.20 (br t, *J* = 5.2 Hz, 4H), 3.85 (dd, *J* = 4.4, 11.2 Hz, 1H), 3.49 (dd, *J* = 7.6, 11.2 Hz, 1H), 3.30 - 3.24 (m, 1H), 3.00 (br s, 4H), 2.27 - 2.11 (m, 4H), 2.05 - 1.39 (m, 4H), 1.30 (d, *J* = 6.8 Hz, 3H), 0.41 (s, 4H)

**Example 97:** LCMS (ESI): [M+H]+ = 603.1; $^1H$ NMR (400 MHz, DMSO-d6) δ ppm 12.88 (s, 1H), 10.50 - 9.92 (m, 1H), 8.72 (dd, *J* = 1.6, 4.0 Hz, 1H), 8.24 (dd, *J* = 1.6, 8.4 Hz, 1H), 8.15 - 8.05 (m, 2H), 7.61 (dd, *J* = 4.0, 8.4 Hz, 1H), 7.30 (d, *J* = 2.0 Hz, 1H), 7.15 (dd, *J* = 2.0, 8.8 Hz, 1H), 5.23 - 4.78 (m, 1H), 4.20 (br t, *J* = 5.2 Hz, 4H), 3.84 (dd, *J* = 4.4, 11.2 Hz, 1H), 3.49 (dd, *J* = 7.6, 11.2 Hz, 1H), 3.29 - 3.21 (m, 1H), 3.00 (br s, 4H), 2.27 - 2.11 (m, 4H), 2.05 - 1.39 (m, 4H), 1.30 (d, *J* = 6.8 Hz, 3H), 0.41 (s, 4H)

**Example 98: 2-{6-azaspiro[2.5]octan-6-yl}-N-[8-(4,4-difluoropiperidin-1-yl)-7-fluoroquinolin-6 -yl]-4- [(2R)-1-hydroxypropane-2-sulfonamido] benzamide**

**Example 99: 2-{6-azaspiro[2.5]octan-6-yl}-N-[8-(4,4-difluoropiperidin-1-yl)-7-fluoroquinolin-6 -yl]-4-[(2S)-1-hydroxypropane-2-sulfonamido]benzamide**

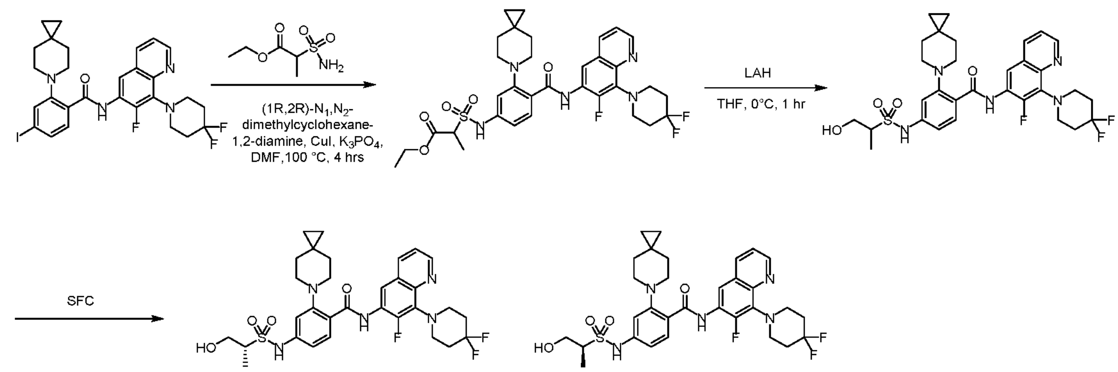


Step 1: Cuprous iodide (13.6 mg, 0.07 mmol) was added to a solution of ethyl 2-sulfamoylpropyl ester (190 mg, 1.05 mmol), trans-N,N-dimethyl-1,2-cyclohexanediamine (100 mg, 0.70 mmol), potassium phosphate (454 mg, 2.10 mmol) in N,N-dimethylformamide (5.0 mL). The mixture was stirred at 50°C for 5 min. Then 2-{6-Azaspiro[2.5]octan-6-yl}-N-[8-(4,4-difluoropiperidin-1-yl)-7-fluoroquinolin-6-y l]-4-iodobenzamide (434 mg, 0.70 mmol) was added to the reaction mixture at 50°C, and the mixture was heated to 100°C with stirring for 4 h under nitrogen atmosphere. Water (10 mL) was added to the reaction solution and extracted with ethyl acetate (10 mL × 2). The combined organic layer was dried over anhydrous sodium sulfate, filtered and concentrated under reduced pressure to give the residue. The residue was purified by flash column chromatography (silica gel, 0-33% gradient of ethyl acetate/petroleum ether) to give ethyl

2-[(3-{6-azaspiro[2.5]octan-6-yl}-4-{[8-(4,4-difluoropiperidin-1-yl)-7-fluoroquinolin-6-yl]aminocarbonyl}phenyl)sulfamoyl]propyl ester (280 mg, 0.412 mmol, yield: 56%) as a white solid. LCMS (ESI): [M+H]+ = 674.2; $^1$H NMR (400 MHz, DMSO-$d_6$) δ = 12.55 - 12.38 (m, 1H), 10.77 - 10.55 (m, 1H), 8.86 - 8.78 (m, 1H), 8.76 - 8.67 (m, 1H), 8.42 - 8.27 (m, 1H), 8.12 - 8.01 (m, 1H), 7.54 - 7.42 (m, 1H), 7.37 - 7.26 (m, 1H), 7.21 - 7.08 (m, 1H), 4.08 (br dd, *J* = 4.4, 7.1 Hz, 2H), 3.67 - 3.51 (m, 5H), 3.11 - 2.92 (m, 4H), 2.24 - 2.11 (m, 4H), 1.68 - 1.54 (m, 4H), 1.51 - 1.45 (m, 3H), 1.19 - 1.08 (m, 3H), 0.48 - 0.25 (m, 4H)

Step 2: Ethyl 2-[(3-{6-azaspiro[2.5]octan-6-yl}-4-{[8-(4,4-difluoropiperidin-1-yl)-7-fluoroquinolin-6-yl]aminocarbonyl}phenyl)sulfamoyl]propyl ester (400.00 mg, 0.63 mmol) was added to a solution of THF (5 mL), lithium tetrahydridoaluminate (33.91 mg, 0.95 mmol) was added portionwise at 0°C, and The mixture was stirred at 0°C for 1 h. The reaction solution was quenched with water (0.05 mL) and 10% aqueous sodium hydroxide solution (0.10 mL), filtered and concentrated under reduced pressure to give a residue. The residue was purified by preparative HPLC (C18, 34-74% gradient of water (formic acid)/acetonitrile) and resolve by chiral SFC (IG, system: carbon dioxide/methanol (0.1% liquor ammonia)) to give 2-{6-azaspiro[2.5]octan-6-yl}-N-[8-(4,4-difluoropiperidin-1-yl)-7-fluoroquinolin-6-yl] -4-[(2R)-1-hydroxypropane-2-sulfonamido]benzamide (61.7 mg, 0.095 mmol, yield: 15%) and 2-{6-azaspiro[2.5]octan-6-yl}-N-[8-(4,4-difluoropiperidin-1-yl)-7-fluoroquinolin-6-yl] -4-[(2R)-1-hydroxypropane-2-sulfonamido]benzamide (69.7 mg, 0.12 mmol, yield: 17%) as white solid compounds.

**Example 98:** LCMS (ESI): [M+H]+ = 632.3; $^1$H NMR (400 MHz, DMSO-$d_6$) δ = 12.52 (br s, 1H), 8.82 (dd, *J* = 1.6, 4.0 Hz, 1H), 8.75 (d, *J* = 8.0 Hz, 1H), 8.33 (dd, *J* = 1.6, 8.4 Hz, 1H), 8.01 (d, *J* = 8.4 Hz, 1H), 7.49 (dd, *J* = 4.2, 8.4 Hz, 1H), 7.27 (br s, 1H), 7.11 (br d, *J* = 8.4 Hz, 1H), 3.90 - 3.78 (m, 1H), 3.58 (br d, *J* = 4.4 Hz, 4H), 3.53 - 3.46 (m, 1H), 3.28 - 3.21 (m, 1H), 2.98 (br t, *J* = 4.8 Hz, 4H), 2.25 - 2.13 (m, 4H), 1.67 - 1.51 (m, 4H), 1.29 (d, *J* = 6.8 Hz, 3H), 0.38 (s, 4H)

**Example 99:** LCMS (ESI): [M+H]+ = 632.7; $^1$H NMR (400 MHz, DMSO-$d_6$) δ = 12.74 - 12.41 (m, 1H), 8.86 - 8.79 (m, 1H), 8.78 - 8.71 (m, 1H), 8.32 (dd, *J* = 1.6, 8.4 Hz, 1H), 7.99 (d, *J* = 8.8 Hz, 1H), 7.49 (dd, *J* = 4.0, 8.4Hz, 1H), 7.23 (s, 1H), 7.06 (br d, *J*= 8.4 Hz, 1H), 3.89 - 3.77 (m, 1H), 3.66 - 3.54 (m, 4H), 3.52 - 3.45 (m, 1H), 3.26 - 3.16 (m, 1H), 3.08 - 2.88 (m, 4H), 2.30 - 2.13 (m, 4H), 1.73 - 1.50 (m, 4H), 1.32 - 1.25 (m, 3H), 0.47 - 0.28 (m, 4H)

**Example 100: N-(2-{6-azaspiro[2.5]octan-6-yl}-4-(2-hydroxyethanesulfonamido)phenyl)-8-(4,4-difluoropiperidin-1-yl)quinoline-6-carboxamide**

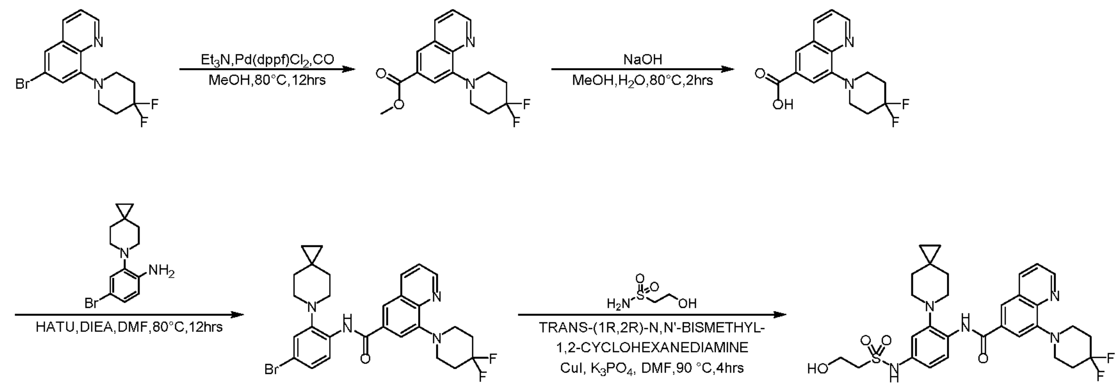


Step 1: 1,1-bis(tert-butylphosphorus)ferrocene palladium chloride (0.69 g, 0.92 mmol) was added to a solution of 6-bromo-8-(4,4-difluoropiperidin-1-yl)quinoline (3.00 g, 9.17 mmol) and triethylamine (2.78 g, 27.5 mmol) in methanol (60 mL), and replace the reaction mixture three times with argon at ambient temperature. Then the reaction system was exchanged with carbon monoxide for three times and finally the reaction solution was stirred at 80°C for 12 h under carbon monoxide atmosphere (50 psi). The reaction mixture was added to water (200 mL) and extracted with ethyl acetate (300 mL × 2). The organic phases were combined and washed once with saturated saline (200 mL), dried over anhydrous magnesium sulfate, filtered and concentrated under reduced pressure to give a residue. The residue was purified by flash column chromatography (silica gel, 0-33% gradient of ethyl acetate/petroleum ether) to give methyl 8-(4,4-difluoropiperidin-1-yl)quinoline-6-carboxylate (2.60 g, 8.53 mmol, yield: 93%) as a white solid. LCMS (ESI): [M+H]+ = 307.1

Step 2: Sodium hydroxide (400 mg, 9.79 mmol) was added to a mixed solution of 8-(4,4-difluoropiperidin-1-yl) quinoline-6-carboxylate (1.00 g, 3.26 mmol) in methanol (10 mL), THF (5 mL) and water (5 mL). The reactionmixture was stirred at 80°C for 2 h. Methanol and THF were removed from the reaction mixture, and the pH was adjusted of the remaining aqueous phase to about 3 with 1 M aqueous hydrochloric acid solution. The white solids precipitated from the solution was collected by filtration and dried under reduced pressure to give 8-(4,4-difluoropiperidin-1-yl)quinoline-6-carboxylic acid (0.90, 3.06 mmol, yield: 94%) as a pure white solid product. LCMS (ESI): [M+H]+ = 293.0

Step 3: Add 2-{ 6-azaspiro[2.5]octan-6-yl }-4-bromoaniline (370 mg, 1.32 mmol) and N,N-diisopropylethylamine

(464 mg, 3.59 mmol) to a solution of 8-(4,4-difluoropiperidin-1-yl)quinoline-6-carboxylic acid (350 mg, 1.20 mmol) in N,N-dimethylformamide (7 mL), add O-(7-azabenzotriazol-1-yl)-N,N,N,N-tetramethylurea hexafluorophosphonate (592 mg, 1.56 mmol) to the reaction vial, and The reactionmixture was stirred at 80°C for 12 h. Add the reaction mixture to water (20 mL) and extracted withethyl acetate (30 mL × 3). Combine the organic phases and wash once with saturated saline (30 mL), dry over anhydrous magnesium sulfate, filter and spin dry the filtrate to give a residue. Dilute the residue with ethyl acetate (20 mL), and then slowly titrate with petroleum ether until the precipitation of white solids, which are then collected by filtration to give the expected product N-(2-{6-azaspiro[2.5]octan-6-yl}-4-bromophenyl)-8-(4,4-difluoropiperidin-1-yl) quin oline-6-carboxamide (150 mg, 0.28 mmol, yield: 23%). LCMS (ESI): [M+H]+ = 556.6

Step 4: 2-hydroxyethyl sulfonamide (50.7 mg, 0.41 mmol) and (1R,2R)-N1,N2-dimethylcyclohexane-1,2-diamine (38.8 mg, 0.27 mmol) were added successively to N,N-dimethylformamide (3.00 mL), then cuprous iodide (26.0 mg, 0.14 mmol) and potassium phosphate (223 mg, 1.08 mmol) were added successively at 25°C. The reaction system was stirred at 50°C for 5 min. Subsequently, N-(2-{6-azaspiro[2.5]octan-6-yl}-4-bromophenyl)-8-(4,4-difluoropiperidin-1-yl)quin oline-6-carboxamide (150 mg, 0.27 mmol) was added in one portion and the mixture was heated to 90°C with stirring for 4 h under nitrogen atmosphere. Liquor ammonia (5 mL) and water (15 mL) were successively added to the reaction mixture and extracted with ethyl acetate (20 mL × 3). The organic phases were combined and washed with saturated saline (20 mL × 1), dried over anhydrous magnesium sulfate, filtered and concentrated under reduced pressure to give a residue. The residue was purified by preparative HPLC (C18, 36-76% gradient of water (formic acid)/acetonitrile) to give N-(2-{6-azaspiro[2.5]octan-6-yl}-4-(2-hydroxyethanesulfonamido)phenyl)-8-(4,4-difl uoropiperidin-1-yl)quinoline-6-carboxamide (80 mg, 0.20 mmol, yield: 49%) as a yellow solid compound. LCMS (ESI): [M+H]+ = 661.2, $^1$H NMR (400 MHz, DMSO-d6) δ ppm 9.68 (d, *J* = 11.6 Hz, 2H), 9.00 (dd, *J* = 1.6, 4.0Hz, 1H), 8.50 (dd, *J* = 1.2, 8.4 Hz, 1H), 8.22 (s, 1H), 8.01 (d, *J* = 8.8 Hz, 1H), 7.73 - 7.61 (m, 2H), 7.14 (d, *J*= 2.0 Hz, 1H), 7.00 (dd, *J* = 2.4, 8.8 Hz, 1H), 4.92 (s, 1H), 3.76 (t, *J* = 6.8 Hz, 2H), 3.58 (br s, 4H), 3.25 (br t, *J* = 6.8 Hz, 2H), 2.88 (br d, *J* = 4.8 Hz, 4H), 2.32 - 2.21 (m, 4H), 1.53 (br s, 4H), 0.34 (s, 4H)

**Example 101: N-(2-amino-4-(4,4-difluoropiperidin-1-yl)-5-fluorobenzo[d]thiazol-6-yl)-4-((2-hyd roxy-1-methylethyl)sulfonamido)-2-(spiro[2.5]oct-5-en-6-yl) benzamide**

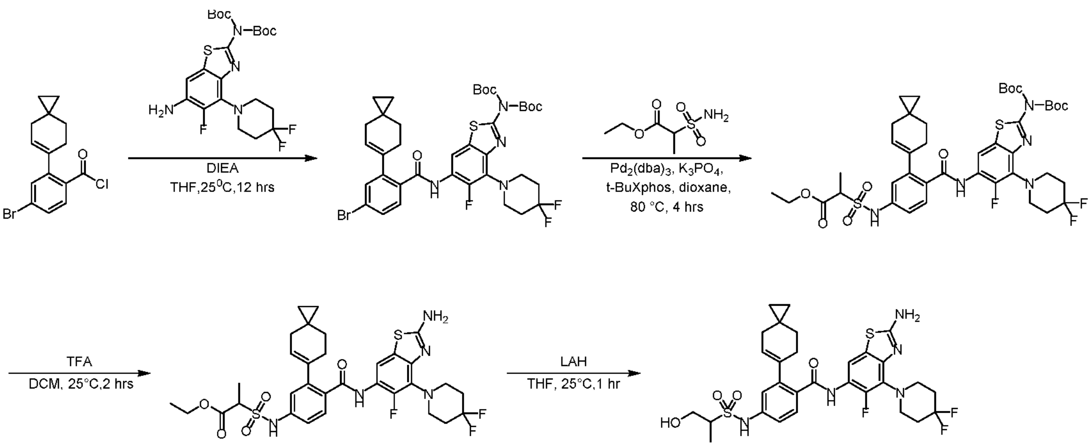


Step 1: Add 4-bromo-2-{spiro[2.5]oct-5-en-6-yl}benzoyl chloride (500 mg, 1.54 mmol) and N,N-diisopropylethylamine (595 mg, 4.61 mmol) to a solution of tert-butyl N-[6-amino-4-(4,4-difluoropiperidin-1-yl)-5-fluoro-1,3-benzothiazol-2-yl]-N-[(tert-bu toxy)carbonyl]carbamate (772 mg, 1.54 mmol) in THF (10 mL), and The reactionmixture was stirred at 25°C for 12 h. Add the reaction mixture to water (60 mL) and extracted withethyl acetate (60 mL × 3). Combine the organic phases and wash once with saturated saline (40 mL), dry over anhydrous magnesium sulfate, filter and spin dry the filtrate to give a residue. The residue was purified by flash column chromatography (silica gel, 0-2% gradient of THF/petroleum ether) to give tert-butyl N-[6-(4-bromo-2-{spiro[2.5]oct-5-en-6-yl}benzoylamino)-4-(4,4-difluoropiperidin-1-yl)-5-fluoro-1,3-benzothiazol-2-yl]-N-[(tert-butoxy)carbonyl]carbamate (0.58 g, 0.74 mmol, yield: 48%) as a yellow solid. LCMS (ESI): [M+H]+ = 793.1; $^1$H NMR (400 MHz, DMSO-d6) δ ppm 9.96 (s, 1H), 8.07 (d, *J* = 6.4 Hz, 1H), 7.57 (dd, *J* = 1.6, 8.4 Hz, 1H), 7.50 (d, *J* = 1.2 Hz, 1H), 7.42 (s, 1H), 5.79 (br s, 1H), 3.40 (br d, *J* = 5.2 Hz, 4H), 2.39 (br s, 2H), 2.18 - 2.02 (m, 4H), 1.97 (br s, 2H), 1.55 (s, 18H), 1.42 (br t, *J* = 6.0 Hz, 2H), 0.32 - 0.11 (m, 4H)

Step 2: 2-di-tert-butylphosphine-2',4',6'-triisopropylbiphenyl (53.6 mg, 0.13 mmol), potassium phosphate (410 mg, 1.89 mmol), and tris(dibenzylideneacetone) dipalladium (59.1 mg, 0.06 mmol) were successively added to a solution of tert-butyl N-[6-(4-bromo-2-{spiro[2.5]oct-5-en-6-yl}benzoylamino)-4-(4,4-difluoropiperidin-1-yl)-5-fluoro-1,3-benzothiazol-2-yl]-N-[(tert-butoxy)carbonyl]carbamate (500 mg, 0.63 mmol) and ethyl 2-sulfamoylpropyl ester (172 mg, 0.95 mmol) in dioxane (10 mL). The reaction solution was exchanged with nitrogen for three times, then heated to 80°C under nitrogen atmosphere and stirred for 4 h. The reaction solution was cooled to ambient temperature and extracted with water (50 mL) and ethyl acetate (80 mL × 3). The organic phases were combined and washed with saturated saline (50 mL), dried over anhydrous magnesium sulfate, filtered and concentrated under reduced pressure to give a residue. The residue was purified by flash column chromatography (silica gel, 0-18% gradient of THF/petroleum ether) to give ethyl 2-({4-[(2-{bis[(tert-butoxy)carbonyl]amino}-4-(4,4-difluoropiperidin-1-yl)-5-fluoro-1, 3-benzothiazol-6-yl)aminocarbonyl]-3-{spiro[2.5]oct-5-en-6-yl}phenyl}sulfamoyl)pr opyl ester (400 mg, 0.45 mmol, yield: 71%) as a yellow solid. LCMS (ESI): [M+H]+ = 892.2

Step 3: Trifluoroacetic acid (2 mL) was added to a solution of ethyl 2-({4-[(2-{bis[(tert-butoxy)carbonyl]amino}-4-(4,4-difluoropiperidin-1-yl)-5-fluoro-1, 3-benzothiazol-6-yl)aminocarbonyl]-3-{spiro[2.5]oct-5-en-6-yl}phenyl}sulfamoyl)pr opyl ester (0.40 g, 0.45 mmol) in dichloromethane (10 mL). The mixture was stirred at 25°C for 2 h. The reaction solution was poured into water (40 mL), the pH was adjusted to 7 by adding 10% aqueous sodium bicarbonate solution (50 mL), and extracted with ethyl acetate (100 mL×3). The combined organic layer was dried over anhydrous magnesium sulfate, filtered and concentrated under reduced pressure to give ethyl 2-(N-(4-((2-amino-4-(4,4-difluoropiperidin-1-yl)-5-fluorobenzo[d]thiazol-6-yl)amino carbonyl)-3-(spiro[2.5]oct-5-en-6-yl)phenyl)sulfamoyl)propyl ester (0.37 g, 0.43 mmol, yield: 95%) as a yellow crude solid. LCMS (ESI): [M+H]+ = 692.2

Step 4: Ethyl 2-(N-(4-((2-amino-4-(4,4-difluoropiperidin-1-yl)-5-fluorobenzo[d]thiazol-6-yl)amino carbonyl)-3-(spiro[2.5]oct-5-en-6-yl)phenyl)sulfamoyl)propyl ester (350 mg, 0.51 mmol) was added to a solution of THF (8 mL), lithium tetrahydridoaluminate (36.1 mg, 1.01 mmol) was added portionwise at 0°C, and The mixture was stirred at 0-25°C for 1 h. The reaction solution was quenched with water (0.05 mL) and 10% aqueous sodium hydroxide solution (0.1 mL), then filtered and concentrated under reduced pressure to give a residue. The residue was purified by preparative HPLC (C18, 24-64% gradient of water (liquor ammonia and ammonium bicarbonate)/acetonitrile) to give N-(2-amino-4-(4,4-difluoropiperidin-1-yl)-5-fluorobenzo[d]thiazol-6-yl)-4-((2-hydrox y-1-methylethyl)sulfonamido)-2-(spiro[2.5] oct-5-en-6-yl) benzamide (60 mg, 0.09 mmol, yield: 18%) as a white solid compound. LCMS (ESI): [M+H]+ = 650.1; $^1$H NMR (400 MHz, DMSO-d6) δ ppm 10.18 - 9.80 (m, 1H), 9.52 (s, 1H), 7.70 (d, *J* = 6.8 Hz, 1H), 7.55 (s, 2H), 7.45 (d, *J* = 8.4 z, 1H), 7.19 (dd, *J* = 2.0, 8.4 Hz, 1H), 7.09 (d, *J* = 2.0 Hz, 1H), 5.75 (br s, 1H), 5.25 - 4.74 (m, 1H), 3.85 (dd, *J* = 4.4, 11.2 Hz, 1H), 3.46 (dd, *J* = 8.0, 10.8 Hz, 1H), 3.39 - 3.34 (m, 4H), 3.20 (dt, *J* = 4.4, 7.2 Hz, 1H), 2.33 (br s, 2H), 2.18 - 2.03 (m, 4H), 1.99 (br s, 2H), 1.42 (br t, *J* = 5.6 Hz, 2H), 1.29 (d, *J* = 6.8 Hz, 3H), 0.37 - 0.17 (m, 4H)

**Example 102: 2-{6-Azaspiro[2.5]octan-6-yl}-N-[8-((3R,5R)-3,5-dimethylmorpholino)-1,7-napht hyridin-6-yl]-4-[1-hydroxypropane-2-sulfonamido]benzamide**

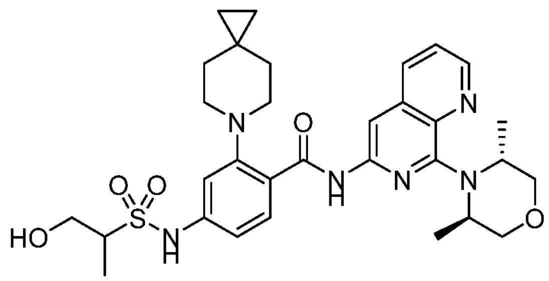

The preparations in **Example 102** follow the synthetic route of **Example 94:** LCMS (ESI): [M+H]+ = 609.3; $^1$H NMR (400 MHz, DMSO) δ 12.97 (s, 1H), 10.21 (s, 1H), 8.77 (dd, J = 4.1, 1.7 Hz, 1H), 8.34-8.24 (m, 2H), 8.10 (d, J = 8.6 Hz, 1H), 7.64 (dd, J = 8.4, 4.1 Hz, 1H), 7.31 (d, J = 2.2 Hz, 1H), 7.17 (dd, J = 8.6, 2.2 Hz, 1H), 5.02 (s, 1H), 4.44 (s, 2H), 3.96 (dd, J = 11.1, 3.4 Hz, 2H), 3.85 (dd, J = 11.3, 4.3 Hz, 1H), 3.62-3.46 (m, 3H), 3.29 (t, J = 7.9 Hz, 1H), 3.02 (d, J = 29.5 Hz, 4H), 2.11-1.38 (m, 4H), 1.30 (dd, J = 6.8, 1.7 Hz, 3H), 0.98 (d, J = 6.2 Hz, 6H), 0.42 (s, 4H).

**Effect Example 1: Enzyme Activity Test**

**Materials:** Human KIF18A (amino acid sequence 1-417) was purchased from Viva Biotech (Shanghai) Ltd.; the ADP-Glo™ protein kinase kit was acquired from Promega, USA; tubulin was sourced from Cytoskeleton, USA; and the 384-well assay plate and Envision multifunctional microplate reader were obtained from PerkinElmer, USA.

**Enzymatic Activity Test:** Dissolve the compound powder in DMSO to prepare a stock solution of 10 mM. Then perform gradient dilution for the compounds in the microplate to achieve a final concentration of 0-10 μM. Then, add 2.5 μL

each of tubulin, compound, ATP, and KIF18A protein to the microplate in sequence for reaction at ambient temperature for 120 min. The final concentrations in the enzyme reaction are 60 μg/mL for microtubules, 25 μM for ATP, and 2.5 nM for KIF18A protein. After the enzyme reaction, add 10 μL of ADP-GLO reaction reagent to each well and incubate at ambient temperature for 30 min. After that, add 20 μL of detection reagent to each well and incubate at ambient temperature for 30 min in the dark. Finally, perform chemiluminescence detection using the PerkinElmer Envision.

**Table 1: Enzymatic Activity Biological Data for Compounds of the Present Invention**

| Example | KIF18A_nM | Example | KIF18A_nM | Example | KIF18A_nM |
|---|---|---|---|---|---|
| 1 | 190 | 35 | 29 | 69 | 1034 |
| 2 | 251 | 36 | 39 | 70 | 43 |
| 3 | 68 | 37 | 121 | 71 | 128 |
| 4 | 29 | 38 | 131 | 72 | >3000 |
| 5 | 28 | 39 | 38 | 73 | >3000 |
| 6 | 241 | 40 | 32 | 74 | >3000 |
| 7 | 100 | 41 | 38 | 75 | 387 |
| 8 | 280 | 42 | 213 | 76 | 46 |
| 9 | 49 | 43 | 28.7 | 77 | 848 |
| 10 | 2755 | 44 | 105 | 78 | 193 |
| 11 | 191 | 45 | 28 | 79 | 943 |
| 12 | 80 | 46 | 39 | 80 | 9 |
| 13 | 27 | 47 | 39 | 81 | 35 |
| 14 | 32 | 48 | 108 | 82 | 33 |
| 15 | 205 | 49 | 1220 | 83 | 27 |
| 16 | 29 | 50 | 1490 | 84 | 40 |
| 17 | 28 | 51 | 455 | 85 | >3000 |
| 18 | 12 | 52 | 149 | 86 | 223 |
| 19 | 13 | 53 | 94 | 87 | 10 |
| 20 | 218 | 54 | 27 | 88 | 41 |
| 21 | 38 | 55 | 250 | 89 | 179 |
| 22 | 1936 | 56 | 34 | 90 | 341 |
| 23 | 42 | 57 | 77 | 91 | 173 |
| 24 | 7 | 58 | 32 | 92 | 2258 |
| 25 | 5 | 59 | 38 | 93 | >3000 |
| 26 | 31 | 60 | 66 | 94 | 255 |
| 27 | 13 | 61 | 15 | 95 | 242 |
| 28 | 46 | 62 | 28 | 96 | 153 |
| 29 | 28 | 63 | 35 | 97 | 153 |
| 30 | 27 | 64 | 116 | 98 | 166 |
| 31 | 71 | 65 | 232 | 99 | 174 |
| 32 | 8 | 66 | 23 | 100 | 24 |
| 33 | 1637 | 67 | 53 | 101 | 139 |
| 34 | 63 | 68 | 306 | 102 | 150 |

## Example 2: Cell Proliferation Activity Test

**Materials and Cells:** OVCAR3 cells were purchased from Nanjing Cobioer Biosciences Co., Ltd. RPMI-1640 medium, fetal bovine serum, and the CyQUANT Direct Cell Proliferation Assay kit were purchased from Thermo Fisher Scientific, USA; 96-well cell culture plates were sourced from Coming, USA.

**Cell Culture:** Culture OVCAR3 cells with RPMI-1640 medium containing 10% fetal bovine serum and incubate them in a 5% $CO_2$ incubator at 37°C. Only cells in the logarithmic growth phase can be used for the test.

### Cell Proliferation Activity Test:

Inoculate OVCAR3 cells into a 96-well cell culture plate at 90 μL per well and incubate overnight in a 5% $CO_2$ incubator at 37°C. Dissolve the compound powder in DMSO to prepare a stock solution of 10 mM. Then perform gradient dilution for the compounds in the microplate to achieve a final concentration of 0-10 μM. Add 10 μL of cell culture medium containing compounds to each well to make the final DMSO content 0.2%. Incubate the cell plates in a 5% $CO_2$ incubator at 37°C for 3 days. Add 100 μL of CyQUANT detection reagent to each well, react at 37°C for 60 min, and perform fluorescence detection using the PerkinElmer Envision.

**Table 2: OVCAR-3 Cell Viability Biological Data for Compounds of the Present Invention**

| Example | OVCAR-3 _IC50_nM | Example | OVCAR-3 _IC50_nM | Example | OVCAR-3 _IC50_nM |
|---|---|---|---|---|---|
| 1 | 1027 | 34 | 166 | 67 | 7 |
| 2 | 899 | 35 | 27 | 68 | 119 |
| 3 | 566 | 36 | 64 | 69 | 1038 |
| 4 | 1348 | 37 | 121 | 70 | 9 |
| 5 | 6 | 38 | NT | 71 | 18 |
| 6 | 1485 | 39 | 15 | 72 | >3000 |
| 7 | >10000 | 40 | 12 | 73 | 1028 |
| 8 | >3000 | 41 | 4 | 74 | 1526 |
| 9 | 55 | 42 | 7 | 75 | 83 |
| 10 | 614 | 43 | 7 | 76 | 34 |
| 11 | 28 | 44 | 21 | 77 | 745 |
| 12 | 80 | 45 | 18 | 78 | 12 |
| 13 | 9 | 46 | 12 | 79 | 47 |
| 14 | 9 | 47 | 48 | 80 | 18 |
| 15 | 71 | 48 | 236 | 81 | 18 |
| 16 | 33 | 49 | 1342 | 82 | 13 |
| 17 | 20 | 50 | NT | 83 | 15 |
| 18 | 16 | 51 | 385 | 84 | 11 |
| 19 | 18 | 52 | 83 | 86 | 34 |
| 20 | 18 | 53 | 4 | 87 | >3000 |
| 21 | 358 | 54 | 6 | 88 | 154 |
| 22 | NT | 55 | 112 | 89 | 32 |
| 23 | >10000 | 56 | 12 | 90 | 96 |
| 24 | 20 | 57 | NT | 91 | 13 |
| 25 | 46 | 58 | 12 | 92 | NT |
| 26 | 592 | 59 | 14 | 93 | NT |
| 27 | 23 | 60 | 18 | 94 | 5.9 |

(continued)

| Example | OVCAR-3 _IC50_nM | Example | OVCAR-3 _IC50_nM | Example | OVCAR-3 _IC50_nM |
|---|---|---|---|---|---|
| 28 | 26 | 61 | 74 | 95 | 4.7 |
| 29 | 13 | 62 | 13 | 96 | 11.3 |
| 30 | 7 | 63 | 11 | 97 | 13.9 |
| 31 | 8 | 64 | 76 | 98 | 17 |
| 32 | 347 | 65 | 420 | 99 | 17 |
| 33 | NT | 66 | 35 | 100 | 11 |
| NT means no test | | | | | |

**Example 3:** CyQuant Cell Proliferation Activity Test

**Materials and Cells:** HT29 cells were purchased from Nanjing Cobioer Biosciences Co., Ltd.; RPMI-1640 medium, fetal bovine serum, Trypsin-EDTA (0.25%), 96-well plates, and CyQuant reagents were purchased from ThermoFisher (USA); DMSO was purchased from SIGMA (USA).

**Cell Culture:** Culture HT29 cells with RPMI-1640 containing 10% fetal bovine serum under the condition of 37°C and 5% $CO_2$. Only cells in the logarithmic growth phase can be used for the test.

**Cell Proliferation Activity Test:** Use the CyQuant reagent to detect the proliferation inhibitory activity of compounds on HT29 cells. Adjust the cell density and inoculate 100 μL per well into the 96-well plate (HT29 at 2000 cells per well), then incubate overnight under the condition of 37°C and 5% $CO_2$. Add compounds of various target concentrations (initial concentration of 3000 nM, 3-fold dilution, 9 concentration gradients), and make the DMSO content 0.2%. Incubate the cell plates at 37°C and 5% $CO_2$ for 3 days. Incubate with CyQuant reagent for 1 h, read the plate by Envision, and calculate $IC_{50}$ using XLFIT.

**Table 3: HT-29 Cell Viability Biological Data for Compounds of the Present Invention**

| Example | HT29 _IC50_nM | Example | HT29 _IC50_nM | Example | HT29 _IC50_nM |
|---|---|---|---|---|---|
| 1 | NT | 36 | 62 | 71 | 17 |
| 2 | NT | 37 | 25 | 72 | >3000 |
| 3 | NT | 38 | 126 | 73 | 1411 |
| 4 | 2016 | 39 | 18 | 74 | 1793 |
| 5 | 7 | 40 | 10 | 75 | 44 |
| 6 | NT | 41 | 4 | 76 | 31 |
| 7 | >10,000 | 42 | 17 | 77 | NT |
| 8 | >3000 | 43 | 17 | 78 | 11 |
| 9 | 37 | 44 | 26 | 79 | 55 |
| 10 | 1267 | 45 | 31 | 80 | 20 |
| 11 | 28 | 46 | 14 | 81 | 8 |
| 12 | 102 | 47 | 40 | 82 | 14 |
| 13 | 13 | 48 | 360 | 83 | NT |
| 14 | 17 | 49 | 1426 | 84 | 25 |
| 15 | 97 | 50 | NT | 85 | NT |
| 16 | 56 | 51 | 387 | 86 | 56 |
| 17 | 30 | 52 | 92 | 87 | >3,000 |
| 18 | 35 | 53 | 6 | 88 | 425 |
| 19 | 28 | 54 | 18 | 89 | 32 |

(continued)

| Example | HT29 _IC50_nM | Example | HT29 _IC50_nM | Example | HT29 _IC50_nM |
|---|---|---|---|---|---|
| 20 | 23 | 55 | 89 | 90 | 106 |
| 21 | NT | 56 | 13 | 91 | 16 |
| 22 | NT | 57 | NT | 92 | NT |
| 23 | NT | 58 | 17 | 93 | NT |
| 24 | 26 | 59 | 22 | 94 | NT |
| 25 | 84 | 60 | 13 | 95 | NT |
| 26 | >3000 | 61 | 111 | 96 | 17 |
| 27 | 33 | 62 | 12 | 97 | 17 |
| 28 | 9 | 63 | 15 | 98 | 21 |
| 29 | 11 | 64 | 82 | 99 | 22 |
| 30 | 10 | 65 | 372 | 100 | 12 |
| 31 | 9 | 66 | 80 | | |
| 32 | 927 | 67 | 13 | | |
| 33 | NT | 68 | 100 | | |
| 34 | 446 | 69 | 1106 | | |
| 35 | 57 | 70 | 16 | | |
| NT means no test | | | | | |

**Example 4:** Experimental testing of cytotoxicity in human primary hepatocytes.

**Materials:** One donor for cytotoxicity testing. Information on human primary hepatocytes is provided in the table below.

| Donor | Race | Source |
|---|---|---|
| 1 | Caucasian | BioIVT |

Note: Hepatocytes of other races may be used, and specific information will be reflected in the test report.

1) CellTiter-Glo Luminescent Cell Viability Assay System was purchased from Promega (Madison, WI).

**Instruments:**

| Instruments | Supplier | Cat# or Model |
|---|---|---|
| 96-well plate | Corning | 354407 |
| Centrifuge | Eppendorf | 5804R&5810R |
| $CO_2$ incubator | Thermo Scientific | 371 |
| Cell counter | Nexcelom Bioscience LLC | Cellmeter K2 |
| Oscillator | IKA | MS3 BASIC |
| Microplate reader | TECAN | INFINITE 200 PRO |

**Experimental Design:**

Warm the following media to 37°C in a water bath: Prepare hepatocyte recovery medium, inoculation medium, and incubation medium according to the table below.

**Recovery Medium**

| Reagent | Initial Concentration | Final Concentration | Volume |
|---|---|---|---|
| Williams' Medium E | - | - | 31.2 mL |
| Isotonic Percoll | - | 30% | 13.5 mL |
| DPBS (10×) | - | - | 1.5 mL |
| GlutaMAX™-1 (100×) | 200 mM/100× | 2 mM | 500 μL |
| HEPES | 1 M | 15 mM | 750 μL |
| FBS | - | 5% | 2.5 mL |
| Human recombination insulin | 4 mg/mL | 4 μg/mL | 50 μL |
| Dexamethasone | 10 mM | 1 μM | 5 μL |

**Inoculation Medium**

| Reagent | Initial Concentration | Final Concentration | Volume |
|---|---|---|---|
| Williams' Medium E | - | - | 45.7 mL |
| FBS | - | 5% | 2.5 mL |
| Dexamethasone | 10 mM | 1 μM | 5 μL |
| Penicillin-streptomycin mixed solution | Penicillin: 10,000 u/mL<br>Streptomycin: 10,000 μg/mL | Penicillin: 100 u/mL<br>Streptomycin: 100 μg/mL | 500 μL |
| Human recombination insulin | 4 mg/mL | 4 μg/mL | 50 μL |
| GlutaMAX™-1 (100×) | 200 mM/100× | 2 mM | 500 μL |
| HEPES | 1 M | 15 mM | 750 μL |

**Incubation Medium**

| Reagent | Initial Concentration | Final Concentration | Volume |
|---|---|---|---|
| Williams' Medium E | | | 48 mL |
| Dexamethasone | 10mM | 0.1 μM | 0.5 μL |
| ITS (100×) | | | 500 μL |
| Penicillin-streptomycin mixed solution | Penicillin: 10,000 u/mL<br>Streptomycin: 10,000 μg/mL | Penicillin: 50 u/mL<br>Streptomycin: 50 μg/mL | 250 μL |
| GlutaMAX™-1 (100×) | 200 mM/100× | 2 mM | 500 μL |

(continued)

| Reagent | Initial Concentration | Final Concentration | Volume |
|---|---|---|---|
| HEPES | 1 M | 15 mM | 750 μL |

**1)** Take one tube of cryopreserved hepatocytes and ensure that the hepatocytes are cryogenically frozen until recovery. Quickly place the hepatocytes in a 37°C water bath and gently shake until all ice crystals are completely dispersed, spray with 75% ethanol and transfer to a biosafety cabinet.
**2)** Transfer the contents of the hepatocyte tube (1 mL, approximately $5 \times 10^6$ cells) into a 50 mL centrifuge tube containing 50 mL of recovery medium and centrifuge at 100 g for 10 min. After centrifugation, aspirate the recovery medium and add sufficient inoculation medium to obtain a cell suspension with a cell density of approximately $1.0 \times 10^6$ cells/mL. Count hepatocytes and determine viable cell density with the cell counter Cellometer®. Make sure that the viability of hepatocytes is greater than 80%. Adjust the cell density to $0.2 \times 10^6$ cells/mL with inoculation medium, and inoculate into 96-well plates coated with collagen I at 100 μL per well. Incubate the plates at 37°C for 4-6 h in a 5% $CO_2$ incubator with 95% relative humidity.
**3)** Prepare a 200X stock solution of the test compound in DMSO (the stock concentration of the test compound can be reduced depending on solubility). Make the final concentration of DMSO 0.5%
**4)** Prepare the working solutions on sterile 96-well plates by adding 2.5 μL of the test compound stock solution to 497.5 μL of hepatocyte culture medium.
**5)** Remove the hepatocyte culture medium from the cell plate, and add 125 μL of working solution to the corresponding wells in triplicate. Incubate the plate at 37°C and 5% $CO_2$ for 72 h. After every 24 h of treatment, replace the medium in cell plates with freshly diluted test and positive control compounds from hepatocyte culture media. Return the plate to the incubator, and add 50 μL of CellTiter-Glo reagent directly into each well of the 96-well plate. Shake on a shaker for 10 min at ambient temperature. After 10 min, transfer 100 μL of the above incubation to a new white and opaque flat-bottom 96-well plate and record the fluorescence.

**Data Analysis:**

All calculations are performed using Microsoft Excel.

The viability of the test compound can be calculated using the following formula:

$$\%\,\text{Vehicle} = \frac{\text{Read}_{\text{Compound}} - \text{Read}_{\text{Blank}}}{\text{Read}_{\text{Vehicle}} - \text{Read}_{\text{Blank}}} \times 100\%$$

The % Vehicle is fitted to the concentration of the test compound, and then GraphPad Prism 5.0 is used to model the data as a sigmoidal dose-response curve with a variable slope. The $IC_{50}$ of this compound is calculated from the curve using the following formula

$$Y=Bottom + (Top-Bottom)/(1+10^\wedge((LogIC50-X)*HillSlope))$$

**Example 5:** Experimental testing of the microsphere model for human primary hepatocytes

**Cells:** Donor information: Male, Caucasian, 36 years old (Lot No. NFX, BioIVT)

**Instruments:**

| Instruments | Brand | Cat. No./Model |
|---|---|---|
| 96-well ultra-low adsorption culture plate | Corning | 4515 |
| Centrifuge | Eppendorf | 5804R |
| Cell incubator | Thermo Scientific | 371 |
| Cell counter | Invitrogen | Countess II |
| Oscillator | IKA | MS 3 |

(continued)

| Instruments | Brand | Cat. No./Model |
|---|---|---|
| Microplate reader | Tecan | Infinite M200 |

**Cell Inoculation and Culture:**

1) Take one tube of cryopreserved hepatocytes and ensure that the hepatocytes are cryogenically frozen until recovery. Quickly place the hepatocytes in a 37°C water bath and gently shake until all ice crystals are completely dispersed, spray with 70% ethanol and transfer to a biosafety cabinet.

2) Transfer the contents of the hepatocyte tube into a 50 mL centrifuge tube containing 50 mL of recovery medium and centrifuge at 100 g for 3 min. After centrifugation, aspirate the recovery medium and add sufficient incubation medium to obtain a cell suspension with a cell density of approximately $1.0 \times 10^6$ cells/mL.

3) Count hepatocytes and determine viable cell density with Cellometer Vision. Make sure that the viability of hepatocytes is greater than 80%. Adjust the cell density to 4000 cells/well with inoculation medium, and inoculate 100 μL per well.

4) Incubate in a cell incubator at 37°C for 7-9 days until microspheres are formed.

**Compound Preparation and Administration:**

1) Dilute the compound with the cell culture medium according to the three-fold dilution method, and the dilution gradient is shown in the table below. The maximum concentration of the control compound is 200 μM, the maximum concentration of the test compound (according to customer requirement) is 5 μM and the final concentration of DMSO is 0.1%.

2) Perform administration after the microspheres are formed. Aspirate the medium in the cell culture plate wells and add 100 μL of the compound working solution before each administration. Perform administration every three days until the fourteenth day after microsphere formation.

**Test Indicator Detection:**

1) After 14 days of culture, remove the cell culture plates from the incubator and transfer 80 μL of supernatant to determine the albumin and lactate dehydrogenase content.

2) Thaw CellTiter-Fluor™ Cell Viability Kit at ambient temperature, add 10 μL of GF-AFC substrate to 10 mL of 2× working solution, and dilute into 1× working solution with an equal volume of PBS.

3) Add 100 μL of working solution to each well of the cell culture plate and incubate at 37°C for 30 min, then pipette 80 μL of supernatant into a 96-well black plate and detect the absorbance at an excitation wavelength of 400 nm and an emission wavelength of 505 nm.

**Data Analysis:**

1) The cell viability (% Vehicle) is calculated using the following formula:

$$\% \text{ Vehicle} = \left( \frac{\text{Read}_{\text{Compound}} - \text{Read}_{\text{Blank}}}{\text{Read}_{\text{Vehicle}} - \text{Read}_{\text{Blank}}} \right) \times 100\%$$

2) The $IC_{50}$ is calculated using GraphPad Prism 8.0.2 with the following software calculation formula:

$$Y=Bottom + (Top-Bottom)/(1+10^{\wedge}((LogIC_{50}-X)*HillSlope))$$

## Claims

1. A compound having the structure of formula (I), or its pharmaceutically acceptable salts, stereoisomers, isotope isomers, prodrugs, hydrates, or solvates:

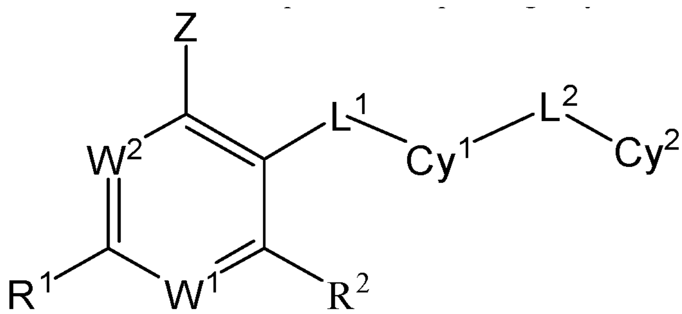

Formula (I)

wherein, $W^1$ represents $CR^{W1}$ or N;
wherein, $W^2$ represents $CR^{W2}$ or N;
wherein, Z represents $-CR^TR^{T'}$ or $-NR^SR^{S'}$;
wherein, $R^T$ and $R^{T'}$, together with the C atom they are attached to, form a ring having a structure selected from the following:

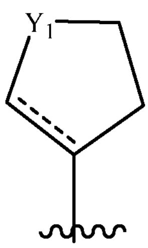 , 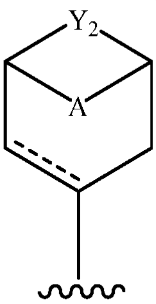 and/or 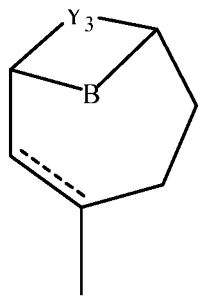 ;

wherein, $R^S$ and $R^{S'}$, together with the N atom they are attached to, form a ring having a structure selected from the following:

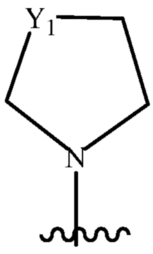, 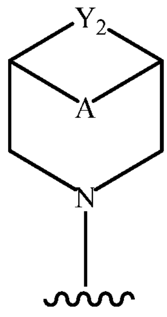 and/or 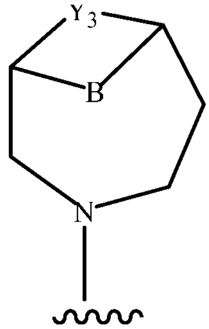

wherein, $Y^1$, $Y^2$ and $Y^3$ each independently represent $-(CR^aR^b)_o-(NR^a)_p-(CR^{a'}R^{b'})_q-$;
wherein, A and B each independently represent $-(CR^aR^b)m-$;
Wherein $L^1$ represents -C(O)NH-, -HNC(O)-, 5-6 membered heteroaryl,

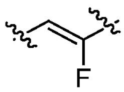, 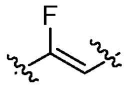, 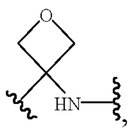, 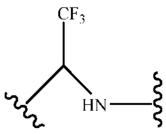, 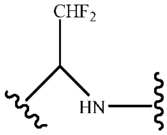, 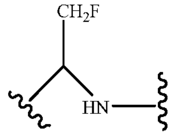;

wherein, $L^2$ represents absence, $-C_1-C_6$ alkylene-, $-NR^a-$, $-NR^a(C_1-C_6$ alkylene)-, $-C(O)NR^a(C_1-C_6$ alkylene)-, -O-, $-O-(C_1-C_6$ alkylene), -S-, -S(O)-, $-S(O)_2-$, $-S(O)_2NR^a-$ or $-S(O)(NR^a)-$;
wherein, $R^1$ represents $L^3-R^3$;
wherein, $R^2$ represents hydrogen, halogen, cyano, nitro, hydroxy $C_1-C_6$ alkyl, $C_1-C_6$ alkyl, $C_3-C_8$ cycloalkyl, $C_1-C_6$ haloalkyl, $-OR^a$, $-SO_3R^a$, $-SR^a$, $-SF_5$, $-S(O)R^a$, -O- $C_1-C_6$ haloalkyl or $-NR^aR^b$;
wherein, $L^3$ represents absence, $C_1-C_6$ alkylene, $-NR^a-$, $-NR^aSO_2-$, $-SO_2NR^a-$, $-S(=O)(NR^a)-$, $-P(O)(OR^a)_2$, $-NR^aP(O)(OR^a)_2$ or $-NR^aP(O)(R^a)_2$;
wherein, $R^3$ represents absence, hydrogen, or $C_1-C_6$ alkyl or $C_3-C_6$ cycloalkyl substituted with 0-3 substituents selected from halogen, $-OR^a$, $-NR^aR^b$, cyano and -O- $C_1-C_6$ haloalkyl;
wherein, $R^{W1}$ and $R^{W2}$ each independently represent hydrogen, halogen, cyano, nitro, hydroxy $C_1-C_6$ alkyl, $C_1-C_6$ alkyl, $C_3-C_8$ cycloalkyl, $C_1-C_6$ haloalkyl, $-OR^a$, $-SO_3R^a$, $-S(O)R^a$, -O- $C_1-C_6$ haloalkyl, $-SR^a$, $-SF_5$ or $-NR^aR^b$;
Wherein $Cy^1$ represents groups with the following structure;

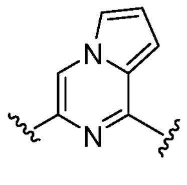 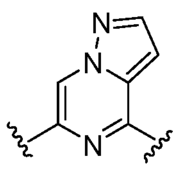 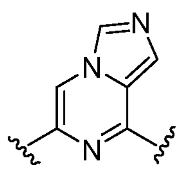 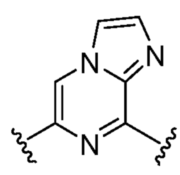

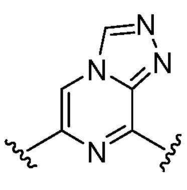 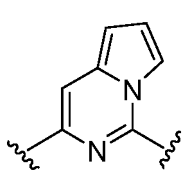 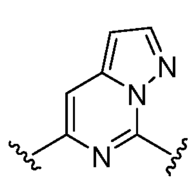 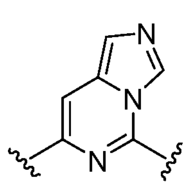

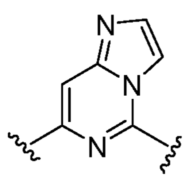 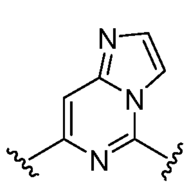 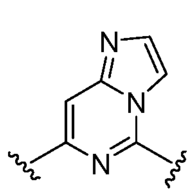 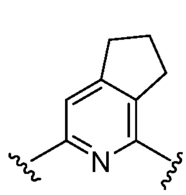

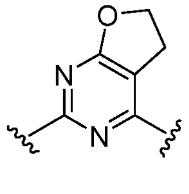 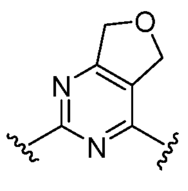 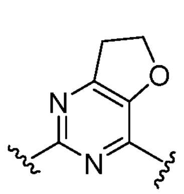 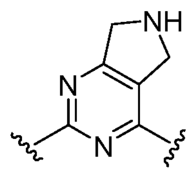

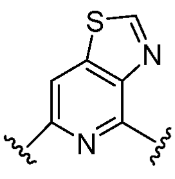 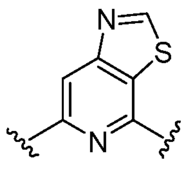 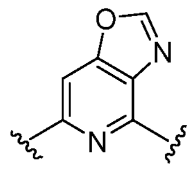 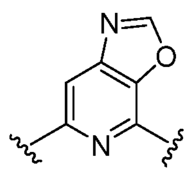

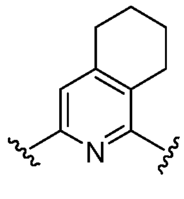 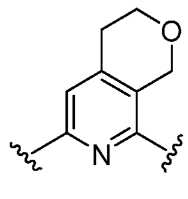 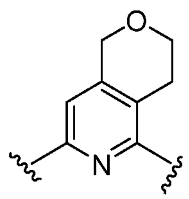 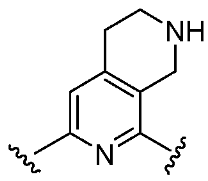

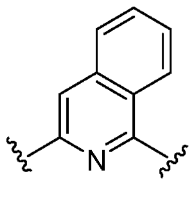 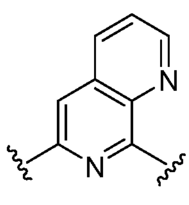 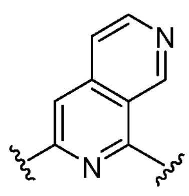 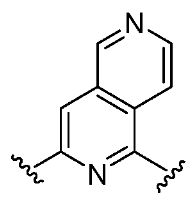

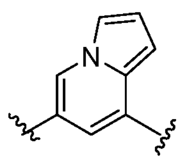 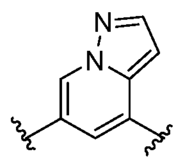 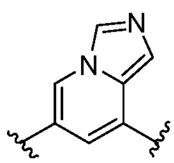 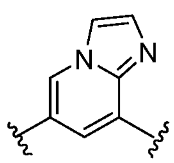

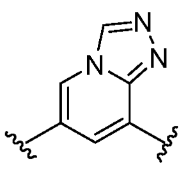 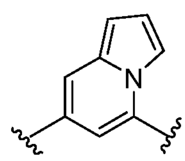 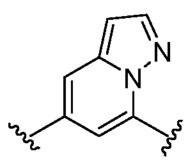 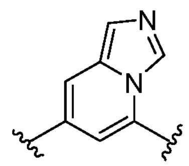

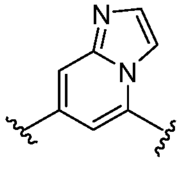 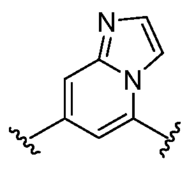 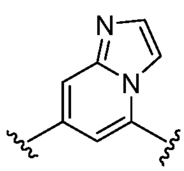 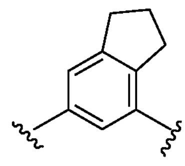

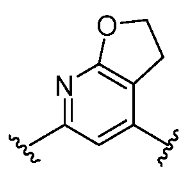 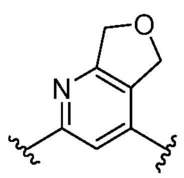 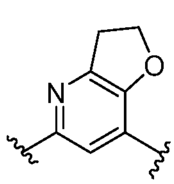 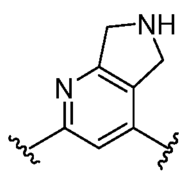

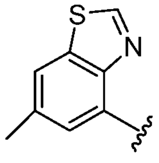 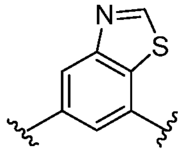 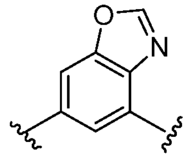 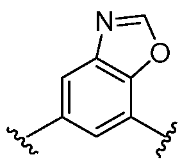

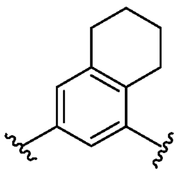 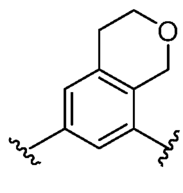 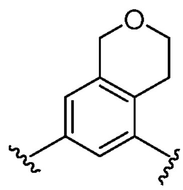 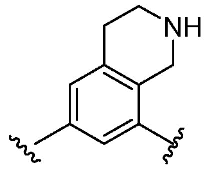

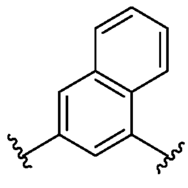 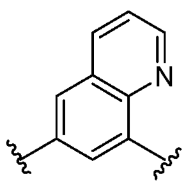 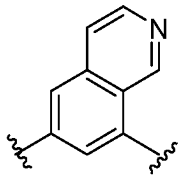 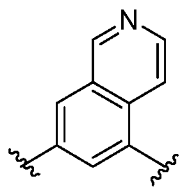

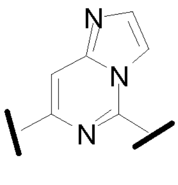 , 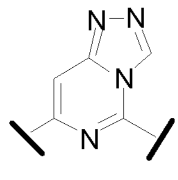 , 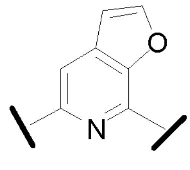 , 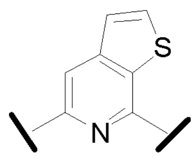

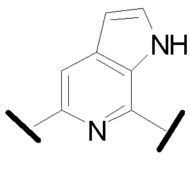 , 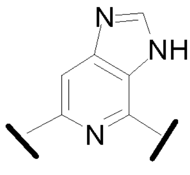 , 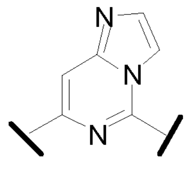 , 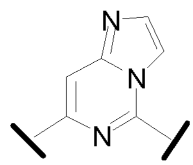

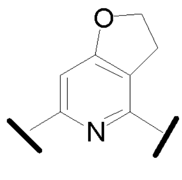 , 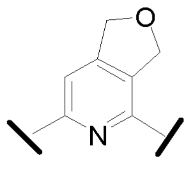 , 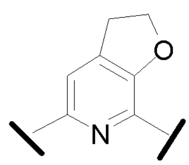 , 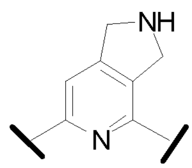

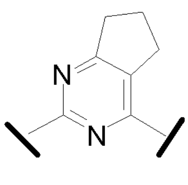 , 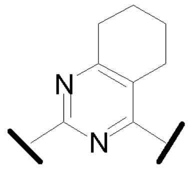 , 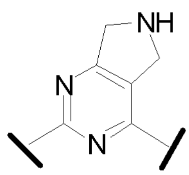 , 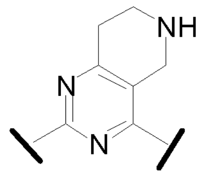

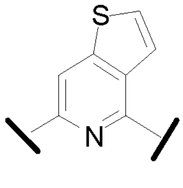 , 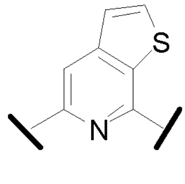 , 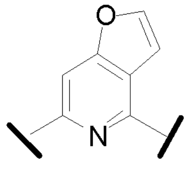 , 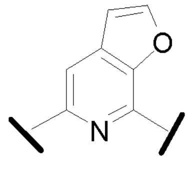

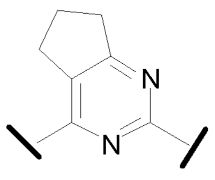,
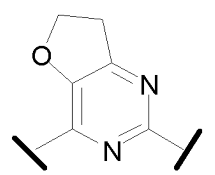,
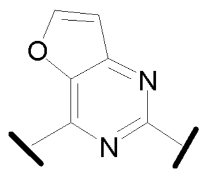,
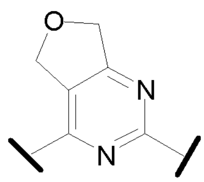

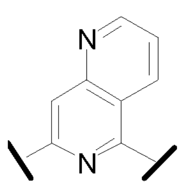,
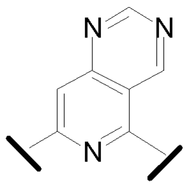,
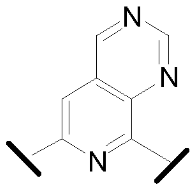
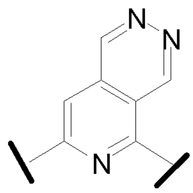

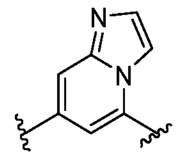
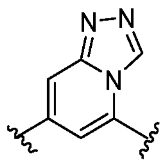
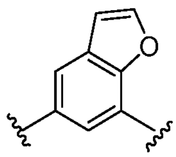
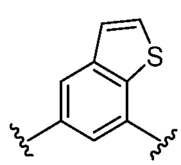

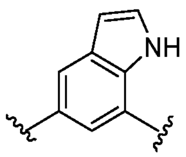
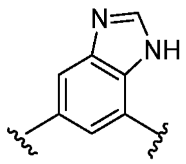
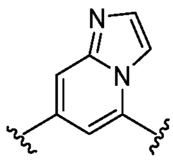
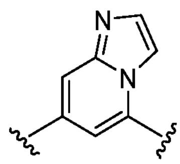

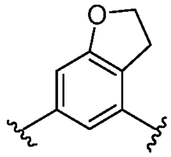
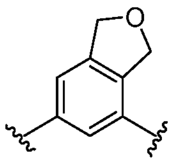
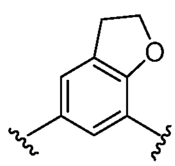
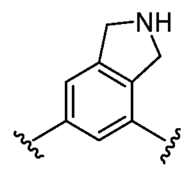

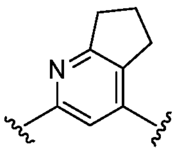
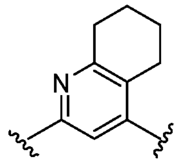
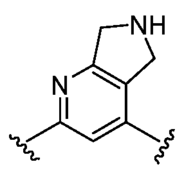
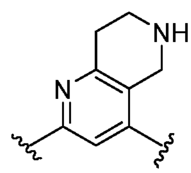

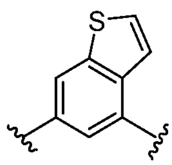
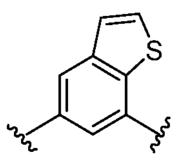
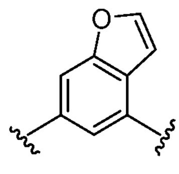
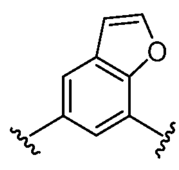

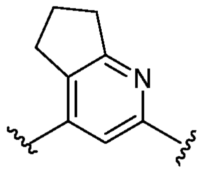
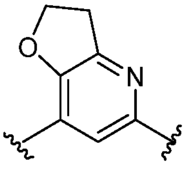
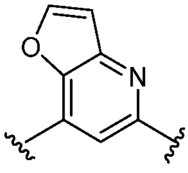
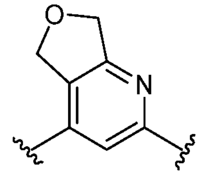

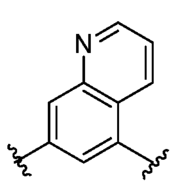
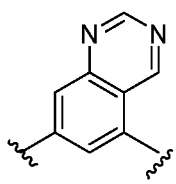
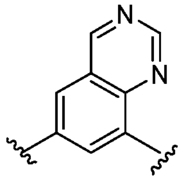
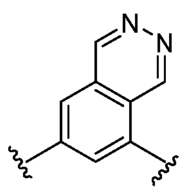
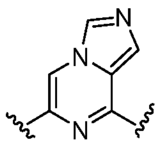
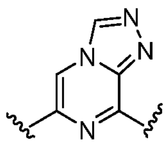
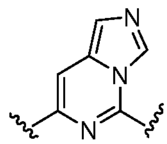
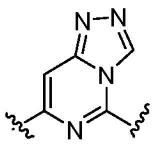
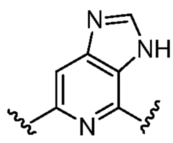
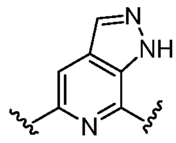
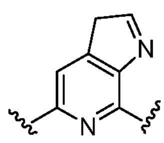
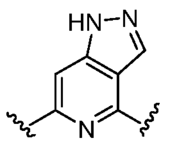
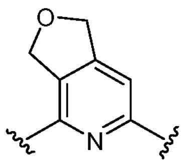
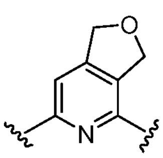
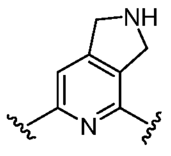
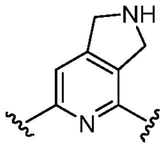
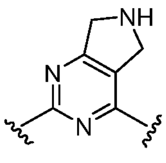
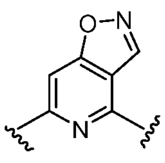
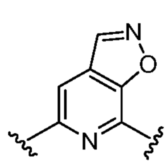
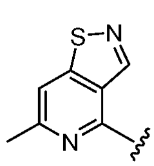
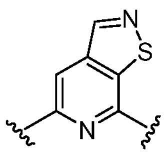
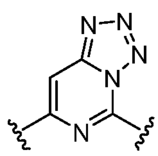
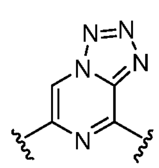
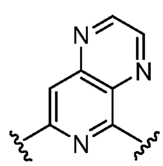
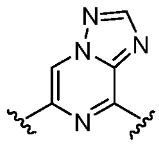
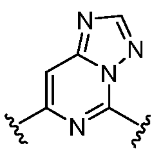
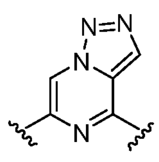
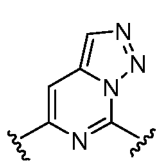
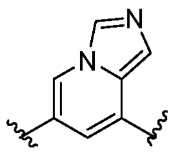
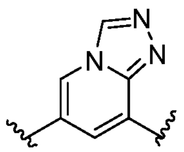
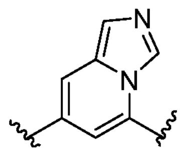
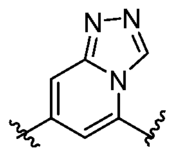
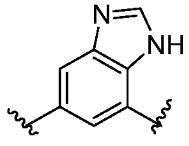
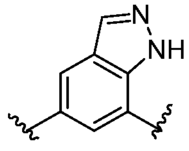
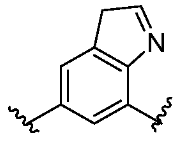
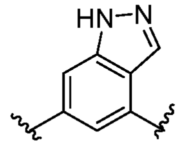

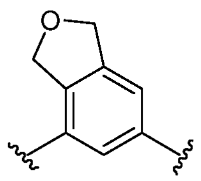 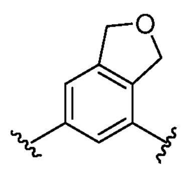 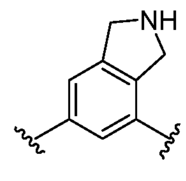 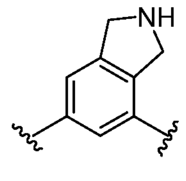

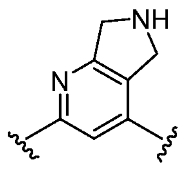 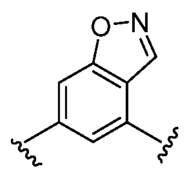 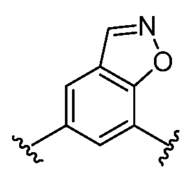 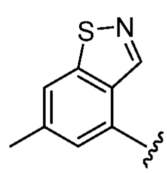

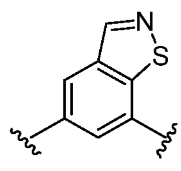 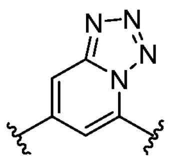 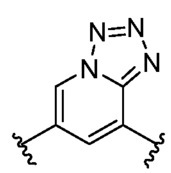 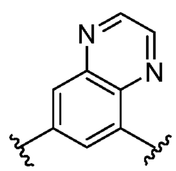

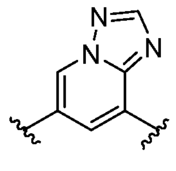 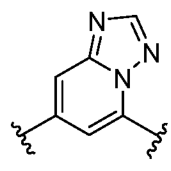 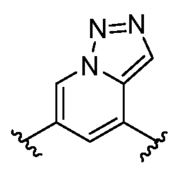 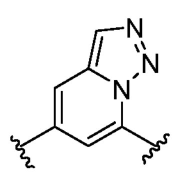

,

The $Cy^1$ may be optionally substituted with 0-3 substituents selected from the following: halogen, $C_1$-$C_6$ alkyl, $C_1$-$C_6$ haloalkyl, hydroxy $C_1$-$C_6$ alkyl, $OR^a$, -O-$C_1$-$C_6$ haloalkyl, 5-6-membered heteroaryl, phenyl, $-SR^a$, $-SF_5$, cyano, nitro, $-NR^aR^b$, $-NR^aC(O)R^b$, $-C(O)NR^aR^b$, $-OC(O)R^a$, $-C(O)R^a$, $-P(O)R^aR^b$, $-C(O)OR^a$, $-S(O)R^a$, $-S(O)_2R^a$, and $-S(O)_2NR^aR^b$;

wherein, $Cy^2$ represents a 3-12-membered saturated or unsaturated monocyclic or bicyclic ring which may optionally contain 0-3 heteroatoms selected from O, N and S; the $Cy^2$ may be optionally substituted with 0-3 substituents selected from the following: halogen, $C_1$-$C_6$ alkyl, $C_1$-$C_6$ haloalkyl, hydroxy $C_1$-$C_6$ alkyl, $-OR^a$, -O-$C_1$-$C_6$ haloalkyl, $-SR^a$, $-SF_5$, cyano, nitro, $-NR^aR^b$, $-NR^aC(O)R^b$, $-C(O)NR^aR^b$, $-OC(O)R^a$, $-C(O)OR^a$, $-S(O)R^a$, $-S(O)_2R^a$ and $-S(O)_2NR^aR^b$;

wherein, $R^a$, $R^b$, $R^{a'}$ and $R^{b'}$ each independently represent hydrogen, halogen,

$C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N; or $R^{a'}$ and $R^{b'}$, together with the atom they are attached to, form a 3-6-membered saturated or unsaturated ring which may optionally contain 0-2 heteroatoms selected from O, S and N;

wherein, the dashed line represents a single or double bond;

wherein, m, o, p and q represent integers from 0 to 3.

**2.** A compound according to claim 1 or its pharmaceutically acceptable salts, stereoisomers, isotope isomers, prodrugs, hydrates, or solvates, wherein $W^1$ represents CH or N.

**3.** A compound according to claim 1 or claim 2, or its pharmaceutically acceptable salts, stereoisomers, isotope isomers, prodrugs, hydrates, or solvates, wherein $W^2$ represents CH or N.

**4.** A compound according to any one of claims 1-3 or its pharmaceutically acceptable salts, stereoisomers, isotope isomers, prodrugs, hydrates, or solvates, wherein $L^3$ represents $-NR^aSO_2-$, $-SO_2NR^a-$ or $-S(=O)(NR^a)-$.

**5.** A compound according to any one of claims 1-4 or its pharmaceutically acceptable salts, stereoisomers, isotope isomers, prodrugs, hydrates, or solvates, wherein $L^3$ represents $-NR^aSO_2-$.

**6.** A compound according to any one of claims 1-5 or its pharmaceutically acceptable salts, stereoisomers, isotope

isomers, prodrugs, hydrates, or solvates, wherein $R^3$ represents hydrogen, or $C_1$-$C_6$ alkyl or $C_3$-$C_6$ cycloalkyl substituted with 0-3 substituents selected from halogen, -$OR^a$, -$NR^aR^b$, cyano and -O-halo $C_1$-$C_6$ alkyl.

7. A compound according to any one of claims 1-6 or its pharmaceutically acceptable salts, stereoisomers, isotope isomers, prodrugs, hydrates, or solvates, wherein $R^3$ represents $C_1$-$C_6$ alkyl substituted with 0-3 substituents selected from halogen, -$OR^a$, -$NR^aR^b$, cyano and -O-halo $C_1$-$C_6$ alkyl.

8. A compound according to any one of claims 1-7 or its pharmaceutically acceptable salts, stereoisomers, isotope isomers, prodrugs, hydrates, or solvates, wherein Z represents -$NR^SR^{S'}$.

9. A compound according to any one of claims 1-8 or its pharmaceutically acceptable salts, stereoisomers, isotope isomers, prodrugs, hydrates, or solvates, wherein Z represents:

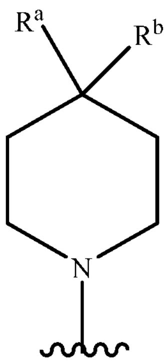 , 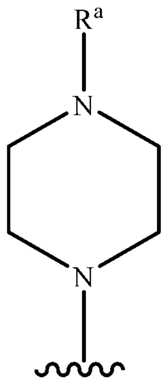 , 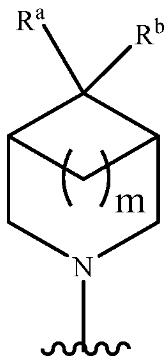 and/or 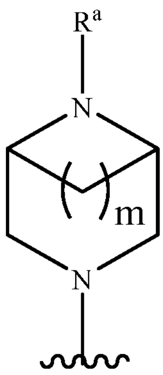 ;

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N.

10. A compound according to any one of claims 1-9 or its pharmaceutically acceptable salts, stereoisomers, isotope isomers, prodrugs, hydrates, or solvates, wherein, Z represents:

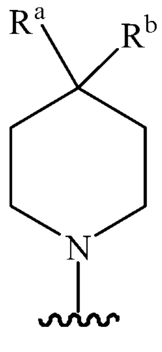

wherein, $R^a$ and $R^b$ each independently represent hydrogen, $C_1$-$C_6$ alkyl, $C_3$-$C_6$ cycloalkyl, $C_1$-$C_6$ haloalkyl or hydroxy $C_1$-$C_6$ alkyl; or $R^a$ and $R^b$, together with the atom they are attached to, form a 3-6-membered saturated or unsaturated ring which may optionally contain 0-2 heteroatoms selected from O, S and N.

11. A compound according to any one of claims 1-10 or its pharmaceutically acceptable salts, stereoisomers, isotope isomers, prodrugs, hydrates, or solvates, wherein Z represents:

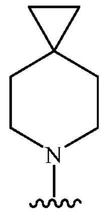 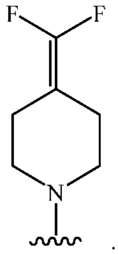.

**12.** A compound according to any one of claims 1-11 or its pharmaceutically acceptable salts, stereoisomers, isotope isomers, prodrugs, hydrates, or solvates, wherein $L^1$ represents: -C(O)NH-, -HNC(O)-, 5-6-membered heteroaryl,

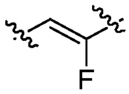,

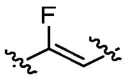, 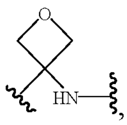, 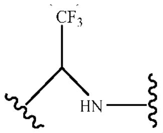, 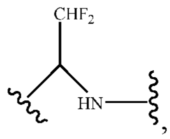, 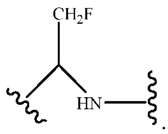.

**13.** A compound according to any one of claims 1-12 or its pharmaceutically acceptable salts, stereoisomers, isotope isomers, prodrugs, hydrates, or solvates, wherein $L^1$ represents -C(O)NH-.

**14.** A compound according to claim 13 or its pharmaceutically acceptable salts, stereoisomers, isotope isomers, prodrugs, hydrates, or solvates, wherein $Cy^1$ represents the following groups:

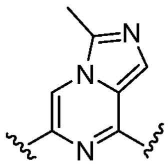 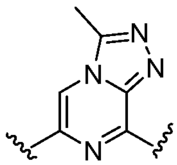 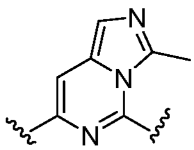 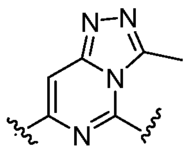

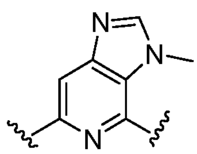 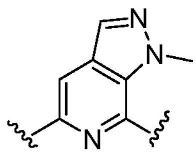 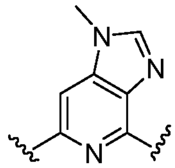 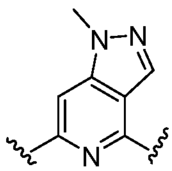

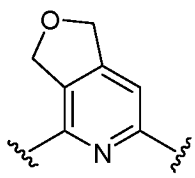 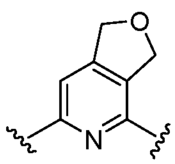 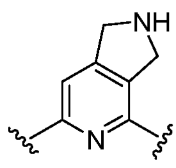 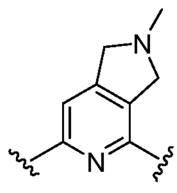

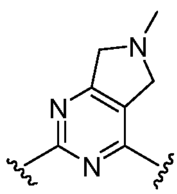 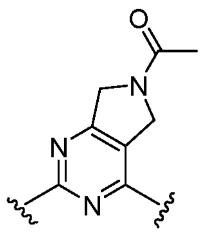 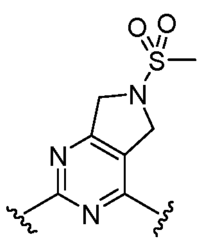 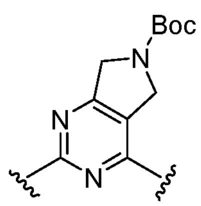

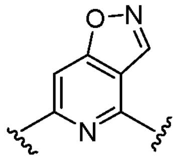 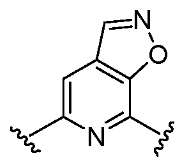 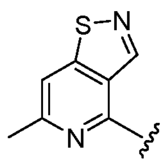 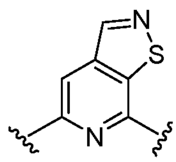

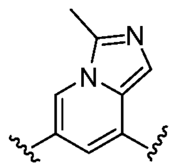 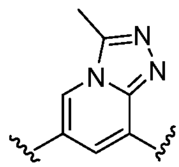 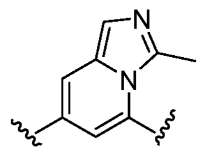 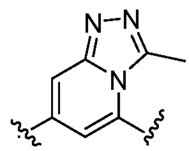

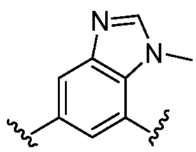 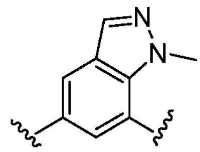 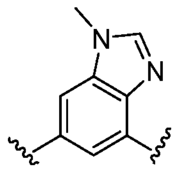 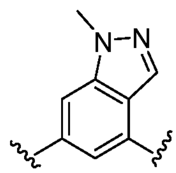

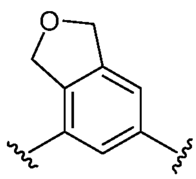 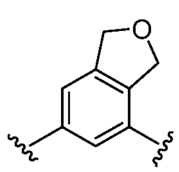 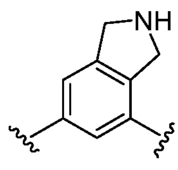 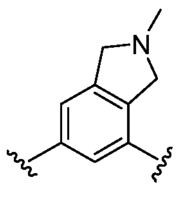

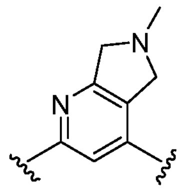 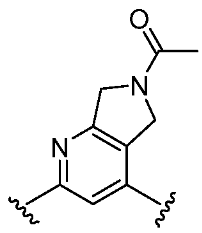 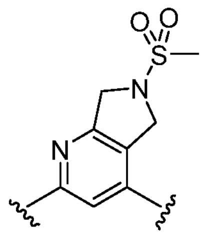 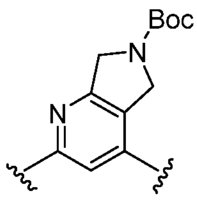

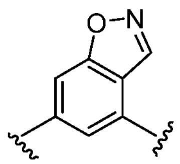 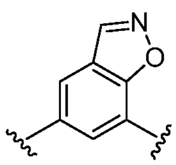 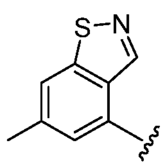 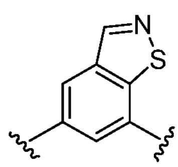 ;

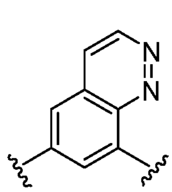 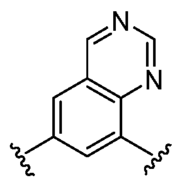 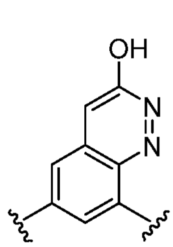 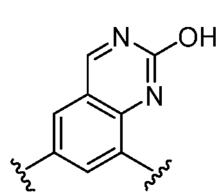

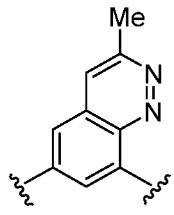 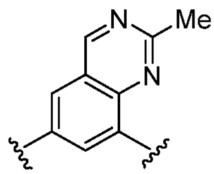 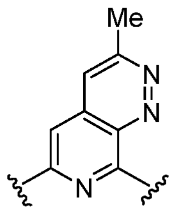 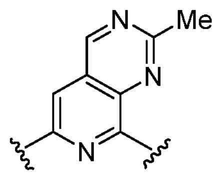

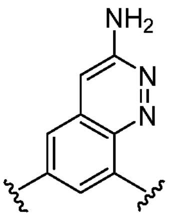 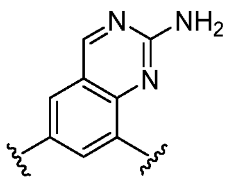 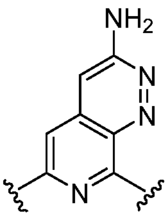 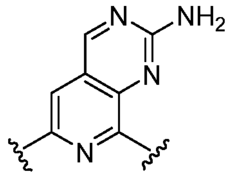

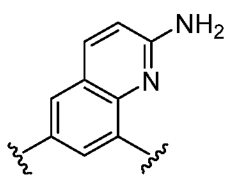 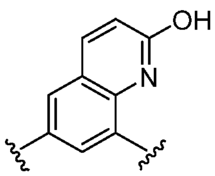 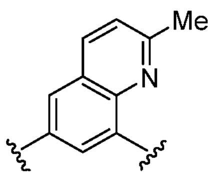 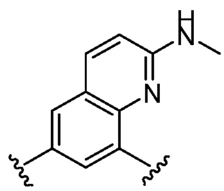

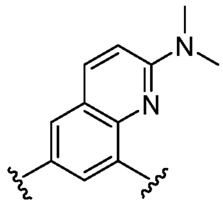 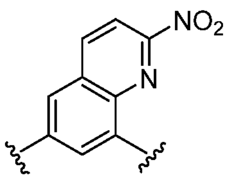 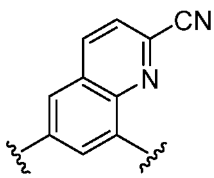 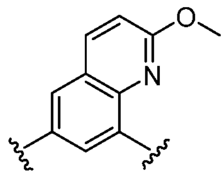

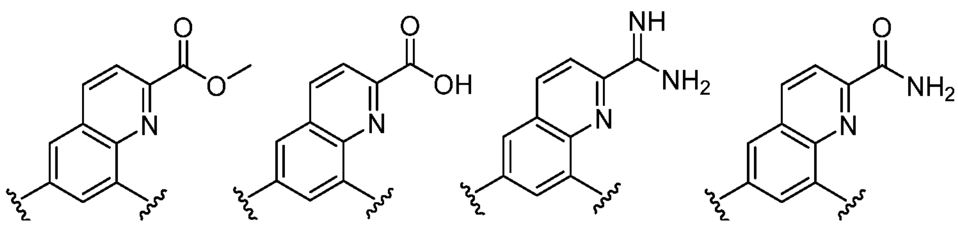

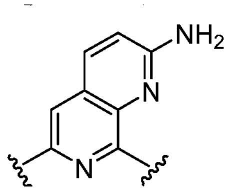 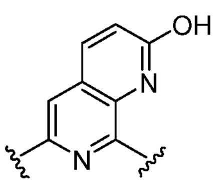 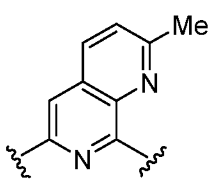 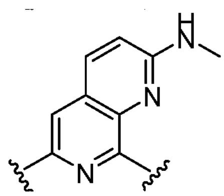

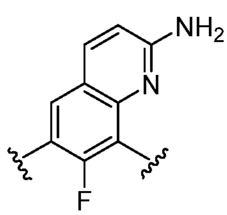 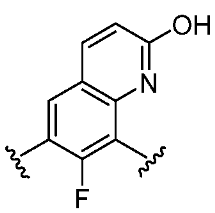 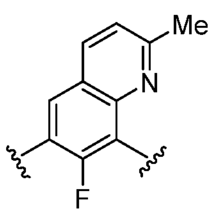 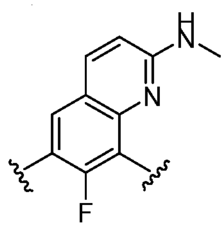

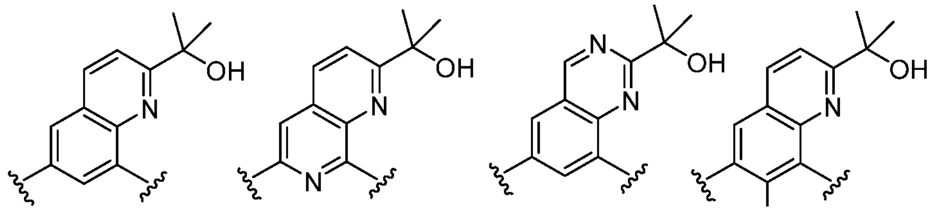

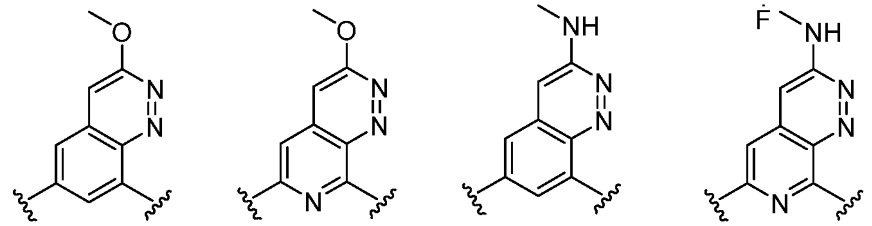

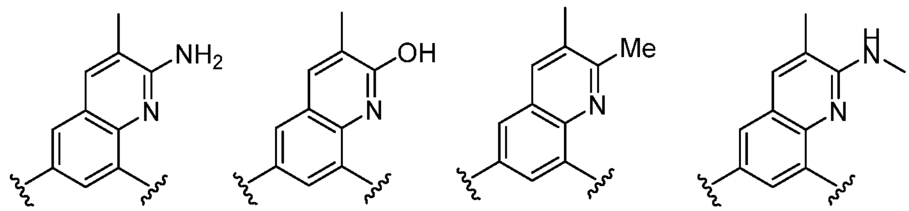

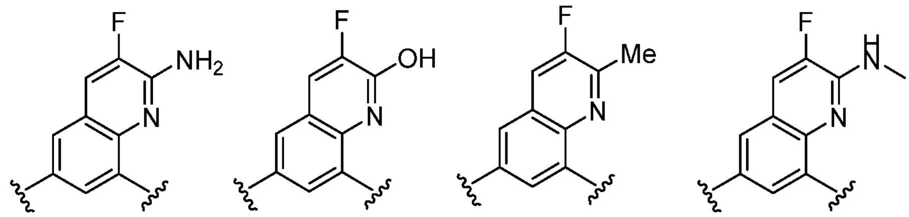

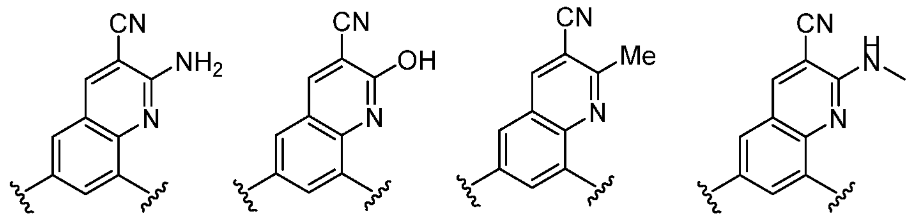

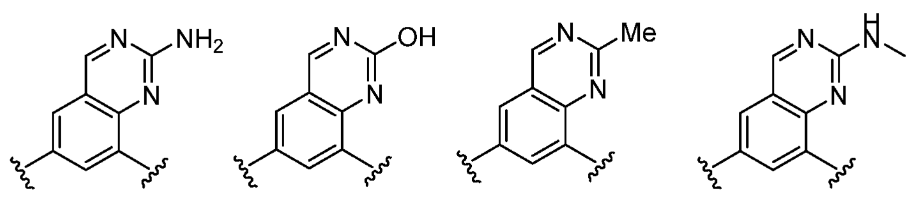

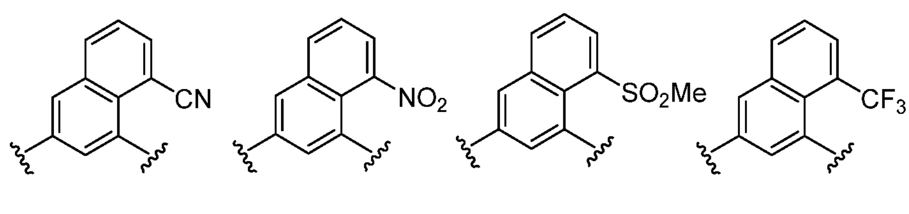

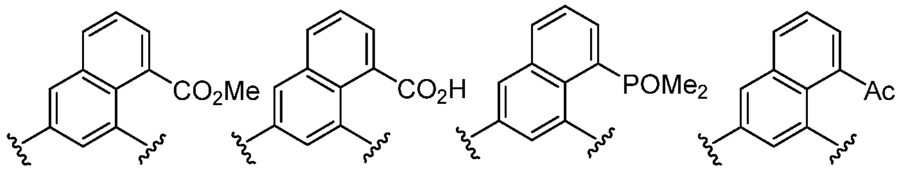

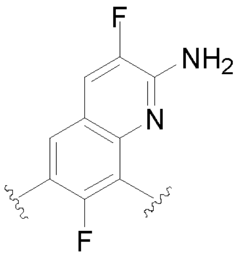 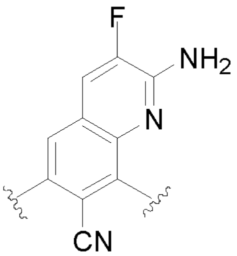 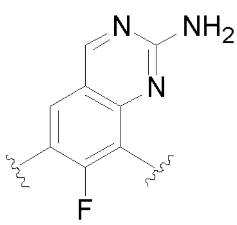 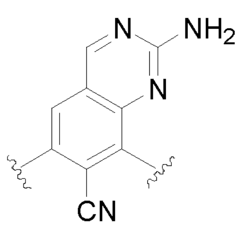

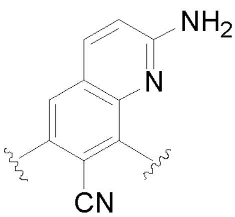 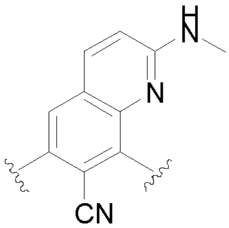 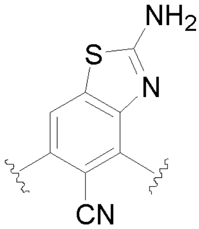 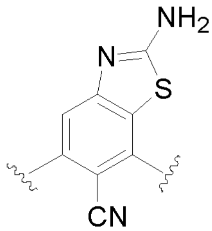

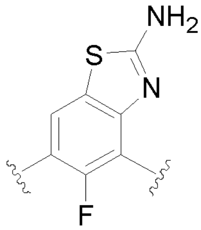 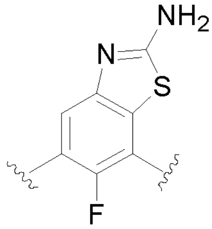

**15.** A compound according to any one of claims 1-14 or its pharmaceutically acceptable salts, stereoisomers, isotope isomers, prodrugs, hydrates, or solvates, wherein $L^1$ represents: -C(O)NH-, -HNC(O)-, 5-6-membered heteroaryl,

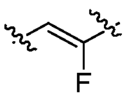, 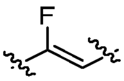, 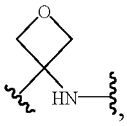, 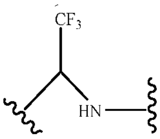, 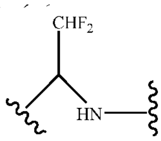, 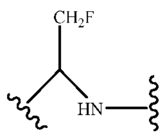.

**16.** A compound according to claim 15 or its pharmaceutically acceptable salts, stereoisomers, isotope isomers, prodrugs, hydrates, or solvates, wherein $L^1$ represents any one of the following groups:

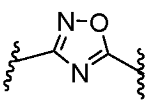, 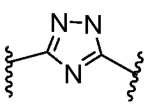, 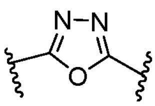, 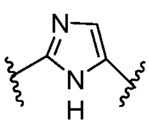, 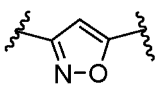,

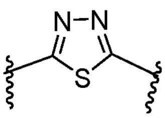, 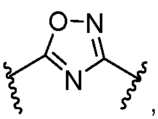, 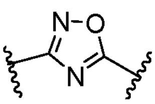, 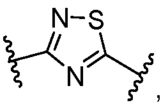, 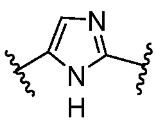

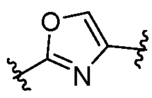 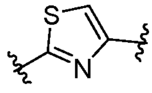 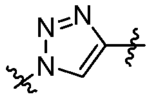 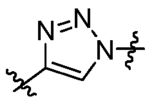 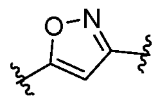

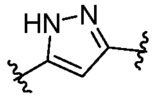 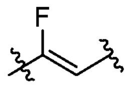 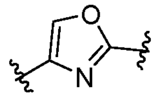 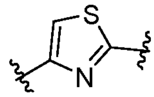 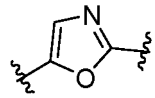

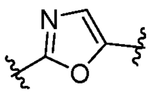 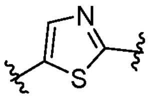 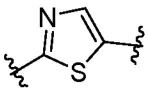 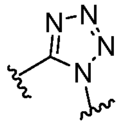 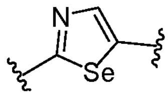

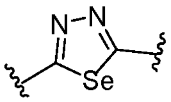 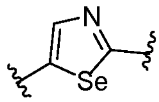 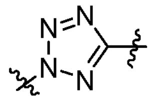 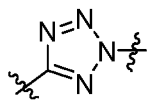 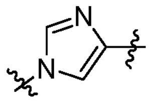

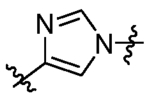 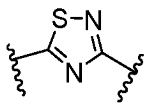

**17.** A compound according to any one of claims 1-16 or its pharmaceutically acceptable salts, stereoisomers, isotope isomers, prodrugs, hydrates, or solvates, wherein $L^2$ represents absence, $-C_1-C_6$ alkylene- or -NH-.

**18.** A compound according to any one of claims 1-17 or its pharmaceutically acceptable salts, stereoisomers, isotope isomers, prodrugs, hydrates, or solvates, wherein $L^2$ represents absence.

**19.** A compound according to any one of claims 1-18 or its pharmaceutically acceptable salts, stereoisomers, isotope isomers, prodrugs, hydrates, or solvates, wherein $Cy^2$ represents a saturated, partially saturated or unsaturated 3-, 4-, 5-, 6-, 7-membered monocyclic ring, or a 3-, 4-, 5-, 6-, 7-membered fused ring, which contains 0-3 N heteroatoms and 0-2 O or S heteroatoms and is substituted with 0-3 substituents selected from the following: halogen, cyano, nitro, hydroxy $C_1-C_6$ alkyl, $C_1-C_6$ alkyl, $C_3-C_8$ cycloalkyl, $C_1-C_6$ haloalkyl, $-OR^a$, $-SO_3R^a$, $-S(O)R^a$, $-O\ C_1-C_6$ haloalkyl, $-SR^a$, $-SF_5$ or $NR^aR^b$-substituted morpholinyl, piperidinyl, azetidine, pyrrolidinyl, cyclopropyl, cyclobutyl, cyclopentyl, cyclohexyl, piperazinyl or tetrahydrofuranyl; wherein, $R^a$ and $R^b$ each independently represent hydrogen, halogen, $C_1-C_6$ alkyl, $C_3-C_8$ cycloalkyl, $C_1-C_6$ haloalkyl or hydroxy $C_1-C_6$ alkyl; or $R^a$ and $R^b$, together with the atom they are attached to, form a 3-6 membered ring which contains 0-2 heteroatoms selected from O, N and S.

**20.** A compound according to any one of claims 1-19 or its pharmaceutically acceptable salts, stereoisomers, isotope isomers, prodrugs, hydrates, or solvates, wherein $Cy^2$ represents:

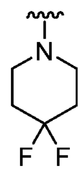 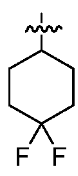 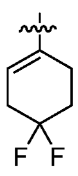 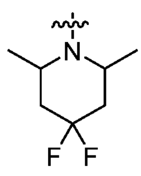 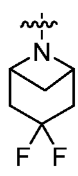 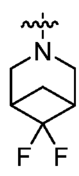 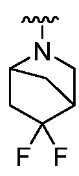

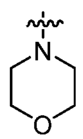 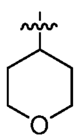 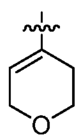 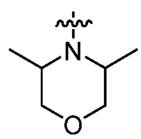 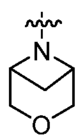 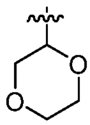 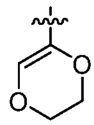

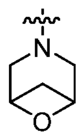 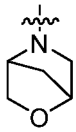 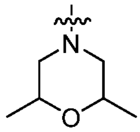 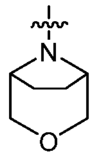 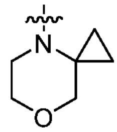 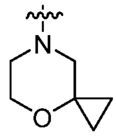 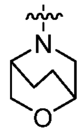

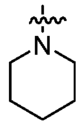 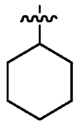 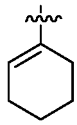 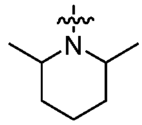 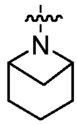 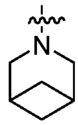 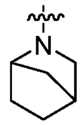

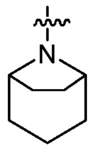 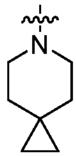 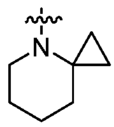 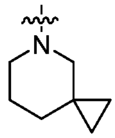 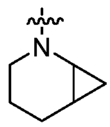 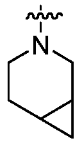 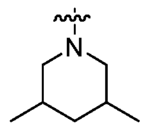

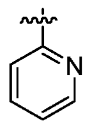 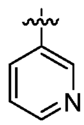 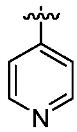 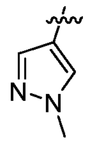 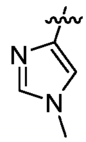

**21.** Compounds with the following structures:

| | | | |
|---|---|---|---|
| 1 | 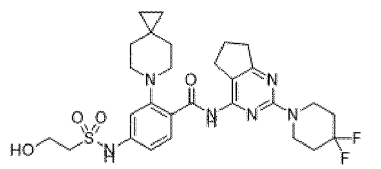 | 2 | 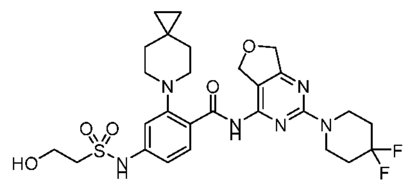 |
| 3 | 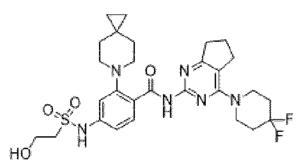 | 4 | 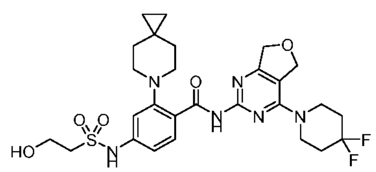 |
| 5 | 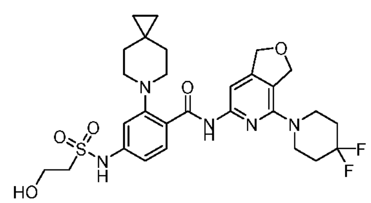 | 6 | 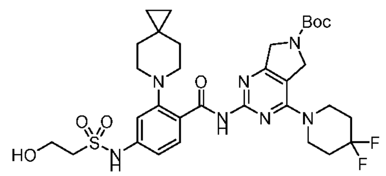 |
| 7 | 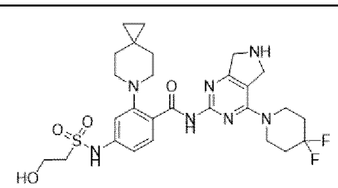 | 8 | 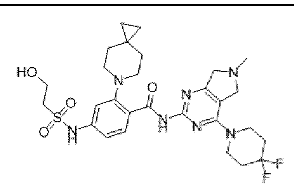 |

(continued)

| | | | |
|---|---|---|---|
| 9 | 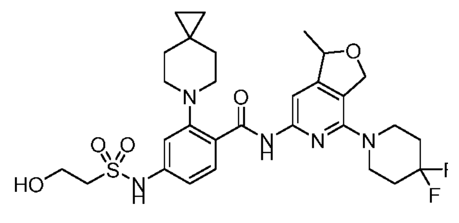 | 10 | 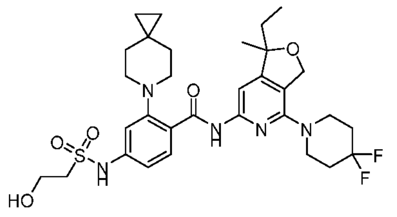 |
| 11 | 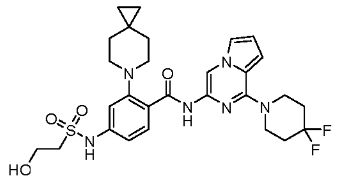 | 12 | 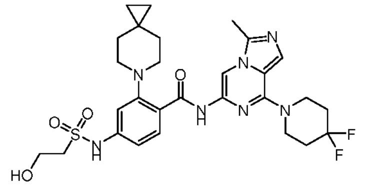 |
| 13 | 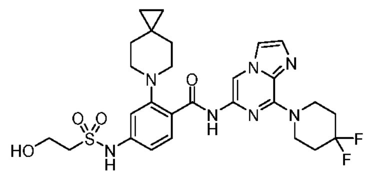 | 14 | 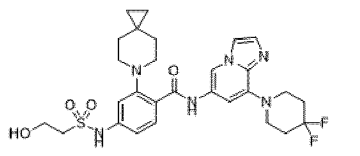 |
| 15 | 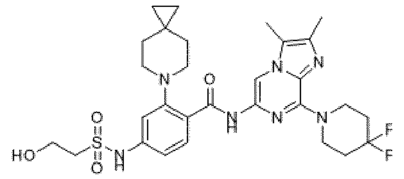 | 16 | 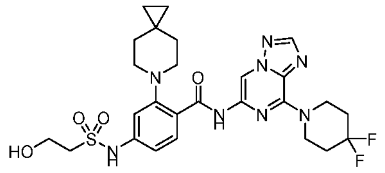 |
| 17 | 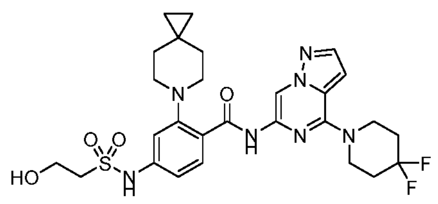 | 18 | 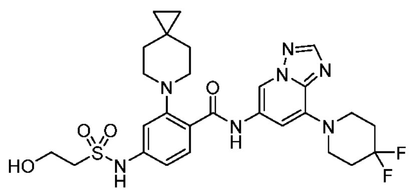 |
| 19 | 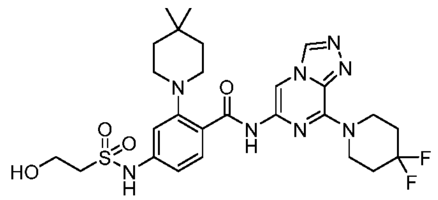 | 20 | 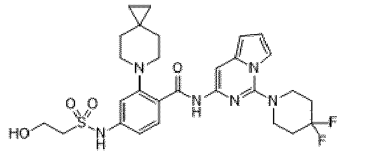 |
| 21 | 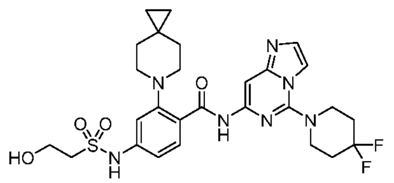 | 22 | 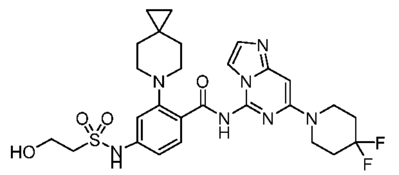 |
| 23 | 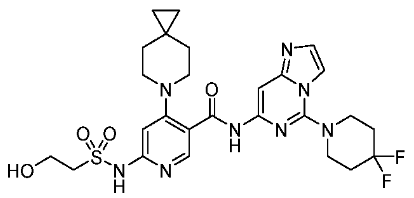 | 24 | 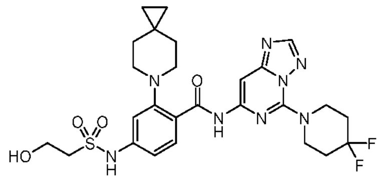 |

(continued)

| | | | |
|---|---|---|---|
| 25 | 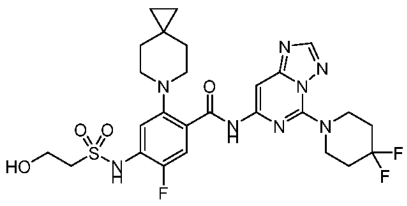 | 26 | 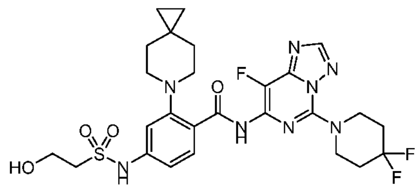 |
| 27 | 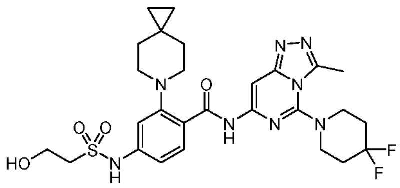 | 28 | 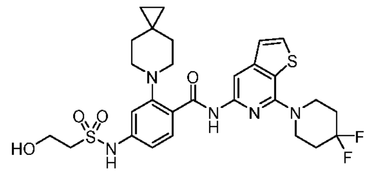 |
| 29 | 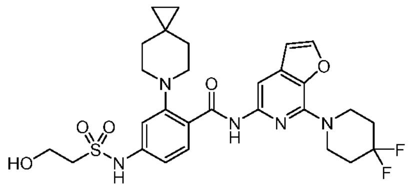 | 30 | 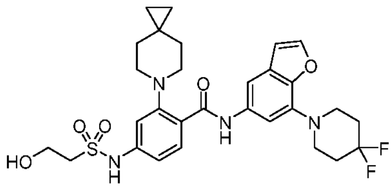 |
| 31 | 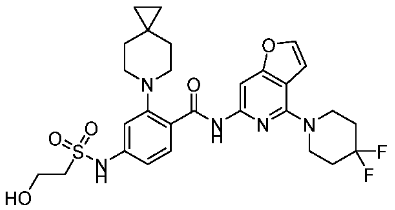 | 32 | 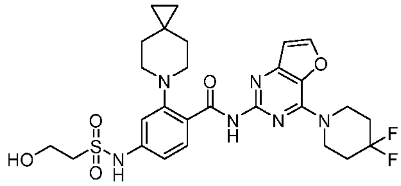 |
| 33 | 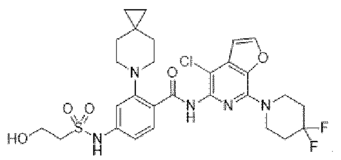 | 34 | 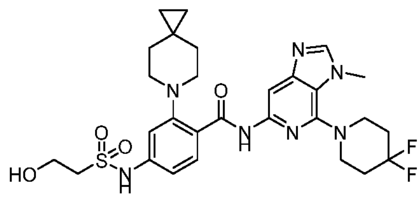 |
| 35 | 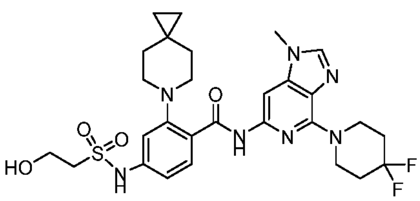 | 36 | 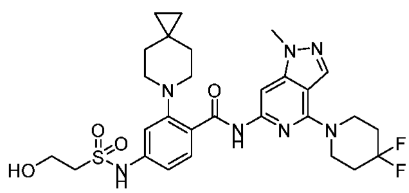 |
| 37 | 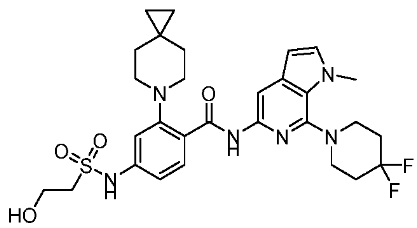 | 38 | 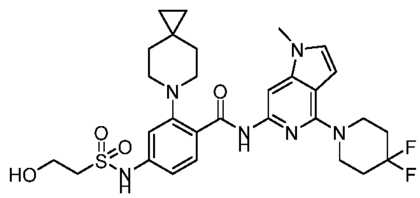 |
| 39 | 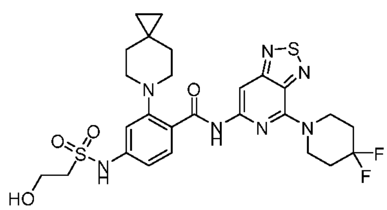 | 40 | 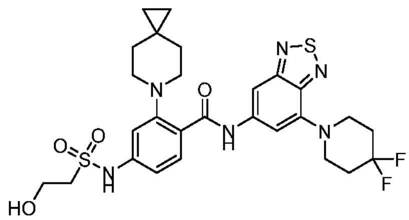 |

(continued)

| | | | |
|---|---|---|---|
| 41 | 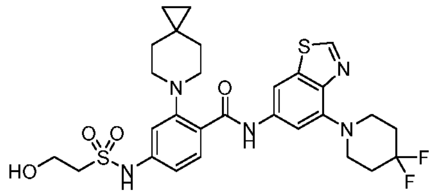 | 42 | 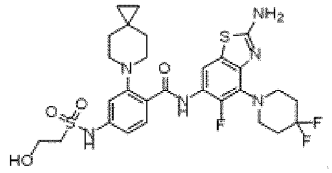 |
| 43 | 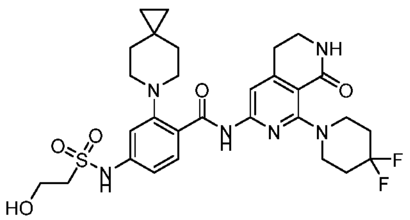 | 44 | 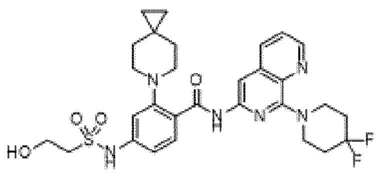 |
| 45 | 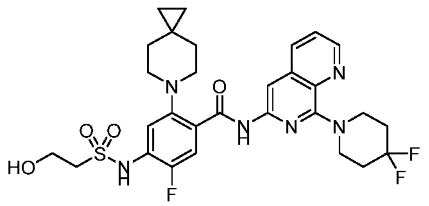 | 46 | 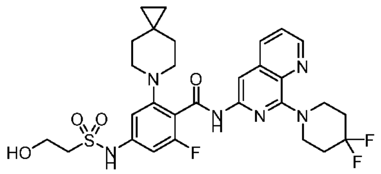 |
| 47 | 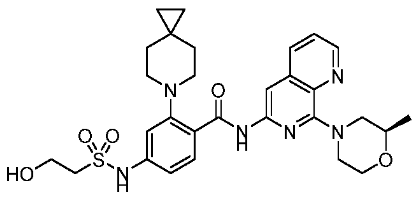 | 48 | 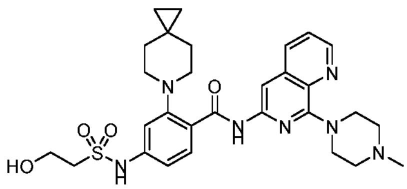 |
| 49 | 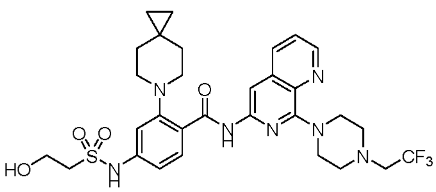 | 50 | 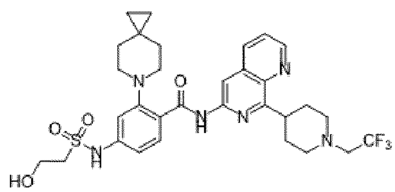 |
| 51 | 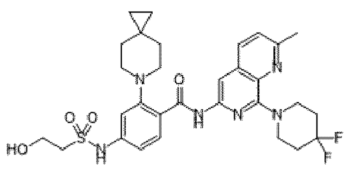 | 52 | 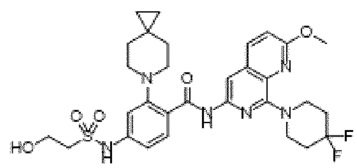 |
| 53 | 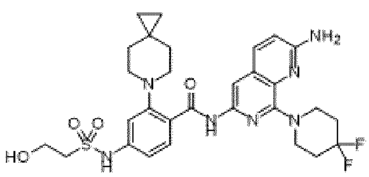 | 54 | 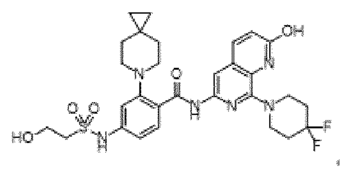 |
| 55 | 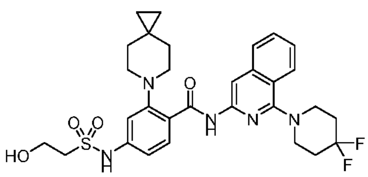 | 56 | 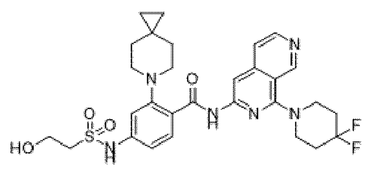 |

(continued)

| | | | |
|---|---|---|---|
| 57 | 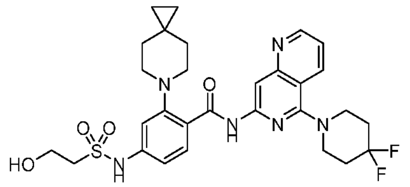 | 58 | 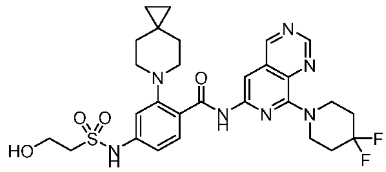 |
| 59 | 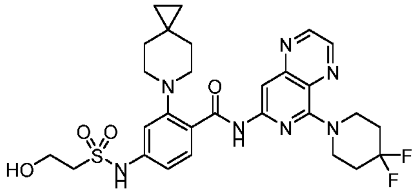 | 60 | 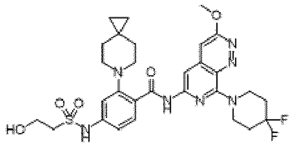 |
| 61 | 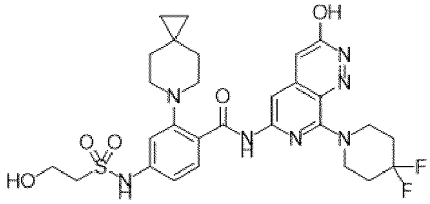 | 62 | 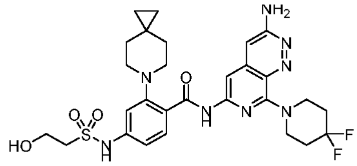 |
| 63 | 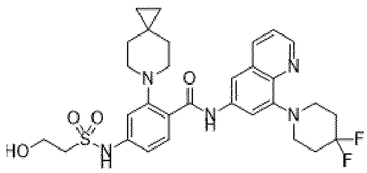 | 64 | 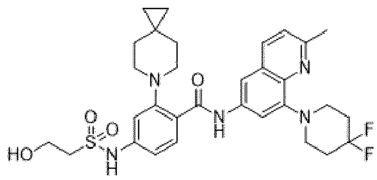 |
| 65 | 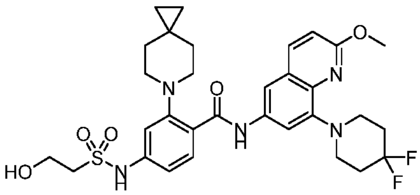 | 66 | 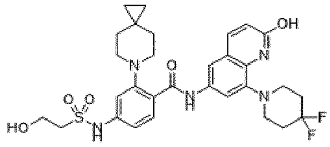 |
| 67 | 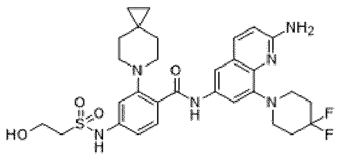 | 68 | 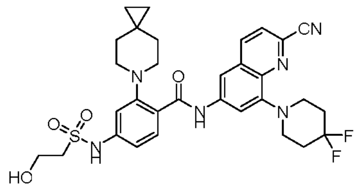 |
| 69 | 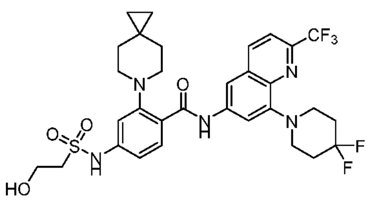 | 70 | 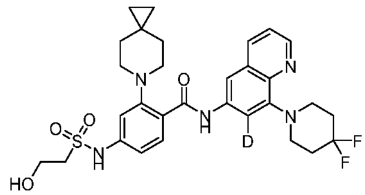 |
| 71 | 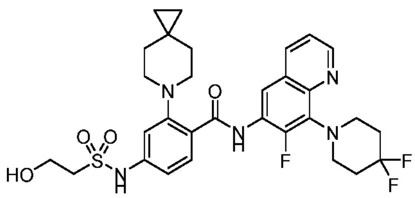 | 72 | 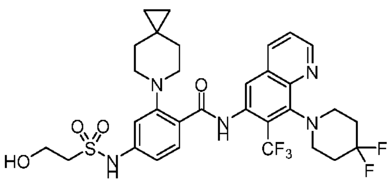 |

(continued)

| | | | |
|---|---|---|---|
| 73 | 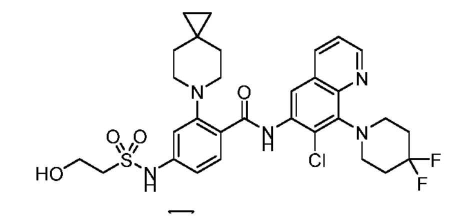 | 74 | 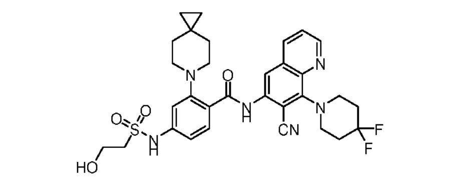 |
| 75 | 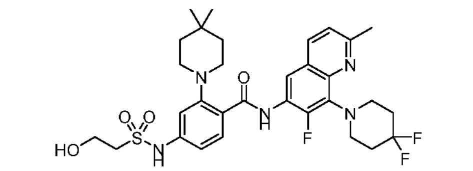 | 76 | 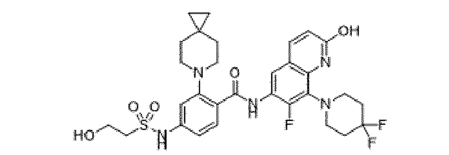 |
| 77 | 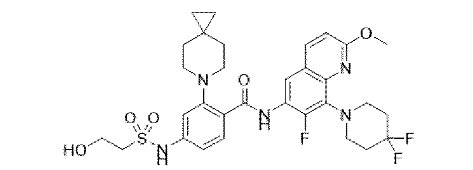 | 78 | 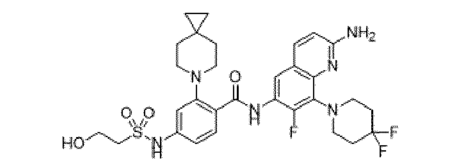 |
| 79 | 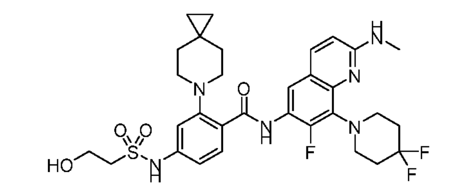 | 80 | 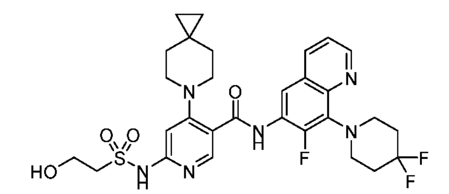 |
| 81 | 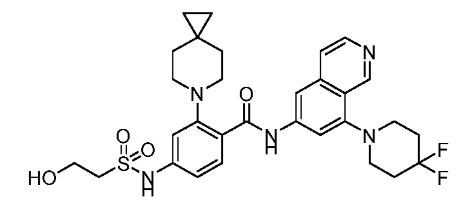 | 82 | 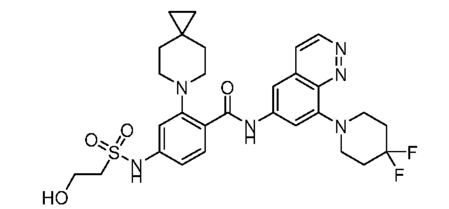 |
| 83 | 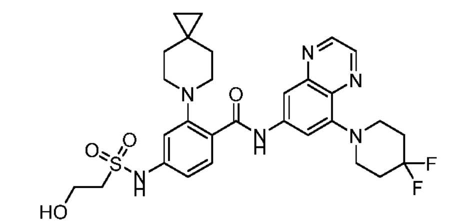 | 84 | 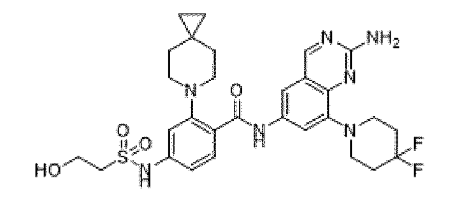 |
| 85 | 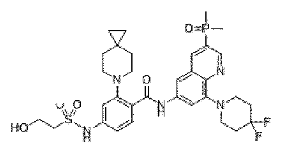 | 86 | 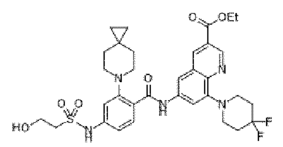 |
| 87 | 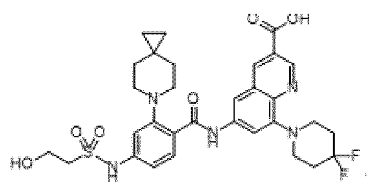 | 88 | 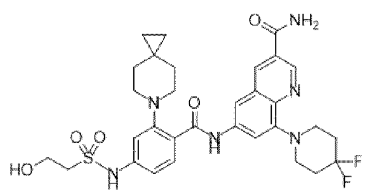 |

(continued)

| | | | |
|---|---|---|---|
| 89 | 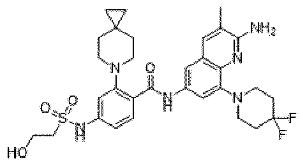 | 90 | 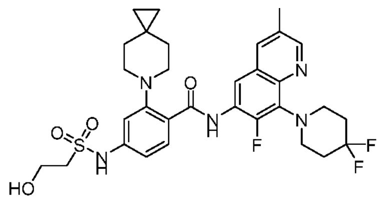 |
| 91 | 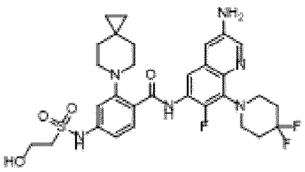 | 92 | 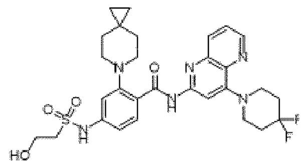 |
| 93 | 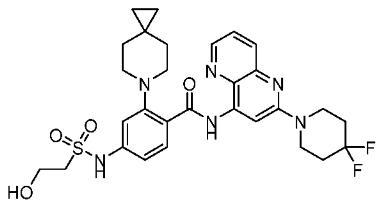 | 94 | 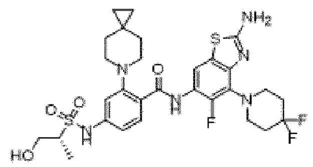 |
| 95 | 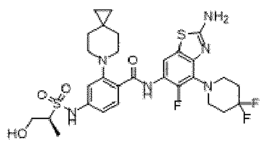 | 96 | 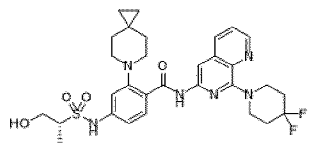 |
| 97 | 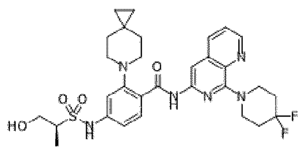 | 98 | 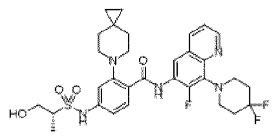 |
| 99 | 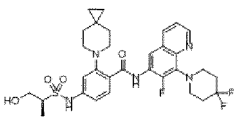 | 100 | 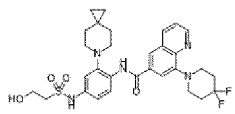 |
| 101 | 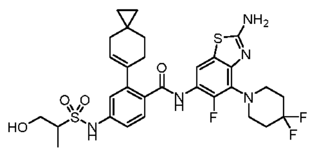 | 102 | 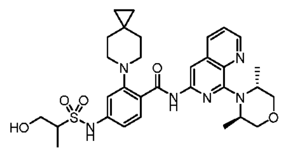 |

## INTERNATIONAL SEARCH REPORT

International application No.
**PCT/CN2023/093528**

### A. CLASSIFICATION OF SUBJECT MATTER

C07D401/14(2006.01)i; C07D487/04(2006.01)i; C07D491/04(2006.01)i; C07D471/04(2006.01)i; C07D498/04(2006.01)i; C07D513/04(2006.01)i; C07D519/00(2006.01)i; C07D491/048(2006.01)i; A61K31/63(2006.01)i; A61K31/438(2006.01)i; A61K31/38(2006.01)i; A61K31/41(2006.01)i; A61P35/00(2006.01)i

According to International Patent Classification (IPC) or to both national classification and IPC

### B. FIELDS SEARCHED

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS; CNTXT; DWPI; WPABS; USTST; EPTXT; WOTXT; CNKI; ISI-WEB OF SCIENCE; STN: 湃隆, 肖贻崧, 谷晓辉, 赖焜民, KIF18A, 癌, 瘤, kinesin, tumor, tumour, cancer, 结构式(I)

### C. DOCUMENTS CONSIDERED TO BE RELEVANT

| Category* | Citation of document, with indication, where appropriate, of the relevant passages | Relevant to claim No. |
|---|---|---|
| X | CN 114391012 A (AMGEN INC.) 22 April 2022 (2022-04-22) claims 1-47 | 1-21 |
| A | CN 114302880 A (AMGEN INC.) 08 April 2022 (2022-04-08) entire document | 1-21 |
| A | WO 2006094237 A2 (SIRTRIS PHARMACEUTICALS INC. et al.) 08 September 2006 (2006-09-08) entire document | 1-21 |
| A | CN 101437519 A (ABBOTT LABORATORIES) 20 May 2009 (2009-05-20) entire document | 1-21 |
| A | US 2011183973 A1 (BALDWIN IAN ROBERT et al.) 28 July 2011 (2011-07-28) entire document | 1-21 |
| A | WO 2021055728 A1 (MERCK SHARP & DOHME CORP.) 25 March 2021 (2021-03-25) entire document | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 July 2023** | **05 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/093528**

**C. DOCUMENTS CONSIDERED TO BE RELEVANT**

| Category* | Citation of document, with indication, where appropriate, of the relevant passages | Relevant to claim No. |
|---|---|---|
| A | US 2022056015 A1 (AMGEN INC.) 24 February 2022 (2022-02-24) entire document | 1-21 |
| A | CN 113226473 A (AMGEN INC.) 06 August 2021 (2021-08-06) entire document | 1-21 |
| A | CN 114269731 A (AMGEN INC.) 01 April 2022 (2022-04-01) entire document | 1-21 |
| A | CN 114401953 A (AMGEN INC.) 26 April 2022 (2022-04-26) entire document | 1-21 |

Form PCT/ISA/210 (second sheet) (July 2022)

**INTERNATIONAL SEARCH REPORT**
**Information on patent family members**

International application No.
**PCT/CN2023/093528**

| Patent document cited in search report | Publication date (day/month/year) | Patent family member(s) | Publication date (day/month/year) |
|---|---|---|---|
| CN 114391012 A | 22 April 2022 | EP 4007638 A1 | 08 June 2022 |
| | | US 2022372018 A1 | 24 November 2022 |
| | | WO 2021026100 A1 | 11 February 2021 |
| | | JP 2022542392 A | 03 October 2022 |
| | | CA 3147276 A1 | 11 February 2021 |
| | | AU 2020325115 A1 | 17 March 2022 |
| CN 114302880 A | 08 April 2022 | CA 3146693 A1 | 11 February 2021 |
| | | US 2022289724 A1 | 15 September 2022 |
| | | EP 4007752 A1 | 08 June 2022 |
| | | JP 2022542967 A | 07 October 2022 |
| | | WO 2021026098 A1 | 11 February 2021 |
| | | AU 2020326627 A1 | 17 March 2022 |
| WO 2006094237 A2 | 08 September 2006 | AU 2006218405 A1 | 08 September 2006 |
| | | EP 1856099 A2 | 21 November 2007 |
| | | WO 2006094237 A3 | 26 October 2006 |
| | | US 2009069301 A1 | 12 March 2009 |
| CN 101437519 A | 20 May 2009 | US 2012053345 A1 | 01 March 2012 |
| | | JP 2009532370 A | 10 September 2009 |
| | | JP 5255559 B2 | 07 August 2013 |
| | | CA 2644910 A1 | 18 October 2007 |
| | | CA 2644910 C | 28 January 2014 |
| | | WO 2007117465 A2 | 18 October 2007 |
| | | WO 2007117465 A3 | 28 August 2008 |
| | | EP 2001480 A2 | 17 December 2008 |
| | | EP 2001480 A4 | 15 June 2011 |
| | | MX 2008012482 A | 10 October 2008 |
| | | US 2007282101 A1 | 06 December 2007 |
| | | US 8008481 B2 | 30 August 2011 |
| | | MX 298183 B | 13 April 2012 |
| US 2011183973 A1 | 28 July 2011 | EP 2280705 A1 | 09 February 2011 |
| | | EP 2280705 B1 | 08 October 2014 |
| | | WO 2009147189 A1 | 10 December 2009 |
| | | JP 2011522003 A | 28 July 2011 |
| | | JP 5502076 B2 | 28 May 2014 |
| | | US 8536169 B2 | 17 September 2013 |
| | | ES 2526966 T3 | 19 January 2015 |
| WO 2021055728 A1 | 25 March 2021 | US 2022402916 A1 | 22 December 2022 |
| | | EP 4031542 A1 | 27 July 2022 |
| | | WO 2021055728 A8 | 11 November 2021 |
| US 2022056015 A1 | 24 February 2022 | EP 3898616 A1 | 27 October 2021 |
| | | WO 2020132649 A1 | 25 June 2020 |
| | | AU 2019404576 A1 | 24 June 2021 |
| | | CA 3123227 A1 | 25 June 2020 |
| | | JP 2022513967 A | 09 February 2022 |
| CN 113226473 A | 06 August 2021 | CA 3123871 A1 | 25 June 2020 |
| | | US 2022106293 A1 | 07 April 2022 |
| | | US 2020239441 A1 | 30 July 2020 |
| | | US 11236069 B2 | 01 February 2022 |
| | | TW 202034924 A | 01 October 2020 |

Form PCT/ISA/210 (patent family annex) (July 2022)

**INTERNATIONAL SEARCH REPORT**
**Information on patent family members**

International application No.
**PCT/CN2023/093528**

| Patent document cited in search report | | | Publication date (day/month/year) | Patent family member(s) | | | Publication date (day/month/year) |
|---|---|---|---|---|---|---|---|
| | | | | JP | 2022513972 | A | 09 February 2022 |
| | | | | WO | 2020132648 | A1 | 25 June 2020 |
| | | | | AU | 2019403486 | A1 | 24 June 2021 |
| | | | | BR | 112021011989 | A2 | 08 September 2021 |
| | | | | KR | 20210106474 | A | 30 August 2021 |
| | | | | EP | 3897852 | A1 | 27 October 2021 |
| | | | | AR | 117490 | A1 | 11 August 2021 |
| | | | | SG | 11202106520 | VA | 29 July 2021 |
| | | | | IN | 202117031747 | A | 10 December 2021 |
| | | | | VN | 82664 | A | 27 December 2021 |
| | | | | HK | 40062253 | A0 | 10 June 2022 |
| CN | 114269731 | A | 01 April 2022 | US | 2022281843 | A1 | 08 September 2022 |
| | | | | JP | 2022542394 | A | 03 October 2022 |
| | | | | CA | 3147451 | A1 | 11 February 2021 |
| | | | | AU | 2020324963 | A1 | 24 February 2022 |
| | | | | WO | 2021026101 | A1 | 11 February 2021 |
| | | | | EP | 4007756 | A1 | 08 June 2022 |
| CN | 114401953 | A | 26 April 2022 | JP | 2022542319 | A | 30 September 2022 |
| | | | | AU | 2020324406 | A1 | 17 March 2022 |
| | | | | CA | 3147272 | A1 | 11 February 2021 |
| | | | | WO | 2021026099 | A1 | 11 February 2021 |
| | | | | EP | 4007753 | A1 | 08 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210520744
- CN 202210878192
- CN 202211183092
- CN 202211537992
- CN 202310071265X
- CN 202310288871
- CN 202310475180


### Non-patent literature cited in the description


- **MI MAYR et al.** *Current Biology*, 2007, vol. 17, 488-98
- **ALLEN L. V. JR. et al.** Remington: The Science and Practice of Pharmacy. Pharmaceutical Press, 2012, vol. 2